(19) Europäisches Patentamt / European Patent Office / Office européen des brevets

(11) EP 4 649 959 A2

(12) **EUROPEAN PATENT APPLICATION**

(43) Date of publication:
**19.11.2025 Bulletin 2025/47**

(21) Application number: **25205018.2**

(22) Date of filing: **13.04.2022**

(51) International Patent Classification (IPC):
***A61K 39/395*** *(2006.01)*

(52) Cooperative Patent Classification (CPC):
**A61P 3/10; A61K 39/39591; A61P 27/02; C07K 16/22;** A61K 2039/545; C07K 2317/24; C07K 2317/35; C07K 2317/524; C07K 2317/526; C07K 2317/71; C07K 2317/76; C07K 2317/90

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.04.2021 US 202163174812 P**

(62) Document number(s) of the earlier application(s) in accordance with Art. 76 EPC:
**22788845.0 / 4 323 399**

(71) Applicant: **Kodiak Sciences Inc.**
**Palo Alto, CA 94304 (US)**

(72) Inventors:
- **EHRLICH, Jason**
  **Palo Alto, California 94304 (US)**
- **VELAZQUEZ-MARTIN, Pablo**
  **Palo Alto, California 94304 (US)**
- **NAOR, Joel**
  **Palo Alto, California 94304 (US)**
- **PERLROTH, Daniel Victor**
  **Palo Alto, California 94304 (US)**
- **LIANG, Hong**
  **Palo Alto, California 94304 (US)**
- **VASQUEZ, Daniel Janer**
  **Palo Alto, California 94304 (US)**

(74) Representative: **De Clercq & Partners**
**Edgard Gevaertdreef 10a**
**9830 Sint-Martens-Latem (BE)**

Remarks:
- The complete document including Reference Table(s) and the Sequence Listing(s) can be downloaded from the EPO website
- This application was filed on 26-09-2025 as a divisional application to the application mentioned under INID code 62.

(54) **METHODS OF TREATING AN EYE DISORDER**

(57) Provided herein are embodiments relating to treating various eye disorders, including, for example, wAMD, DME, RVO, and DR.

EP 4 649 959 A2

## Description

FIELD

The present disclosure relates to antibodies and conjugates thereof and methods of using and manufacturing said antibodies, conjugates thereof, and other protein conjugates.

BACKGROUND

Vascular endothelial growth factor (VEGF) stimulates vascular endothelial cell growth and induces vascular permeability. These biologic activities give it a central role in angiogenesis, both in normal and pathologic conditions. Inappropriate over-expression of VEGF has played a key role in retinal vascular diseases such as diabetic retinopathy (DR), diabetic macular edema (DME), wet age-related macular degeneration (wAMD), and retinal vein occlusion (RVO). In addition, increased retinal VEGF expression has been demonstrated in patients with retinal ischemic diseases. Inhibition of inappropriate VEGF activity is an "antiangiogenic" approach to treatment of these diseases and has been an effective method of preserving and improving visual acuity in patients with these retinal vascular diseases.

Intravitreal antiangiogenic therapy is currently the primary treatment for DME, wAMD, and macular edema due to RVO. However, standard treatment of these eye disorders with therapeutic VEGF-A inhibitors such as intravitreal aflibercept and intravitreal ranibizumab involve dosing every month or every 8 weeks (after initial monthly loading doses), depending on the eye disorder. Thus, real world outcomes have fallen short of expectation because of the burden involved in monthly visits to the retina specialist for evaluation and treatment. There is a medical need to achieve therapeutic results with fewer and/or less frequent intravitreal injections.

SUMMARY

Provided herein are methods of treating various eye disorders by administering an anti-VEGF antibody or anti-VEGF protein to a subject having an eye disorder. Some embodiments provided herein allow one to overcome certain limitations in the prior art by providing new therapeutic methods for use in subjects with eye disorders characterized by aberrant growth of vascular tissue mediated at least in part by vascular endothelial growth factor (VEGF). In some embodiments, the anti-VEGF antibody of the present disclosure may be an anti-VEGF antibody conjugate (e.g., KSI-301), or anti-VEGF protein conjugate, that includes a polymeric moiety that extends the half-life (e.g., ocular half-life, etc.) of the antibody or protein when administered to a subject. In some embodiments, methods of the present disclosure may provide for a course of treatment for an eye disorder that includes fewer doses (e.g., less frequent administration) of the anti-VEGF antibody conjugates or anti-VEGF protein conjugates than conventional anti-VEGF therapies, to achieve a therapeutic effect of the anti-VEGF therapy on the subject.

In some embodiments, provided herein is a method for treating an eye disorder, wherein the method comprises administering an anti-VEGF antibody conjugate (e.g., KSI-301), or anti-VEGF protein conjugate (e.g., an aflibercept biopolymer conjugate), to a subject in need of treating an eye disorder at a first loading dose, a second loading dose, and optionally, a third loading dose, waiting at least one month between loading doses, whereby the subject retains a therapeutic result of the anti-VEGF antibody conjugate, e.g., KSI-301 therapy for at least 24 weeks after a final loading dose. Optionally, the eye disorder is diabetic retinopathy (DR).

In some embodiments, provided herein is method for treating diabetic retinopathy (DR), wherein the method comprises: administering anti-VEGF antibody conjugate (e.g., KSI-301), or anti-VEGF protein conjugate (e.g., an aflibercept biopolymer conjugate), to a subject with RVO at a first loading dose; repeating the loading dose once or twice, waiting 12 weeks between the first and second loading dose, and waiting 24 weeks after the second dose, whereby the subject retains a therapeutic result of the anti-VEGF antibody conjugate, e.g., KSI-301 therapy for at least 12 weeks and ideally for at least 24 weeks after the final loading dose.

In some embodiments, provided herein is a method of improving perfusion of an eye, the method comprising: identifying a subject with DR, and administering at least 2 loading doses of the anti-VEGF antibody conjugate (e.g., KSI-301) or anti-VEGF protein conjugate (e.g., an aflibercept biopolymer conjugate), to the subject, where the loading doses are administered four weeks apart; providing one or more further doses of the anti-VEGF antibody conjugate, e.g., KSI-301, or anti-VEGF protein conjugate (e.g., aflibercept biopolymer conjugate), to the subject every 12 weeks.

Optionally, if there is decline in eye health, such as the eye developing macular edema at week 8 after administration of the second loading dose, then subsequent doses are administered to the subject every 8 weeks.

In some embodiments, provided herein is a method of improving perfusion of an eye, the method comprising: identifying a subject with DR, and administering at least a first and second loading dose of the anti-VEGF antibody conjugate (e.g., KSI-301) or anti-VEGF protein conjugate (e.g., aflibercept biopolymer conjugate), to the subject; evaluating the subject’s eye health through regular ophthalmic exams, and administering at subsequent doses of the

anti-VEGF antibody conjugate (e.g., KSI-301) or anti-VEGF protein conjugate (e.g., aflibercept biopolymer conjugate) to the subject once every 12 weeks.

Optionally, if the subject has a decline in eye health, then administration of subsequent doses of the anti-VEGF antibody conjugate, e.g., KSI-301 or anti-VEGF protein conjugate (e.g., aflibercept biopolymer conjugate) is given at least once every 8 weeks.

In some embodiments, provided is a method of improving perfusion in the eye, the method comprising: identifying a subject with diabetic retinopathy, and administering at least a first and second dose of the anti-VEGF antibody conjugate, wherein the second loading dose is administered 4 weeks after the first loading dose; and administering subsequent doses of the anti-VEGF antibody conjugate or anti-VEGF protein conjugate to the subject once every 8 or 12 weeks.

In some embodiments, provided herein is a method of improving perfusion of an eye, the method comprising: identifying a subject with wet Age-related Macular Degeneration (wAMD), wherein the subject is currently being treated with therapeutic agents for wAMD, where the therapeutic agents are not KSI-301, and wherein a loading dose of an anti-VEGF antibody conjugate like KSI-301 is thereafter administered.

In some embodiments, provided herein is a method of treating a subject with wAMD, the method comprising: administering 1-3 loading doses of the anti-VEGF antibody conjugate (e.g., KSI-301), or anti-VEGF protein conjugate (e.g., an aflibercept biopolymer conjugate), to a subject with wAMD; where each loading dose is administered every 4 weeks; and wherein the eye is examined after the last loading dose.

In some embodiments, the eye is examined 12 weeks after the third loading dose and if the eye is dry, administering doses of the anti-VEGF antibody conjugate at a dosing schedule of once every 16 weeks. For eyes which show wetness, after the 12 week exam following the third loading dose, administering doses of the anti-VEGF antibody conjugate at a dosing schedule of once every 8 weeks.

In some embodiments, provided herein is a method of treating a subject with wAMD, the method comprising: identifying a subject with wAMD, wherein the subject has active choroidal neovascularization (CNV) wherein the CNV components affect the central subfield of the subject's eye.

In some embodiments, provided herein is a method of treating an eye disorder wherein the eye disorder is wAMD, comprising: administering 1-3 loading doses of the anti-VEGF antibody conjugate (e.g., KSI-301), or anti-VEGF protein conjugate (e.g., an aflibercept biopolymer conjugate), to a subject with wAMD; where each loading dose is administered every 4 weeks, and wherein the eye is examined using luminance measurements during subsequent ophthalmic examinations. Where the luminance is above a predetermined value, the method can optionally alter the treatment timeline to decrease dose frequency; additionally, where luminance is below a predetermined value, the method can similarly alter the treatment timeline to increase dose frequency.

In some embodiments, provided herein is a method of treating an eye disorder, comprising: administering an anti-VEGF antibody conjugate, e.g., KSI-301 or anti-VEGF protein conjugate (e.g., and aflibercept biopolymer conjugate) to a subject in need of treating an eye disorder at a first loading dose, wherein the eye disorder is wAMD; and optionally repeating the loading dose once or twice for a total of up to 3 loading doses, and wherein the eye is examined to determine the composition of fluid in the eye. In some embodiments, the eye is examined at least 4 weeks after the last loading dose. In some embodiments, if the amount of fluid has stabilized and is below a predetermined level, a subsequent administration of the anti-VEGF antibody conjugate or anti-VEGF protein conjugate therapy is administered at a first dose frequency. In some embodiments, this first dose frequency is every 4 weeks; in some embodiments, this first dose frequency may also be longer than every 4 weeks. In some embodiments, if the amount of fluid is above a predetermined level, a subsequent administration of the anti-VEGF antibody conjugate therapy, or anti-VEGF protein conjugate therapy is administered at a second dose frequency; wherein the second dose frequency is more frequent than the first dose frequency.

In some embodiments, provided herein is a method of disease modification of an eye disorder, wherein the eye disorder is Diabetic Macular Edema (DME), wherein the method comprises: administering 1-3 loading doses of an anti-VEGF antibody polymer conjugate or anti-VEGF protein conjugate therapy, wherein the method further comprises administering between 1-8 maintenance doses of the anti-VEGF antibody polymer conjugate or anti-VEGF protein conjugate therapy, and wherein 7 or fewer doses are administered to the subject within a year of the first administration of the anti-VEGF antibody polymer conjugate or anti-VEGF protein conjugate therapy.

In some embodiments, provided herein is a method of treating an eye disorder, wherein the disorder is DME, the method comprising: administering 2-3 loading doses of an anti-VEGF antibody polymer conjugate or anti-VEGF protein conjugate to a subject having DME, and administering an additional dose of the anti-VEGF antibody polymer conjugate or anti-VEGF protein conjugate every 3-15 months. In some embodiments, the dosing schedule of the anti-VEGF antibody polymer conjugate or anti-VEGF protein conjugate is determined by ophthalmic evaluation using Optical Coherence Tomography (OCT) or OCT-A. In some embodiments, where the eye is dry as determined by OCT or OCT-A, the dosing schedule is modified to become less frequent. In some embodiments, where the eye is wet as determined by OCT or OCT-A, the dosing schedule is modified to become more frequent.

In some embodiments, provided herein is a method of treating an eye disorder, the method comprising:

administering 2-3 loading doses of an anti-VEGF antibody polymer conjugate or anti-VEGF protein conjugate to a subject having DME, and administering an additional dose of the anti-VEGF antibody polymer conjugate or anti-VEGF protein conjugate every 3-15 months. In some embodiments, the additional dose schedule of the anti-VEGF antibody polymer conjugate or anti-VEGF protein conjugate is determined by ophthalmic evaluation of retinal thickness, wherein the dosing schedule becomes less frequent where the measured retinal thickness falls below a predetermined level of retinal thickness, or more frequent where the measured retinal thickness falls above a predetermined level. In some embodiments, the predetermined retinal thickness is 320 microns. In some embodiments, the predetermined retinal thickness is 350 microns. In some embodiments, the ophthalmic evaluation also includes Optical Coherence Tomography (OCT) or OCT-A, wherein the dosing schedule becomes more frequent where the eye is found to be wet using OCT or OCT-A, and wherein the dosing schedule becomes less frequent where the eye is found to be dry using OCT or OCT-A.

In some embodiments, provided herein is a method of treating a subject with DME, DR, or retinal vein occlusion (RVO), the method comprising: administering 2-3 loading doses of an anti-VEGF antibody polymer conjugate or anti-VEGF protein conjugate; obtaining an ophthalmic evaluation a minimum of 4, 8, 12, 16, 20, 24, 36, or optionally 52 weeks after a last loading dose, and placing the patient on a fixed dosing schedule of the anti-VEGF antibody polymer conjugate or anti-VEGF protein conjugate a minimum of every 4, 8, 12, 16, 20, 24, 36, 48, or 52 weeks. Optionally, the ophthalmic evaluation includes a Best Corrected Visual Acuity (BCVA) score assessment, wherein the improvement of BCVA score alters the subject's dosing schedule to be less frequent, and wherein the decrease of BCVA score alters the dosing schedule to be more frequent. In some embodiments, the ophthalmic evaluation also includes Optical Coherence Tomography (OCT) or OCT-A, wherein the dosing schedule becomes more frequent where the eye is found to increase retinal thickness by 30-50 microns using OCT or OCT-A, and wherein the dosing schedule becomes less frequent where the eye is found to have decreased retinal thickness by 30-50 microns using OCT or OCT-A. In some embodiments, the ophthalmic evaluation also includes Optical Coherence Tomography (OCT) or OCT-A, wherein the dosing schedule becomes more frequent where the eye is found to increase retinal thickness by 30, 40, 50 microns, or by a thickness within a range defined by any two of the preceding values, using OCT or OCT-A, and wherein the dosing schedule becomes less frequent where the eye is found to have decreased retinal thickness by 30, 40, 50 microns, or by a thickness within a range defined by any two of the preceding values, using OCT or OCT-A. In some embodiments, the ophthalmic evaluation also includes Optical Coherence Tomography (OCT) or OCT-A, wherein the dosing schedule becomes more frequent where the eye is found to increase retinal thickness by 40 microns, using OCT or OCT-A, and wherein the dosing schedule becomes less frequent where the eye is found to have decreased retinal thickness by 40 microns, using OCT or OCT-A.

In some embodiments, provided herein is a method of treating a subject with DME, DR, or RVO, the method comprising: administering 1-3 loading doses of an anti-VEGF antibody polymer conjugate or anti-VEGF protein conjugate to a subject; providing a follow-on application of the anti-VEGF antibody conjugate, e.g., KSI-301, or anti-VEGF protein conjugate (e.g., aflibercept biopolymer conjugate), no sooner than 8 weeks after a last loading dose or after a last follow-on application of the anti-VEGF antibody polymer conjugate, wherein the loading doses are administered to the subject on a monthly basis, and wherein the subject is above 16 years of age.

In some embodiments, a method of treating a subject with DME, DR or RVO is provided. The method comprises administering 1-3 loading doses of an anti-VEGF antibody polymer conjugate to a subject with DME, DR or RVO, not administering more than 3 loading doses to the subject, and providing a follow-on application of the anti-VEGF antibody polymer conjugate at a point in time after a last loading dose or after a last follow-on application of the anti-VEGF antibody polymer conjugate, wherein the loading doses are administered to the subject on a monthly basis, and wherein the subject is above 16 years of age.

In some embodiments, a method of treating diabetic retinopathy is provided. The method comprises: identifying a subject with diabetic retinopathy, administering a first dose of an anti-VEGF antibody polymer conjugate to the subject, administering a second dose of the anti-VEGF antibody polymer conjugate to the subject 4 weeks after the first dose, administering one or more subsequent doses to the subject no more frequently than once every 12 weeks, unless, an eye of the subject has a decline in eye health at week 8 from the second loading dose, then administering the anti-VEGF antibody polymer conjugate once every 8 weeks.

In some embodiments, provided herein is a method of treating a subject with DME, DR, or RVO, the method comprising: administering 1-3 loading doses of an anti-VEGF antibody polymer conjugate to a subject; providing a follow-on application of the anti-VEGF antibody polymer conjugate at a point in time no sooner than 8 or 12 weeks after a last loading dose or after a last follow-on application of the anti-VEGF antibody polymer conjugate, wherein the loading doses are administered to the subject on a monthly basis, and wherein an improvement of Best Corrected Visual Acuity (BCVA) is observed during subsequent ophthalmic evaluation. In some embodiments, the improvement of BCVA is greater than at 5, or at least 6.8 to 7.2 letters post treatment. In some embodiments, the subject has DME and the BCVA is at least 65 letters, e.g., >75 letters. In some embodiments, the subject has RVO and the BCVA is at least 65 letters, e.g., >80 letters, e.g., >90 letters, or e.g., >100 letters

In some embodiments, provided herein is a method of treating a subject with DME, DR, or RVO, the method comprising: administering 1-3 loading doses of an anti-VEGF antibody polymer conjugate or anti-VEGF protein conjugate

to a subject; providing between 0-1 follow-on applications of the anti-VEGF antibody conjugate, e.g., KSI-301, or anti-VEGF protein conjugate (e.g., aflibercept biopolymer conjugate), no sooner than 8 or 12 weeks after a last loading dose or after a last follow-on application of the anti-VEGF antibody polymer conjugate, wherein the loading doses are administered to the subject on a monthly basis.

In some embodiments, a method of treating diabetic retinopathy is provided, the method comprising: identifying a subject with diabetic retinopathy; administering a first dose of an anti-VEGF antibody polymer conjugate to the subject; administering a second dose of the anti-VEGF antibody polymer conjugate to the subject 8 weeks after the first dose; administering a third dose of the anti-VEGF antibody polymer conjugate to the subject 12 weeks after the second dose; and administering a subsequent dose of the anti-VEGF antibody polymer conjugate to the subject no more frequently than once every 24 weeks after the third dose.

In some embodiments, a method of treating diabetic retinopathy is provided, the method comprising: identifying a subject with diabetic retinopathy; administering a first dose of an anti-VEGF antibody polymer conjugate to the subject; administering a second dose of the anti-VEGF antibody polymer conjugate to the subject 8 weeks after the first dose; administering a third dose of the anti-VEGF antibody polymer conjugate to the subject 12 weeks after the second dose; and administering one or more subsequent doses of the anti-VEGF antibody polymer conjugate to the subject either once every 12 weeks or once every 24 weeks after the third loading dose.

In some embodiments, a method of treating diabetic retinopathy is provided, the method comprising: identifying a subject with diabetic retinopathy; administering a first dose of an anti-VEGF antibody polymer conjugate to the subject; administering a second dose of the anti-VEGF antibody polymer conjugate to the subject 8 weeks after the first dose; and administering one or more subsequent doses to the subject no more frequently than once every 12 weeks, unless, an eye of the subject develops macular edema, then administering the anti-VEGF antibody polymer conjugate once every 4-12 weeks.

In some embodiments, a method of treating diabetic retinopathy is provided, the method comprising: identifying a subject with diabetic retinopathy; administering a first dose of an anti-VEGF antibody polymer conjugate to the subject; administering a second dose of the anti-VEGF antibody polymer conjugate to the subject 8 weeks after the first dose; if the subject has a decline in eye health 8 weeks after the second dose administering at least one subsequent dose of the anti-VEGF antibody polymer conjugate at a frequency of at least once every 8 weeks, and if the subject maintains eye health or it improves, then administering at least one subsequent dose of the anti-VEGF antibody polymer conjugate at a frequency of no more than once every 12 weeks.

In some embodiments, a method of treating diabetic retinopathy is provided, the method comprising: identifying a subject with diabetic retinopathy; administering a first dose of an anti-VEGF antibody polymer conjugate to the subject; administering a second dose of the anti-VEGF antibody polymer conjugate to the subject 4 weeks after the first dose; and administering one or more subsequent doses to the subject no more frequently than once every 12 weeks or once every 8 weeks.

In some embodiments, a method of treating a subject having wet AMD is provided, the method comprising: identifying a subject having wet AMD and receiving a therapy for wet AMD, wherein the therapy is not achieving the goal of treatment; wherein the treatment is not KSI-301; and administering KSI-301 to the subject in an amount and at a frequency sufficient to treat the wAMD.

In some embodiments, a method of treating wAMD is provided, the method comprising: identifying a subject with wet AMD; administering a first loading dose of an anti-VEGF antibody polymer conjugate to the subject; administering a second loading dose of the anti-VEGF antibody polymer conjugate to the subject 4 weeks after the first loading dose; administering a third loading dose of the anti-VEGF antibody polymer conjugate to the subject 4 weeks after the second loading dose; and evaluating an eye of the subject 12 weeks after the third loading dose, wherein if the eye is dry extending maintenance treatments to once every 16 weeks or every 20 weeks and wherein if the eye is wet providing a maintenance treatment once every 4 or 8 weeks.

In some embodiments, a method of treating wet AMD is provided, the method comprising: identifying a subject with wet AMD; administering a first loading dose of an anti-VEGF antibody polymer conjugate to the subject; administering a second loading dose of the anti-VEGF antibody polymer conjugate to the subject 4 weeks after the first loading dose; administering a third loading dose of the anti-VEGF antibody polymer conjugate to the subject 4 weeks after the second loading dose; and evaluating an eye of the subject using low-luminance visual acuity (LLVA) at 16 weeks after the first loading dose, wherein if the eye has a LLVA to BCVA difference of 33 or more letters, the subject will receive an increased dose frequency of maintenance doses and if the eye has a LLVA to BCVA difference of less than 33 letters, the subject will receive a decreased frequency of maintenance doses. In some embodiments, evaluating an eye of the subject involves using a low-luminance visual acuity assessment (LLVA) at 16 weeks after the third loading dose, wherein if the eye has a LLVA) to BCVA difference of 33 or more letters, the subject will receive an increased dose frequency (e.g. every 4 to 8 weeks) of maintenance doses and if the eye has LLVA to BCVA difference of less than 33 letters, the subject will receive a decreased frequency of maintenance doses (e.g. every 12 to 24 weeks) the subject will receive a decreased frequency of maintenance doses.

In some embodiments, a method of treating a subject with wet AMD is provided, the method comprising: administering 1-3 loading doses of an anti-VEGF antibody polymer conjugate to a subject; determining if an amount of fluid in an eye of the subject 4 weeks after a last loading dose is stabilized in the eye, wherein if the subject has stabilized fluid, administering a maintenance dose at a first frequency, and wherein if the subject has an increase in fluid administering a maintenance dose at a second frequency, wherein the second frequency is more frequent than the first frequency.

In some embodiments, a method of treating DME is provided, the method comprising: administering 1-3 loading doses of an anti-VEGF antibody polymer conjugate to a subject having DME; and administering 1-6 maintenance doses of the anti-VEGF antibody polymer conjugate to the subject, wherein 7 or fewer total doses are administered to the subject in a year.

In some embodiments, a method of treating DME is provided, the method comprising: administering 2-3 loading doses of an anti-VEGF antibody polymer conjugate to a subject having DME; and administering a maintenance dose of the anti-VEGF antibody polymer conjugate to the subject every 3-15 months, depending on whether the eye is dry as determined by OCT or OCT-A measurement.

In some embodiments, a method of treating DME is provided, the method comprising: administering 2-3 loading doses of an anti-VEGF antibody polymer conjugate to a subject having DME; and administering a maintenance dose of the anti-VEGF antibody polymer conjugate to the subject every 3-15 months, depending on whether retina thickness in the subject is above 320 microns. In some embodiments, the method includes administering the maintenance dose of the anti-VEGF antibody polymer conjugate to the subject every 3-15 months, depending on whether retina thickness in the subject is above 350 microns.

In some embodiments, a method of preventing vision complications is provided, the method comprising: administering 2-3 loading doses of an anti-VEGF antibody polymer conjugate to a subject having DME; and administering a maintenance dose of the anti-VEGF antibody polymer conjugate to the subject every 3-15 months, depending on: a) whether retina thickness in the subject is above 320 microns; and/or b) whether the eye is dry as determined by OCT or OCT-A measurement. In some embodiments, the method includes administering the maintenance dose of the anti-VEGF antibody polymer conjugate to the subject every 3-15 months, depending on: a) whether retina thickness in the subject is above 350 microns; and/or b) whether the eye is dry as determined by OCT or OCT-A measurement.

In some embodiments, a method of treating wAMD, DME, or RVO is provided, the method comprising: administering 2-3 loading doses of an anti-VEGF antibody polymer conjugate to a subject having wAMD, DME. Or RVO; obtaining a vision assessment of the subject after 4, 8, 12, 16, 20, 24, 36, 48, or 52 weeks after a last loading dose; and based on the vision assessment of the patient, placing the patient on a fixed regimen of the anti-VEGF antibody polymer conjugate once every 4, 8, 12, 16, 20, 24, 36, 48, or 52 weeks.

In some embodiments, a method of treating wAMD, DME, or RVO is provided, the method comprising: administering 1-3 loading doses of an anti-VEGF antibody polymer conjugate to a subject with DME, DR or RVO; not administering more than 3 loading doses to the subject; providing a follow-on application of the anti-VEGF antibody polymer conjugate at a point in time no sooner than 8 or 12 weeks after a last loading dose or after a last follow-on application of the anti-VEGF antibody polymer conjugate, wherein the loading doses are administered to the subject on a monthly basis, and wherein the subject is above 16 years of age.

In some embodiments, a method of treating a subject with DME, DR or RVO is provided. The method comprises administering 1-3 loading doses of an anti-VEGF antibody polymer conjugate to a subject with DME, DR or RVO, not administering more than 3 loading doses to the subject, and providing a follow-on application of the anti-VEGF antibody polymer conjugate at a point in time after a last loading dose or after a last follow-on application of the anti-VEGF antibody polymer conjugate, wherein the loading doses are administered to the subject on a monthly basis, and wherein the subject is above 16 years of age.

In some embodiments, a method of treating wAMD, DME, or RVO is provided, the method comprising: administering 1-3 loading doses of an anti-VEGF antibody polymer conjugate to a subject with DME, DR or RVO; not administering more than 3 loading doses to the subject; providing a follow-on application of the anti-VEGF antibody polymer conjugate at a point in time no sooner than 8 or 12 weeks after a last loading dose or after a last follow-on application of the anti-VEGF antibody polymer conjugate, wherein the loading doses are administered to the subject on a monthly basis. In some embodiments, an improvement of Best Corrected Visual Acuity (BCVA) is greater than at least 5 or at least 6.8 to 7.2 letters post treatment.

In some embodiments, a method of treating wAMD, DME, or RVO is provided, the method comprising: administering 1-3 loading doses of an anti-VEGF antibody polymer conjugate to a subject with DME, DR, or RVO; not administering more than 3 loading doses; and providing between 0-1 follow-on application of the anti-VEGF antibody polymer conjugate at a point in time no sooner than 8 or 12 weeks after a last loading dose or after a last follow-on application of the anti-VEGF antibody polymer conjugate, wherein the loading doses are administered to the subject on a monthly basis.

Also provided is a method of treating diabetic retinopathy, comprising: identifying a subject with diabetic retinopathy; administering 1-3 loading doses of an anti-VEGF antibody polymer conjugate to the subject; administering

one or more subsequent doses of the anti-VEGF antibody polymer conjugate to the subject no more frequently than once every 24 weeks after the last loading dose, wherein at least 4 doses of the anti-VEGF antibody polymer conjugate is administered to the subject in the first year of treatment.

Provided herein is a method of treating a subject having wAMD, comprising: identifying a subject having wAMD; and administering an anti-VEGF antibody polymer conjugate to the subject no more frequently than once every 4 weeks.

Further provided is a method of treating DME, comprising: identifying a subject having DME; administering 2-3 monthly loading doses of an anti-VEGF antibody polymer conjugate to the subject; and administering one or more subsequent doses of the anti-VEGF antibody polymer conjugate to the subject every 2-6 months, depending on: whether retina thickness in the subject is above 320 microns (e.g., above 330 microns, above 340 microns, or above 350 microns); and/or whether the eye is dry as determined by OCT or OCT-A measurement, wherein at least 4 doses of the anti-VEGF antibody polymer conjugate is administered to the subject in the first year of treatment.

Also provided is a method of treating RVO, comprising: identifying a subject having RVO; administering 2 monthly loading doses of an anti-VEGF antibody polymer conjugate to the subject; and administering one or more subsequent doses of the anti-VEGF antibody polymer conjugate to the subject no more frequently than once every 2 months, wherein at least 4 doses of the anti-VEGF antibody polymer conjugate is administered to the subject in the first year of treatment.

Also provided is a method of treating wAMD, including: identifying a subject having wAMD; administering 3 monthly loading doses of an anti-VEGF antibody polymer conjugate (e.g., KSI-301) to the subject; evaluating retina thickness and/or BCVA of the subject at 12 weeks after the third loading dose to thereby determine that the subject has improved visual acuity; evaluating retina thickness and/or BCVA of the subject at 16 weeks after the third loading dose to thereby determine that the subject has improved visual acuity; and administering a maintenance dose of the anti-VEGF antibody polymer conjugate to the subject 20 weeks after the third loading dose; and determining that the subject will receive one or more maintenance doses no more frequently than once every 20 weeks, wherein the subject has improved visual acuity when at least one of: retina thickness is reduced by about 50 microns or more compared to retina thickness before administering the first loading dose; and BCVA is increased by about 3 or more compared to BCVA before administering the first loading dose. In some embodiments, the subject has improved visual acuity when at least one of: retina thickness is reduced by about 40 microns or more compared to retina thickness before administering the first loading dose; and BCVA is increased by about 3 or more compared to BCVA before administering the first loading dose.

Provided herein is a pharmaceutical composition comprising: an anti-VEGF antibody polymer conjugate; 12.5 mM sodium phosphate buffer; and 0.025% (w/w) polysorbate 20, at a pH of about 6.5, wherein approximately 40-55 mg/mL of antibody mass is present and approximately 259-356 mg/mL of total mass of the antibody polymer conjugate is present in the composition.

Also provided is a pharmaceutical composition comprising: an anti-VEGF antibody polymer conjugate; 12.5 mM sodium phosphate buffer; and 0.025% (w/w) polysorbate 20, at a pH of about 6.5, wherein approximately 50 mg/mL of antibody mass is present and approximately 324 mg/mL of total mass of the antibody polymer conjugate is present in the composition.

Further provided herein is a pharmaceutical composition comprising: a) an anti-VEGF antibody polymer conjugate, 12.5 mM sodium phosphate buffer with 0.025% (w/w) polysorbate 20, pH 6.5, wherein the antibody polymer conjugate is present in the composition at a concentration of 50 mg/mL (based on antibody mass), equivalent to 324 mg/mL of total mass of the antibody polymer conjugate, including 50 mg/mL OG1950 Antibody Intermediate and 274 mg/mL OG1802 Biopolymer Intermediate; b) an anti-VEGF antibody polymer conjugate, 10-15 mM sodium phosphate buffer with 0.01-0.5% (w/w) polysorbate 20, pH 6.5, wherein the antibody polymer conjugate is present in the composition at a concentration of about 50 mg/mL (based on antibody mass), equivalent to about 324 mg/mL of total mass of the antibody polymer conjugate; c) an anti-VEGF antibody polymer conjugate, 12.5 mM sodium phosphate buffer with optional 0.025% (w/w) polysorbate 20, pH 6.5, wherein the antibody polymer conjugate is present in the composition at a concentration of 50 mg/mL (based on antibody mass), equivalent to 324 mg/mL of total mass of the antibody polymer conjugate, including 50 mg/mL OG1950 Antibody Intermediate and 274 mg/mL OG1802 Biopolymer Intermediate; d) an anti-VEGF antibody polymer conjugate, sodium phosphate buffer with polysorbate 20, pH 6.5, wherein the antibody polymer conjugate is present in the composition at a concentration of 50 mg/mL (based on antibody mass), equivalent to about 324 mg/mL of total mass of the antibody-biopolymer conjugate, including about 50 mg/mL OG1950 Antibody Intermediate and about 274 mg/mL OG1802 Biopolymer Intermediate; e) an anti-VEGF antibody polymer conjugate, 10-15 mM sodium phosphate buffer with 0.01-0.5% (w/w) polysorbate 20, pH 6.5, wherein the antibody polymer conjugate is present in the composition at a concentration of about 40-55 mg/mL (based on antibody mass), equivalent to about 259-356 mg/mL of total mass of the antibody-biopolymer conjugate; f) an anti-VEGF antibody polymer conjugate, 10-15 mM sodium phosphate buffer with 0.01-0.5% (w/w) polysorbate 20, pH 6.5, wherein the antibody polymer conjugate is present in the composition at a concentration of about 45-52.5 mg/mL (based on antibody mass), equivalent to about 292-340 mg/mL of total mass of the antibody-biopolymer conjugate; or g) an anti-VEGF antibody polymer conjugate, 10-15 mM sodium phosphate buffer with 0.01-0.5% (w/w) polysorbate 20, pH 6.5, wherein the antibody polymer conjugate is present in the composition at a concentration of about 40-55 mg/mL (based on antibody mass), equivalent to about

259-356 mg/mL of total mass of the antibody-biopolymer conjugate, wherein the antibody polymer conjugate comprises an antibody comprising i) VH and VL in FIG. 64, or ii) 6 CDRs within the VH and VL of FIG. 64.

## BRIEF DESCRIPTION OF THE DRAWINGS

**FIG. 1** is a graph showing extended half-life of KSI-301 *in vivo.*

**FIG. 2** is a graph showing *in vivo* retinal bioavailability of KSI-301.

**FIG. 3** is a graph showing rapid systemic clearance of intravenously administered KSI-301.

**FIG. 4** is a graph showing the therapeutic effect of a single intravitreal administration of KSI-301in patients with diabetic macular edema (DME), according to some non-limiting embodiments of the present disclosure.

**FIG. 5** is a schematic diagram representing a KSI-301 intravitreal administration schedule in age-related macular degeneration (wAMD), diabetic macular edema (DME), and retinal vein occlusion (RVO), according to some non-limiting embodiments of the present disclosure.

**FIG. 6** is a set of graphs showing sustained therapeutic effects of KSI-301 after intravitreal administration of loading doses of KSI-301 to patients with wet age-related macular degeneration (wAMD), according to some non-limiting embodiments of the present disclosure.

**FIG. 7** is a set of graphs showing the schedule of intravitreal administration of KSI-301 received by individual patients treated for wAMD, according to some non-limiting embodiments of the present disclosure. 4% (1/25) retreated before 3 months; 5% (1/20) retreated at 3 months; 90% (19/21) have gone longer than 3 months after the last loading dose; and 80% (11/14) reach 4 months or longer until first retreatment.

**FIG. 8** is a set of graphs showing sustained therapeutic effects of KSI-301 after intravitreal administration of loading doses of KSI-301 to patients with diabetic macular edema (DME), according to some non-limiting embodiments of the present disclosure.

**FIG. 9** is a set of graphs showing the schedule of intravitreal administration of KSI-301received by individual patients treated for DME, according to some non-limiting embodiments of the present disclosure.

**FIG. 10** is a set of graphs showing sustained therapeutic effects of KSI-301after intravitreal administration of loading doses of KSI-301 to patients with retinal vein occlusion (RVO), according to some non-limiting embodiments of the present disclosure.

**FIG. 11** is a graph showing the schedule of intravitreal administration of KSI-301 received by individual patients treated for RVO, according to some non-limiting embodiments of the present disclosure.

**FIG. 12** is a collection of images showing sustained improvement in retinal health after intravitreal administration of loading doses of KSI-301 to patients with wAMD (left column), DME (middle column), and RVO (right column), according to some non-limiting embodiments of the present disclosure.

**FIG. 13** is a schematic diagram of the structure of KSI-301, according to some non-limiting embodiments of the present disclosure, where each heavy chain of the anti-VEGF-A antibody is denoted by the letter H, and each light chain of the anti-VEGF-A antibody is denoted by the letter L; the polymer is bonded to the anti-VEGF-A antibody through a sulfhydryl at C443 according to EU numbering, which bond is depicted on one of the heavy chains above; PC is

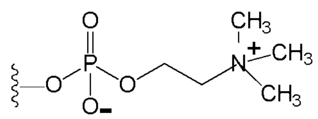

,

where the curvy line indicates the point of attachment to the rest of the polymer; and n1, n2, n3, n4, n5, n6, n7, n8 and n9 are the same or different such that the sum of n1, n2, n3, n4, n5, n6, n6, n7, n8 and n9 is 2500 plus or minus 15%.

**FIG. 14** depicts an amino acid sequence of the heavy and light chains of KSI-301, according to some non-limiting embodiments of the present disclosures.

**FIG. 15** is a set of amino acid sequences for various antibodies.

**FIG. 16** is a graph showing the proportion of patients with differing levels of diabetic retinopathy severity, measured on a standardized photographic reading scale.

**FIGs. 17A** and **17B** display the efficacy of KSI-301 in Wet AMD and the direct effect on the choroidal neovascularization. **FIG. 17A** displays the efficacy of KSI-301 in Wet AMD, and the change from baseline to week in median BCVA and OCT CST.

**FIGs. 18A-18D** show the results in a DME patient with disease modification post 3 loading doses, with significant

DRSS improvement and reperfusion representing disease modification.

**FIG. 19** shows the results of an RVO patient that after 3 loading doses with no additional doses required for at least 5 months, representing possible disease modification.

**FIG. 20** displays a set of OCT images of a patient showing the effect of 3 loading doses lasting 8 weeks until diseases recurs and the patient receives retreatment.

**FIGs. 21A-21C** depict the results from a single-dose bioactivity study.

**FIG. 22** is a set of graphs showing the schedule of intravitreal administration of KSI-301 received by individual patients treated for wAMD, according to some non-limiting embodiments of the present disclosure.

**FIG. 23** is a set of graphs showing sustained therapeutic effects of KSI-301 after intravitreal administration of loading doses of KSI-301 to patients with wet age-related macular degeneration (wAMD), according to some non-limiting embodiments of the present disclosure.

**FIG. 24** is a set of graphs showing sustained therapeutic effects of KSI-301 after intravitreal administration of loading doses of KSI-301 to patients with wet age-related macular degeneration (wAMD), but without high pigment epithelial detachment, according to some non-limiting embodiments of the present disclosure.

**FIG. 25** is a set of graphs showing sustained therapeutic effects of KSI-301 after intravitreal administration of loading doses of KSI-301 to patients with wet age-related macular degeneration (wAMD), according to some non-limiting embodiments of the present disclosure.

**FIG. 26** is a set of graphs showing sustained therapeutic effects of KSI-301 after intravitreal administration of loading doses of KSI-301 to patients with wet age-related macular degeneration (wAMD), but without high pigment epithelial detachment, according to some non-limiting embodiments of the present disclosure.

**FIG. 27** is a set of graphs showing the schedule of intravitreal administration of KSI-301 received by individual patients treated for DME, according to some non-limiting embodiments of the present disclosure.

**FIG. 28** is a set of graphs showing sustained therapeutic effects of KSI-301 after intravitreal administration of loading doses of KSI-301 to patients with diabetic macular edema (DME), according to some non-limiting embodiments of the present disclosure.

**FIG. 29** is a set of graphs showing sustained therapeutic effects of KSI-301 after intravitreal administration of loading doses of KSI-301 to patients with diabetic macular edema (DME), according to some non-limiting embodiments of the present disclosure.

**FIG. 30** is a graph showing the schedule of intravitreal administration of KSI-301 received by individual patients treated for RVO, according to some non-limiting embodiments of the present disclosure.

**FIG. 31** is a set of graphs showing sustained therapeutic effects of KSI-301 after intravitreal administration of loading doses of KSI-301 to patients with retinal vein occlusion (RVO), according to some non-limiting embodiments of the present disclosure.

**FIG. 32** is a set of graphs showing sustained therapeutic effects of KSI-301 after intravitreal administration of loading doses of KSI-301 to patients with retinal vein occlusion (RVO), according to some non-limiting embodiments of the present disclosure.

**FIG. 33** is a schematic diagram representing an antibody binding construct A intravitreal administration schedule in age-related macular degeneration (wAMD), diabetic macular edema (DME), and retinal vein occlusion (RVO), according to some non-limiting embodiments of the present disclosure.

**FIG. 34** is a set of graphs showing the schedule of intravitreal administration of KSI-301 received by individual patients treated for wAMD, according to some non-limiting embodiments of the present disclosure.

**FIG. 35** is a set of graphs showing sustained therapeutic effects of KSI-301 after intravitreal administration of loading doses of KSI-301 to patients with wAMD, according to some non-limiting embodiments of the present disclosure.

**FIG. 36** is a set of graphs showing the schedule of intravitreal administration of KSI-301 received by individual patients treated for DME, according to some non-limiting embodiments of the present disclosure.

**FIG. 37** is a set of graphs showing sustained therapeutic effects of KSI-301after intravitreal administration of loading doses of KSI-301 to patients with DME, according to some non-limiting embodiments of the present disclosure.

**FIG. 38** is a set of graphs showing the schedule of intravitreal administration of KSI-301 received by individual patients treated for RVO, according to some non-limiting embodiments of the present disclosure.

**FIG. 39** is a set of graphs showing sustained therapeutic effects of KSI-301 after intravitreal administration of loading doses of KSI-301 to patients with retinal vein occlusion (RVO), according to some non-limiting embodiments of the present disclosure.

**FIG. 40** is a schematic diagram representing a Phase 2 study design for KSI-301 treatment in treatment-naive wAMD patients and comparison with a standard of care treatment, according to some non-limiting embodiments of the present disclosure.

**FIGS. 41A and 41B** are a collection of graphs showing hypothetical schedule of treatment and probability of remaining on Q20W dosing, based on data from patients from the Phase 1b study but applying the Phase 2 retreatment criteria, according to some non-limiting embodiments of the present disclosure.

**FIG. 42** is a flow chart depicting an embodiment of a method of the present disclosure.

**FIG. 43** is a schematic diagram representing KSI-301 intravitreal administration schedule in age-related macular degeneration (wAMD), diabetic macular edema (DME), and retinal vein occlusion (RVO), according to some non-limiting embodiments of the present disclosure.

**FIG. 44** is a set of graphs showing sustained therapeutic effects of KSI-301 administered to patients with wet age-related macular degeneration (wAMD), according to some non-limiting embodiments of the present disclosure.

**FIG. 45A** is a set of graphs showing the schedule of intravitreal administration of KSI-301 received by individual patients treated for wAMD, according to some non-limiting embodiments of the present disclosure.

**FIG. 45B** is a table summarizing the administration interval of KSI-301 in wAMD patients shown in FIG. 45A.

**FIG. 46** is a collection of OCT images of a wAMD patient treated with KSI-301, according to some non-limiting embodiments of the present disclosure.

**FIG. 47** is a graph showing benchmarking of KSI-301 in wAMD against a standard-of-care treatment, according to some non-limiting embodiments of the present disclosure.

**FIG. 48** is a graph showing benchmarking of KSI-301 in wAMD against a standard-of-care treatment, according to some non-limiting embodiments of the present disclosure.

**FIG. 49** is a set of graphs showing sustained therapeutic effects of KSI-301 administered to patients with diabetic macular edema (DME), according to some non-limiting embodiments of the present disclosure.

**FIG. 50A** is a set of graphs showing the schedule of intravitreal administration of KSI-301 received by individual patients treated for DME, according to some non-limiting embodiments of the present disclosure.

**FIG. 50B** is a table summarizing the administration interval of KSI-301 in DME patients shown in FIG. 50A.

**FIG. 51** is a collection of OCT images of a DME patient treated with KSI-301, according to embodiments of the present disclosure.

**FIG. 52** is a collection of OCT images of a DME patient treated with KSI-301, according to some non-limiting embodiments of the present disclosure.

**FIG. 53** is a set of graphs showing sustained therapeutic effects of KSI-301 administered to patients with retinal vein occlusion (RVO), according to some non-limiting embodiments of the present disclosure.

**FIG. 54A** is a set of graphs showing the schedule of intravitreal administration of KSI-301 received by individual patients treated for RVO, according to some non-limiting embodiments of the present disclosure.

**FIG. 54B** is a table summarizing the administration interval of KSI-301 in RVO patients shown in FIG. 54A.

**FIG. 55** is a graph showing benchmarking of KSI-301 in RVO against a standard-of-care treatment, according to some non-limiting embodiments of the present disclosure.

**FIG. 56** is a collection of OCT images of a CRVO patient treated with KSI-301, according to some non-limiting embodiments of the present disclosure.

**FIG. 57** is a schematic diagram representing an anti-VEGF antibody conjugate intravitreal administration schedule in age-related macular degeneration (wAMD), according to some non-limiting embodiments of the present disclosure.

**FIG. 58** is a schematic diagram representing an anti-VEGF antibody conjugate intravitreal administration schedule in diabetic macular edema (DME), according to some non-limiting embodiments of the present disclosure.

**FIG. 59** is a schematic diagram representing an anti-VEGF antibody conjugate intravitreal administration schedule in retinal vein occlusion (RVO), according to some non-limiting embodiments of the present disclosure.

**Fig. 60** is a graph showing sustained therapeutic effects of KSI-301 after intravitreal administration of loading doses and subsequent doses of KSI-301 to patients with DME, according to some non-limiting embodiments of the present disclosure.

**Fig. 61** is a set of graphs showing the proportion of patients requiring follow-on doses after administration of loading doses of KSI-301 to patients with DME and the average treatment interval required for patients requiring subsequent follow-on doses of KSI-301.

**Fig. 62** is a graph showing sustained therapeutic effects of KSI-301 after intravitreal administration of loading doses and subsequent doses of KSI-301 to patients with RVO, according to some non-limiting embodiments of the present disclosure.

**Fig. 63** is a set of graphs showing the proportion of patients requiring follow-on doses after administration of loading doses of KSI-301 to patients with RVO and the average treatment interval required for patients requiring subsequent follow-on doses of KSI-301.

**Fig. 64** is an amino acid sequence for some embodiments of OG1950.

**FIG. 65** is a schematic diagram showing patient disposition in a randomized, multicenter study of KSI-301, according to some non-limiting embodiments of the present disclosure.

**FIG. 66** is a table showing the baseline patient demographics in a randomized, multicenter study of KSI-301, according to some non-limiting embodiments of the present disclosure.

**FIG. 67** is a table showing ocular baseline characteristics for patients in a randomized, multicenter study of KSI-301, according to some non-limiting embodiments of the present disclosure.

**FIGs. 68A** and **68B** are a collection of graphs showing average therapeutic effects of KSI-301 administered to patients with wAMD, according to some non-limiting embodiments of the present disclosure.

**FIG. 69** is a chart and a graph showing sustained therapeutic effects of intravitreal KSI-301 in a patient subgroup, according to some non-limiting embodiments of the present disclosure.

**FIG. 70** is a chart and a graph showing therapeutic effects of intravitreal KSI-301 in different patient subgroups, according to some non-limiting embodiments of the present disclosure.

**FIG. 71** is a chart and a graph showing sustained therapeutic effects of intravitreal KSI-301 in a patient subgroup, according to some non-limiting embodiments of the present disclosure.

**FIG. 72** is a chart and a graph showing therapeutic effects of intravitreal KSI-301 in different patient subgroups, according to some non-limiting embodiments of the present disclosure.

## DETAILED DESCRIPTION

In some embodiments, provided herein are methods of treating an eye disorder by administering an anti-VEGF antibody to a subject having an eye disorder. The anti-VEGF antibody of the present disclosure may be an anti-VEGF antibody conjugate that includes a polymeric moiety that extends the half-life of the antibody when administered to a subject (e.g., KSI-301). The antibody conjugate can retain therapeutic efficacy after administration for a longer time period compared to an antibody without the polymeric moiety. Thus, in some embodiments, the methods of the present disclosure may provide for a course of treatment for an eye disorder that includes fewer doses (e.g., less frequent administration) of the anti-VEGF antibody conjugates than conventional anti-VEGF antibody therapies, to achieve a therapeutic effect of the anti-VEGF therapy on the subject. In some embodiments, the present methods may encourage better patient compliance with the treatment course especially when the eye disorder treatment involves intravitreal administration of the therapeutic agent. In some embodiments, the present methods can achieve an increased therapeutic effect compared to alternative standard therapies, at a comparable, increased, or decreased dosing schedules of the anti-VEGF therapy. In some embodiments, the present methods may allow for long lasting or even lifelong therapeutic effect. In some embodiments, the present methods may allow for personalized dosing schedules of the anti-VEGF therapy according to specific disease progression markers as evaluated by physicians and ophthalmic examination. In some embodiments, the methods provided herein not only stop disease progression, but can reverse disease progression.

In some embodiments, the methods herein can include administering one or more doses of an anti-VEGF antibody conjugate (e.g., KSI-301 which includes an antibody conjugated to a phosphorylcholine polymer, as provided herein), or an anti-VEGF protein conjugate (e.g., aflibercept biopolymer conjugate) to a subject (e.g., human or other mammalian patient) in need of treating an eye disorder, to thereby treat the eye disorder. The anti-VEGF antibody conjugate or anti-VEGF protein conjugate, when administered to the subject, can provide a long-lasting therapeutic effect that allows for a dosing schedule with longer intervals between dosing than has been previously used with anti-VEGF therapies. Additionally, aggressive treatment with shorter dosing schedules can provide enhanced therapeutic efficacy due to the long half-life of the anti-VEGF antibody conjugate or anti-VEGF protein conjugate within the eye. In some embodiments, a therapeutic result of the anti-VEGF antibody conjugate therapy or anti-VEGF protein conjugate therapy, once achieved by administration of one or more doses (e.g., loading dose and/or maintenance dose) of the anti-VEGF antibody conjugate or anti-VEGF protein conjugate, is retained by the subject without requiring any additional dose thereafter, e.g., retained for the rest of the subject's life. The long-lasting nature of the anti-VEGF antibody conjugate and differences in individual patient physiology can also allow for custom dosing schedules of the anti-VEGF antibody conjugate or the anti-VEGF protein conjugate - based on physician opinion and clinical ophthalmic examination. Where a physician has observed continued benefits and lessened disease indicia, they may order a relaxed dosing schedule with longer intervals. Where a physician has observed continued degradation and increased disease indicia, they may order a more aggressive dosing schedule with shorter intervals between doses. The benefits of custom dosing schedules based on ophthalmic examination follow-ups allows for patients to receive effective therapeutics, while also decreasing patient burdens associated with shorter dosing intervals.

### Definitions

A "dosing schedule" is a clinical regimen for administration of a therapeutic agent. Examples of dosing schedules include administration of a therapeutic agent every day, every week, every 4 weeks, or as needed due to monitoring of patient symptoms and symptomology. Dosing schedules can vary due to the severity of the underlying disorder or disease state.

A "dose frequency" is a clinical regimen for administration of a therapeutic agent. The dose frequency may be every day, every week, every 4 weeks, or as needed due to monitoring of patient symptoms and symptomology. Dose frequencies can vary due to the severity of the underlying disorder or disease state.

A "Ophthalmic Exam" is a comprehensive series of tests performed by a physician to assess vision and eye

health of a patient. Commonly, Ophthalmic exams include patient histories, refraction tests to check vision, optical coherence tomography, and other assessments to aid a physician in rendering a professional opinion regarding a patient's eye health. During an Ophthalmic Exam, a physician may use imaging techniques including but not limited to Fundus Photograph, SD-OCT, OCT-A, and Fluorescein Angiography (FA) to characterize anatomical features of the eye. A physician may further characterize whether anatomical structures within the eye are exudative or not, where structures that appear to "leak" bodily fluids would be "wet," while non-exudative structures would be "dry". On imaging an eye, the physician may also characterize finding excess intra and/or subretinal fluid and/or subretinal hyperreflective material (SHRM) affecting the central subfield of the eye as "wet", while the lack of excess intra and/or subretinal fluid affecting the central subfield of the eye as "dry."

A "neovascular disorder" is a disorder or disease state characterized by altered, dysregulated or unregulated angiogenesis. Examples of neovascular disorders include neoplastic transformation (e.g. cancer) and ocular neovascular disorders including diabetic retinopathy, age-related macular degeneration, and retinal vein occlusion.

An "ocular neovascular" disorder is a disorder characterized by altered, dysregulated or unregulated angiogenesis in the eye of a patient. Such disorders include retinal vein occlusion, optic disc neovascularization, iris neovascularization, retinal neovascularization, choroidal neovascularization, corneal neovascularization, vitreal neovascularization, glaucoma, pannus, pterygium, macular edema, diabetic retinopathy, diabetic macular edema, vascular retinopathy, retinal degeneration, uveitis, inflammatory diseases of the retina, and proliferative vitreoretinopathy.

The term antibody includes intact antibodies and binding fragments thereof. A binding fragment refers to a molecule other than an intact antibody that comprises a portion of an intact antibody that binds the antigen to which the intact antibody binds. Examples of binding fragments include Fv, Fab', Fab'-SH, F(ab')2; diabodies; linear antibodies; single-chain antibody molecules (e.g. scFv); and multispecific antibodies formed from antibody fragments. scFv antibodies are described in Houston JS. 1991. Methods in Enzymol. 203:46-96. In addition, antibody fragments comprise single chain polypeptides having the characteristics of a VH domain, namely being able to assemble together with a VL domain, or of a VL domain, namely being able to assemble together with a VH domain to a functional antigen binding site and thereby providing the antigen binding property of full length antibodies.

Specific binding of an antibody to its target antigen(s) means an affinity of at least $10^6$, $10^7$, $10^8$, $10^9$, or $10^{10}$ $M^{-1}$. Specific binding is detectably higher in magnitude and distinguishable from non-specific binding occurring to at least one unrelated target. Specific binding can be the result of formation of bonds between particular functional groups or particular spatial fit (e.g., lock and key type) whereas nonspecific binding is usually the result of van der Waals forces. Specific binding does not however necessarily imply that an antibody or fusion protein binds one and only one target.

A basic antibody structural unit is a tetramer of subunits. Each tetramer includes two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. This variable region is initially expressed linked to a cleavable signal peptide. The variable region without the signal peptide is sometimes referred to as a mature variable region. Thus, for example, a light chain mature variable region means a light chain variable region without the light chain signal peptide. However, reference to a variable region does not mean that a signal sequence is necessarily present; and in fact signal sequences are cleaved once the antibodies or fusion proteins have been expressed and secreted. A pair of heavy and light chain variable regions defines a binding region of an antibody. The carboxy-terminal portion of the light and heavy chains respectively defines light and heavy chain constant regions. The heavy chain constant region is primarily responsible for effector function. In IgG antibodies, the heavy chain constant region is divided into CH1, hinge, CH2, and CH3 regions. The CH1 region binds to the light chain constant region by disulfide and noncovalent bonding. The hinge region provides flexibility between the binding and effector regions of an antibody and also provides sites for intermolecular disulfide bonding between the two heavy chain constant regions in a tetramer subunit. The CH2 and CH3 regions are the primary site of effector functions and FcR binding.

Light chains are classified as either kappa or lambda. Heavy chains are classified as gamma, mu, alpha, delta, or epsilon, and define the antibody's isotype as IgG, IgM, IgA, IgD and IgE, respectively. Within light and heavy chains, the variable and constant regions are joined by a "J" segment of about 12 or more amino acids, with the heavy chain also including a "D" segment of about 10 or more amino acids. (See generally, Fundamental Immunology (Paul, W., ed., 2nd ed. Raven Press, N.Y., 1989), Ch. 7) (incorporated by reference in its entirety for all purposes).

The mature variable regions of each light/heavy chain pair form the antibody binding site. Thus, an intact antibody has two binding sites, i.e., is divalent. In natural antibodies, the binding sites are the same. However, bispecific antibodies can be made in which the two binding sites are different (see, e.g., Songsivilai S, Lachmann PC. 1990. Bispecific antibody: a tool for diagnosis and treatment of disease. Clin Exp Immunol. 79:315-321; Kostelny SA, Cole MS, Tso JY. 1992. Formation of bispecific antibody by the use of leucine zippers. J Immunol. 148: 1547-1553). The variable regions all exhibit the same general structure of relatively conserved framework regions (FR) joined by three hypervariable regions, also called complementarity determining regions or CDRs. The CDRs from the two chains of each pair are aligned by the framework regions, enabling binding to a specific epitope. From N-terminal to C-terminal, both light and heavy chains comprise the domains FRl, CDRl, FR2, CDR2, FR3, CDR3 and FR4. For convenience, the variable heavy CDRs can be

referred to as $CDR_H1$, $CDR_H2$ and $CDR_H3$; the variable light chain CDRs can be referred to as $CDR_L1$, $CDR_L2$ and $CDR_L3$. The assignment of amino acids to each domain is in accordance with the definitions of Kabat EA, et al. 1987 and 1991. Sequences of Proteins of Immunological Interest (National Institutes of Health, Bethesda, MD) or Chothia C, Lesk AM. 1987. Canonical Structures for the Hypervariable Regions of Immunoglobulins. J Mol Biol 196:901-917; Chothia C, et al. 1989. Conformations of Immunoglobulin Hypervariable Regions. Nature 342:877-883. Kabat also provides a widely used numbering convention (Kabat numbering) in which corresponding residues between different heavy chain variable regions or between different light chain variable regions are assigned the same number. Although Kabat numbering can be used for antibody constant regions, EU numbering is more commonly used, as is the case in this application. Although specific sequences are provided for exemplary antibodies disclosed herein, it will be appreciated that after expression of protein chains one to several amino acids at the amino or carboxy terminus of the light and/or heavy chain, particularly a heavy chain C-terminal lysine residue, may be missing or derivatized in a proportion or all of the molecules.

The term "epitope" refers to a site on an antigen to which an antibody or extracellular trap segment binds. An epitope on a protein can be formed from contiguous amino acids or noncontiguous amino acids juxtaposed by tertiary folding of one or more proteins. Epitopes formed from contiguous amino acids (also known as linear epitopes) are typically retained on exposure to denaturing solvents whereas epitopes formed by tertiary folding (also known as conformational epitopes) are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. See, e.g., Epitope Mapping Protocols, in Methods in Molecular Biology, Vol. 66, Glenn E. Morris, Ed. (1996).

Antibodies that recognize the same or overlapping epitopes can be identified in a simple immunoassay showing the ability of one antibody to compete with the binding of another antibody to a target antigen. The epitope of an antibody can also be defined by X-ray crystallography of the antibody (or Fab fragment) bound to its antigen to identify contact residues.

Alternatively, two antibodies have the same epitope if all amino acid mutations in the antigen that reduce or eliminate binding of one antibody reduce or eliminate binding of the other. Two antibodies have overlapping epitopes if some amino acid mutations that reduce or eliminate binding of one antibody reduce or eliminate binding of the other.

Competition between antibodies is determined by an assay in which an antibody under test inhibits specific binding of a reference antibody to a common antigen (see, e.g., Junghans et al., Cancer Res. 50: 1495, 1990). A test antibody competes with a reference antibody if an excess of a test antibody (e.g., at least 2x, 5x, 10x, 20x or 100x) inhibits binding of the reference antibody by at least 50%. In some embodiments the test antibody inhibits binding of the reference antibody by 75%, 90%, or 99% as measured in a competitive binding assay. Antibodies identified by competition assay (competing antibodies) include antibodies binding to the same epitope as the reference antibody and antibodies binding to an adjacent epitope sufficiently proximal to the epitope bound by the reference antibody for steric hindrance to occur.

The term "patient" includes human and other mammalian subjects that receive either prophylactic or therapeutic treatment.

For purposes of classifying amino acids substitutions as conservative or nonconservative, amino acids are grouped as follows: Group I (hydrophobic side chains): met, ala, val, leu, ile; Group II (neutral hydrophilic side chains): cys, ser, thr; Group III (acidic side chains): asp, glu; Group IV (basic side chains): asn, gin, his, lys, arg; Group V (residues influencing chain orientation): gly, pro; and Group VI (aromatic side chains): trp, tyr, phe. Conservative substitutions involve substitutions between amino acids in the same class. Non-conservative substitutions constitute exchanging a member of one of these classes for a member of another.

Percentage sequence identities are determined with antibody sequences maximally aligned by the Kabat numbering convention for a variable region or EU numbering for a constant region. After alignment, if a subject antibody region (e.g., the entire mature variable region of a heavy or light chain) is being compared with the same region of a reference antibody, the percentage sequence identity between the subject and reference antibody regions is the number of positions occupied by the same amino acid in both the subject and reference antibody region divided by the total number of aligned positions of the two regions, with gaps not counted, multiplied by 100 to convert to percentage. Sequence identities of other sequences can be determined by aligning sequences using algorithms, such as BESTFIT, FASTA, and TFASTA in the Wisconsin Genetics Software Package Release 7.0, Genetics Computer Group, 575 Science Dr., Madison, WI, using default gap parameters, or by inspection, and the best alignment (*i.e.,* resulting in the highest percentage of sequence similarity over a comparison window). Percentage of sequence identity is calculated by comparing two optimally aligned sequences over a window of comparison, determining the number of positions at which the identical residues occurs in both sequences to yield the number of matched positions, dividing the number of matched positions by the total number of positions in the window of comparison (*i.e.,* the window size), and multiplying the result by 100 to yield the percentage of sequence identity.

Compositions or methods "comprising" one or more recited elements may include other elements not specifically recited. For example, a composition that comprises antibody may contain the antibody alone or in combination with other ingredients.

The term "antibody-dependent cellular cytotoxicity", or ADCC, is a mechanism for inducing cell death that depends upon the interaction of antibody-coated target cells (i.e., cells with bound antibody) with immune cells possessing lytic activity (also referred to as effector cells). Such effector cells include natural killer cells, monocytes/macrophages and neutrophils. ADCC is triggered by interactions between the Fc region of an antibody bound to a cell and Fcγ receptors, particularly FcγRI and FcγRIII, on immune effector cells such as neutrophils, macrophages and natural killer cells. The target cell is eliminated by phagocytosis or lysis, depending on the type of mediating effector cell. Death of the antibody-coated target cell occurs as a result of effector cell activity.

The term opsonization also known as "antibody-dependent cellular phagocytosis", or ADCP, refers to the process by which antibody-coated cells are internalized, either in whole or in part, by phagocytic immune cells (e.g., macrophages, neutrophils and dendritic cells) that bind to an immunoglobulin Fc region.

The term "complement-dependent cytotoxicity" or CDC refers to a mechanism for inducing cell death in which an Fc effector domain(s) of a target-bound antibody activates a series of enzymatic reactions culminating in the formation of holes in the target cell membrane. Typically, antigen-antibody complexes such as those on antibody-coated target cells bind and activate complement component Clq which in turn activates the complement cascade leading to target cell death. Activation of complement may also result in deposition of complement components on the target cell surface that facilitate ADCC by binding complement receptors (e.g., CR3) on leukocytes.

A humanized antibody is a genetically engineered antibody in which the CDRs from a non-human "donor" antibody are grafted into human "acceptor" antibody sequences (see, e.g., Queen, US 5,530,101 and 5,585,089; Winter, US 5,225,539, Carter, US 6,407,213, Adair, US 5,859,205 6,881,557, Foote, US 6,881,557). The acceptor antibody sequences can be, for example, a mature human antibody sequence, a composite of such sequences, a consensus sequence of human antibody sequences, or a germline region sequence. Thus, a humanized antibody is an antibody having some or all CDRs entirely or substantially from a donor antibody and variable region framework sequences and constant regions, if present, entirely or substantially from human antibody sequences. Similarly a humanized heavy chain has at least one, two and usually all three CDRs entirely or substantially from a donor antibody heavy chain, and a heavy chain variable region framework sequence and heavy chain constant region, if present, substantially from human heavy chain variable region framework and constant region sequences. Similarly a humanized light chain has at least one, two and usually all three CDRs entirely or substantially from a donor antibody light chain, and a light chain variable region framework sequence and light chain constant region, if present, substantially from human light chain variable region framework and constant region sequences. Other than nanobodies and dAbs, a humanized antibody comprises a humanized heavy chain and a humanized light chain. A CDR in a humanized antibody is substantially from a corresponding CDR in a non-human antibody when at least 85%, 90%, 95% or 100% of corresponding residues (as defined by Kabat) are identical between the respective CDRs. The variable region framework sequences of an antibody chain or the constant region of an antibody chain are substantially from a human variable region framework sequence or human constant region respectively when at least 85, 90, 95 or 100% of corresponding residues defined by Kabat are identical.

Although humanized antibodies often incorporate all six CDRs (which can be as defined by Kabat) from a mouse antibody, they can also be made with less than all CDRs (e.g., at least 3, 4, or 5 CDRs from a mouse antibody) (e.g., De Pascalis R, Iwahashi M, Tamura M, et al. 2002. Grafting "Abbreviated" Complementary-Determining Regions Containing Specificity-Determining Residues Essential for Ligand Contact to Engineer a Less Immunogenic Humanized Monoclonal Antibody. J Immunol. 169:3076-3084; Vajdos FF, Adams CW, Breece TN, Presta LG, de Vos AM, Sidhu, SS. 2002. Comprehensive functional maps of the antigen-binding site of an anti-ErbB2 antibody obtained with shotgun scanning mutagenesis. J Mol Biol. 320: 415-428; Iwahashi M, Milenic DE, Padlan EA, et al. 1999. CDR substitutions of a humanized monoclonal antibody (CC49): Contributions of individual CDRs to antigen binding and immunogenicity. Mol Immunol. 36:1079-1091; Tamura M, Milenic DE, Iwahashi M, et al. 2000. Structural correlates of an anticarcinoma antibody: Identification of specificity-determining regions (SDRs) and development of a minimally immunogenic antibody variant by retention of SDRs only. J Immunol. 164:1432-1441).

A chimeric antibody is an antibody in which the mature variable regions of light and heavy chains of a non-human antibody (e.g., a mouse) are combined with human light and heavy chain constant regions. Such antibodies substantially or entirely retain the binding specificity of the mouse antibody, and are about two-thirds human sequence.

A veneered antibody is a type of humanized antibody that retains some and usually all of the CDRs and some of the non-human variable region framework residues of a non-human antibody but replaces other variable region framework residues that may contribute to B- or T-cell epitopes, for example exposed residues (Padlan EA. 1991. A possible procedure for reducing the immunogenicity of antibody variable domains while preserving their ligand-binding properties. Mol Immunol. 28:489-98) with residues from the corresponding positions of a human antibody sequence. The result is an antibody in which the CDRs are entirely or substantially from a non-human antibody and the variable region frameworks of the non-human antibody are made more human-like by the substitutions. A human antibody can be isolated from a human, or otherwise result from expression of human immunoglobulin genes (e.g., in a transgenic mouse, in vitro or by phage display). Methods for producing human antibodies include the trioma method of Östberg L, Pursch E. 1983. Human x (mouse x human) hybridomas stably producing human antibodies. Hybridoma 2:361-367; Östberg, U.S. Patent No.

4,634,664; and Engleman et al., US Patent 4,634,666, use of transgenic mice including human immunoglobulin genes (see, e.g., Lonberg et al., WO93/12227 (1993); US 5,877,397, US 5,874,299, US 5,814,318, US 5,789,650, US 5,770,429, US 5,661,016, US 5,633,425, US 5,625,126, US 5,569,825, US 5,545,806, Nature 148, 1547-1553 (1994), Nature Biotechnology 14, 826 (1996), Kucherlapati, WO 91/10741 (1991) and phage display methods (see, .e.g. Dower et al., WO 91/17271 and McCafferty et al., WO 92/01047, US 5,877,218, US 5,871,907, US 5,858,657, US 5,837,242, US 5,733,743 and US 5,565,332.

"Polymer" refers to a series of monomer groups linked together. A polymer is composed of multiple units of a single monomer (a homopolymer) or different monomers (a heteropolymer). High MW polymers are prepared from monomers that include, but are not limited to, acrylates, methacrylates, acrylamides, methacrylamides, styrenes, vinyl-pyridine, vinyl-pyrrolidone and vinyl esters such as vinyl acetate. Additional monomers are useful in high MW polymers . When two different monomers are used, the two monomers are called "comonomers," meaning that the different monomers are copolymerized to form a single polymer. The polymer can be linear or branched. When the polymer is branched, each polymer chain is referred to as a "polymer arm." The end of the polymer arm linked to the initiator moiety is the proximal end, and the growing-chain end of the polymer arm is the distal end. On the growing chain-end of the polymer arm, the polymer arm end group can be the radical scavenger, or another group.

"Initiator" refers to a compound capable of initiating a polymerization using monomers or comonomers. The polymerization can be a conventional free radical polymerization or a controlled/"living" radical polymerization, such as Atom Transfer Radical Polymerization (ATRP), Reversible Addition-Fragmentation-Termination (RAFT) polymerization or nitroxide mediated polymerization (NMP). The polymerization can be a "pseudo" controlled polymerization, such as degenerative transfer. When the initiator is suitable for ATRP, it contains a labile bond which can be homolytically cleaved to form an initiator fragment, I, being a radical capable of initiating a radical polymerization, and a radical scavenger, I', which reacts with the radical of the growing polymer chain to reversibly terminate the polymerization. The radical scavenger I' is typically a halogen, but can also be an organic moiety, such as a nitrile. In some embodiments, the initiator contains one of more 2-bromoisobutyrate groups as sites for polymerization via ATRP.

A "chemical linker" refers to a chemical moiety that links two groups together, such as a half-life extending moiety and a protein. The linker can be cleavable or non-cleavable. Cleavable linkers can be hydrolyzable, enzymatically cleavable, pH sensitive, photolabile, or disulfide linkers, among others. Other linkers include homobifunctional and heterobifunctional linkers. A "linking group" is a functional group capable of forming a covalent linkage consisting of one or more bonds to a bioactive agent. Non-limiting examples include those illustrated in Table 1 of WO2013059137 (incorporated by reference).

The term "reactive group" refers to a group that is capable of reacting with another chemical group to form a covalent bond, i.e. is covalently reactive under suitable reaction conditions, and generally represents a point of attachment for another substance. The reactive group is a moiety, such as maleimide or succinimidyl ester, is capable of chemically reacting with a functional group on a different moiety to form a covalent linkage. Reactive groups generally include nucleophiles, electrophiles and photoactivatable groups.

"Phosphorylcholine," also denoted as "PC," refers to the following:

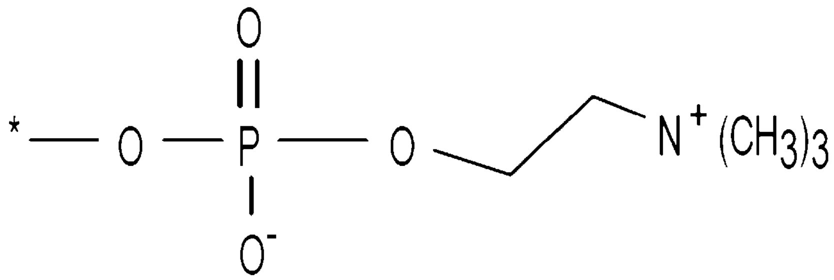

where * denotes the point of attachment. The phosphorylcholine is a zwitterionic group and includes salts (such as inner salts), and protonated and deprotonated forms thereof.

"Phosphorylcholine containing polymer" is a polymer that contains phosphorylcholine. "Zwitterion containing polymer" refers to a polymer that contains a zwitterion.

Poly(acryloyloxyethyl phosphorylcholine) containing polymer refers to a polymer containing 2-(acryloyloxy) ethyl-2-(trimethylammonium)ethyl phosphate (HEA-PC shown below in Example 6) as monomer.

Poly(methacryloyloxyethyl phosphorylcholine) containing polymer refers to a polymer containing 2-(methacryloyloxy)ethyl-2-(trimethylammonium)ethyl phosphate (HEMA-PC or MPC) as monomer (see below):

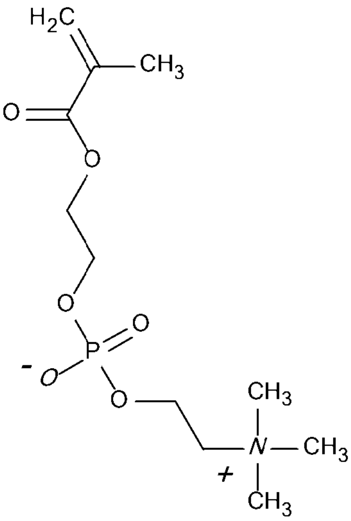

As used herein, "MPC" and "HEMA-PC" are interchangeable.

"Molecular weight" in the context of the polymer can be expressed as either a number average molecular weight, or a weight average molecular weight or a peak molecular weight. Unless otherwise indicated, all references to molecular weight herein refer to the peak molecular weight. These molecular weight determinations, number average (Mn), weight average (Mw) and peak (Mp), can be measured using size exclusion chromatography or other liquid chromatography techniques. Other methods for measuring molecular weight values can also be used, such as the use of end-group analysis or the measurement of colligative properties (e.g., freezing-point depression, boiling-point elevation, or osmotic pressure) to determine number average molecular weight, or the use of light scattering techniques, ultracentrifugation or viscometry to determine weight average molecular weight. In some embodiments, the molecular weight is measured by SEC-MALS (size exclusion chromatography - multi angle light scattering). In some embodiments, the polymeric reagents are typically polydisperse (*i.e.,* number average molecular weight and weight average molecular weight of the polymers are not equal), and can possess low polydispersity values of, for example, less than about 1.5, as judged, for example, by the PDI value derived from the SEC-MALS measurement. In some embodiments, the polydispersities (PDI) are in the range of about 1.4 to about 1.2. In some embodiments the PDI is less than about 1.15, 1.10, 1.05, or 1.03.

The phrase "a" or "an" entity refers to one or more of that entity; for example, a compound refers to one or more compounds or at least one compound. As such, the terms "a" (or "an"), "one or more", and "at least one" can be used interchangeably herein.

"About" means variation one might see in measurements taken among different instruments, samples, and sample preparations.

"Protected," "protected form," "protecting group" and "protective group" refer to the presence of a group (*i.e.,* the protecting group) that prevents or blocks reaction of a particular chemically reactive functional group in a molecule under certain reaction conditions. Protecting groups vary depending upon the type of chemically reactive group being protected as well as the reaction conditions to be employed and the presence of additional reactive or protecting groups in the molecule, if any. Suitable protecting groups include those such as found in the treatise by Greene et al., "Protective Groups In Organic Synthesis," 3rd Edition, John Wiley and Sons, Inc., New York, 1999.

"Alkyl" refers to a straight or branched, saturated, aliphatic radical having the number of carbon atoms indicated. For example, $C_1$-$C_6$ alkyl includes, but is not limited to, methyl, ethyl, propyl, isopropyl, butyl, isobutyl, sec-butyl, tert-butyl, pentyl, isopentyl, hexyl, etc. Other alkyl groups include, but are not limited to heptyl, octyl, nonyl, decyl, etc. Alkyl can include any number of carbons, such as 1-2, 1-3, 1-4, 1-5, 1-6, 1-7, 1-8, 1-9, 1-10, 2-3, 2-4, 2-5, 2-6, 3-4, 3-5, 3-6, 4-5, 4-6 and 5-6. The alkyl group is typically monovalent, but can be divalent, such as when the alkyl group links two moieties together.

The term "lower" referred to above and hereinafter in connection with organic radicals or compounds respectively defines a compound or radical which can be branched or unbranched with up to and including 7 or up to and including 4 and (as unbranched) one or two carbon atoms.

"Alkylene" refers to an alkyl group, as defined above, linking at least two other groups, *i.e.,* a divalent hydrocarbon radical. The two moieties linked to the alkylene can be linked to the same atom or different atoms of the alkylene. For instance, a straight chain alkylene can be the bivalent radical of $-(CH_2)_n$, where n is 1, 2, 3, 4, 5 or 6. Alkylene groups include, but are not limited to, methylene, ethylene, propylene, isopropylene, butylene, isobutylene, sec-butylene, pentylene and hexylene.

Substituents for the alkyl and heteroalkyl radicals (including those groups often referred to as alkylene, alkenyl,

heteroalkylene, heteroalkenyl, alkynyl, cycloalkyl, heterocycloalkyl, cycloalkenyl, and heterocycloalkenyl) can be a variety of groups selected from: -OR', =O, =NR', =N-OR', -NR'R", -SR', -halogen, -SiR'R"R"', -OC(O)R', -C(O)R', $-CO_2R'$, -CONR'R", -OC( O)NR'R", -NR"C(O)R', -NR'-C(O)NR"R"', $-NR"C(O)_2R'$, $-NH-C(NH_2)=NH$, $-NR'C(NH_2)=N$ H, $-NH-C(NH_2)=NR'$, -S(O)R', $-S(O)_2R'$, $-S(O)_2NR'R"$, -CN and $-NO_2$ in a number ranging from zero to (2m'+1), where m' is the total number of carbon atoms in such radical. R', R" and R‴ each independently refer to hydrogen, unsubstituted $(C_1-C_8)$alkyl and heteroalkyl, unsubstituted aryl, aryl substituted with 1-3 halogens, unsubstituted alkyl, alkoxy or thioalkoxy groups, or aryl-$(C_1-C_4)$alkyl groups. When R' and R" are attached to the same nitrogen atom, they can be combined with the nitrogen atom to form a 5-, 6-, or 7-membered ring. For example, -NR'R" is meant to include 1-pyrrolidinyl and 4-morpholinyl. The term "alkyl" is include groups such as haloalkyl (*e.g.,* $-CF_3$ and $-CH_2CF_3$) and acyl (*e.g.,* $-C(O)CH_3$, $-C(O)CF_3$, $-C(O)CH_2OCH_3$, and the like). In some embodiments, the substituted alkyl and heteroalkyl groups have from 1 to 4 substituents. In some embodiments, the substituted akyl and heteroalkyl groups have 1, 2 or 3 substituents. Exceptions are those perhalo alkyl groups (e.g., pentafluoroethyl and the like) .

Substituents for the alkyl and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) can be one or more of a variety of groups selected from, but not limited to: -OR', =O, =NR', =N-OR', -NR'R", -SR', -halogen, -SiR'R"R‴, -OC(O)R', -C(O)R', $-CO_2R'$, -CONR'R", -OC( O)NR'R", -NR"C(O)R', -NR'-C(O)NR"R"', $-NR"C(O)_2R'$, -NR-C(NR'R"R‴) =NR⁗, -NR-C( NR'R")=NR'", -S(O)R', $-S(O)_2R'$, $-S(O)_2NR'R"$, $-NRSO_2R'$, -CN and $-NO_2$ in a number ranging from zero to (2m'+1), where m' is the total number of carbon atoms in such radical. R', R", R"' and R⁗ each independently refer to hydrogen, substituted or unsubstituted heteroalkyl, substituted or unsubstituted aryl, e.g., aryl substituted with 1-3 halogens, substituted or unsubstituted alkyl, alkoxy or thioalkoxy groups, or arylalkyl groups. When a compound includes more than one R group, for example, each of the R groups is independently selected as are each R', R", R'" and R"" groups when more than one of these groups is present. When R' and R" are attached to the same nitrogen atom, they can be combined with the nitrogen atom to form a 5-, 6-, or 7-membered ring. For example, -NR'R" is meant to include, but not be limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl (*e.g.,* $-CF_3$ and $-CH_2CF_3$) and acyl (*e.g.,* $-C(O)CH_3$, $-C(O)CF_3$, $-C(O)CH_2OCH_3$, and the like).

"Alkoxy" refers to alkyl group having an oxygen atom that either connects the alkoxy group to the point of attachment or is linked to two carbons of the alkoxy group. Alkoxy groups include, for example, methoxy, ethoxy, propoxy, iso-propoxy, butoxy, 2-butoxy, iso-butoxy, sec-butoxy, tert-butoxy, pentoxy, hexoxy, etc. The alkoxy groups can be further substituted with a variety of substituents described within. For example, the alkoxy groups can be substituted with halogens to form a "halo-alkoxy" group.

"Carboxyalkyl" means an alkyl group (as defined herein) substituted with a carboxy group. The term "carboxycycloalkyl" means a cycloalkyl group (as defined herein) substituted with a carboxy group. The term alkoxyalkyl means an alkyl group (as defined herein) substituted with an alkoxy group. The term "carboxy" employed herein refers to carboxylic acids and their esters.

"Haloalkyl" refers to alkyl as defined above where some or all of the hydrogen atoms are substituted with halogen atoms. Halogen (halo) represents chloro or fluoro, but may also be bromo or iodo. For example, haloalkyl includes trifluoromethyl, fluoromethyl, 1,2,3,4,5-pentafluoro-phenyl, etc. The term "perfluoro" defines a compound or radical which has all available hydrogens that are replaced with fluorine. For example, perfluorophenyl refers to 1,2,3,4,5-pentafluorophenyl, perfluoromethyl refers to 1,1,1-trifluoromethyl, and perfluoromethoxy refers to 1,1,1-trifluoromethoxy.

"Fluoro-substituted alkyl" refers to an alkyl group where one, some, or all hydrogen atoms have been replaced by fluorine.

"Cytokine" is a member of a group of protein signaling molecules that may participate in cell-cell communication in immune and inflammatory responses. Cytokines are typically small, water-soluble glycoproteins that have a mass of about 8-35 kDa.

"Cycloalkyl" refers to a cyclic hydrocarbon group that contains from about 3 to 12, from 3 to 10, or from 3 to 7 endocyclic carbon atoms. Cycloalkyl groups include fused, bridged and spiro ring structures.

"Endocyclic" refers to an atom or group of atoms which comprise part of a cyclic ring structure.

"Exocyclic" refers to an atom or group of atoms which are attached but do not define the cyclic ring structure.

"Cyclic alkyl ether" refers to a 4 or 5 member cyclic alkyl group having 3 or 4 endocyclic carbon atoms and 1 endocyclic oxygen or sulfur atom (*e.g.,* oxetane, thietane, tetrahydrofuran, tetrahydrothiophene); or a 6 to 7 member cyclic alkyl group having 1 or 2 endocyclic oxygen or sulfur atoms (*e.g.,* tetrahydropyran, 1,3-dioxane, 1,4-dioxane, tetrahydrothiopyran, 1,3-dithiane, 1,4-dithiane, 1,4-oxathiane).

"Alkenyl" refers to either a straight chain or branched hydrocarbon of 2 to 6 carbon atoms, having at least one double bond. Examples of alkenyl groups include, but are not limited to, vinyl, propenyl, isopropenyl, 1-butenyl, 2-butenyl, isobutenyl, butadienyl, 1-pentenyl, 2-pentenyl, isopentenyl, 1,3-pentadienyl, 1,4-pentadienyl, 1-hexenyl, 2-hexenyl, 3-hexenyl, 1,3-hexadienyl, 1,4-hexadienyl, 1,5-hexadienyl, 2,4-hexadienyl, or 1,3,5-hexatrienyl. Alkenyl groups can also have from 2 to 3, 2 to 4, 2 to 5, 3 to 4, 3 to 5, 3 to 6, 4 to 5, 4 to 6 and 5 to 6 carbons. The alkenyl group is typically monovalent,

but can be divalent, such as when the alkenyl group links two moieties together.

"Alkenylene" refers to an alkenyl group, as defined above, linking at least two other groups, *i.e.,* a divalent hydrocarbon radical. The two moieties linked to the alkenylene can be linked to the same atom or different atoms of the alkenylene. Alkenylene groups include, but are not limited to, ethenylene, propenylene, isopropenylene, butenylene, isobutenylene, sec-butenylene, pentenylene and hexenylene.

"Alkynyl" refers to either a straight chain or branched hydrocarbon of 2 to 6 carbon atoms, having at least one triple bond. Examples of alkynyl groups include, but are not limited to, acetylenyl, propynyl, 1-butynyl, 2-butynyl, isobutynyl, sec-butynyl, butadiynyl, 1-pentynyl, 2-pentynyl, isopentynyl, 1,3-pentadiynyl, 1,4-pentadiynyl, 1-hexynyl, 2-hexynyl, 3-hexynyl, 1,3-hexadiynyl, 1,4-hexadiynyl, 1,5-hexadiynyl, 2,4-hexadiynyl, or 1,3,5-hexatriynyl. Alkynyl groups can also have from 2 to 3, 2 to 4, 2 to 5, 3 to 4, 3 to 5, 3 to 6, 4 to 5, 4 to 6 and 5 to 6 carbons. The alkynyl group is typically monovalent, but can be divalent, such as when the alkynyl group links two moieties together.

"Alkynylene" refers to an alkynyl group, as defined above, linking at least two other groups, *i.e.,* a divalent hydrocarbon radical. The two moieties linked to the alkynylene can be linked to the same atom or different atoms of the alkynylene. Alkynylene groups include, but are not limited to, ethynylene, propynylene, butynylene, sec-butynylene, pentynylene and hexynylene.

"Cycloalkyl" refers to a saturated or partially unsaturated, monocyclic, fused bicyclic or bridged polycyclic ring assembly containing from 3 to 12 ring atoms, or the number of atoms indicated. Monocyclic rings include, for example, cyclopropyl, cyclobutyl, cyclopentyl, cyclohexyl, and cyclooctyl. Bicyclic and polycyclic rings include, for example, norbornane, decahydronaphthalene and adamantane. For example, $C_{3-8}$cycloalkyl includes cyclopropyl, cyclobutyl, cyclopentyl, cyclohexyl, cyclooctyl, and norbornane.

"Cycloalkylene" refers to a cycloalkyl group, as defined above, linking at least two other groups, *i.e.,* a divalent hydrocarbon radical. The two moieties linked to the cycloalkylene can be linked to the same atom or different atoms of the cycloalkylene. Cycloalkylene groups include, but are not limited to, cyclopropylene, cyclobutylene, cyclopentylene, cyclohexylene, and cyclooctylene.

"Heterocycloalkyl" refers to a ring system having from 3 ring members to about 20 ring members and from 1 to about 5 heteroatoms such as N, O and S. Additional heteroatoms can also be useful, including, but not limited to, B, Al, Si and P. The heteroatoms can also be oxidized, such as, but not limited to, -S(O)- and $-S(O)_2-$. For example, heterocycle includes, but is not limited to, tetrahydrofuranyl, tetrahydrothiophenyl, morpholino, pyrrolidinyl, pyrrolinyl, imidazolidinyl, imidazolinyl, pyrazolidinyl, pyrazolinyl, piperazinyl, piperidinyl, indolinyl, quinuclidinyl and 1,4-dioxa-8-aza-spiro[4.5]dec-8-yl.

"Heterocycloalkylene" refers to a heterocyclalkyl group, as defined above, linking at least two other groups. The two moieties linked to the heterocycloalkylene can be linked to the same atom or different atoms of the heterocycloalkylene.

"Aryl" refers to a monocyclic or fused bicyclic, tricyclic or greater, aromatic ring assembly containing 6 to 16 ring carbon atoms. For example, aryl may be phenyl, benzyl or naphthyl. "Arylene" means a divalent radical derived from an aryl group. Aryl groups can be mono-, di- or tri-substituted by one, two or three radicals selected from alkyl, alkoxy, aryl, hydroxy, halogen, cyano, amino, amino-alkyl, trifluoromethyl, alkylenedioxy and oxy-$C_2$-$C_3$-alkylene; all of which are optionally further substituted, for instance as hereinbefore defined; or 1- or 2-naphthyl; or 1- or 2-phenanthrenyl. Alkylenedioxy is a divalent substitute attached to two adjacent carbon atoms of phenyl, e.g. methylenedioxy or ethylenedioxy. Oxy-$C_2$-$C_3$-alkylene is also a divalent substituent attached to two adjacent carbon atoms of phenyl, e.g. oxyethylene or oxypropylene. An example for oxy- $C_2$-$C_3$-alkylene-phenyl is 2,3-dihydrobenzofuran-5-yl.

In some embodiments the aryl is naphthyl, phenyl or phenyl mono- or disubstituted by alkoxy, phenyl, halogen, alkyl or trifluoromethyl, especially phenyl or phenyl-mono- or disubstituted by alkoxy, halogen or trifluoromethyl, and in particular phenyl.

Examples of substituted phenyl groups as R are, *e.g.* 4-chlorophen-1-yl, 3,4-dichlorophen-1-yl, 4-methoxyphen-1-yl, 4-methylphen-1-yl, 4-aminomethylphen-1-yl, 4-methoxyethylaminomethylphen-1-yl, 4-hydroxyethylaminomethylphen-1-yl, 4-hydroxyethyl-(methyl)-aminomethylphen-1-yl, 3-aminomethylphen-1-yl, 4-N-acetylaminomethylphen-1-yl, 4-aminophen-1-yl, 3-aminophen-1-yl, 2-aminophen-1-yl, 4-phenyl-phen-1-yl, 4-(imidazol-1-yl)-phenyl, 4-(imidazol-1-ylmethyl)-phen-1-yl, 4-(morpholin-1-yl)-phen-1-yl, 4-(morpholin-1-ylmethyl)-phen-1-yl, 4-(2-methoxyethylaminomethyl)-phen-1-yl and 4-(pyrrolidin-1-ylmethyl)-phen-1-yl, 4-(thiophenyl)-phen-1-yl, 4-(3-thiophenyl)-phen-1-yl, 4-(4-methylpiperazin-1-yl)-phen-1-yl, and 4-(piperidinyl)-phenyl and 4-(pyridinyl)-phenyl optionally substituted in the heterocyclic ring.

"Arylene" refers to an aryl group, as defined above, linking at least two other groups. The two moieties linked to the arylene are linked to different atoms of the arylene. Arylene groups include, but are not limited to, phenylene.

"Arylene-oxy" refers to an arylene group, as defined above, where one of the moieties linked to the arylene is linked through an oxygen atom. Arylene-oxy groups include, but are not limited to, phenylene-oxy.

Similarly, substituents for the aryl and heteroaryl groups are varied and are selected from: -halogen, -OR', -OC(O)R', -NR'R", -SR', -R', -CN, $-NO_2$, $-CO_2R'$, -CONR'R", -C(O)R', -OC(O)NR'R", -NR"C(O)R',

-NR"C(O)$_2$R', ,-NR'-C(O)NR"R"', -NH-C(NH$_2$)=NH, -NR'C(NH$_2$)=NH, -NH-C(NH$_2$)=NR', -S(O)R', -S(O)$_2$R ', -S(O)$_2$NR'R", -N$_3$, -CH(Ph)$_2$, perfluoro(C$_1$-C$_4$)alkoxy, and perfluoro(C$_1$-C$_4$)alkyl, in a number ranging from zero to the total number of open valences on the aromatic ring system; and where R', R" and R"' are independently selected from hydrogen, (C$_1$-C$_8$)alkyl and heteroalkyl, unsubstituted aryl and heteroaryl, (unsubstituted aryl)-(C$_1$-C$_4$)alkyl, and (unsubstituted aryl)oxy-(C$_1$-C$_4$)alkyl.

Two of the substituents on adjacent atoms of the aryl or heteroaryl ring may optionally be replaced with a substituent of the formula -T-C(O)-(CH$_2$)$_q$-U-, wherein T and U are independently -NH-, -O-, -CH$_2$- or a single bond, and q is an integer of from 0 to 2. Alternatively, two of the substituents on adjacent atoms of the aryl or heteroaryl ring may optionally be replaced with a substituent of the formula -A-(CH$_2$)$_r$-B-, wherein A and B are independently -CH$_2$-, -O-, -NH-, -S-, -S(O)-, -S(O)$_2$-, -S(O)$_2$NR'- or a single bond, and r is an integer of from 1 to 3. One of the single bonds of the new ring so formed may optionally be replaced with a double bond. Alternatively, two of the substituents on adjacent atoms of the aryl or heteroaryl ring may optionally be replaced with a substituent of the formula -(CH$_2$)$_s$-X-(CH$_2$)$_t$-, where s and t are independently integers of from 0 to 3, and X is -O-, -NR'-, -S-, -S(O)-, -S(O)$_2$-, or -S(O)$_2$NR'-. The substituent R' in -NR'- and -S(O)$_2$NR'- is selected from hydrogen or unsubstituted (C$_1$-C$_6$)alkyl.

"Heteroaryl" refers to a monocyclic or fused bicyclic or tricyclic aromatic ring assembly containing 5 to 16 ring atoms, where from 1 to 4 of the ring atoms are a heteroatom each N, O or S. For example, heteroaryl includes pyridyl, indolyl, indazolyl, quinoxalinyl, quinolinyl, isoquinolinyl, benzothienyl, benzofuranyl, furanyl, pyrrolyl, thiazolyl, benzothiazolyl, oxazolyl, isoxazolyl, triazolyl, tetrazolyl, pyrazolyl, imidazolyl, thienyl, or any other radicals substituted, especially mono- or di-substituted, by e.g. alkyl, nitro or halogen. Pyridyl represents 2-, 3- or 4-pyridyl, advantageously 2- or 3-pyridyl. Thienyl represents 2- or 3-thienyl. In some embodiments, quinolinyl represents 2-, 3- or 4-quinolinyl. In some embodiments, isoquinolinyl represents 1-, 3- or 4-isoquinolinyl. In some embodiments, benzopyranyl, benzothiopyranyl can represent 3-benzopyranyl or 3-benzothiopyranyl, respectively. In some embodiments, thiazolyl can represent 2- or 4-thiazolyl. In some embodiments, triazolyl can be 1-, 2- or 5-(1,2,4-triazolyl). In some embodiments, tetrazolyl can be 5-tetrazolyl.

In some embodiments, heteroaryl is pyridyl, indolyl, quinolinyl, pyrrolyl, thiazolyl, isoxazolyl, triazolyl, tetrazolyl, pyrazolyl, imidazolyl, thienyl, furanyl, benzothiazolyl, benzofuranyl, isoquinolinyl, benzothienyl, oxazolyl, indazolyl, or any of the radicals substituted, especially mono- or di-substituted.

The term "heteroalkyl" refers to an alkyl group having from 1 to 3 heteroatoms such as N, O and S. Additional heteroatoms can also be useful, including, but not limited to, B, Al, Si and P. The heteroatoms can also be oxidized, such as, but not limited to, -S(O)- and -S(O)$_2$-. For example, heteroalkyl can include ethers, thioethers, alkyl-amines and alkylthiols.

The term "heteroalkylene" refers to a heteroalkyl group, as defined above, linking at least two other groups. The two moieties linked to the heteroalkylene can be linked to the same atom or different atoms of the heteroalkylene.

"Electrophile" refers to an ion or atom or collection of atoms, which may be ionic, having an electrophilic center, *i.e.,* a center that is electron seeking, capable of reacting with a nucleophile. An electrophile (or electrophilic reagent) is a reagent that forms a bond to its reaction partner (the nucleophile) by accepting both bonding electrons from that reaction partner.

"Nucleophile" refers to an ion or atom or collection of atoms, which may be ionic, having a nucleophilic center, *i.e.,* a center that is seeking an electrophilic center or capable of reacting with an electrophile. A nucleophile (or nucleophilic reagent) is a reagent that forms a bond to its reaction partner (the electrophile) by donating both bonding electrons. A "nucleophilic group" refers to a nucleophile after it has reacted with a reactive group. Non limiting examples include amino, hydroxyl, alkoxy, haloalkoxy and the like.

"Maleimido" refers to a pyrrole-2,5-dione-1-yl group having the structure:

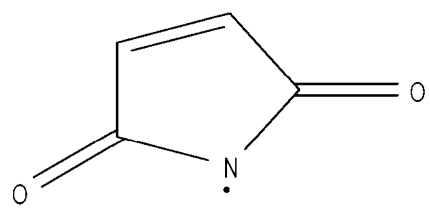

, which upon reaction with a sulfhydryl (e.g., a thio alkyl) forms an -S-maleimido group having the structure

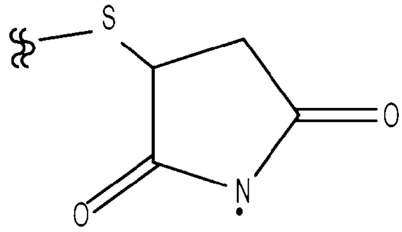

, where "•" indicates the point of attachment for the maleimido group and "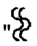 "indicates the point of attachment of the sulfur atom the thiol to the remainder of the original sulfhydryl bearing group.

For the purpose of this disclosure, "naturally occurring amino acids" found in proteins and polypeptides are L-alanine, L-arginine, L-asparagine, L-aspartic acid, L-cysteine, L-glutamine, L-glutamic acid, L-glycine, L-histidine, L-isoleucine, L-leucine, L-lysine, L-methionine, L-phenylalanine, L-proline, L-serine, L-threonine, L-tryptophan, L-tyrosine, and or L-valine. "Non-naturally occurring amino acids" found in proteins are any amino acid other than those recited as naturally occurring amino acids. Non-naturally occurring amino acids include, without limitation, the D isomers of the naturally occurring amino acids, and mixtures of D and L isomers of the naturally occurring amino acids. Other amino acids, such as N-alpha- methyl amino acids (e.g. sarcosine), 4-hydroxyproline, desmosine, isodesmosine, 5-hydroxylysine, epsilon-N-methyllysine, 3-methylhistidine, although found in naturally occurring proteins, are considered to be non-naturally occurring amino acids found in proteins for the purpose of this disclosure as they are generally introduced by means other than ribosomal translation of mRNA.

"Linear" in reference to the geometry, architecture or overall structure of a polymer, refers to polymer having a single polymer arm.

"Branched," in reference to the geometry, architecture or overall structure of a polymer, refers to a polymer having 2 or more polymer "arms" extending from a core structure contained within an initiator. The initiator may be employed in an atom transfer radical polymerization (ATRP) reaction. A branched polymer may possess 2 polymer chains (arms), 3 polymer arms, 4 polymer arms, 5 polymer arms, 6 polymer arms, 7 polymer arms, 8 polymer arms, 9 polymer arms or more. Each polymer arm extends from a polymer initiation site. Each polymer initiation site is capable of being a site for the growth of a polymer chain by the addition of monomers. For example and not by way of limitation, using ATRP, the site of polymer initiation on an initiator is typically an organic halide undergoing a reversible redox process catalyzed by a transition metal compound such as cuprous halide. In some embodiments, the halide is a bromine.

"Pharmaceutically acceptable excipient" refers to an excipient that can be included in compositions and that causes no significant adverse toxicological effect on the patient and is approved or approvable by the FDA for therapeutic use, particularly in humans. Non-limiting examples of pharmaceutically acceptable excipients include water, NaCl, normal saline solutions, lactated Ringer's, normal sucrose, normal glucose and the like.

Therapeutic proteins are administered in an effective regime meaning a dosage, route of administration and frequency of administration that delays the onset, reduces the severity, inhibits further deterioration, and/or ameliorates at least one sign or symptom of a disorder. If a patient is already suffering from a disorder, the regime can be referred to as a therapeutically effective regime. If the patient is at elevated risk of the disorder relative to the general population but is not yet experiencing symptoms, the regime can be referred to as a prophylactically effective regime. In some instances, therapeutic or prophylactic efficacy can be observed in an individual patient relative to historical controls or past experience in the same patient. In other instances, therapeutic or prophylactic efficacy can be demonstrated in a preclinical or clinical trial in a population of treated patients relative to a control population of untreated patients.

The "biological half-life" of a substance is a pharmacokinetic parameter which specifies the time required for one half of the substance to be removed from a tissue or an organism following introduction of the substance.

"OG1786" is a 9-arm initiator used for polymer synthesis, which depicts that salt form of OG1786 with trifluororacetic acid. OG1786 may be used as other salts are used or as the free base.

"OG1801" is an approximately (+/- 15%) 750 kDa polymer (either by Mn or Mp) made using OG1786 as an initiator for ATRP synthesis using the monomer HEMA-PC.

"OG1802" is OG1801 with a maleimide functionality added wherein each of $n_1$, $n_2$, $n_3$, $n_4$, $n_5$, $n_6$, $n_7$, $n_8$ and $n_9$ is an integer (positive) (from 0 up to about 3000) such that the total molecular weight of the polymer is (Mw) 750,000 $\pm$ 15% Daltons.

"BCVA" denotes Best Corrected Visual Acuity.

"OCT-A" denotes OCT-Angiography.

"SD-OCT" denotes Spectral Domain Optical Coherence Tomography.

Multi-angle light scattering (**MALS**) is a technique of analyzing macromolecules where the laser light impinges on the molecule, the oscillating electric field of the light induces an oscillating dipole within it. This oscillating dipole will re-radiate light and can be measured using a MALS detector such as Wyatt miniDawn TREOS. The intensity of the radiated

light depends on the magnitude of the dipole induced in the macromolecule which in turn is proportional to the polarizability of the macromolecule, the larger the induced dipole, and hence, the greater the intensity of the scattered light. Therefore, in order to analyze the scattering from a solution of such macromolecules, one should know their polarizability relative to the surrounding medium (e.g., the solvent). This may be determined from a measurement of the change, $\Delta n$, of the solution's refractive index $n$ with the molecular concentration change, $\Delta c$, by measuring the ***dn/dc*** (=$\Delta n/\Delta c$) value using a Wyatt Optilab T-rEX differential refractometer. Two molar weight parameters that MALS determination employ are number average molecular weight (Mn) and weight average molecular weight (Mw) where the polydispersity index (PDI) equals Mw divided by Mn. SEC also allows another average molecular weight determination of the peak molecular weight Mp which is defined as the molecular weight of the highest peak at the SEC.

The PDI is used as a measure of the broadness of a molecular weight distribution of a polymer and bioconjugate which is derived from conjugation of a discrete protein (e.g. OG1950) to a polydisperse biopolymer (e.g., OG1802). For a protein sample, its polydispersity is close to 1.0 due to the fact that it is a product of translation where every protein molecule in a solution is expected to have almost the same length and molar mass. In contrast, due to the polydisperse nature of the biopolymer where the various length of polymer chains are synthesized during the polymerization process, it is very important to determine the PDI of the sample as one of its quality attribute for narrow distribution of molecular weight.

Size exclusion chromatography (**SEC**) is a chromatography technique in which molecules in solution are separated by their size. Typically an aqueous solution is applied to transport the sample through the column which is packed with resins of various pore sizes. The resin is expected to be inert to the analyte when passing through the column and the analytes separate from each other based on their unique size and the pore size characteristics of the selected column.

Coupling the **SEC** with **MALS** or **SEC/MALS** provides accurate distribution of molar mass and size (root mean square radius) as opposed to relying on a set of SEC calibration standards. This type of arrangement has many advantages over traditional column calibration methods. Since the light scattering and concentration are measured for each eluting fraction, the molar mass and size can be determined independently of the elution position. This is particularly relevant for species with non-globular shaped macromolecules such as the biopolymers (OG1802) or bioconjugates (e.g., KSI-301); such species typically do not elute in a manner that might be described by a set of column calibration standards.

In some embodiments, a SEC/MALS analysis includes a Waters HPLC system with Alliance 2695 solvent delivery module and Waters 2996 Photodiole Array Detector equipped with a Shodex SEC-HPLC column (7.8x300mm). This is connected online with a Wyatt miniDawn TREOS and Wyatt Optilab T-rEX differential refractometer. The Empower software from Waters can be used to control the Waters HPLC system and the ASTRA V 6.1.7.16 software from Wyatt can be used to acquire the MALS data from the Wyatt miniDawn TREOS, dn/dc data from the T-rEX detector and the mass recovery data using the A280 absorbance signal from the Waters 2996 Photodiole Array detector. SEC can be carried out at 1ml/min in 1xPBS pH 7.4, upon sample injection, the MALS and RI signals can be analyzed by the ASTRA software for determination of absolute molar mass (Mp, **Mw**, **Mn**) and polydisperse index (**PDI**). In addition, the calculation also involves the input dn/dc values for polymer and protein as 0.142 and 0.183, respectively. For KSI-301 dn/dc value, the dn/dc is calculated based on the weighted MW of the polymer and the protein to be about 0.148 using the formula below:

$$\text{Conjugate } \mathbf{dn/dc} = 0.142 \text{ x } [\ \text{MW}\mathit{polymer}\ /(\text{MW}\mathit{polymer}+\text{MW}\mathit{protein})] + 0.183 \text{ x } [\text{MW}\mathit{protein}\ /(\text{MW}\mathit{polymer}+\text{MW}\mathit{protein})]$$

where MW*polymer* for OG1802 is 800 kDa and the MW*protein* for OG1950 is 146 kDa.

"KSI-301" is a bioconjugate of a recombinant, mammalian cell expressed full-length humanized anti-VEGF monoclonal antibody which is covalently conjugated to a branched high molecular weight phosphorylcholine based biopolymer. In some embodiments, KSI-301 is supplied as a preservative free, sterile, aqueous solution in a single-use glass vial at a concentration of 50 mg/mL (based on antibody mass). FIG. 14 displays the amino acid sequence of the antibody portion of KSI-301. KSI-301 is an anti-vascular endothelial growth factor (VEGF) biopharmaceutical with an extended ocular half-life. KSI-301 is a bioconjugate of two intermediates: (1) OG1950 antibody intermediate, a recombinant, full-length humanized, anti-huVEGF A monoclonal antibody, and (2) OG1802 biopolymer intermediate, a phosphorylcholine biopolymer. The addition of OG1802, an inert biopolymer, increases the size of the biologic, thereby extending the ocular pharmacokinetics (PK) of KSI-301 beyond that of currently approved anti-huVEGF-A therapeutics. Nonclinical studies with KSI-301 indicate that it appropriately binds with high affinity to huVEGF-A whose binding to huVEGF Receptors 1 and 2 (huVEGFR) is then inhibited. This in turn abrogates huVEGF-A mediated function.

Pharmacokinetic studies have been conducted in rabbit which demonstrate that KSI-301 has extended ocular half-life, penetrates ocular tissues well and is distributed to the retina and choroid. In rabbit, KSI-301 has an ocular half-life of approximately 11 days, which is significantly longer than the reported rabbit half-life measured for aflibercept and ranibizumab, which are 4 to 5 and 3 to 4 days, respectively (CovanceStudy 8376321, Park 2016). A series of non-clinical

GLP repeat dose (4-week dosing intervals) toxicology studies in cynomolgus monkeys testing the ocular and systemic safety of KSI-301 have been conducted through 26 weeks (7 intravitreal doses) and 10 weeks (3 intravenous doses), respectively. Results show that KSI-301 was well tolerated up to the maximum dose tested of 5 mg/eye (intravitreal) and 5 mg/kg (intravenous) in the ocular and systemic studies, respectively. Together, data extrapolated from non-clinical PK and toxicology studies indicate that KSI-301 can be safely and effectively dosed in human subjects. In some embodiments, the route of administration is via an intravitreal injection. In some embodiments, an anti-VEGF antibody conjugate (e.g., KSI-301) can be administered every 3 - 4 months, after a loading dose completion, or even less frequently.

In some embodiments, the molecule to be administered in any one or more of the methods provided herein is any one of the molecules disclosed in U.S. Pat. Pub. No. 2017/0190766, herein incorporated by reference in its entirety.

FIG. 64 displays the amino acid sequence of the antibody portion of KSI-301 with a terminal lysine removed. Terminal lysine removal is a post-translational modification in antibodies. The lysine residues at the heavy chain C-terminus of recombinant IgGs are removed (often to a large extent) during cell culture by carboxypeptidases that are endogenous to CHO host cells.. For all antibody sequences recited herein, although specific sequences (which may be longer) are provided for exemplary antibodies disclosed herein, it will be appreciated that after expression of protein chains, one to several amino acids at the amino or carboxy terminus of the light and/or heavy chain, particularly a heavy chain C-terminal lysine residue, may be missing or derivatized in a proportion or all of the molecules. Thus, in some embodiments, any of the antibody sequences provided herein may be modified by this lysine clip, which can include the modified version as shown in FIG. 64 (with one or more of the underlined residues being removed). In some embodiments, the lysine in the heavy chain of FIG. 64 is removed. In some embodiments, the GK is removed. In some embodiments, the PGK is removed. In some embodiments, the SPGK is removed. As will be appreciated by those in the art, across a population of molecules, the lysine clip may vary and not be complete. Thus, for example, 90, 95, 98, 99, or 100% of the molecules may have one or more of the clip versions, while 10, 5, 2, 1, or down to 0% may be full length (including any range defined between any two of the preceding values). Thus, as used herein, KSI-301 includes any one of and all options for the heavy chain variations outlined in FIG. 64. Any reference to the antibody portion of KSI-301 or an amino acid sequence of an anti-VEGF antibody heavy chain is intended to encompass, but not limited to, a polypeptide expressed recombinantly from a nucleic acid construct encoding the heavy chain amino acid sequence without any of the post-translational modifications noted above (e.g., encoding the amino acid sequence of SEQ ID NO:1).

As used herein, any time "anti-VEGF antibody" or "anti-VEGF antibody conjugate" is referenced, an anti-VEGF protein, such as an anti-VEGF fusion protein, e.g., aflibercept, is also contemplated. Thus, as disclosed herein, any time "anti-VEGF antibody conjugate" is referenced, an anti-VEGF protein, e.g., aflibercept, covalently bonded to a phosphorylcholine containing biopolymer (e.g., OG1802) as disclosed herein, is also contemplated. In the various embodiments disclosed herein, any reference to an anti-VEGF antibody conjugate therapy, also contemplates an anti-VEGF protein, e.g., aflibercept, conjugate therapy. In the various embodiments of methods of treating an eye disorder, disclosed herein, any reference to an anti-VEGF antibody conjugate, also contemplates an aflibercept biopolymer conjugate.

Methods of Treatment

In some embodiments, a method of treating diabetic retinopathy is provided. The method comprises identifying a subject with diabetic retinopathy, administering a first dose of an anti-VEGF antibody polymer conjugate (e.g., KSI-301) (or anti-VEGF protein conjugate (e.g., an aflibercept biopolymer conjugate)) to the subject, administering a second dose of the anti-VEGF antibody polymer conjugate to the subject 8 weeks after the first dose, administering a third dose of the anti-VEGF antibody polymer conjugate to the subject 12 weeks after the second dose. The method can further include administering a subsequent dose of the anti-VEGF antibody polymer conjugate to the subject no more frequently than once every 24 weeks after the third dose. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is +1, 2, 3, 4, 5, 6, 7, 8, 9, 10, 11, 12, 13, or 14 days.

In some embodiments, a method includes administering a first dose of an anti-VEGF antibody conjugate (e.g., KSI-301) (or anti-VEGF protein conjugate (e.g., an aflibercept biopolymer conjugate)) to a subject (e.g., human or other mammalian patient) in need of treating DR, and subsequently administering at least one more, but no more than three more of the doses to achieve a therapeutic result (e.g., improved vision, reduced symptoms, etc.) of the anti-VEGF therapy. In some embodiments, subsequent doses of the anti-VEGF antibody conjugate or anti-VEGF protein conjugate are administered no more frequently than 24 weeks after the final dose was administered. In some embodiments, subsequent doses of the anti-VEGF antibody conjugate or anti-VEGF protein conjugate are administered no more frequently than: 24 weeks, at least 28 weeks, at least 32 weeks, at least 36 weeks, at least 40 weeks, at least 44 weeks, at least 48 weeks, including at least 52 weeks after the final dose was administered. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no

sooner than the designated point in time. In some embodiments, the dosing is ±1, 2, 3, 4, 5, 6, 7, 8, 9, 10, 11, 12, 13, or 14 days.

In some embodiments, a method of treating diabetic retinopathy is provided. The method comprises identifying a subject with diabetic retinopathy; administering a first dose of an anti-VEGF antibody polymer conjugate (e.g., KSI-301 or an aflibercept biopolymer conjugate), to the subject, administering a second dose of the anti-VEGF antibody polymer conjugate to the subject 8 weeks after the first dose, administering a third dose of the anti-VEGF antibody polymer conjugate to the subject 12 weeks after the second dose, and administering one or more subsequent doses of the anti-VEGF antibody polymer conjugate to the subject either once every 12 weeks after the third dose or once every 24 weeks after the third dose. The 12 or 24 weeks can depend upon the effectiveness of the therapy in the subject. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, the eye disorder is DR and the method includes administering a first dose of an anti-VEGF antibody conjugate (e.g., KSI-301), or anti-VEGF protein conjugate (e.g., an aflibercept biopolymer conjugate), to a subject (e.g., human patient) in need of treating DR, and subsequently administering a second dose and a third dose to achieve a therapeutic result (e.g., improved vision, reduced symptoms, etc.) of the anti-VEGF therapy, where the second loading dose is administered 12 weeks after administration of the first loading dose, and the third loading dose is administered 24 weeks after the second loading dose. In some embodiments, the second loading dose is administered no more than 4 weeks, 8 weeks, or 12 weeks after the first loading dose. In some embodiments, the third loading dose is administered no more than 4 weeks, 8 weeks, or 12 weeks after the first loading dose. In some embodiments, a subsequent dose (e.g., maintenance dose) of the anti-VEGF antibody conjugate after the final loading dose is administered. In some embodiments, the subsequent doses may be administered to the subject no more frequently than once every 8 weeks, e.g., every 10 weeks, every 12 weeks, every 14 weeks, every 16 weeks, every 18 weeks, every 20 weeks, every 24 weeks, every 28 weeks, every 32 weeks, every 36 weeks, every 40 weeks, every 44 weeks, every 48 weeks, including every 52 weeks. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of treating diabetic retinopathy is provided. The method comprises identifying a subject with diabetic retinopathy, administering a first dose of an anti-VEGF antibody polymer conjugate (e.g., KSI-301or an aflibercept biopolymer conjugate) to the subject, administering a second dose of the anti-VEGF antibody polymer conjugate to the subject 4 weeks after the first dose, and administering one or more subsequent doses to the subject no more frequently than once every 12 weeks, unless, an eye of the subject has a decline in eye health, or develops macular edema at week 8 from the second loading dose, then administering the anti-VEGF antibody polymer conjugate once every 8 weeks. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days. In some embodiments, the decline in eye health is that the eye's diabetic retinopathy severity is stable or worsening as determined by physical examination and imaging of the eye. In some embodiments, the decline in eye health is that the eye's diabetic retinopathy severity is worsening as determined by physical examination and imaging of the eye.

In some embodiments, the eye disorder is DR and the method includes administering a first dose of an anti-VEGF antibody conjugate (e.g., KSI-301), or anti-VEGF protein conjugate (e.g., an aflibercept biopolymer conjugate), to a subject (e.g., human patient) and their eye ("treated eye") in need of treating DR, and subsequently administering a second dose to achieve a therapeutic result (e.g., improved vision, reduced symptoms, etc.) of the anti-VEGF therapy. In some embodiments the second dose is administered at least 4 weeks after the administration of the first dose. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is +1, 2, 3, 4, 5, 6, 7, 8, 9, 10, 11, 12, 13, or 14 days.

In some embodiments, the subject undergoes an ophthalmic examination no sooner than 4 weeks (e.g., every 10 weeks, every 12 weeks, every 14 weeks, every 16 weeks, every 18 weeks, every 20 weeks, every 24 weeks, every 28 weeks, every 32 weeks, every 36 weeks, every 40 weeks, every 44 weeks, every 48 weeks, including every 52 weeks) after the last loading dose was administered. In some embodiments, the subject may be administered a subsequent dose. In some embodiments, the subsequent dose may be administered no more frequently than once every 12 weeks (e.g., every 16 weeks, every 20 weeks, every 24 weeks, every 28 weeks, every 32 weeks, every 36 weeks, every 40 weeks, including every 52 weeks after the final loading dose) if the subject's treated eye is determined to be dry. In some embodiments the

subsequent dose may be administered no more frequently than once every 8 weeks e.g., every 12 weeks, every 16 weeks, every 20 weeks, every 24 weeks, every 28 weeks, every 32 weeks, every 36 weeks, every 40 weeks, including every 52 weeks after the final loading dose if the subject's treated eye is determined to be wet, or exudative. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of treating diabetic retinopathy is provided. The method comprises identifying a subject with diabetic retinopathy, administering a first dose of an anti-VEGF antibody polymer conjugate (e.g., KSI-301) to the subject, administering a second dose of the anti-VEGF antibody polymer conjugate to the subject 4 weeks after the first dose, if the subject has a decline in eye health 8 weeks after the second dose, administering at least one subsequent dose of the anti-VEGF antibody polymer conjugate at a frequency of at least once every 8 weeks, and if the subject maintains eye health or it improves, then administering at least one subsequent dose of the anti-VEGF antibody polymer conjugate at a frequency of no more than once every 12 weeks. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, provided herein are methods of treating a subject with an eye disorder, where that eye disorder is DR, where the method includes administering a first dose of an anti-VEGF antibody conjugate (e.g., KSI-301), or anti-VEGF protein conjugate (e.g., an aflibercept biopolymer conjugate), to a subject (e.g., human patient) and their eye ("treated eye") in need of treating DR, and subsequently administering a second dose to achieve a therapeutic result (e.g., improved vision, reduced symptoms, etc.) of the anti-VEGF therapy. In some embodiments the second dose is administered at least 4 weeks after the administration of the first dose. In some embodiments, if the subject has a decline in eye health before 4 weeks after the second dose(e.g., before week 8), then subject is administered subsequent doses of the anti-VEGF antibody polymer conjugate at least once every 8 weeks, every 12 weeks, every 16 weeks, every 20 weeks, every 24 weeks, every 28 weeks, every 32 weeks, every 36 weeks, every 40 weeks, including every 52 weeks after the final dose noted above. In some embodiments, if the subject has no decline in eye health, then subject is administered subsequent doses of the anti-VEGF antibody polymer conjugate at least once every 12 weeks, every 16 weeks, every 20 weeks, every 24 weeks, every 28 weeks, every 32 weeks, every 36 weeks, every 40 weeks, including every 52 weeks after the final loading dose. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of treating diabetic retinopathy is provided. The method comprises: identifying a subject with diabetic retinopathy, administering a first dose of an anti-VEGF antibody polymer conjugate (e.g., KSI-301) to the subject, administering a second dose of the anti-VEGF antibody polymer conjugate to the subject 4 weeks after the first dose, administering one or more subsequent doses to the subject no more frequently than once every 12 weeks or once every 8 weeks. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, provided herein are methods of treating a subject with an eye disorder, where that eye disorder is DR, where the method includes administering a first dose of an anti-VEGF antibody conjugate (e.g., KSI-301), or anti-VEGF protein conjugate (e.g., an aflibercept biopolymer conjugate), to a subject (e.g., human patient) and their eye ("treated eye") in need of treating DR, and subsequently administering a second dose to achieve a therapeutic result (e.g., improved vision, reduced symptoms, etc.) of the anti-VEGF therapy. In some embodiments the second dose is administered at least 4 weeks after the administration of the first dose. In some embodiments, subsequent doses are administered no less than once every 8 weeks e.g., every 12 weeks, every 16 weeks, every 20 weeks, every 24 weeks, every 28 weeks, every 32 weeks, every 36 weeks, every 40 weeks, including every 52 weeks after the final loading dose. In some embodiments, subsequent doses are administered no less than once every 12 weeks e.g., every 16 weeks, every 20 weeks, every 24 weeks, every 28 weeks, every 32 weeks, every 36 weeks, every 40 weeks, including every 52 weeks after the final dose (second dose). In some embodiments, an anti-VEGF protein conjugate (e.g., an aflibercept biopolymer conjugate) can be used in place of the denoted KSI-301 or anti-VEGFVEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, the subject is not retreated with the anti-VEGF antibody conjugate more frequently than once every 12 weeks (e.g., after administering the last loading dose).

In some embodiments, any of the diabetic retinopathy (DR) embodiments (e.g., therapies) provided herein can

instead be applied to the broader genus of diabetic eye disease. Thus, the present disclosure envisions and discloses by this short-hand all of the disclosed DR embodiments for the treatment of diabetic eye diseases generally. Diabetic eye disease includes diabetic retinopathy (proliferative and non-proliferative), as well as DME.

In some embodiments, a method of treating a subject having wAMD is provided. The method comprises identifying a subject having wAMD and receiving a therapy for wAMD, wherein the therapy is not achieving the goal of treatment; wherein the treatment is not KSI-301; and administering KSI-301 to the subject in an amount and at a frequency sufficient to treat the wAMD. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, the eye disorder is wAMD and the method includes administering a dose or doses of the anti-VEGF antibody conjugate, e.g., KSI-301, or anti-VEGF protein conjugate (e.g., an aflibercept biopolymer conjugate), after identifying a subject having wAMD and currently undergoing a therapy for wAMD, for which adequate treatment has not been achieved. In some embodiments, the therapy is not achieving a measurable reduction in disease state of wAMD. In some embodiments, the administration of KSI-301 in a dose or through a treatment regimen is sufficient to treat the wAMD to the previously unsuccessful therapy. In some embodiments, the frequency of administration of KSI-301is any of the frequencies for wAMD as provided herein for KSI-301. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, the subject having wAMD has experienced wAMD as a recurrent condition. In some embodiments, the subject having wAMD is on an approved therapy for wAMD wherein the approved therapy is not KSI-301. In some embodiments, after treatment with KSI-301, the goal of treatment is halting or reversing the loss of vision in the subject. In some embodiments, after treatment with an approved therapy for wAMD, the goal of treatment is halting or reversing the loss of vision in the subject. In some embodiments, sufficient treatment can be defined as a gain in Best Corrected Visual Acuity letters after administration of KSI-301. In some embodiments, BCVA is assessed at least 4 weeks (e.g., at least 8 weeks) after the first administration of KSI-301. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of treating wet AMD is provided. The method comprises identifying a subject with wet AMD, administering a first loading dose of an anti-VEGF antibody polymer conjugate (e.g., KSI-301 to the subject, administering a second loading dose of the anti-VEGF antibody polymer conjugate to the subject 4 weeks after the first loading dose, administering a third loading dose of the anti-VEGF antibody polymer conjugate to the subject 4 weeks after the second loading dose, and evaluating an eye of the subject 12 weeks after the third loading dose, wherein if the eye is dry extending maintenance treatments to once every 16 weeks and wherein if the eye is wet providing a maintenance treatment once every 8 weeks. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, the decision of whether or not to administer or continue to administer the anti-VEGF antibody polymer conjugate is based on low luminance at week 16 in Daylight. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, the eye disorder is wAMD and the method of the present disclosure includes administering a first loading dose of an anti-VEGF antibody conjugate (e.g., KSI-301) (or anti-VEGF protein conjugate (e.g., an aflibercept biopolymer conjugate)) to a subject (e.g., human or other mammalian patient) in need of treating wAMD or DME, and subsequently administering a second dose, and subsequently administering a third dose, where the second loading dose is at least 4 weeks after the first loading dose, and the third loading dose is at least 4 weeks after the second loading dose. In some embodiments, the subject undergoes an ophthalmic examination no sooner than 4 weeks (e.g., every 10 weeks, every 12 weeks, every 14 weeks, every 16 weeks, every 18 weeks, every 20 weeks, every 24 weeks, every 28 weeks, every 32 weeks, every 36 weeks, every 40 weeks, every 44 weeks, every 48 weeks, including every 52 weeks) after the last loading dose was administered. In some embodiments, the subject may be administered a subsequent dose. In some embodiments, the subsequent dose may be administered no more frequently than once every 16 weeks (e.g., every 16 weeks, every 20 weeks, every 24 weeks, every 28 weeks, every 32 weeks, every 36 weeks, every 40 weeks, including every 52 weeks after the final loading dose) if the subject's treated eye is determined to be dry. In some embodiments the subsequent dose may be administered no more frequently than once every 12 weeks e.g., every 12 weeks, every 16 weeks, every 20 weeks, every 24 weeks, every 28 weeks, every 32 weeks, every 36 weeks, every 40 weeks, including every 52 weeks after the final loading dose if the subject's treated eye is determined to be wet, or

exudative. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of treating wet AMD is provided. The method comprises identifying a subject with wet AMD; administering a first loading dose of an anti-VEGF antibody polymer conjugate (e.g., KSI-301), to the subject; administering a second loading dose of the anti-VEGF antibody polymer conjugate to the subject 4 weeks after the first loading dose; administering a third loading dose of the anti-VEGF antibody polymer conjugate to the subject 4 weeks after the second loading dose; and evaluating an eye of the subject using low-luminance visual acuity (LLVA) at 16 weeks after the first loading dose, wherein if the eye has a LLVA to BCVA difference of 33 or more letters , the subject will receive an increased dose frequency (e.g. every 4 to 8 weeks) of maintenance doses and if the eye has a LLVA to BCVA difference of less than 33 letters, the subject will receive a decreased frequency of maintenance doses (e.g. every 12 to 24 weeks). In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, the eye disorder is wAMD and the method of the present disclosure includes administering a first loading dose of an anti-VEGF antibody conjugate (e.g., KSI-301) (or anti-VEGF protein conjugate (e.g., an aflibercept biopolymer conjugate)) to a subject (e.g., human or other mammalian patient) in need of treating wAMD, and subsequently administering a second dose, and subsequently administering a third dose, where the second loading dose is at least 4 weeks after the first loading dose, and the third loading dose is at least 4 weeks after the second loading dose. In some embodiments, the subject undergoes an ophthalmic examination no sooner than 4 weeks (e.g., every 6 weeks, every 8 weeks, every 10 weeks, every 12 weeks, every 14 weeks, every 16 weeks, every 18 weeks, every 20 weeks, every 24 weeks, every 28 weeks, every 32 weeks, every 36 weeks, every 40 weeks, every 44 weeks, every 48 weeks, including every 52 weeks) after the last loading dose was administered. In some embodiments, the subject may be administered a subsequent dose. In some embodiments, the subject may have a treatment schedule wherein administration of subsequent doses of the anti-VEGF antibody polymer conjugate is administered no more than every 4 weeks (e.g., every 8 weeks, every 12 weeks, every 16 weeks, every 20 weeks, every 24 weeks, every 28 weeks, every 32 weeks, every 36 weeks, every 40 weeks, including every 52 weeks after the final loading dose) In some embodiments, the treatment schedule may be adjusted to administer doses more frequently (e.g. original dose frequency every 8 weeks, adjusted dose frequency every 4 weeks) if the subject's treated eye is determined to have a LLVA to BCVA difference of 33 or more letters. In some embodiments, treatment may be more frequent if the treated eye is determined to still have disease activity, such as intraretinal fluid measured on OCT or OCT-A. In some embodiments the treatment schedule may be adjusted to administer doses less frequently (e.g. original dose frequency every 8 weeks, adjusted dose frequency every 12 weeks) after the final loading dose if the subject's treated eye is determined to have a LLVA to BCVA difference of less than 33 letters. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of treating a subject with wet AMD is provided. The method comprises administering 1-3 loading doses of an anti-VEGF antibody polymer conjugate (e.g., KSI-301), to a subject, determining if an amount of fluid in an eye of the subject 4 weeks after a last loading dose is stabilized in the eye. If the subject has stabilized fluid, administering a maintenance dose at a first frequency, and wherein if the subject has an increase in fluid administering a maintenance dose at a second frequency, wherein the second frequency is more frequent than the first frequency. In some embodiments, the fluid stays within 10% or 20% of the best value. In some embodiments, if the fluid has changed by more than 10% or 20%, then a more frequent administration is provided. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, the eye disorder is wAMD and the method of the present disclosure includes administering a first loading dose of an anti-VEGF antibody conjugate (e.g., KSI-301) (or anti-VEGF protein conjugate (e.g., an aflibercept biopolymer conjugate)) to a subject (e.g., human or other mammalian patient) in need of treating wAMD or DME, and subsequently administering a second dose, and subsequently administering a third dose, where the second loading dose is at least 4 weeks after the first loading dose, and the third loading dose is at least 4 weeks after the second loading dose. In some embodiments, the subject undergoes an ophthalmic examination no sooner than 4 weeks (e.g., every 10 weeks, every 12 weeks, every 14 weeks, every 16 weeks, every 18 weeks, every 20 weeks, every 24 weeks, every 28 weeks, every 32 weeks, every 36 weeks, every 40 weeks, every 44 weeks, every 48 weeks, including every 52

weeks) after the last loading dose was administered. In some embodiments, the subject may be administered a subsequent dose. In some embodiments, the subject may have a treatment schedule wherein administration of subsequent doses of the anti-VEGF antibody polymer conjugate is administered no more than every 4 weeks (e.g., every 8 weeks, every 12 weeks, every 16 weeks, every 20 weeks, every 24 weeks, every 28 weeks, every 32 weeks, every 36 weeks, every 40 weeks, including every 52 weeks after the final loading dose) In some embodiments, the treatment schedule may be adjusted to administer doses more frequently (e.g. original dose frequency every 8 weeks, adjusted dose frequency every 4 weeks) if the subject's treated eye is determined to have an increase in non-stabilized fluid within the eye. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

DME

In some embodiments, a method of treating DME is provided. The method comprises administering 1-3 loading doses of an anti-VEGF antibody polymer conjugate (e.g., KSI-301), to a subject having DME and administering 1-7 maintenance doses of the anti-VEGF antibody polymer conjugate to the subject, wherein 7 or fewer total doses are administered to the subject in a year. In some embodiments, fewer than 7, 6, 5, 4, 3, or 2 doses are administered to the subject in a year. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, the total number of injections (including loading and maintenance doses) of the anti-VEGF antibody conjugate (e.g., KSI-301) administered to the subject with DME in the first year of treatment is 7 times or less, 6 times or less, 5 times or less, 4 times or less, 3 times or less, 2 times or less, or once, in order to retain the therapeutic result of the anti-VEGF antibody conjugate therapy. In some embodiments, the total number of injections (including loading and maintenance doses) of the anti-VEGF antibody conjugate (e.g., KSI-301) administered to the subject with DME in the first two years of treatment is 7 times or less, 6 times or less, 5 times or less, 4 times or less, 3 times or less, 2 times or less, or once, in order to retain the therapeutic result of the anti-VEGF antibody conjugate therapy. In some embodiments, the total number of injections (including loading and maintenance doses) of the anti-VEGF antibody conjugate (e.g., KSI-301) administered to the subject with DME in the first three years of treatment is 23 times or less, 22 times or less, 21 times or less, 20 times or less, 19 times or less, 18 times or less, 17 times or less, 16 times or less, 15 times or less, 14 times or less, 13 times or less, 12 times or less, 11 times or less, 10 times or less, 9 times or less, 8 times or less, 7 times or less, 6 times or less, 5 times or less, 4 times or less, 3 times or less, 2 times or less, or once, in order to retain the therapeutic result of the anti-VEGF antibody conjugate therapy. In some embodiments the total number of loading doses is 3 times or less, 2 times or less, or once, while maintenance doses are administered 7 times or less, 6 times or less, 5 times or less, 4 times or less, 3 times or less, 2 times or less, or once, in order to retain the therapeutic result of the anti-VEGF antibody conjugate therapy. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of treating DME is provided. The method comprises administering 2-3 loading doses of an anti-VEGF antibody polymer conjugate (e.g., KSI-301), to a subject having DME; and administering a maintenance dose of the anti-VEGF antibody polymer conjugate to the subject every 2-15 months, depending on whether the eye is dry as determined by OCT or OCT-A measurement. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days. In some embodiments, no further maintenance doses are required after the 2 or 3 loading doses.

In some embodiments, presented herein is a method of treating a subject with an eye disorder, where the eye disorder is DME, where the method includes administering between 1 to 3 loading doses, but no more than 3 loading doses, of an anti-VEGF antibody conjugate (e.g., KSI-301), or anti-VEGF protein conjugate (e.g., an aflibercept biopolymer conjugate), to a subject, and providing a follow-on application of the anti-VEGF antibody conjugate or anti-VEGF protein at a schedule of every 3 to every 15 (e.g., every 4 months, every 5 months, every 6 months, every 7 months, every 8 months, every 9 months, every 10 months, every 11 months, every 12 months, every 13 months, every 14 months) based on results from an ophthalmic exam. In some embodiments, the ophthalmic exam may include measurement and assessment of eye health using any suitable method, including, but not limited to, OCT-angiography (OCT-A), fluorescein angiogram or ultrawide-field fluorescein angiogram, and may measure various eye anatomical

features, including, but not limited to, choroid status, retinal thickness, reflectivity, luminance, or fluid turbidity and status. In some embodiments, where the eye appears exudative or wet as determined by OCT-A measurement, the follow-on application of the anti-VEGF antibody conjugate or anti-VEGF protein is administered every 3 to every 15 (e.g., every 4 months, every 5 months, every 6 months, every 7 months, every 8 months, every 9 months, every 10 months, every 11 months, every 12 months, every 13 months, every 14 months). In some embodiments, where the eye appears dry as determined by OCT-A measurement, the follow-on application of the anti-VEGF antibody conjugate or anti-VEGF protein is administered every 3 to every 15 (e.g., every 4 months, every 5 months, every 6 months, every 7 months, every 8 months, every 9 months, every 10 months, every 11 months, every 12 months, every 13 months, every 14 months). In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of treating DME is provided. The method comprises: administering 2-3 loading doses of an anti-VEGF antibody polymer conjugate (e.g., KSI-301) to a subject having DME; and administering a maintenance dose of the anti-VEGF antibody polymer conjugate to the subject every 2-15 months, depending on whether retina thickness in the subject is above 320 microns. In some embodiments, the method includes administering the maintenance dose of the anti-VEGF antibody polymer conjugate to the subject every 2-15 months, depending on whether retina thickness in the subject is above 350 microns. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is +1, 2, 3, 4, 5, 6, 7, 8, 9, 10, 11, 12, 13, or 14 days. In some embodiments, no further maintenance doses are required after the 2 or 3 loading doses.

In some embodiments, presented herein is a method of treating a subject with an eye disorder, where the eye disorder is DME, where the method includes administering between 1 to 3 loading doses, but no more than 3 loading doses, of an anti-VEGF antibody conjugate (e.g., KSI-301), or anti-VEGF protein conjugate (e.g., an aflibercept biopolymer conjugate), to a subject, and providing a follow-on application of the anti-VEGF antibody conjugate or anti-VEGF protein at a schedule of every 3 to every 15 months (e.g., every 4 months, every 5 months, every 6 months, every 7 months, every 8 months, every 9 months, every 10 months, every 11 months, every 12 months, every 13 months, every 14 months) based on results from an ophthalmic exam. In some embodiments, the ophthalmic exam may include measurement and assessment of eye health using any suitable method, including, but not limited to, OCT-angiography (OCT-A), fluorescein angiogram or ultrawide-field fluorescein angiogram, and may measure various eye anatomical features, including, but not limited to, choroid status, retinal thickness, reflectivity, luminance, or fluid turbidity and status. In some embodiments, where the eye appears to have a retina thickness above 320 microns, e.g., above 330 microns, above 340 microns, above 350 microns, or a thickness in a range defined by any two of the preceding values, the follow-on application of the anti-VEGF antibody conjugate or anti-VEGF protein is administered every 2 to every 15 months (e.g., every 4 months, every 5 months, every 6 months, every 7 months, every 8 months, every 9 months, every 10 months, every 11 months, every 12 months, every 13 months, every 14 months, or at an interval within a range defined by any two of the preceding values). In some embodiments where the eye appears to have a retina thickness below 320 microns, e.g., above 330 microns, above 340 microns, above 350 microns, or a thickness in a range defined by any two of the preceding values, the follow-on application of the anti-VEGF antibody conjugate or anti-VEGF protein is administered every 3 to every 15 (e.g., every 4 months, every 5 months, every 6 months, every 7 months, every 8 months, every 9 months, every 10 months, every 11 months, every 12 months, every 13 months, every 14 months, or at an interval within a range defined by any two of the preceding values). In some embodiments, an anti-VEGF protein conjugate (e.g., an aflibercept biopolymer conjugate) can be used in place of the denoted KSI-301 or anti-VEGF antibody polymer conjugate. In some embodiments, the follow-on application of the anti-VEGF antibody conjugate or anti-VEGF protein is provided based on the retinal thickness being above 320 microns. In some embodiments, the follow-on application of the anti-VEGF antibody conjugate or anti-VEGF protein is provided based on the retinal thickness being above 350 microns. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days. In some embodiments, no further maintenance doses are required after the 2 or 3 loading doses.

In some embodiments, retina thickness and BCVA is determined every 4 weeks after the administration of the last loading dose. In some embodiments, evaluation of retina thickness and BCVA is determined every 4 weeks for 20 weeks. In some embodiments the retina thickness is determined by OCT or OCT-A measurements, and retina thickness is first determined prior to administering the anti-VEGF antibody polymer conjugate. In some embodiments, a second retina thickness measurement, can be determined 4 weeks after administration of the last loading dose. In some embodiments, the second retina thickness can be compared to the retina thickness measurement taken prior to administering the anti-VEGF antibody polymer conjugate, and where the second retina thickness is ≥ 75 microns compared to the retina thickness measurement taken prior to administering the anti-VEGF antibody polymer conjugate, the subsequent

administration of the anti-VEGF antibody polymer conjugate is no more than every 8 weeks. In some embodiments, the second retina thickness can be compared to the retina thickness measurement taken prior to administering the anti-VEGF antibody polymer conjugate, and where the second retina thickness is ≥ 40 microns compared to the retina thickness measurement taken prior to administering the anti-VEGF antibody polymer conjugate, the subsequent administration of the anti-VEGF antibody polymer conjugate is no more than every 8 weeks. In some embodiments BCVA is measured prior to administration of the anti-VEGF antibody polymer conjugate. In some embodiments, a second BCVA measurement can be determined 4 weeks after administration of the last loading dose. In some embodiments, where the second retina thickness is ≥ 50 microns compared to the retina thickness measurement taken prior to administering the anti-VEGF antibody polymer conjugate and wherein there is a decrease in the second BCVA of ≥ 5 letters compared to the BCVA measured prior to the administration of the anti-VEGF antibody polymer conjugate, then administration of the anti-VEGF antibody polymer conjugate and thereafter administering the anti-VEGF antibody polymer conjugate no more than every 8 weeks should occur. In some embodiments, wherein no increase of retina thickness >30 microns is observed compared to the retina thickness measurement taken prior to administering the anti-VEGF antibody polymer conjugate, then no maintenance dose of the anti-VEGF antibody polymer conjugate is administered to the subject for at least 4 weeks. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of preventing vision complications is provided. The method comprises: administering 2-3 loading doses of an anti-VEGF antibody polymer conjugate (e.g., KSI-301) to a subject having DME or proliferative diabetic retinopathy; and administering a maintenance dose of the anti-VEGF antibody polymer conjugate to the subject every 3-15 months, depending on: a) whether retina thickness in the subject is above 320 microns; and/or b) whether the eye is dry as determined by OCT or OCT-A measurement, and/or c) there is evidence of proliferative diabetic retinopathy. In some embodiments, an anti-VEGF protein conjugate (e.g., an aflibercept biopolymer conjugate) can be used in place of the denoted KSI-301 or anti-VEGF antibody polymer conjugate. In some embodiments, the retina thickness is more than 320, or more than 350 microns, or more than 400 microns. In some embodiments, the retina thickness is more than 350 microns. In some embodiments, the retina thickness is more than 320 microns. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, where the eye appears exudative or wet as determined by OCT or OCT-A measurement, the follow-on application of the anti-VEGF antibody conjugate or anti-VEGF protein is administered every 3 to every 15 (e.g., every 4 months, every 5 months, every 6 months, every 7 months, every 8 months, every 9 months, every 10 months, every 11 months, every 12 months, every 13 months, every 14 months). In some embodiments, where the eye appears dry as determined by OCT or OCT-A measurement, the follow-on application of the anti-VEGF antibody conjugate or anti-VEGF protein is administered every 3 to every 15 (e.g., every 4 months, every 5 months, every 6 months, every 7 months, every 8 months, every 9 months, every 10 months, every 11 months, every 12 months, every 13 months, every 14 months). In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is +1, 2, 3, 4, 5, 6, 7, 8, 9, 10, 11, 12, 13, or 14 days.

In some embodiments, a method of treating wAMD, DME, or RVO is provided. The method comprises administering 2-3 loading doses of an anti-VEGF antibody polymer conjugate (e.g., KSI-301) to a subject having wAMD, DME or RVO; obtaining a vision assessment of the subject after 4, 8, 12, 16, 20, 24, 36, 48, or 52 weeks after a last loading dose; and based on the vision assessment of the patient, placing the patient on a fixed regimen of the anti-VEGF antibody polymer conjugate once every 4, 8, 12, 16, 20, 24, 36, 48, or 52 weeks. In some embodiments, the vision assessment is based on a change in the number of letters of 3-5. In some embodiments, the vision assessment is based on a change in OCT or OCT-A of at least 30-50 microns. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, dosing for RVO can be in line with that depicted in FIG. 59.

In some embodiments, presented herein is a method of treating a subject with an eye disorder, where the eye disorder is DME, DR, or RVO, where the method includes administering between 1 to 3 loading doses, but no more than 3 loading doses, of an anti-VEGF antibody conjugate (e.g., KSI-301), or anti-VEGF protein conjugate (e.g., an aflibercept biopolymer conjugate), to a subject, and providing a follow-on application of the anti-VEGF antibody conjugate or anti-VEGF protein at a schedule of once every 4 to every 52 weeks (e.g., every 4 weeks or more, 8 weeks or more, e.g., 10 weeks or more, 12 weeks or more, 14 weeks or more, 16 weeks or more, 18 weeks or more, 20 weeks or more, 24 weeks or more, 28 weeks or more, 32 weeks or more, 36 weeks or more, 40 weeks or more, at least 44 weeks or more, at least 48 weeks or more, including 52 weeks) from the final loading dose based on results from an ophthalmic exam. In some embodiments, the ophthalmic exam may include measurement and assessment of eye health using any suitable method,

including, but not limited to, OCT-angiography (OCT-A), fluorescein angiogram or ultrawide-field fluorescein angiogram, and may measure various eye anatomical features, including, but not limited to, choroid status, retinal thickness, reflectivity, luminance, or fluid turbidity and status. In some embodiments, the ophthalmic exam is run by a physician 4 weeks, 8 weeks, 12 weeks, 16 weeks, 20 weeks, 24 weeks, 30 weeks, 36 weeks, 42 weeks, 48 weeks, or 52 weeks after a last loading dose. In some embodiments, the ophthalmic exam further includes evaluation of vision based on visual acuity. In some embodiments, visual acuity may be monitored using any suitable method. In some embodiments, the visual acuity is measured by best corrected visual acuity (BCVA) using e.g., ETDRS letters or Snellen chart, etc. In some embodiments, the therapeutic result may include an improvement in BCVA measured by ETDRS letters of 3 letters or more, e.g., 4 letters or more, 5 letters or more, , 6 letters or more, 7 letters or more, 8 letters or more, 9 letters or more, 10 letters or more, 12 letters or more, 15 letters or more, 18 letters or more, 20 letters or more, 22 letters, or more, including 25 letters or more, or by a number within a range defined by any two of the preceding values, compared to pre-treatment. In some embodiments, the retinal thickness may be measured using any suitable method, including, but not limited to, optical coherence tomography (OCT) or OCT-A. In some embodiments, the therapeutic result may include a reduction in retinal thickness of about 25 μm or more, e.g., about 30 μm or more, about 40 μm or more, about 50 μm or more, about 75 μm or more, about 100 μm or more, about 125 μm or more, about 150 μm or more, about 175 μm or more, about 200 μm or more, about 225 μm or more, about 250 μm or more, about 275 μm or more, about 300 μm or more, about 325 μm or more, about 350 μm or more, about 375 μm or more, about 400 μm or more, or a reduction within a range defined by any two of the preceding values, compared to pre-treatment. In some embodiments, the therapeutic result may include a reduction in the rate of increase in retinal thickness by at least 10%, e.g., at least 15%, at least 25%, at least 50%, at least 75%, at least 90%, including about 100%, or any percentage in a range defined by any two of the preceding values, over pre-treatment. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of treating a subject with DME, DR or RVO, the method comprising: administering 1-3 loading doses of an anti-VEGF antibody polymer conjugate (e.g., KSI-301) to a subject with DME, DR or RVO; not administering more than 3 loading doses to the subject; and providing a follow-on application of the anti-VEGF antibody polymer conjugate at a point in time no sooner than 8 weeks after a last loading dose or after a last follow-on application of the anti-VEGF antibody polymer conjugate, wherein the loading doses are administered to the subject on a monthly basis, and wherein the subject is above 16 years of age. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of treating a subject with DME, DR or RVO is provided. The method comprises administering 1-3 loading doses of an anti-VEGF antibody polymer conjugate to a subject with DME, DR or RVO, not administering more than 3 loading doses to the subject, and providing a follow-on application of the anti-VEGF antibody polymer conjugate at a point in time after a last loading dose or after a last follow-on application of the anti-VEGF antibody polymer conjugate, wherein the loading doses are administered to the subject on a monthly basis, and wherein the subject is above 16 years of age. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of treating a subject with DME or DR, the method comprising: administering 1-3 loading doses of an anti-VEGF antibody polymer conjugate (e.g., KSI-301) to a subject with DME, DR or RVO; not administering more than 3 loading doses to the subject; and providing a follow-on application of the anti-VEGF antibody polymer conjugate at a point in time after a last loading dose or after a last follow-on application of the anti-VEGF antibody polymer conjugate, wherein the loading doses are administered to the subject on a monthly basis, and wherein the subject is above 16 years of age. In some embodiments, the follow-on application can occur after 6, 7, 8, 9, 10, 11, 12, 13, or 14 weeks or more after the loading doses. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of treating a subject with DME, DR or RVO is provided. The method comprises administering 1-3 loading doses of an anti-VEGF antibody polymer conjugate to a subject with DME, DR or RVO, not administering more than 3 loading doses to the subject, and providing a follow-on application of the anti-VEGF antibody polymer conjugate at a point in time after a last loading dose or after a last follow-on application of the anti-VEGF antibody polymer conjugate, wherein the loading doses are administered to the subject on a monthly basis, and wherein the subject is above 16 years of age. In some embodiments, the follow-on application can occur after 6, 7, 8, 9, 10, 11, 12, 13, or 14 weeks or more after the loading doses. In some embodiments, an anti-VEGF protein conjugate (e.g., an aflibercept biopolymer conjugate) can be used in place of the denoted KSI-301 or anti-VEGF antibody polymer conjugate. In some

embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, presented herein is a method of treating a subject with an eye disorder, where the eye disorder is DME, DR, or RVO, where the method includes administering between 1 to 3 loading doses, but no more than 3 loading doses, of an anti-VEGF antibody conjugate (e.g., KSI-301), or anti-VEGF protein conjugate (e.g., an aflibercept biopolymer conjugate), to a subject, and providing a follow-on application of the anti-VEGF antibody conjugate or anti-VEGF protein no sooner than 4, 5, 6, 7, 8, 9, 10, 11, 12, 13, 14, 15, 16 weeks or longer after the last loading dose was administered. In some embodiments, the follow-on application of the anti-VEGF antibody conjugate or anti-VEGF protein conjugate is provided no sooner than 8 weeks, e.g., no sooner than 12 weeks, no sooner than 16 weeks, no sooner than 18 weeks, no sooner than 20 weeks, no sooner than 24 weeks, no sooner than 28 weeks, no sooner than 32 weeks, no sooner than 36 weeks, including no sooner than 40 weeks after the last loading dose was administered. In some embodiments, the subject is above 16 years of age (e.g. above 17 years of age, above 18 years of age, above 20 years of age, above 25 years of age, above 30 years of age, above 35 years of age, above 40 years of age, above 45 years of age, above 50 years of age, above 55 years of age, above 60 years of age, above 65 years of age, above 70 years of age, above 75 years of age, above 80 years of age, above 85 years of age, above 90 years of age, above 95 years of age, above 100 years of age, above 105 years of age, above 110 years of age, above 115 years of age, above 120 years of age, above 125 years of age, above 130 years of age. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of treating DME, DR, or RVO is provided. The method comprises administering 1-3 loading doses of an anti-VEGF antibody polymer conjugate (e.g., KSI-301) to a subject with DME, DR or RVO, not administering more than 3 loading doses to the subject, providing a follow-on application of the anti-VEGF antibody polymer conjugate at a point in time no sooner than 8 or 12 weeks after a last loading dose or after a last follow-on application of the anti-VEGF antibody polymer conjugate, wherein the loading doses are administered to the subject on a monthly basis, and wherein an improvement of Best Corrected Visual Acuity (BCVA) is greater than at least 5, or at least 6.8 to 7.2 letters post treatment. In some embodiments, an anti-VEGF protein conjugate (e.g., an aflibercept biopolymer conjugate) can be used in place of the denoted KSI-301 or anti-VEGF antibody polymer conjugate. In some embodiments, the subject has DME and the improvement achieves a BCVA score of at least 65 letters, e.g., >75 letters. In some embodiments, the subject has RVO and the improvement achieves a BCVA score of at least 65 letters, e.g., >80, >90, >95, >99, 100 letters. In some embodiments, the subject has DME and the improvement is 1 to 30 letters. In some embodiments, the subject has wAMD and the improvement is 1 to 35 letters. In some embodiments, the subject has RVO and the improvement is 1 to 40 letters. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days. In some embodiments, no further maintenance doses are required after the 2 or 3 loading doses.

In some embodiments, presented herein is a method of treating a subject with an eye disorder, where the eye disorder is DME, DR, or RVO, where the method includes administering between 1 to 3 loading doses, but no more than 3 loading doses, of an anti-VEGF antibody conjugate (e.g., KSI-301), or anti-VEGF protein conjugate (e.g., an aflibercept biopolymer conjugate), to a subject, and providing a follow-on application of the anti-VEGF antibody conjugate or anti-VEGF protein no sooner than 12 weeks after the last loading dose was administered. In some embodiments, the loading doses are given every 4 weeks. In some embodiments, the follow-on application of the anti-VEGF antibody conjugate or anti-VEGF protein conjugate is provided no sooner than 12 weeks, e.g., no sooner than 12 weeks, no sooner than 16 weeks, no sooner than 18 weeks, no sooner than 20 weeks, no sooner than 24 weeks, no sooner than 28 weeks, no sooner than 32 weeks, no sooner than 36 weeks, including no sooner than 40 weeks after the last loading dose was administered. In some embodiments, the subject has a measured improvement in Best Corrected Visual Acuity (BCVA) greater than 5 letters, or 6.8 to 7.2 letters post treatment. In some embodiments, the subject has DME and a measured Best Corrected Visual Acuity (BCVA) to a BCVA score of at least 65 letters after treatment (e.g., >75 letters, >80 letter, >90 letter, >95 letters, or 100 letters. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of treating a subject with DME, DR, or RVO is provided. The method comprises administering 1-3 loading doses of an anti-VEGF antibody polymer conjugate (e.g., KSI-301) to a subject with DME, DR, or RVO; not administering more than 3 loading doses; and providing between 0-1 follow-on application of the anti-VEGF antibody polymer conjugate at a point in time no sooner than 8 or 12 weeks after a last loading dose or after a last follow-on application of the anti-VEGF antibody polymer conjugate, wherein the loading doses are administered to the subject on a monthly basis. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, presented herein is a method of treating a subject with an eye disorder, where the eye disorder is DME, DR, or RVO, where the method includes administering between 1 to 3 loading doses, but no more than 3 loading doses, of an anti-VEGF antibody conjugate (e.g., KSI-301), or anti-VEGF protein conjugate (e.g., an aflibercept biopolymer conjugate), to a subject, and providing between 0-1 follow-on application(s) of the anti-VEGF antibody conjugate or anti-VEGF protein no sooner than 8 or 12 weeks after the last loading dose was administered, for a total of up to 4 total doses (e.g. 3 total doses, 2 total doses, including 1 total dose for therapeutic effect). In some embodiments, the loading doses are given every 4 weeks. In some embodiments, the follow-on application of the anti-VEGF antibody conjugate or anti-VEGF protein conjugate is provided no sooner than 12 weeks, e.g., no sooner than 12 weeks, no sooner than 16 weeks, no sooner than 18 weeks, no sooner than 20 weeks, no sooner than 24 weeks, no sooner than 28 weeks, no sooner than 32 weeks, no sooner than 36 weeks, including no sooner than 40 weeks after the last loading dose was administered. In some embodiments, an anti-VEGF protein conjugate (e.g., an aflibercept biopolymer conjugate) can be used in place of the denoted KSI-301 or anti-VEGF antibody polymer conjugate. In some embodiments, the dosing is approximate. In some embodiments, the dosing is no sooner than the designated point in time. In some embodiments, the dosing is $\pm$1, 2, 3, 4, 5, 6, 7, 8, 9, 10, 11, 12, 13, or 14 days.

In some embodiments, for any of the indications herein, the subject can have the health of their eye determined in part by vision assessment, where the vision assessment measures the decrease of Best Corrected Visual Acuity (BCVA). In some embodiments, the decrease in BCVA is > 3-5 letters. In some embodiments, the dosing is approximate. In some embodiments, the dosing is no sooner than the designated point in time. In some embodiments, the dosing is $\pm$1, 2, 3, 4, 5, 6, 7, 8, 9, 10, 11, 12, 13, or 14 days.

In some embodiments, for any of the indications herein, the subject can have the health of their eye determined in part by imaging the eye, including but not limited to OCT-A, SD-OCT, and FA. In some embodiments, the subject's eye can be classified as wet or dry based on physical examination and imaging the eye. In some embodiments, imaging of the eye by OCT or OCT-A allows for classification of the subject's eye as wet or dry based on observation of excess intraretinal or excess subretinal fluid affecting the central subfield of the eye, where if there is excess intraretinal or subretinal fluid affecting the central subfield of the eye then the eye is wet, and where if there is no excess intraretinal or subretinal fluid affecting the central subfield of the eye then the eye is dry. In some embodiments, the dosing is approximate. In some embodiments, the dosing is no sooner than the designated point in time. In some embodiments, the dosing is $\pm$1, 2, 3, 4, 5, 6, 7, 8, 9, 10, 11, 12, 13, or 14 days.

In some embodiments, the subject can have DME, and an improvement of BCVA of at least 65 letters, e.g., >75 letters. In some embodiments, the subject can have wAMD, and an improvement of BCVA of at least 60 letters, e.g., >75 letters. In some embodiments, the subject can have RVO, and an improvement of BCVA of at least 65 letters, e.g., >80 letters. In some embodiments, the subject can have DME, and an improvement of 15 to 25 letters. In some embodiments, the subject can have wAMD, and the improvement is 10 to 22 letters. In some embodiments, the subject can have RVO, and the improvement is 15 to 30 letters.

## Additional Embodiments

In some embodiments, the therapeutic result comprises one or more of: improved visual acuity, reduced retinal thickness, improved perfusion in at least one eye, improved diabetic retinopathy severity score compared to pre-treatment levels; reduced rate of vitreous hemorrhage, reduced rate of diabetic macular edema, reduced rate of tractional retinal detachment, and/or reduced rate of anterior segment neovascularization (ASNV), after the initiation of treatment. In some embodiments, the therapeutic result is compared to an assessment after the loading phase, to assess the stability of retinal thickness and vision.

In some embodiments, the treatment can include for:

1. Neovascular (Wet) Age-related Macular Degeneration (wAMD): monthly treatment regimen
2. Treatment-naive Diabetic Macular Edema (DME): dosing regimen, covering Q8W after loading doses (previous patent only covers Q12W)

In some embodiments, for DME and RVO, one treats with 2-3 monthly loading doses only, and provides a therapeutic benefit for at least 1 yr.

In some embodiments, a method of treating an eye disorder is provided. The method comprises administering an anti-VEGF antibody conjugate to a subject in need of treating an eye disorder at a first loading dose, wherein the eye disorder is diabetic retinopathy (DR); and repeating the loading dose at least once, but not more than three times, whereby the subject retains a therapeutic result of the anti-VEGF antibody conjugate therapy for at least 24 weeks after a final loading dose, and where pan-retinal photocoagulation can be safely deferred for at least 6 months.

In some embodiments, a method of treating an eye disorder is provided. The method comprising: administering an anti-VEGF antibody conjugate to a subject in need of treating an eye disorder at a first loading dose, wherein the eye

disorder is diabetic retinopathy (DR); and repeating the loading dose at least once, but not more than three times, whereby the subject retains a therapeutic result of the anti-VEGF antibody conjugate therapy for at least 24 weeks after a final loading dose, and wherein the subject is a type 1 or type 2 diabetic with a HbA1c of <= 12.0%.

In some embodiments, the therapeutic population comprises patients with no history of cataract surgery within 2 months, no history of YAG laser capsulotomy, or no history of uncontrolled glaucoma.

In some embodiments, the subject is going through concomitant therapy. In some embodiments, the therapeutic population comprises patients not currently taking intravitreal or periocular steroids. In some embodiments, the therapeutic population comprises patients not currently being treated with concurrent macular laser photocoagulation.

In some embodiments, a method of treating an eye disorder is provided. The method comprises administering to a subject in need of treating an eye disorder a therapeutically effective amount of the anti-VEGF antibody conjugate at a dosing schedule of Q4W or longer, wherein the eye disorder is Wet Age-related Macular Degeneration (wAMD) thereby treating the eye disorder, and wherein said subject has not been treated previously for wAMD. In some embodiments, wherein the subject has subretinal hyperreflective material (SHRM) affecting the central subfield. In some embodiments, the subject has an eye without one or more of: angioid streaks, prior trauma, ocular histoplasmosis, fibrosis of >50% of the foveal center of said eye, subretinal blood affecting 50% or more of the foveal center of said eye, retinal pigment epithelium tears, prior macular laser treatment, other approved treatment for wAMD, cataract surgery, glaucoma surgery, Yttrium-Aluminum Garnet (YAG) laser capsulotomy within past 2 months, uncontrolled glaucoma, uveitis, significant media opacities, cataracts, aphakia, prior vitrectomy, corneal transplant, or any other active retinal disease. In some embodiments, the subject does not have: uncontrolled blood pressure (>180 mmHg systolic / >100 mmHg diastolic), kidney failure, recent history of myocardial infarction, stroke, congestive heart failure, or any acute coronary event. In some embodiments, the subject does not currently take intravitreal or periocular steroids. In some embodiments, the subject is not currently being treated with concurrent macular laser photocoagulation. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of treating a subject with DME, DR or RVO is provided. The method comprises: administering 1-3 loading doses of an anti-VEGF antibody conjugate to a subject with DME, DR or RVO; not administering more than 3 loading doses to the subject; providing a follow-on application of the anti-VEGF antibody conjugate at a point in time no sooner than 8 weeks after a last loading dose or after a last follow-on application of the anti-VEGF antibody conjugate, wherein the loading doses are administered to the subject on a monthly basis, and wherein the subject can have pan-retinal photocoagulation safely deferred for at least 6 months. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of treating a subject with DME, DR or RVO is provided. The method comprising: administering 1-3 loading doses of an anti-VEGF antibody conjugate to a subject with DME, DR or RVO; not administering more than 3 loading doses to the subject; providing a follow-on application of the anti-VEGF antibody conjugate at a point in time no sooner than 8 weeks after a last loading dose or after a last follow-on application of the anti-VEGF antibody conjugate, wherein the loading doses are administered to the subject on a monthly basis, and wherein the subject is a type 1 or type 2 diabetic with HbAlc of <= 12.0%. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

Also provided herein is a method of treating diabetic retinopathy that includes: identifying a subject with diabetic retinopathy; administering 1-3 loading doses (e.g., 3 loading doses) of an anti-VEGF antibody polymer conjugate to the subject; administering one or more subsequent doses of the anti-VEGF antibody polymer conjugate to the subject no more frequently than once every 24 weeks after the last loading dose, wherein at least 4 or 5 doses (e.g., including loading doses and any subsequent doses) of the anti-VEGF antibody polymer conjugate is administered to the subject in the first year of treatment. In some embodiments, the loading doses are administered once every one month or two months. In some embodiments, the anti-VEGF antibody polymer conjugate is KSI-301.

Also provided herein is a method of treating a subject having wAMD that includes: identifying a subject having wAMD; and administering an anti-VEGF antibody polymer conjugate (e.g., KSI-301) to the subject no more frequently than once every 4 weeks. In some embodiments, the anti-VEGF antibody polymer conjugate is administered to the subject once every 4 weeks regardless of improvement in visual acuity observed after administering the anti-VEGF antibody polymer conjugate.

Also provided is a method of treating DME, including: identifying a subject having DME; administering 2-3 monthly loading doses (e.g., 3 monthly loading doses) of an anti-VEGF antibody polymer conjugate to the subject; and administering one or more subsequent doses of the anti-VEGF antibody polymer conjugate to the subject every 2-6 months, depending on: whether retina thickness in the subject is above 320 microns (e.g., above 330 microns, above 340 microns, or above 350 microns); and/or whether the eye is dry as determined by OCT or OCT-A measurement, wherein at least 4, 5, 6, 7 or more doses (e.g., including loading doses and any subsequent doses) of the anti-VEGF antibody polymer

conjugate is administered to the subject in the first year of treatment. In some embodiments, the anti-VEGF antibody polymer conjugate is KSI-301. In some embodiments, the one or more subsequent doses of the anti-VEGF antibody polymer conjugate is administered to the subject every 2, 3, 4, 5, 6 months, or with a time interval defined by any two of the preceding values. In some embodiments, the method includes administering the one or more subsequent doses of the anti-VEGF antibody polymer conjugate to the subject every 2-6 months, depending on whether retina thickness in the subject is above 350 microns; and/or whether the eye is dry as determined by OCT or OCT-A measurement.

Provided herein is a method of treating RVO, including: identifying a subject having RVO; administering 2 monthly loading doses of an anti-VEGF antibody polymer conjugate to the subject; and administering one or more subsequent doses of the anti-VEGF antibody polymer conjugate to the subject no more frequently than once every 2 months, wherein at least 4, 5, 6, 7 or more doses (e.g., including loading doses and any subsequent doses) of the anti-VEGF antibody polymer conjugate is administered to the subject in the first year of treatment. In some embodiments, the anti-VEGF antibody polymer conjugate is KSI-301. In some embodiments, the anti-VEGF antibody polymer conjugate is administered to the subject once every 2 months regardless of improvement in visual acuity observed after administering the anti-VEGF antibody polymer conjugate.

Also provided is a method of treating wAMD, including: identifying a subject having wAMD; administering 3 monthly loading doses of an anti-VEGF antibody polymer conjugate (e.g., KSI-301) to the subject; evaluating retina thickness and/or BCVA of the subject at 12 weeks after the third loading dose to thereby determine that the subject has improved visual acuity; evaluating retina thickness and/or BCVA of the subject at 16 weeks after the third loading dose to thereby determine that the subject has improved visual acuity; and administering a maintenance dose of the anti-VEGF antibody polymer conjugate to the subject 20 weeks after the third loading dose; and determining that the subject will receive one or more maintenance doses no more frequently than once every 20 weeks. In some embodiments, the subject has improved visual acuity when retina thickness is reduced by about 40, 50, 60, 70, 80, 90, 100, 110, 120 microns or more, or by a thickness in a range defined by any two of the preceding values, compared to the subject's retina thickness before administering the first loading dose. In some embodiments, the subject has improved visual acuity when BCVA is increased by about 3, 4, 5, 6, 7, 8 or more, or by an amount in a range defined by any two of the preceding values, compared to BCVA before administering the first loading dose. In some embodiments, a subject having wAMD who will exhibit sustained improvement in visual acuity from an anti-VEGF antibody polymer conjugate therapy (e.g., KSI-301 therapy) can be identified. In some embodiments, the subject is identified by administering 3 monthly loading doses of an anti-VEGF antibody polymer conjugate (e.g., KSI-301) to the subject; and evaluating retina thickness and/or BCVA of the subject at 12 and/or at 16 weeks after the third loading dose. If the subject retains the therapeutic benefit of the anti-VEGF antibody polymer conjugate therapy 16 weeks after the third loading dose (e.g., without receiving any additional treatment after the third loading dose), the subject is determined to be one who exhibits sustained improvement in visual acuity from the anti-VEGF antibody polymer conjugate therapy. In some embodiments, the subject is administered one or more maintenance doses of the anti-VEGF antibody polymer conjugate therapy no more frequently than once every 20 weeks.

In some embodiments, for any of the indications herein, the subject can have pan-retinal photocoagulation safely deferred for at least 6 months. In some embodiments, for any of the indications herein, the subject is a type 1 or type 2 diabetic with HbAlc of <= 12.0%. In some embodiments, for any of the indications herein, the subject has a central subfield thickness (CST) of > 320 microns. In some embodiments, for any of the indications herein, the subject has a central subfield thickness (CST) of > 350 microns. In some embodiments, for any of the indications herein, the therapeutic population comprises patients not currently taking intravitreal or periocular steroids. In some embodiments, for any of the indications herein, the therapeutic population comprises patients not currently being treated with concurrent macular laser photocoagulation. In some embodiments, the dosing is approximate. In some embodiments, the dosing is no sooner than the designated point in time. In some embodiments, the dosing is $\pm$1, 2, 3, 4, 5, 6, 7, 8, 9, 10, 11, 12, 13, or 14 days.

In some embodiments, a method of the present disclosure includes administering one or more doses of an anti-VEGF antibody conjugate (e.g., KSI-301, or the embodiment in FIG. 14 conjugated to a phosphorylcholine polymer, as provided herein, or the construct depicted in FIG. 13), or an anti-VEGF protein conjugate (e.g., aflibercept biopolymer conjugate) to a subject (e.g., human or other mammalian patient) in need of treating an eye disorder, to thereby treat the eye disorder. The anti-VEGF antibody conjugate or anti-VEGF protein conjugate, when administered to the subject, may provide a long-lasting therapeutic effect that allows for a dosing schedule with longer intervals between dosing than has been previously used with anti-VEGF therapies. In some embodiments, a therapeutic result of the anti-VEGF antibody conjugate therapy or anti-VEGF protein conjugate therapy, once achieved by administration of one or more doses (e.g., loading dose and/or maintenance dose) of the anti-VEGF antibody conjugate or anti-VEGF protein conjugate, is retained by the subject without requiring any additional dose thereafter, e.g., retained for the rest of the subject's life.

With reference to Fig. 42, an embodiment of a method of the present disclosure is described. The method **4200** can include administering **4210** an anti-VEGF antibody conjugate (e.g., KSI-301) to a subject in need of treating an eye disorder (e.g., wAMD, DME, or RVO) at a first loading dose. Then, the loading dose can be repeated **4220** at least once (e.g., repeated once, twice, three times, etc.). After administration of the loading doses, the therapeutic result of the anti-VEGF antibody conjugate, e.g., KSI-301, therapy can be retained by the subject for at least 8 weeks, e.g., at least 12

weeks, at least 16 weeks, at least 20 weeks, at least 24 weeks or longer, after the final loading dose. In some embodiments, the method includes administering **4230** one or more subsequent doses (e.g., maintenance doses) of the anti-VEGF antibody conjugate, e.g., KSI-301, to the subject at least 8 weeks, e.g., at least 12 weeks, at least 16 weeks, at least 20 weeks, at least 24 weeks, after administering the final loading dose. In some embodiments, the method includes administering an anti-VEGF protein, e.g., aflibercept, conjugate (in lieu of the anti-VEGF antibody conjugate) to a subject in need of treating an eye disorder, according to any of the methods disclosed herein.

In some embodiments, the method includes administering a first loading dose of an anti-VEGF antibody conjugate (e.g., KSI-301) or anti-VEGF protein conjugate (e.g., an aflibercept biopolymer conjugate) to a subject (e.g., human or other mammalian patient) in need of treating an eye disorder, and subsequently administering at least one more, but no more than two more of the loading doses to achieve a therapeutic result (e.g., improved vision, slowing disease progression, reduced symptoms, improved retinal health, etc.) of the anti-VEGF therapy that lasts for an extended period of time. In some embodiments, the patient may not require retreatment of the eye disorder for an extended period of time upon receiving the final loading dose. In some embodiments, administration of the anti-VEGF antibody conjugate (or anti-VEGF protein conjugate (e.g., an aflibercept biopolymer conjugate)) may provide for a therapeutic result of the anti-VEGF therapy without any loading doses, to treat a subject having an eye disorder (e.g., non-proliferative diabetic retinopathy).

As used herein, any time "anti-VEGF antibody" or "anti-VEGF antibody conjugate" is referenced, an anti-VEGF protein, such as an anti-VEGF fusion protein, e.g., aflibercept, is also contemplated. Thus, as disclosed herein, any time "anti-VEGF antibody conjugate" is referenced, an anti-VEGF protein, e.g., aflibercept, covalently bonded to a phosphorylcholine containing biopolymer (e.g., OG1802) as disclosed herein, is also contemplated. In the various embodiments disclosed herein, any reference to an anti-VEGF antibody conjugate therapy, also contemplates an anti-VEGF protein, e.g., aflibercept, conjugate therapy. In the various embodiments of methods of treating an eye disorder, disclosed herein, any reference to an anti-VEGF antibody conjugate, also contemplates an aflibercept biopolymer conjugate.

A "loading dose" has its ordinary and customary meaning as understood by a person of ordinary skill in the art, in view of the present disclosure. A loading dose may refer to an amount of a therapeutic agent administered to a subject, either before a therapeutic effect of the agent is observed in the subject, or before a desired level of therapeutic effect of the agent is achieved in the subject. A loading dose is typically administered at the beginning of a course of treatment with the therapy. In some embodiments, the loading dose is administered more frequently or at shorter intervals compared to later doses that are for maintenance of a therapeutic result. The time period during which a subject receives one or more loading doses may be referred to as a loading phase. In some embodiments, a subject is not monitored for disease progression or status (e.g., not assessed for visual acuity, retinal thickness, etc.) during the loading phase. In some embodiments, a therapeutic result (as disclosed herein) of the anti-VEGF antibody conjugate therapy (e.g., KSI-301 therapy) has not reached a desired or threshold level during the loading phase. The loading dose may be one of a series of loading doses administered to the subject, e.g., during the loading phase. A "final loading dose" may refer to the last loading dose in a series of loading doses administered to the subject, at and/or after which a desired level of therapeutic effect of the agent is achieved. Thus, where the subject is given one loading dose, the final loading dose is the first loading dose. Where the subject is given two loading doses, the final loading dose is the second loading dose. Likewise, where the subject is given three loading doses, the final loading dose is the third loading dose, and so on. A dose of the therapeutic agent administered to a subject after the loading phase may be referred to as a maintenance dose or a retreatment dose. "Maintenance dose" and "retreatment dose" are used herein interchangeably. In some embodiments provided herein, the loading doses can be adequate without as frequent need for, or any need for, subsequent retreatment or maintenance doses. In some embodiments, a series of loading doses is administered to a subject at a higher frequency than a series of maintenance (or retreatment) doses administered to the subject. In some embodiment, the loading dose(s) given may be sufficient to keep disease activity under control in the subject, without requiring a maintenance (or retreatment) dose. In some embodiments, for example, with DME, DR, wet AMD, or RVO, the first dose administered can be sufficient on its own. Thus, for DME, the first dose administered to the subject can be the loading dose, or no specific loading doses are required (as all doses are the same).

In some embodiments, the therapeutic result of the anti-VEGF therapy achieved by the methods disclosed herein is sufficiently retained so as not to require a maintenance dose at the scheduled time point in a predetermined dosing schedule. In some embodiments, a subject has at least 30%, at least 35%, at least 40%, at least 45%, at least 50%, at least 55%, at least 60%, at least 65%, at least 70%, at least 75%, at least 80%, at least 85%, at least 90%, at least 95%, or higher chance, or percentage between any two of the preceding values, of not requiring a subsequent dose, e.g., a maintenance dose, of an anti-VEGF antibody conjugate (e.g. KSI-301), or anti-VEGF protein conjugate (e.g., an aflibercept biopolymer conjugate), until about 2 months or longer after receiving the last loading dose or the last maintenance dose. In some embodiments, a subject has at least 30%, at least 35%, at least 40%, at least 45%, at least 50%, at least 55%, at least 60%, at least 65%, at least 70%, at least 75%, at least 80%, at least 85%, at least 90%, at least 95%, or higher chance, or percentage between any two of the preceding values, of not requiring a subsequent dose, e.g., a maintenance dose, of the anti-VEGF antibody conjugate until about 3 months or longer after receiving the last loading dose or the last maintenance

dose. In some embodiments, a subject has at least 30%, at least 35%, at least 40%, at least 45%, at least 50%, at least 55%, at least 60%, at least 65%, at least 70%, at least 75%, at least 80%, at least 85%, at least 90%, at least 95%, or higher chance, or percentage between any two of the preceding values, of not requiring a subsequent dose, e.g., a maintenance dose, of the anti-VEGF antibody conjugate until about 4 months or longer after receiving the last loading dose or the last maintenance dose. In some embodiments, a subject has at least 30%, at least 35%, at least 40%, at least 45%, at least 50%, at least 55%, at least 60%, at least 65%, at least 70%, at least 75%, at least 80%, at least 85%, at least 90%, at least 95%, or higher chance, or percentage between any two of the preceding values, of not requiring a subsequent dose, e.g., a maintenance dose, of the anti-VEGF antibody conjugate until about 5 months or longer after receiving the last loading dose or the last maintenance dose. In some embodiments, a subject has at least 30%, at least 35%, at least 40%, at least 45%, at least 50%, at least 55%, at least 60%, at least 65%, at least 70%, at least 75%, at least 80%, at least 85%, at least 90%, at least 95%, or higher chance, or percentage between any two of the preceding values, of not requiring a subsequent dose, e.g., a maintenance dose, of the anti-VEGF antibody conjugate until about 6 months or longer after receiving the last loading dose or the last maintenance dose. In some embodiments, a subject has at least 30%, at least 35%, at least 40%, at least 45%, at least 50%, at least 55%, at least 60%, at least 65%, at least 70%, at least 75%, at least 80%, at least 85%, at least 90%, at least 95%, or higher chance, or percentage between any two of the preceding values, of not requiring a subsequent dose, e.g., a maintenance dose, of the anti-VEGF antibody conjugate until about 7 months or longer after receiving the last loading dose or the last maintenance dose. In some embodiments, a subject has at least 30%, at least 35%, at least 40%, at least 45%, at least 50%, at least 55%, at least 60%, at least 65%, at least 70%, at least 75%, at least 80%, at least 85%, at least 90%, at least 95%, or higher chance, or percentage between any two of the preceding values, of not requiring a subsequent dose, e.g., a maintenance dose, of the anti-VEGF antibody conjugate until about 8 months or longer after receiving the last loading dose or the last maintenance dose. In some embodiments, a subject has at least 30%, at least 35%, at least 40%, at least 45%, at least 50%, at least 55%, at least 60%, at least 65%, at least 70%, at least 75%, at least 80%, at least 85%, at least 90%, at least 95%, or higher chance, or percentage between any two of the preceding values, of not requiring a subsequent dose, e.g., a maintenance dose, of the anti-VEGF antibody conjugate until about 9 months or longer after receiving the last loading dose or the last maintenance dose. In some embodiments, a subject has at least 30%, at least 35%, at least 40%, at least 45%, at least 50%, at least 55%, at least 60%, at least 65%, at least 70%, at least 75%, at least 80%, at least 85%, at least 90%, at least 95%, or higher chance, or percentage between any two of the preceding values, of not requiring a subsequent dose, e.g., a maintenance dose, of the anti-VEGF antibody conjugate until about 10 months or longer after receiving the last loading dose or the last maintenance dose. In some embodiments, a subject has at least 30%, at least 35%, at least 40%, at least 45%, at least 50%, at least 55%, at least 60%, at least 65%, at least 70%, at least 75%, at least 80%, at least 85%, at least 90%, at least 95%, or higher chance, or percentage between any two of the preceding values, of not requiring a subsequent dose, e.g., a maintenance dose, of the anti-VEGF antibody conjugate until about 12 months or longer after receiving the last loading dose or the last maintenance dose. In some embodiments, a subject has at least 30%, at least 35%, at least 40%, at least 45%, at least 50%, at least 55%, at least 60%, at least 65%, at least 70%, at least 75%, at least 80%, at least 85%, at least 90%, at least 95%, or higher chance, or percentage between any two of the preceding values, of not requiring a subsequent dose, e.g., a maintenance dose, of the anti-VEGF antibody conjugate until about 14 months or longer after receiving the last loading dose or the last maintenance dose. In some embodiments, a subject has at least 30%, at least 35%, at least 40%, at least 45%, at least 50%, at least 55%, at least 60%, at least 65%, at least 70%, at least 75%, at least 80%, at least 85%, at least 90%, at least 95%, or higher chance, or percentage between any two of the preceding values, of not requiring a subsequent dose, e.g., a maintenance dose, of the anti-VEGF antibody conjugate until about 16 months or longer after receiving the last loading dose or the last maintenance dose. In some embodiments, a subject has at least 30%, at least 35%, at least 40%, at least 45%, at least 50%, at least 55%, at least 60%, at least 65%, at least 70%, at least 75%, at least 80%, at least 85%, at least 90%, at least 95%, or higher chance, or percentage between any two of the preceding values, of not requiring a subsequent dose, e.g., a maintenance dose, of the anti-VEGF antibody conjugate until about 18 months or longer after receiving the last loading dose or the last maintenance dose.

After receiving the last dose (e.g., the final loading dose, or any dosing that occurred last), the subject may retain a therapeutic result of the anti-VEGF therapy for a sustained period of time without the subject receiving a subsequent dose (e.g., a maintenance dose) of the antibody conjugate. A therapeutic result of the anti-VEGF therapy may include an improvement in one or more of visual acuity or retinal health (e.g., retinal thickness, extent of retinal perfusion, etc.) at or around the time of the final loading dose compared to before or at the time of the first loading dose. Any suitable therapeutic result of an anti-VEGF therapy may be used according to methods of the present disclosure. Suitable measures for determining therapeutic results include, e.g., visual acuity, retinal thickness, perfusion in at least one eye, diabetic retinopathy severity score (DRSS), disease activity of the eye disorder, or any combination thereof. In some embodiments, wAMD, DME, RVO, or DR disease activity includes one or more of increased intraretinal fluid, increased subretinal fluid, new intraretinal hemorrhage, new exudates.

In some embodiments, the therapeutic result includes an improvement, or at least a slowed decline, in visual acuity. Visual acuity may be monitored using any suitable method. In some embodiments, the visual acuity is measured by

best corrected visual acuity (BCVA) using e.g., ETDRS letters or Snellen chart, etc. In some embodiments, the therapeutic result may include an improvement in BCVA measured by ETDRS letters of 3 letters or more, e.g., 4 letters or more, 5 letters or more, 6 letters or more, 7 letters or more, 8 letters or more, 9 letters or more, 10 letters or more, 12 letters or more, 15 letters or more, 18 letters or more, 20 letters or more, 22 letters, or more, including 25 letters or more, or by a number within a range defined by any two of the preceding values, compared to pre-treatment. In some embodiments, the therapeutic result may include a reduction in the rate of deterioration of BCVA by at least 10%, e.g., at least 15%, at least 25%, at least 50%, at least 75%, at least 90%, including about 100%, or any percentage in a range defined by any two of the preceding values, over pre-treatment.

In some embodiments, the therapeutic result includes a reduction, or at least a slowed increase, in retinal thickness (e.g., central subfield thickness). The retinal thickness may be measured using any suitable method, including, but not limited to, optical coherence tomography (OCT) or OCT-A. In some embodiments, the therapeutic result may include a reduction in retinal thickness of about 25 μm or more, e.g., about 30 μm or more, about 40 μm or more, about 50 μm or more, about 75 μm or more, about 100 μm or more, about 125 μm or more, about 150 μm or more, about 175 μm or more, about 200 μm or more, about 225 μm or more, about 250 μm or more, about 275 μm or more, about 300 μm or more, about 325 μm or more, about 350 μm or more, about 375 μm or more, about 400 μm or more, or a reduction within a range defined by any two of the preceding values, compared to pre-treatment. In some embodiments, the therapeutic result may include a reduction in the rate of increase in retinal thickness by at least 10%, e.g., at least 15%, at least 25%, at least 50%, at least 75%, at least 90%, including about 100%, or any percentage in a range defined by any two of the preceding values, over pre-treatment.

In some embodiments, the therapeutic result includes improved perfusion, or at least a reduction in the rate of expansion of non-perfusion, of the retina. Perfusion may be monitored using any suitable method. Suitable methods include, without limitation, OCT-angiography (OCT-A), fluorescein angiogram or ultrawide-field fluorescein angiogram. The degree of perfusion, or non-perfusion, may be measured using any suitable measure. In some embodiments, non-perfusion area or area of capillary non-perfusion is measured. In some embodiments, an ischemic index is calculated by dividing the non-perfusion area by the total retinal area. In some embodiments, the presence or absence of retinal non-perfusion in retinal quadrants on the angiogram is measured. In some embodiments, the therapeutic result may include a reduction in the area of non-perfusion of at least 10%, e.g., at least 15%, at least 25%, at least 50%, at least 75%, at least 90%, including about 100%, or any percentage in a range defined by any two of the preceding values, over pre-treatment. In some embodiments, the therapeutic result may include a reduction in the rate of progressive non-perfusion of at least 10%, e.g., at least 15%, at least 25%, at least 50%, at least 75%, at least 90%, including about 100%, or any percentage in a range defined by any two of the preceding values, over pre-treatment.

In some embodiments, the therapeutic result includes improved, or prevented worsening of, diabetic retinopathy severity score (DRSS). In some embodiments, the therapeutic result may include an improved DRSS of 2 steps or more, or 3 steps or more compared to pre-treatment. In some embodiments, the therapeutic result may include preventing worsening of DRSS by 2 steps or more, or 3 steps or more compared to pre-treatment.

The therapeutic result is retained if the level of visual acuity or retinal health (e.g., retinal thickness, degree of non-perfusion, etc.) does not worsen by more than a predetermined amount compared to the improved level. In some embodiments, the therapeutic result is retained if the level of visual acuity or retinal health does not revert by 30% or more, e.g., 50% or more, 75% or more, 90% or more, including 100% or more to the pretreatment level of visual acuity or retinal health after the last dose (e.g., final loading dose). In some embodiments, the therapeutic result is retained if the rate of change of visual acuity or retinal health does not revert by 30% or more, e.g., 50% or more, 75% or more, 90% or more, including 100% or more to the pretreatment level of the rate of change of visual acuity or retinal health after the last dose (e.g., final loading dose).

In some embodiments, the therapeutic result includes an improvement in visual acuity. In some embodiments the therapeutic result may be retained if BCVA does not fall by 3 letters or more, 4 letters or more, 5 letters or more, 6 letters or more, 7 letters or more, 8 letters or more, 9 letters or more, or 10 letters or more from the BCVA score at the time of the final loading dose (e.g., at Week 12 after three monthly loading doses). In some embodiments the therapeutic result may be retained if BCVA does not fall by 3 letters or more, 4 letters or more, 5 letters or more, 6 letters or more, 7 letters or more, 8 letters or more, 9 letters or more, or 10 letters or more from the BCVA score measured at the last assessment (e.g., 4 weeks ago). In some embodiments the therapeutic result may be retained if BCVA does not fall by 3 letters or more, 4 letters or more, 5 letters or more, 6 letters or more, 7 letters or more, 8 letters or more, 9 letters or more, or 10 letters or more from the best measured BCVA score, or the average of the 2 best measured BCVA scores, of the subject.

In some embodiments, the therapeutic result includes a reduction in retinal thickness (e.g., central subfield thickness). In some embodiments, the therapeutic result may be retained if retinal thickness (e.g., central subfield thickness) does not increase by 25 μm or more, 30 μm or more, 40 μm or more, 50 μm or more, 75 μm or more, 100 μm or more, 125 μm or more, or 150 μm or more from the retinal thickness at the time of the last dose (e.g., final loading dose) (e.g., at Week 12 after three monthly loading doses). In some embodiments, the therapeutic result may be retained if retinal thickness (e.g., central subfield thickness) does not increase by 25 μm or more, 30 μm or more, 40 μm or more, 50 μm or

more, 75 μm or more, 100 μm or more, 125 μm or more, or 150 μm or more from the retinal thickness measured at the last assessment (e.g., 4 weeks ago). In some embodiments, a retained therapeutic result includes retinal thickness that is not greater than 150 μm, 125 μm, 100 μm, 75 μm, 50 μm, 40 μm, or 30 μm, compared to the lowest measured retinal thickness of the subject.

In some embodiments, the therapeutic result includes improved perfusion of the retina. In patients with DR, DME, and RVO, the retina can have an area of non-perfusion, or absence of blood flow. In some embodiments, non-perfusion is visualized on angiograms. In some embodiments, a therapeutic result of the anti-VEGF antibody conjugate, e.g., KSI-301, administration, or anti-VEGF protein conjugate (e.g., an aflibercept biopolymer conjugate) administration, according to methods of the present disclosure, includes regression of non-perfusion, or re-perfusion of the retina. In some embodiments, the therapeutic result may be retained if the area of non-perfusion is reduced by 10% or more, e.g., 15% or more, 25% or more, 50% or more, 75% or more, 90% or more, or about 100% relative to the area of non-perfusion at the time of the last dose (e.g., final loading dose). In some embodiments, the therapeutic result may be retained if the area of non-perfusion is not increased by 10% or more, e.g., 15% or more, 25% or more, 50% or more, 75% or more, 90% or more, or about 100% relative to the area of non-perfusion at the time of the last dose (e.g., final loading dose). In some embodiments, the therapeutic result may be retained if the area of non-perfusion is not increased by 10% or more, e.g., 15% or more, 25% or more, 50% or more, 75% or more, 90% or more, or about 100% relative to the area of non-perfusion measured at the last assessment (e.g., 4 weeks ago). In some embodiments, the therapeutic result may be retained if the area of non-perfusion is not increased by 10% or more, e.g., 15% or more, 25% or more, 50% or more, 75% or more, 90% or more, or about 100% relative to the smallest area of non-perfusion measured in the subject.

According to methods of the present disclosure, a therapeutic result of the anti-VEGF therapy after the last dose (e.g., final loading dose) may be retained for at least 8 weeks or more, e.g., at least 10 weeks or more, at least 12 weeks or more, at least 14 weeks or more, at least 16 weeks or more, at least 20 weeks or more, at least 24 weeks or more, at least 28 weeks or more, at least 32 weeks or more, at least 36 weeks or more, at least 40 weeks or more, at least 44 weeks or more, at least 48 weeks or more, including at least 52 weeks or more, after the last dose (e.g., final loading dose) was administered. In some embodiments, the therapeutic result may be retained for a time period of between 8 weeks to 1 year, e.g., between 8 weeks to 40 weeks, between 8 weeks to 32 weeks, including between 12 weeks to 28 weeks, after the final loading dose was administered. This can be for any one or more of the eye disorders provided herein, including, for example, RVO, DME, DR, and/or wAMD.

In some embodiments, the method includes administering one, two, or three loading doses of the anti-VEGF antibody conjugate (e.g., KSI-301), or anti-VEGF protein conjugate (e.g., an aflibercept biopolymer conjugate), to the subject, and administering one or more subsequent doses (e.g., maintenance doses) of the anti-VEGF antibody conjugate, e.g., KSI-301, (or anti-VEGF protein conjugate (e.g., an aflibercept biopolymer conjugate)) after the final loading dose. The subsequent doses may be administered to the subject no more frequently than once every 8 weeks, e.g., every 10 weeks, every 12 weeks, every 14 weeks, every 16 weeks, every 18 weeks, every 20 weeks, every 24 weeks, every 28 weeks, every 32 weeks, every 40 weeks, including every 52 weeks. Whether to administer a subsequent dose of the anti-VEGF antibody conjugate may be determined based on any convenient reason. In some embodiments, the subsequent dose may be administered based on a predetermined schedule (e.g., a schedule determined before the subject is administered any of the one or more of the loading doses). In some embodiments, the subsequent dose may be administered based on a predetermined schedule based on the severity of the eye disorder, the subject's previous response, or lack thereof, to other therapies for the eye disorder, or any other clinically relevant factors associated with the subject. In some embodiments, the subsequent dose may be administered based on the outcome of one or more assessment tests for ocular health and/or function carried out on the subject during the course of treatment with the anti-VEGF antibody conjugate. In some embodiments, the subsequent dose may be administered based on the outcome of one or more assessment tests carried out on the subject every 4 or more weeks, e.g., every 6 or more weeks, every 8 or more weeks, every 10 or more weeks, every 12 or more weeks, every 16 or more weeks, every 20 or more weeks, every 24 or more weeks, every 28 or more weeks, every 32 or more weeks, every 36 or more weeks, including every 40 or more weeks. In some embodiments, the subsequent dose may be administered if one or more assessment tests indicates a diminishment of the therapeutic result of the anti-VEGF therapy that is greater than a predetermined threshold.

As used herein, "Q4W", "Q8W" and the like refer to a dosing schedule, and have the ordinary and customary meaning to one of ordinary skill in the art. The number may indicate the number of the unit of time specified by the subsequent letter. "W" indicates a unit of a week; "M" specifies an interval of a month. Thus, Q4W refers to a dosing interval of 4 weeks, which also includes a dosing interval of one month; Q8W refers to a dosing interval of 8 weeks, which also includes a dosing interval of two months; and so on. As used herein, specification of a dosing schedule does not necessarily imply a number of doses beyond two, unless indicated otherwise. In some embodiments, a dosing schedule refers to the dosing schedule for maintenance doses (including the interval between the last loading dose, and the first maintenance dose). A reference to a dosing schedule being "longer" or "shorter" (e.g., "Q12W or longer") refers to the time interval between doses being longer than that specified (e.g., a dosing interval of 12 weeks or longer).

In some embodiments, the total number of injections (including loading and maintenance doses) of the anti-VEGF antibody conjugate (e.g., KSI-301) administered to the subject in the first year of treatment is 10 times or less, 9 times or less, 8 times or less, 7 times or less, 6 times or less, 5 times or less, 4 times or less, 3 times or less, 2 times or less, or once, in order to retain the therapeutic result of the anti-VEGF antibody conjugate therapy. As used herein, "first year of treatment" and similar reference to the first "X" years of treatment denotes the time period measured from the first dose (e.g., first loading dose) of the anti-VEGF antibody polymer conjugate administered to the subject under the particular treatment regimen or schedule. In some embodiments, the time period is measured from the first dose (e.g., first loading dose) of the anti-VEGF antibody polymer conjugate administered to a treatment naïve subject (e.g., a subject who has not received an anti-VEGF therapy before being administered the anti-VEGF antibody polymer conjugate of the present disclosure). In some embodiments, the time period is measured from the first dose (e.g., first loading dose) of the anti-VEGF antibody polymer conjugate administered to a subject who has previously been treated with an anti-VEGF therapy (e.g., an anti-VEGF therapy that does not include the anti-VEGF antibody polymer conjugate of the present disclosure) but has not responded to the anti-VEGF therapy. In some embodiments, the total number of injections (including loading and maintenance doses) of the anti-VEGF antibody conjugate (e.g., KSI-301) administered to the subject in the first two years of treatment is 10 times or less, 9 times or less, 8 times or less, 7 times or less, 6 times or less, 5 times or less, 4 times or less, 3 times or less, 2 times or less, or once, in order to retain the therapeutic result of the anti-VEGF antibody conjugate therapy. In some embodiments, the total number of injections (including loading and maintenance doses) of the anti-VEGF antibody conjugate (e.g., KSI-301) administered to the subject in the first three years of treatment is 10 times or less, 9 times or less, 8 times or less, 7 times or less, 6 times or less, 5 times or less, 4 times or less, 3 times or less, 2 times or less, or once, in order to retain the therapeutic result of the anti-VEGF antibody conjugate therapy. In any method of the present disclosure, in some embodiments, at least 4 doses (e.g., loading doses and any subsequent doses) of the anti-VEGF antibody polymer conjugate is administered to the subject in the first year of treatment.

In some embodiments, the total number of maintenance doses of the anti-VEGF antibody conjugate (e.g., KSI-301) administered to the subject in a one-year period for treatment is 7 times or less, 6 times or less, 5 times or less, 4 times or less, 3 times or less, 2 times or less, once or less, or zero, in order to retain the therapeutic result of the anti-VEGF antibody conjugate therapy. In some embodiments, the total number of maintenance doses of the anti-VEGF antibody conjugate (e.g., KSI-301) administered to the subject in a two-year period for treatment is 7 times or less, 6 times or less, 5 times or less, 4 times or less, 3 times or less, 2 times or less, once or less, or zero, in order to retain the therapeutic result of the anti-VEGF antibody conjugate therapy. In some embodiments, the total number of maintenance doses of the anti-VEGF antibody conjugate (e.g., KSI-301) administered to the subject in a three-year period for treatment is 7 times or less, 6 times or less, 5 times or less, 4 times or less, 3 times or less, 2 times or less, once or less, or zero, in order to retain the therapeutic result of the anti-VEGF antibody conjugate therapy.

In some embodiments, the total number of injections (including loading and maintenance doses) of the anti-VEGF antibody conjugate (e.g., KSI-301) administered to the subject with wAMD in the first year of treatment is 8 times or less, 7 times or less, 6 times or less, 5 times or less, 4 times or less, 3 times or less, 2 times or less, or once, in order to retain the therapeutic result of the anti-VEGF antibody conjugate therapy. In some embodiments, the total number of injections (including loading and maintenance doses) of the anti-VEGF antibody conjugate (e.g., KSI-301) administered to the subject with wAMD in the first two years of treatment is 8 times or less, 7 times or less, 6 times or less, 5 times or less, 4 times or less, 3 times or less, 2 times or less, or once, in order to retain the therapeutic result of the anti-VEGF antibody conjugate therapy. In some embodiments, the total number of injections (including loading and maintenance doses) of the anti-VEGF antibody conjugate (e.g., KSI-301) administered to the subject with wAMD in the first three years of treatment is 8 times or less, 7 times or less, 6 times or less, 5 times or less, 4 times or less, 3 times or less, 2 times or less, or once, in order to retain the therapeutic result of the anti-VEGF antibody conjugate therapy.

In some embodiments, the total number of maintenance doses of the anti-VEGF antibody conjugate (e.g., KSI-301) administered to the subject with wAMD in a one-year period for treatment is 4 times or less, 3 times or less, 2 times or less, once or less, or zero, in order to retain the therapeutic result of the anti-VEGF antibody conjugate therapy. In some embodiments, the total number of maintenance doses of the anti-VEGF antibody conjugate (e.g., KSI-301) administered to the subject with wAMD in a two-year period for treatment is 4 times or less, 3 times or less, 2 times or less, once or less, or zero, in order to retain the therapeutic result of the anti-VEGF antibody conjugate therapy. In some embodiments, the total number of maintenance doses of the anti-VEGF antibody conjugate (e.g., KSI-301) administered to the subject with wAMD in a three-year period for treatment is 4 times or less, 3 times or less, 2 times or less, once or less, or zero, in order to retain the therapeutic result of the anti-VEGF antibody conjugate therapy.

In some embodiments, the total number of injections (including loading and maintenance doses) of the anti-VEGF antibody conjugate (e.g., KSI-301) administered to the subject with DME in the first year of treatment is 8 times or less, 7 times or less, 6 times or less, 5 times or less, 4 times or less, 3 times or less, 2 times or less, or once, in order to retain the therapeutic result of the anti-VEGF antibody conjugate therapy. In some embodiments, the total number of injections (including loading and maintenance doses) of the anti-VEGF antibody conjugate (e.g., KSI-301) administered to the subject with DME in the first two years of treatment is 8 times or less, 7 times or less, 6 times or less, 5 times or less, 4 times or less, 3 times or less, 2 times or less, or once, in order to retain the therapeutic result of the anti-VEGF antibody conjugate

therapy. In some embodiments, the total number of injections (including loading and maintenance doses) of the anti-VEGF antibody conjugate (e.g., KSI-301) administered to the subject with DME in the first three years of treatment is 8 times or less, 7 times or less, 6 times or less, 5 times or less, 4 times or less, 3 times or less, 2 times or less, or once, in order to retain the therapeutic result of the anti-VEGF antibody conjugate therapy.

In some embodiments, the total number of maintenance doses of the anti-VEGF antibody conjugate (e.g., KSI-301) administered to the subject with DME in a one-year period during treatment is 4 times or less, 3 times or less, 2 times or less, once or less, or zero, in order to retain the therapeutic result of the anti-VEGF antibody conjugate therapy. In some embodiments, the total number of maintenance doses of the anti-VEGF antibody conjugate (e.g., KSI-301) administered to the subject with DME in a two-year period during treatment is 4 times or less, 3 times or less, 2 times or less, once or less, or zero, in order to retain the therapeutic result of the anti-VEGF antibody conjugate therapy. In some embodiments, the total number of maintenance doses of the anti-VEGF antibody conjugate (e.g., KSI-301) administered to the subject with DME in a three-year period during treatment is 4 times or less, 3 times or less, 2 times or less, once or less, or zero, in order to retain the therapeutic result of the anti-VEGF antibody conjugate therapy.

In some embodiments, the total number of injections (including loading and maintenance doses) of the anti-VEGF antibody conjugate (e.g., KSI-301) administered to the subject with RVO in the first year of treatment is 8 times or less, 7 times or less, 6 times or less, 5 times or less, 4 times or less, 3 times or less, 2 times or less, or once, in order to retain the therapeutic result of the anti-VEGF antibody conjugate therapy. In some embodiments, the total number of injections (including loading and maintenance doses) of the anti-VEGF antibody conjugate (e.g., KSI-301) administered to the subject with RVO in the first two years of treatment is 8 times or less, 7 times or less, 6 times or less, 5 times or less, 4 times or less, 3 times or less, 2 times or less, or once, in order to retain the therapeutic result of the anti-VEGF antibody conjugate therapy. In some embodiments, the total number of injections (including loading and maintenance doses) of the anti-VEGF antibody conjugate (e.g., KSI-301) administered to the subject with RVO in the first three years of treatment is 8 times or less, 7 times or less, 6 times or less, 5 times or less, 4 times or less, 3 times or less, 2 times or less, or once, in order to retain the therapeutic result of the anti-VEGF antibody conjugate therapy.

In some embodiments, the total number of maintenance doses of the anti-VEGF antibody conjugate (e.g., KSI-301) administered to the subject with DME in a one-year period during treatment is 5 times or less, 4 times or less, 3 times or less, 2 times or less, once or less, or zero, in order to retain the therapeutic result of the anti-VEGF antibody conjugate therapy. In some embodiments, the total number of maintenance doses of the anti-VEGF antibody conjugate (e.g., KSI-301) administered to the subject with DME in a two-year period during treatment is 5 times or less, 4 times or less, 3 times or less, 2 times or less, once or less, or zero, in order to retain the therapeutic result of the anti-VEGF antibody conjugate therapy. In some embodiments, the total number of maintenance doses of the anti-VEGF antibody conjugate (e.g., KSI-301) administered to the subject with DME in a three-year period during treatment is 5 times or less, 4 times or less, 3 times or less, 2 times or less, once or less, or zero, in order to retain the therapeutic result of the anti-VEGF antibody conjugate therapy.

In some embodiments, a first subsequent dose is administered at a first time after the last loading dose, and a second subsequent dose is administered at a second period of time after the first subsequent dose, where no other dose is administered between the last loading dose and the first subsequent dose, or between the first subsequent dose and the second subsequent dose. The second period of time between the first and second subsequent doses may be the same or different from the first period of time between the last loading dose and the first subsequent dose. In some embodiments, the first time period is 8 weeks or more, e.g., 10 weeks or more, 12 weeks or more, 14 weeks or more, 16 weeks or more, 18 weeks or more, 20 weeks or more, 24 weeks or more, 28 weeks or more, 32 weeks or more, 36 weeks or more, 40 weeks or more, at least 44 weeks or more, at least 48 weeks or more, including 52 weeks or more. In some embodiments, the second period of time is longer than the first period of time by 0 weeks or more, e.g., by 4 weeks or more, by 6 weeks or more, by 8 weeks or more, by 10 weeks or more, by 12 weeks or more, by 16 weeks or more, by 20 weeks or more, including by 24 weeks or more. The timing for administering the second subsequent dose may depend on the outcome of one or more assessments for ocular health and/or function of the subject.

Any suitable amount of the anti-VEGF antibody conjugate, or anti-VEGF protein conjugate (e.g., an aflibercept biopolymer conjugate), may be administered to the subject in a subsequent dose (e.g., maintenance dose). In some embodiments, the subsequent dose includes about 1 mg or more, e.g., about 1.25 mg or more, about 1.5 mg or more, about 1.75 mg or more, about 2 mg or more, about 2.5 mg or more, about 3 mg or more, about 3.5 mg or more, about 4 mg or more, about 4.5 mg of more, including about 5 mg or more (by weight of the anti-VEGF antibody portion) of the anti-VEGF antibody conjugate. In some embodiments, the subsequent dose includes from about 1 mg to about 10 mg, e.g., about 1 mg to about 7.5 mg, about 1.25 mg to about 5 mg, including about 2 mg to about 5 mg (by weight of the anti-VEGF antibody portion) of the anti-VEGF antibody conjugate.

As the therapeutic result of the anti-VEGF therapy is retained for a sustained period of time after the last dose (e.g., final loading dose), the subject may not need to receive a dose of the anti-VEGF antibody conjugate, or anti-VEGF protein conjugate (e.g., an aflibercept biopolymer conjugate), while the therapeutic effect lasts. In some embodiments, no further administration of the anti-VEGF antibody conjugate, or anti-VEGF protein conjugate (e.g., an aflibercept

biopolymer conjugate), is made to the subject within 4 weeks or more, e.g., within 6 weeks or more, within 8 weeks or more, within 10 weeks or more, within 12 weeks or more, within 14 weeks or more, within 16 weeks or more, within 20 weeks or more, within 24 weeks or more, within 28 weeks or more, within 32 weeks or more, within 36 weeks or more, within 40 weeks or more, within 44 weeks or more, within 48 weeks or more, including within 52 weeks or more, after the last dose (e.g., final loading dose).

A method of the present disclosure can include administering a fewer number of injections (of the anti-VEGF antibody conjugate, such as KSI-301, or anti-VEGF protein conjugate (e.g., an aflibercept biopolymer conjugate)) after the last loading dose compared to the number of injections in a standard of care treatment, to maintain a therapeutic result. In some embodiments, the average number of injections (e.g., of the anti-VEGF antibody conjugate) administered after the last loading dose is about 1 or less, about 0.9 or less, about 0.8 or less, about 0.7 or less, about 0.5 or less, about 0.45 or less, about 0.4 or less, about 0.35 or less, about 0.3 or less, about 0.25 or less, about 0.2 or less, about 0.18 or less, about 0.17 or less, or a number within a range defined by any two of the preceding values, over 16 weeks. In some embodiments, the average number of injections (e.g., of the anti-VEGF antibody conjugate) administered after the last loading dose is about 1 or less, about 0.9 or less, about 0.8 or less, about 0.7 or less, about 0.5 or less, about 0.45 or less, about 0.4 or less, about 0.35 or less, about 0.3 or less, about 0.25 or less, about 0.2 or less, about 0.18 or less, about 0.17 or less, or a number within a range defined by any two of the preceding values, over 12 weeks. In some embodiments, the average number of injections (of the anti-VEGF antibody conjugate, e.g., KSI-301) administered after the last loading dose is about 1 or less, about 0.9 or less, about 0.8 or less, about 0.7 or less, about 0.5 or less, about 0.45 or less, about 0.4 or less, about 0.35 or less, about 0.3 or less, about 0.25 or less, about 0.2 or less, about 0.18 or less, about 0.17 or less, or a number within a range defined by any two of the preceding values, over 8 weeks.

In some embodiments, the subject has DME, and the average number of injections (of the anti-VEGF antibody conjugate, e.g., KSI-301) administered after the last loading dose is about 1.75 or less, about 1.5 or less, about 1.25 or less, about 1 or less, about 0.9 or less, about 0.8 or less, about 0.7 or less, about 0.5 or less, about 0.45 or less, about 0.4 or less, about 0.35 or less, about 0.3 or less, about 0.25 or less, about 0.2 or less, or a number within a range defined by any two of the preceding values, over 16 weeks. In some embodiments, the subject has DME, and the average number of injections (of the anti-VEGF antibody conjugate, e.g., KSI-301) administered after the last loading dose is about 1.75 or less, about 1.5 or less, about 1.25 or less, about 1 or less, about 0.9 or less, about 0.8 or less, about 0.7 or less, about 0.5 or less, about 0.45 or less, about 0.4 or less, about 0.35 or less, about 0.3 or less, about 0.25 or less, about 0.2 or less, or a number within a range defined by any two of the preceding values, over 12 weeks. In some embodiments, the subject has DME, and the average number of injections (of the anti-VEGF antibody conjugate, e.g., KSI-301) administered after the last loading dose is about 1.75 or less, about 1.5 or less, about 1.25 or less, about 1 or less, about 0.9 or less, about 0.8 or less, about 0.7 or less, about 0.5 or less, about 0.45 or less, about 0.4 or less, about 0.35 or less, about 0.3 or less, about 0.25 or less, about 0.2 or less, or a number within a range defined by any two of the preceding values, over 8 weeks.

In some embodiments, the subject has RVO, and the average number of injections (of the anti-VEGF antibody conjugate, e.g., KSI-301) administered after the last loading dose is about 2.75 or less, about 2.5 or less, about 2.25 or less, about 2 or less, about 1.75 or less, about 1.5 or less, about 1.25 or less, about 1 or less, about 0.9 or less, about 0.8 or less, about 0.7 or less, about 0.5 or less, about 0.45 or less, or a number within a range defined by any two of the preceding values, over 16 weeks. In some embodiments, the subject has RVO, and the average number of injections (of the anti-VEGF antibody conjugate, e.g., KSI-301) administered after the last loading dose is about 2.75 or less, about 2.5 or less, about 2.25 or less, about 2 or less, about 1.75 or less, about 1.5 or less, about 1.25 or less, about 1 or less, about 0.9 or less, about 0.8 or less, about 0.7 or less, about 0.5 or less, about 0.45 or less, or a number within a range defined by any two of the preceding values, over 12 weeks. In some embodiments, the subject has RVO, and the average number of injections (of the anti-VEGF antibody conjugate, e.g., KSI-301) administered after the last loading dose is about 2.75 or less, about 2.5 or less, about 2.25 or less, about 2 or less, about 1.75 or less, about 1.5 or less, about 1.25 or less, about 1 or less, about 0.9 or less, about 0.8 or less, about 0.7 or less, about 0.5 or less, about 0.45 or less, or a number within a range defined by any two of the preceding values, over 8 weeks.

In some embodiments, the eye disorder treated by the present methods include one or more of age-related macular degeneration (AMD), diabetic macular edema (DME), retinal vein occlusion (RVO) (e.g., central retinal vein occlusion (CRVO) and branched central retinal vein occlusion (BRVO)), diabetic retinopathy (DR) (e.g., non-proliferative DR and proliferative DR) and presumed ocular histoplasmosis syndrome. In some embodiment, a subject to be treated by methods of the present disclosure has wet AMD. In some embodiments, the subject has wet AMD without a high pigment epithelial detachment (PED). In some embodiments, a subject has high PED when the baseline central subfield retinal thickness (CST) in an eye of the subject is 500 microns or greater.

In some embodiments, a subject with the eye disorder has, before receiving a treatment according to methods of the present disclosure, a CST of about 200 microns or more, about 250 microns or more, about 275 microns or more, about 300 microns or more, about 325 microns or more, about 350 microns or more, about 375 microns or more, about 400 microns or more, about 425 microns or more, about 450 microns or more, about 475 microns or more, about 500 microns or more, about 525 microns or more, about 550 microns or more, about 575 microns or more, about 600 microns or more,

about 625 microns or more, about 650 microns or more, about 675 microns or more, about 700 microns or more, about 725 microns or more, about 750 microns or more, about 775 microns or more, about 800 microns or more, about 825 microns or more, about 850 microns or more, about 875 microns or more, about 900 microns or more, or a distance within a range defined by any two of the preceding values. The CST can be measured by, e.g., optical coherence tomography (OCT) or OCT-A.

In some embodiments, a subject with the eye disorder has, before receiving a treatment according to methods of the present disclosure, a BCVA, in ETDRS letters, of about 80 or less, about 75 or less, about 70 or less, about 68 or less, about 66 or less, about 64 or less, about 62 or less, about 60 or less, about 58 or less, about 56 or less, about 54 or less, about 52 or less, about 50 or less, about 48 or less, about 46 or less, about 44 or less, about 42 or less, about 40 or less, or value within a range defined by any two of the preceding values.

In some embodiments, a method of the present disclosure includes administering a first loading dose of an anti-VEGF antibody conjugate (e.g., KSI-301) (or anti-VEGF protein conjugate (e.g., an aflibercept biopolymer conjugate)) to a subject (e.g., human or other mammalian patient) in need of treating wAMD or DME, and subsequently administering at least one more, but no more than two more of the loading doses to achieve a therapeutic result (e.g., improved vision, reduced symptoms, etc.) of the anti-VEGF therapy, where the therapeutic result is retained for at least 12 weeks after the final loading dose was administered. In some embodiments, the therapeutic result is retained for at least 14 weeks, e.g., at least 16 weeks, at least 20 weeks, at least 24 weeks, at least 28 weeks, at least 32 weeks, at least 36 weeks, at least 40 weeks, at least 44 weeks, at least 48 weeks, including at least 52 weeks after the final loading dose was administered. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, the eye disorder is wAMD and the method includes administering a subsequent dose of the anti-VEGF antibody conjugate, e.g., KSI-301, or anti-VEGF protein conjugate (e.g., an aflibercept biopolymer conjugate), after about 24 months of the final loading dose. In some embodiment, the eye disorder is wet AMD without a high pigment epithelial detachment (PED). In some embodiments, a subject has high PED when the baseline central subfield retinal thickness (CST) in an eye of the subject is 500 microns or greater. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a subject treated by the present methods has RVO. In some embodiments, a method of the present disclosure includes administering a first loading dose of an anti-VEGF antibody conjugate (e.g., KSI-301), or anti-VEGF protein conjugate (e.g., an aflibercept biopolymer conjugate), to a subject (e.g., human patient) in need of treating RVO, and subsequently administering one more loading dose to achieve a therapeutic result (e.g., improved vision, reduced symptoms, etc.) of the anti-VEGF therapy, where the therapeutic result is retained for at least 8 weeks after the final loading dose was administered. In some embodiments, the therapeutic result is retained for at least 10 weeks, e.g., at least 12 weeks, at least 14 weeks, at least 16 weeks, at least 20 weeks, at least 24 weeks, at least 28 weeks, at least 32 weeks, at least 36 weeks, at least 40 weeks, including at least 52 weeks after the final loading dose was administered. In some embodiments, the method further includes administering one or more subsequent doses (e.g., maintenance doses) of the anti-VEGF antibody conjugate, e.g., KSI-301, after the final loading dose. The subsequent doses may be administered to the subject no more frequently than once every 8 weeks, e.g., every 10 weeks, every 12 weeks, every 14 weeks, every 16 weeks, every 18 weeks, every 20 weeks, every 24 weeks, every 28 weeks, every 32 weeks, every 36 weeks, every 40 weeks, every 44 weeks, every 48 weeks, including every 52 weeks. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

Also provided herein are methods of treating a subject with an eye disorder, where the method includes administering between 1 to 3 loading doses, but no more than 3 loading doses, of an anti-VEGF antibody conjugate (e.g., KSI-301), or anti-VEGF protein conjugate (e.g., an aflibercept biopolymer conjugate), to a subject, and providing a follow-on application of the anti-VEGF antibody conjugate or anti-VEGF protein conjugate no sooner than 14 weeks after the last loading dose was administered. In some embodiments, the follow-on application of the anti-VEGF antibody conjugate or anti-VEGF protein conjugate is provided no sooner than 16 weeks, e.g., no sooner than 18 weeks, no sooner than 20 weeks, no sooner than 24 weeks, no sooner than 28 weeks, no sooner than 32 weeks, no sooner than 36 weeks, including no sooner than 40 weeks after the last loading dose was administered. In some embodiments, the eye disorder treated is DME. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

Also provided are methods of treating a subject for an eye disorder, where the method includes administering to a subject in need of treating an eye disorder a monthly loading dose of an anti-VEGF antibody conjugate (e.g., KSI-301), or anti-VEGF protein conjugate (e.g., an aflibercept biopolymer conjugate), over one, two or three months, and administering one or more subsequent doses of the anti-VEGF antibody conjugate no less than every 8 weeks after the final loading dose. In some embodiments, the eye disorder is AMD (e.g., wAMD), DME, RVO or DR. In some embodiments, the eye disorder is RVO. In some embodiments, the eye disorder is wAMD, DME or DR, and the subsequent doses of the anti-VEGF antibody conjugate or anti-VEGF protein conjugate is administered no less than every 12 weeks, e.g., every 16 weeks, every 20 weeks, every 24 weeks, every 28 weeks, every 32 weeks, every 36 weeks, every 40 weeks, including every 52 weeks after the final loading dose. In some embodiments, the eye disorder is wAMD, and the subsequent doses of the anti-VEGF antibody conjugate or anti-VEGF protein conjugate is administered no less than every 12 weeks. In some embodiment, the eye disorder is wet AMD without a high pigment epithelial detachment (PED). In some embodiments, a subject has high PED when the baseline central subfield retinal thickness (CST) in an eye of the subject is 500 microns or greater. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of the present disclosure includes administering at least one and up to three loading doses of an anti-VEGF antibody conjugate (e.g., KSI-301), or anti-VEGF protein conjugate (e.g., an aflibercept biopolymer conjugate), to a subject in need of treating an eye disorder, to thereby achieve a therapeutic result of the anti-VEGF therapy, assessing the ocular health of the subject after the final loading dose to determine whether the therapeutic result of the anti-VEGF therapy is retained, and not administering any subsequent doses of the anti-VEGF antibody conjugate or anti-VEGF protein conjugate for at least 8 weeks after the final loading dose until upon a determination that the anti-VEGF therapy is no longer retained. In some embodiments, the eye disorder is AMD (e.g., wAMD), DME or RVO. In some embodiments, the eye disorder is RVO. In some embodiments, the eye disorder is wAMD or DME, and the subsequent doses of the anti-VEGF antibody conjugate or anti-VEGF protein conjugate is administered at least 12 weeks after the final loading dose. In some embodiments, the eye disorder is wAMD and the method includes administering a subsequent dose of the anti-VEGF antibody conjugate, e.g., KSI-301, or anti-VEGF protein conjugate (e.g., an aflibercept biopolymer conjugate), after about 24 months of the final loading dose. In some embodiment, the eye disorder is wet AMD without a high pigment epithelial detachment (PED). In some embodiments, a subject has high PED when the baseline central subfield retinal thickness (CST) in an eye of the subject is 500 microns or greater. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, a method of the present disclosure includes administering at least one and up to three loading doses of an anti-VEGF antibody conjugate (e.g., KSI-301), or anti-VEGF protein conjugate (e.g., an aflibercept biopolymer conjugate), to a subject in need of treating an eye disorder, to achieve a therapeutic result (e.g., improved vision, reduced symptoms, etc.) of the anti-VEGF therapy, and administering one or more subsequent doses of the anti-VEGF antibody conjugate or anti-VEGF protein conjugate no less than every 8 weeks after the final loading dose, where the therapeutic result of the anti-VEGF therapy is retained as effectively as administering the one or more subsequent doses of the anti-VEGF antibody conjugate or anti-VEGF protein conjugate every 4 weeks after the final loading dose. In some embodiments, the eye disorder is AMD (e.g., wAMD), DME or RVO. In some embodiments, the eye disorder is RVO. In some embodiments, the eye disorder is wAMD or DME, and the subsequent doses of the anti-VEGF antibody conjugate is administered no less than every 12 weeks after the final loading dose, where the therapeutic result of the anti-VEGF therapy is retained as effectively as administering the one or more subsequent doses of the anti-VEGF antibody conjugate every 4 weeks after the final loading dose. In some embodiments, the eye disorder is wAMD and the method includes administering a subsequent dose of the anti-VEGF antibody conjugate, e.g., KSI-301, or anti-VEGF protein conjugate (e.g., an aflibercept biopolymer conjugate), after about 24 months of the final loading dose. In some embodiment, the eye disorder is wet AMD without a high pigment epithelial detachment (PED). In some embodiments, a subject has high PED when the baseline central subfield retinal thickness (CST) in an eye of the subject is 500 microns or greater. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

According to some embodiments, a method of the present disclosure includes administering at least one and up to three loading doses of an anti-VEGF antibody conjugate (e.g., KSI-301), or anti-VEGF protein conjugate (e.g., an aflibercept biopolymer conjugate), to a subject in need of treating an eye disorder, to achieve a therapeutic result (e.g., improved vision, reduced symptoms, etc.) of the anti-VEGF therapy, and administering one or more subsequent doses of the anti-VEGF antibody conjugate no less than every 12 weeks after the final loading dose, where the therapeutic result of the anti-VEGF therapy is retained as effectively as the therapeutic result a standard or care treatment for the eye disorder, where the standard of care treatment includes administering three monthly loading doses of a standard of care therapeutic, and administering one or more subsequent doses of the standard of care therapeutic every 8 weeks, or every 4 weeks, after the final loading dose of the standard of care therapeutic. In some embodiments, the eye disorder is wAMD. In some embodiment, the eye disorder is wet AMD without a high pigment epithelial detachment (PED). In some embodiments, a subject has high PED when the baseline central subfield retinal thickness (CST) in an eye of the subject is 500 microns or greater. In some embodiments, the standard of care therapeutic is aflibercept. In some embodiments, the subsequent doses of the anti-VEGF antibody conjugate is administered no less than every 16 weeks, e.g., no less than every 20 weeks, no less than every 24 weeks, no less than every 28 weeks, including no less than every 32 weeks, after the final loading dose of the anti-VEGF antibody conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11,

12, 13, or 14 days.

In some embodiments, provided herein are methods of reperfusion of an eye in a subject suffering from DME, where the method includes identifying the subject with DME, DR or RVO, administering at least 2 loading doses of an anti-VEGF antibody conjugate (e.g., KSI-301), or anti-VEGF protein conjugate (e.g., an aflibercept biopolymer conjugate), to the subject, and providing one or more further doses of the anti-VEGF antibody conjugate or anti-VEGF protein conjugate to the subject until the subject displays reperfusion in at least one eye. Reperfusion of an eye may be monitored using any suitable measure. In some embodiments, reperfusion of the eye is determined by an increase in blood vessel count, e.g., in retinal tissue, compared to a pretreatment state. The reperfusion of the eye may be monitored using any suitable method, including, but not limited to, OCT-angiography (OCT-A), fluorescein angiogram or ultrawide-field fluorescein angiogram. In some embodiments, the method provides for improved perfusion of an eye as measured by reduction in the area of non-perfusion of at least 10%, e.g., at least 20%, at least 30%, at least 50%, at least 75%, at least 90%, including about 100%, over a pre-treatment area of non-perfusion. In some embodiments, the method provides for reperfusion of an eye as measured by a reduction in the area of non-perfusion of between 10% and 100%, e.g., between 10% and 75%, between 10% and 50%, including between 10% and 30%, over a pre-treatment area of non-perfusion. In some embodiments, the improved perfusion provides at least 10% recovery, e.g., at least 20% recovery, at least 30% recovery, at least 50% recovery, at least 75% recovery, at least 90% recovery, including approximately 100% recovery of visual acuity in the subject over the pretreatment level. In some embodiments, the improved perfusion provides between 10% and 100% recovery, e.g., between 10% and 90% recovery, between 10% and 75% recovery, between 10% and 50% recovery, including between 10% and 30% recovery of visual acuity in the subject over the pretreatment level. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, the loading doses may be administered to the subject at any suitable time interval to achieve the desired therapeutic result. In some embodiments, the loading doses are administered with 3 weeks or more, e.g., 4 weeks or more, one month or more, 5 weeks or more, 6 weeks or more, 8 weeks or more, 12 weeks or more, including 16 weeks or more between each loading dose. Where there are more than two loading doses, the time period between each loading dose may be the same or may be different. In some embodiments, some of the loading doses may be administered at the same interval, and some other loading doses may be administered at a different interval. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, any suitable amount of the anti-VEGF antibody conjugate or anti-VEGF protein conjugate may be administered to the subject in a loading dose. In some embodiments, the loading dose includes about 1 mg or more, e.g., about 1.25 mg or more, about 1.5 mg or more, about 1.75 mg or more, about 2 mg or more, about 2.5 mg or more, about 3 mg or more, about 3.5 mg or more, about 4 mg or more, about 4.5 mg of more, including about 5 mg or more (by weight of the anti-VEGF antibody portion) of the anti-VEGF antibody conjugate. In some embodiments, the loading dose includes from about 1 mg to about 10 mg, e.g., about 1 mg to about 7.5 mg, about 1.25 mg to about 5 mg, including about 2 mg to about 5 mg (by weight of the anti-VEGF antibody portion) of the anti-VEGF antibody conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

Also provided herein are methods of treating an eye disorder by administering an anti-VEGF antibody conjugate (e.g., KSI-301), or anti-VEGF protein conjugate (e.g., an aflibercept biopolymer conjugate), to a subject in need of treating an eye disorder, where a single dose of the anti-VEGF antibody conjugate or anti-VEGF protein conjugate is sufficient to obtain a therapeutic effect of the anti-VEGF therapy. In some embodiments, a lasting therapeutic effect is obtained by a single dose of the anti-VEGF antibody conjugate or anti-VEGF protein conjugate, without administering any loading dose (e.g., without having a loading phase with monthly loading doses in the treatment schedule). In some embodiments, the eye disorder is non-proliferative DR. In some embodiments, the VEGF antibody conjugate or anti-VEGF protein is administered to a patient having non-proliferative DR to improve perfusion in at least one eye of the subject. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

Any suitable amount of the anti-VEGF antibody conjugate or anti-VEGF protein conjugate may be administered to the subject in a dose. In some embodiments, the dose includes 1 mg or more, e.g., 1.25 mg or more, 1.5 mg or more, 1.75 mg or more, 2 mg or more, 2.5 mg or more, 3 mg or more, 3.5 mg or more, 4 mg or more, 4.5 mg of more, including 5 mg or more (by weight of the anti-VEGF antibody portion) of the anti-VEGF antibody conjugate. In some embodiments, the dose includes from 1 mg to 10 mg, e.g., 1 mg to 7.5 mg, 1.25 mg to 5 mg, including 2 mg to 5 mg (by weight of the anti-VEGF antibody portion) of the anti-VEGF antibody conjugate. In some embodiments, the dosing is approximate. In some embodiments, the dosing is no sooner than the designated point in time. In some embodiments, the dosing is ±1, 2, 3, 4, 5, 6, 7, 8, 9, 10, 11, 12, 13, or 14 days.

In some embodiments, methods of the present disclosure provide for an anti-VEGF therapy for an eye disorder, where there is reduced risk of intraocular inflammation (e.g., blepharitis, infectious conjunctivitis, keratitis, scleritis,

endophthalmitis). In some embodiments, intravitreal administration of the anti-VEGF antibody conjugate (e.g., KSI-301), or anti-VEGF protein conjugate (e.g., an aflibercept biopolymer conjugate), is associated with a reduced risk of intraocular inflammation, e.g., compared to a standard of care treatment for the eye disorder. In some embodiments, intravitreal administration of the anti-VEGF antibody conjugate (e.g., KSI-301), or anti-VEGF protein conjugate (e.g., an aflibercept biopolymer conjugate), does not cause intraocular inflammation. In some embodiments, the dosing is approximate. In some embodiments, the dosing is no sooner than the designated point in time. In some embodiments, the dosing is +1, 2, 3, 4, 5, 6, 7, 8, 9, 10, 11, 12, 13, or 14 days.

In some embodiments, retreating wAMD involves one or more of:

Increase in CST ≥75 μm with a decrease in BCVA of ≥ 5 letters compared to Week 12, *OR*
Decrease in BCVA of > 5 letters compared to Day 1, due to worsening wAMD activity, *OR*
Decrease in BCVA of ≥ 10 letters compared to the best prior BCVA, due to worsening wAMD activity.

In some embodiments, retreating wAMD involves one or more of:

Increase in CST ≥ 50 μm with a decrease in BCVA of ≥ 5 letters compared to Week 12, *OR*
Decrease in BCVA of > 10 letters compared to the best prior BCVA, due to worsening wAMD activity, OR
Increase of ≥ 75 microns compared to Week 12, *OR*
New Macular Hemorrhage.

In some embodiments, retreating DME and RVO involves one or more of:

Increase in CST ≥75 μm with a decrease in BCVA of ≥ 5 letters compared to Week 12 or the prior visit, *OR*
Decrease in BCVA of ≥ 10 letters compared to the best prior BCVA, due to worsening DME/RVO disease activity.

In some embodiments, no more than 0, 1, 2, 3, 4, 5, or 6 retreatment events is required for a one year duration for a subject, including, over a 1, 2, 3 or 4 year duration.

In some embodiments, retreatment occurs when one or more of the above criteria occurs. In some embodiments, a retreatment is a treatment that follows the last loading dose or treatment for a subject. A loading dose is a dose that is provided initially to bring the amount of drug in the patient or subject up to a desired level to have an initial therapeutic effect. In contrast, a retreatment dose, or maintenance dose, is a dose that is provided to return the therapeutic effect of the drug to the subject, after a prior dose (last loading dose or retreatment/maintenance dose) has degraded in effectiveness (or there is reactivation of the disease), or on a pre-determined interval.

In some embodiments, the method involves administering a re-treatment with intravitreal injection of an antibody or conjugate thereof (e.g., KSI-301) if at least one of the following re-treatment criteria is met. In some embodiments, the re-treatment criteria includes an increase in OCT or OCT-A central subfield retinal thickness (CST) ≥ 30 μm, 40 μm, 50 μm, 60 μm, 70 μm, 75 μm or more, or an increase in thickness in a range defined by any two of the preceding values. In some embodiments, the criteria are related to signs of disease recurrence and/or vision loss due to disease recurrence. In some embodiments, the subject has wAMD and the criteria is one or more of: increase in OCT or OCT-A central subfield retinal thickness (CST) ≥ 75 μm with a decrease in BCVA of ≥ 5 letters compared to Week 12; decrease in BCVA of ≥ 10 letters compared to Day 1, due to worsening wAMD disease activity (e.g. increased intraretinal fluid, increased subretinal fluid, new intraretinal hemorrhage, new subretinal hemorrhage); and/or decrease in BCVA of ≥ 10 letters compared to the best prior BCVA, due to worsening wAMD disease activity. In some embodiments, the subject has wAMD and the criteria is one or more of: increase in OCT or OCT-A central subfield retinal thickness (CST) ≥ 40 μm with a decrease in BCVA of ≥ 5 letters compared to Week 12; decrease in BCVA of ≥ 10 letters compared to Day 1, due to worsening wAMD disease activity (e.g. increased intraretinal fluid, increased subretinal fluid, new intraretinal hemorrhage, new subretinal hemorrhage); and/or decrease in BCVA of ≥ 10 letters compared to the best prior BCVA, due to worsening wAMD disease activity. In some embodiments, the subject has DME or RVO, and the criteria is one or more of: increase in OCT or OCT-A central subfield retinal thickness (CST) ≥ 75 μm with a decrease in BCVA of ≥ 5 letters compared to Week 12 or the prior visit (4-week span between visits); and/or decrease in BCVA of ≥ 10 letters compared to the best prior BCVA, due to worsening DME/RVO disease activity (e.g. increased intraretinal fluid, increased subretinal fluid, new intraretinal hemorrhage, new exudates). In some embodiments, the subject has DME or RVO, and the criteria is one or more of: increase in OCT or OCT-A central subfield retinal thickness (CST) ≥ 40 μm with a decrease in BCVA of ≥ 5 letters compared to Week 12 or the prior visit (4-week span between visits); and/or decrease in BCVA of ≥ 10 letters compared to the best prior BCVA, due to worsening DME/RVO disease activity (e.g. increased intraretinal fluid, increased subretinal fluid, new intraretinal hemorrhage, new exudates).

In some embodiments, the method involves administering a re-treatment with intravitreal injection of an antibody or conjugate thereof (e.g., KSI-301) if at least one of the following re-treatment criteria is met. In some embodiments, the

criteria are related to signs of disease recurrence and/or vision loss due to disease recurrence. In some embodiments, the subject has wAMD, and the criteria is one or more of: increase in OCT or OCT-A central subfield retinal thickness (CST) ≥50 μm with a decrease in BCVA of ≥ 5 letters compared to Week 12; decrease in BCVA of ≥ 10 letters compared to the best prior BCVA, due to worsening wAMD disease activity (e.g. increased intraretinal fluid, increased subretinal fluid, new intraretinal hemorrhage, new subretinal hemorrhage); increase in OCT or OCT-A central subfield retinal thickness (CST) ≥ 75 μm; and/or new macular hemorrhage. In some embodiments, the subject has wAMD, and the criteria is one or more of: increase in OCT or OCT-A central subfield retinal thickness (CST) ≥40 μm with a decrease in BCVA of ≥ 5 letters compared to Week 12; decrease in BCVA of ≥ 10 letters compared to the best prior BCVA, due to worsening wAMD disease activity (e.g. increased intraretinal fluid, increased subretinal fluid, new intraretinal hemorrhage, new subretinal hemorrhage); increase in OCT or OCT-A central subfield retinal thickness (CST) ≥ 40 μm; and/or new macular hemorrhage.

In some embodiments, no retreatment dose is given to a subject for at least 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52 or more weeks following the last loading dose. In some embodiments, 1 loading dose is administered and no retreatment dose is given to a subject for at least 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52 or more weeks following the loading dose. In some embodiments, 2 loading doses are given to a subject and no retreatment dose is given for at least 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52 or more weeks following the last loading dose. In some embodiments, 3 loading doses are given to a subject and no retreatment dose is given for at least 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52 or more weeks following the last loading dose. In some embodiments, 1-2 loading doses are given to a subject and no retreatment dose is given for at least 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52 or more weeks following the last loading dose. In some embodiments, 1-3 loading doses are given to a subject and 1, 2, 3, or 4 retreatment dose are given for at least 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52 or more weeks following the last loading dose. In some embodiments, no more than 2, 3, 4, or 5 loading doses are administered to a subject. In some embodiments, no $4^{th}$ loading dose is provided to the subject. In some embodiments, the above treatment approaches allow for the subject's vision to stay improved at or close to the level achieved following the $1^{st}$ $2^{nd}$ or $3^{rd}$ loading dose. In some embodiments, the above treatment approaches allow for the subject's vision to stay improved to the point of not requiring a retreatment of the subject.

In some embodiments, the methods of the present disclosure can provide a long dosing interval and still sustain a therapeutic result of the anti-VEGF antibody conjugate (e.g., KSI-301) therapy, or anti-VEGF protein conjugate (e.g., an aflibercept biopolymer conjugate) therapy. In some embodiments, at least 75% of patients treated according to methods of the present disclosure can be on a Q4M dosing interval. In some embodiments, at least 90% of patients treated according to methods of the present disclosure can be on a Q4M dosing interval. In some embodiments, at least 50%, at least 55%, at least 60%, at least 65%, at least 70%, at least 75%, at least 80%, at least 85%, at least 90% of patients treated according to methods of the present disclosure can be on a Q4M dosing interval. In some embodiments, between about 40 to about 95%, e.g., between about 45 to about 90%, between about 50 to about 85%, between about 55 to about 85%, between about 60 to about 85%, between about 65 to about 85%, including between about 70 to about 80% of patients treated according to methods of the present disclosure can be on a Q4M dosing interval. In some embodiments, at least 75% of patients treated according to methods of the present disclosure can be on a Q5M dosing interval. In some embodiments, at least 50%, at least 55%, at least 60%, at least 65%, at least 70%, at least 75%, at least 80%, at least 85%, at least 90% of patients treated according to methods of the present disclosure can be on a Q5M dosing interval. In some embodiments, between about 40 to about 95%, e.g., between about 45 to about 90%, between about 50 to about 85%, between about 55 to about 85%, between about 60 to about 85%, between about 65 to about 85%, including between about 70 to about 80% of patients treated according to methods of the present disclosure can be on a Q5M dosing interval. In some embodiments, at least 55% of patients treated according to methods of the present disclosure can be on a Q6M dosing interval. In some embodiments, at least 70% of patients treated according to methods of the present disclosure can be on a Q6M dosing interval. In some embodiments, at least 75% of patients treated according to methods of the present disclosure can be on a Q6M dosing interval. In some embodiments, at least 30%, at least 35%, at least 40%, at least 45%, at least 50%, at least 55%, at least 60%, at least 65%, at least 70%, at least 75%, or at least 80% of patients treated according to methods of the present disclosure can be on a Q6M dosing interval. In some embodiments, between about 30 to about 85%, e.g., between about 30 to about 80%, between about 35 to about 75%, between about 40 to about 70%, between about 45 to about 65%, between about 50 to about 65%, including between about 50 to about 60% of patients treated according to methods of the present disclosure can be on a Q6M dosing interval.

In some embodiments, a patient (e.g., patient having an eye disorder, such as, but not limited to wAMD, RVO or DME) treated according to methods of the present disclosure has at least 75% chance of maintaining a therapeutic result of anti-VEGF therapy on a Q5M dosing interval. In some embodiments, a patient (e.g., patient having an eye disorder, such

as, but not limited to wAMD, RVO or DME) treated according to methods of the present disclosure has at least 50%, at least 55%, at least 60%, at least 65%, at least 70%, at least 75%, or at least 80% chance of maintaining a therapeutic result of anti-VEGF therapy on a Q5M dosing interval. In some embodiments, a patient (e.g., patient having an eye disorder, such as, but not limited to wAMD, RVO or DME) treated according to methods of the present disclosure has between about 40 to about 95%, e.g., between about 45 to about 90%, between about 50 to about 85%, between about 55 to about 85%, between about 60 to about 85%, between about 65 to about 85%, including between about 70 to about 80% chance of maintaining a therapeutic result of anti-VEGF therapy on a Q5M dosing interval. In some embodiments, a patient (e.g., patient having an eye disorder, such as, but not limited to wAMD, RVO or DME) treated according to methods of the present disclosure has at least 55% chance of maintaining a therapeutic result of anti-VEGF therapy on a Q6M dosing interval. In some embodiments, a patient (e.g., patient having an eye disorder, such as, but not limited to wAMD, RVO or DME) treated according to methods of the present disclosure has at least 70% chance of maintaining a therapeutic result of anti-VEGF therapy on a Q6M dosing interval. In some embodiments, a patient (e.g., patient having an eye disorder, such as, but not limited to wAMD, RVO or DME) treated according to methods of the present disclosure has at least 80% chance of maintaining a therapeutic result of anti-VEGF therapy on a Q6M dosing interval. In some embodiments, a patient (e.g., patient having an eye disorder, such as, but not limited to wAMD, RVO or DME) treated according to methods of the present disclosure has at least 30%, at least 35%, at least 40%, at least 45%, at least 50%, at least 55% chance of maintaining a therapeutic result of anti-VEGF therapy on a Q6M dosing interval. In some embodiments, a patient (e.g., patient having an eye disorder, such as, but not limited to wAMD, RVO or DME) treated according to methods of the present disclosure has between about 30 to about 85%, e.g., between about 30 to about 80%, between about 35 to about 75%, between about 40 to about 70%, between about 45 to about 65%, between about 50 to about 65%, including between about 50 to about 60% chance of maintaining a therapeutic result of anti-VEGF therapy on a Q6M dosing interval.

In some embodiments, a patient having RVO treated according to methods of the present disclosure has at least 75% chance of maintaining a therapeutic result of anti-VEGF therapy on a Q4M dosing interval. In some embodiments, a patient having RVO treated according to methods of the present disclosure has at least 80% chance of maintaining a therapeutic result of anti-VEGF therapy on a Q5M dosing interval. In some embodiments, a patient having RVO treated according to methods of the present disclosure has at least 90% chance of maintaining a therapeutic result of anti-VEGF therapy on a Q5M dosing interval. In some embodiments, a patient having RVO treated according to methods of the present disclosure has at least 50%, at least 55%, at least 60%, at least 65%, at least 70%, at least 75%, at least 80% or at least 900% chance of maintaining a therapeutic result of anti-VEGF therapy on a Q4M dosing interval. In some embodiments, a patient having RVO treated according to methods of the present disclosure has between about 40 to about 95%, e.g., between about 45 to about 95%, between about 50 to about 95%, between about 55 to about 95%, between about 60 to about 95%, between about 65 to about 95%, including between about 70 to about 90% chance of maintaining a therapeutic result of anti-VEGF therapy on a Q4M dosing interval.

In some embodiments, 50% or more, 55% or more, 60% or more, 65% or more, 70% or more, 75% or more, 80% or more, 85% or more, 90% or more, 95% or more, or about 100%, or a percentage within a range defined by any two of the preceding values, of subjects (e.g., subjects having an eye disorder, such as, but not limited to wAMD, RVO or DME) administered 1, 2, 3, 4, 5, or 6 loading doses (loading doses of an anti-VEGF antibody conjugate, e.g., KSI-301), according to some methods of the present disclosure, do not receive a retreatment dose (e.g., a retreatment dose that would be administered due to disease activity meeting one or more retreatment criteria) for at least about 28 weeks after the last loading dose, or after the last retreatment dose. In some embodiments, 50% or more, 55% or more, 60% or more, 65% or more, 70% or more, 75% or more, 80% or more, 85% or more, 90% or more, 95% or more, or about 100%, or a percentage within a range defined by any two of the preceding values, of subjects (e.g., subjects having an eye disorder, such as, but not limited to wAMD, RVO or DME) administered 3 loading doses (loading doses of the anti-VEGF antibody conjugate, e.g., KSI-301), according to some methods of the present disclosure, do not receive a retreatment dose (e.g., a retreatment dose that would be administered due to disease activity meeting one or more retreatment criteria) for at least about 28 weeks after the last loading dose, or after the last retreatment dose.

In some embodiments, a subject (e.g., a subject having an eye disorder, such as, but not limited to wAMD, RVO or DME) who has been administered 1, 2, 3, 4, 5, or 6 loading doses (of the anti-VEGF antibody conjugate, e.g., KSI-301), according to some methods of the present disclosure, has a chance of 50% or more, 55% or more, 60% or more, 65% or more, 70% or more, 75% or more, 80% or more, 85% or more, 90% or more, 95% or more, or about 100%, or a percentage within a range defined by any two of the preceding values, of not receiving a retreatment dose (e.g., a retreatment dose that would be administered due to disease activity meeting one or more retreatment criteria) for at least about 28 weeks after the last loading dose, or after the last retreatment dose. In some embodiments, a subject having wAMD, who has been administered 3 loading doses (of the anti-VEGF antibody conjugate, e.g., KSI-301), according to some methods of the present disclosure, has a chance of about 70% or higher of not receiving a retreatment dose (e.g., a retreatment dose that would be administered due to disease activity meeting one or more retreatment criteria) for at least about 28 weeks after the last loading dose, or after the last retreatment dose. In some embodiments, a subject (e.g., a subject having an eye disorder, such as, but not limited to wAMD, RVO or DME) who has been administered 3 loading doses (of the anti-VEGF

antibody conjugate, e.g., KSI-301), according to some methods of the present disclosure, has a chance of 50% or more, 55% or more, 60% or more, 65% or more, 70% or more, 75% or more, 80% or more, 85% or more, 90% or more, 95% or more, or about 100%, or a percentage within a range defined by any two of the preceding values, of not receiving a retreatment dose (e.g., a retreatment dose that would be administered due to disease activity meeting one or more retreatment criteria) for at least about 28 weeks after the last loading dose, or after the last retreatment dose.

In some embodiments, 50% or more, 55% or more, 60% or more, 65% or more, 70% or more, 75% or more, 80% or more, 85% or more, 90% or more, 95% or more, or about 100%, or a percentage within a range defined by any two of the preceding values, of subjects (e.g., subjects having an eye disorder, such as, but not limited to wAMD, RVO or DME) administered 1, 2, 3, 4, 5, or 6 loading doses (loading doses of an anti-VEGF antibody conjugate, e.g., KSI-301), according to some methods of the present disclosure, do not receive a retreatment dose (e.g., a retreatment dose that would be administered due to disease activity meeting one or more retreatment criteria) for at least about 24 weeks after the last loading dose, or after the last retreatment dose. In some embodiments, 50% or more, 55% or more, 60% or more, 65% or more, 70% or more, 75% or more, 80% or more, 85% or more, 90% or more, 95% or more, or about 100%, or a percentage within a range defined by any two of the preceding values, of subjects (e.g., subjects having an eye disorder, such as, but not limited to wAMD, RVO or DME) administered 3 loading doses (loading doses of the anti-VEGF antibody conjugate, e.g., KSI-301), according to some methods of the present disclosure, do not receive a retreatment dose (e.g., a retreatment dose that would be administered due to disease activity meeting one or more retreatment criteria) for at least about 24 weeks after the last loading dose, or after the last retreatment dose.

In some embodiments, a subject (e.g., a subject having an eye disorder, such as, but not limited to wAMD, RVO or DME) who has been administered 1, 2, 3, 4, 5, or 6 loading doses (of the anti-VEGF antibody conjugate, e.g., KSI-301), according to some methods of the present disclosure, has a chance of 50% or more, 55% or more, 60% or more, 65% or more, 70% or more, 75% or more, 80% or more, 85% or more, 90% or more, 95% or more, or about 100%, or a percentage within a range defined by any two of the preceding values, of not receiving a retreatment dose (e.g., a retreatment dose that would be administered due to disease activity meeting one or more retreatment criteria) for at least about 24 weeks after the last loading dose, or after the last retreatment dose. In some embodiments, a subject (e.g., a subject having an eye disorder, such as, but not limited to wAMD, RVO or DME) who has been administered 3 loading doses (e.g., of KSI-301), according to some methods of the present disclosure, has a chance of 50% or more, 55% or more, 60% or more, 65% or more, 70% or more, 75% or more, 80% or more, 85% or more, 90% or more, 95% or more, or about 100%, or a percentage within a range defined by any two of the preceding values, of not receiving a retreatment dose (e.g., a retreatment dose that would be administered due to disease activity meeting one or more retreatment criteria) for at least about 24 weeks after the last loading dose, or after the last retreatment dose.

In some embodiments, 50% or more, 55% or more, 60% or more, 65% or more, 70% or more, 75% or more, 80% or more, 85% or more, 90% or more, 95% or more, or about 100%, or a percentage within a range defined by any two of the preceding values, of subjects (e.g., subjects having an eye disorder, such as, but not limited to wAMD, RVO or DME) administered 1, 2, 3, 4, 5, or 6 loading doses (loading doses of an anti-VEGF antibody conjugate, e.g., KSI-301), according to some methods of the present disclosure, do not receive a retreatment dose (e.g., a retreatment dose that would be administered due to disease activity meeting one or more retreatment criteria) for at least about 20 weeks after the last loading dose, or after the last retreatment dose. In some embodiments, 50% or more, 55% or more, 60% or more, 65% or more, 70% or more, 75% or more, 80% or more, 85% or more, 90% or more, 95% or more, or about 100%, or a percentage within a range defined by any two of the preceding values, of subjects (e.g., subjects having an eye disorder, such as, but not limited to wAMD, RVO or DME) administered 3 loading doses (e.g., loading doses of anti-VEGF antibody conjugate, e.g., KSI-301), according to some methods of the present disclosure, do not receive a retreatment dose (e.g., a retreatment dose that would be administered due to disease activity meeting one or more retreatment criteria) for at least about 20 weeks after the last loading dose, or after the last retreatment dose.

In some embodiments, a subject (e.g., a subject having an eye disorder, such as, but not limited to wAMD, RVO or DME) who has been administered 1, 2, 3, 4, 5, or 6 loading doses (of the anti-VEGF antibody conjugate, e.g., KSI-301), according to some methods of the present disclosure, has a chance of 50% or more, 55% or more, 60% or more, 65% or more, 70% or more, 75% or more, 80% or more, 85% or more, 90% or more, 95% or more, or about 100%, or a percentage within a range defined by any two of the preceding values, of not receiving a retreatment dose (e.g., a retreatment dose that would be administered due to disease activity meeting one or more retreatment criteria) for at least about 20 weeks after the last loading dose, or after the last retreatment dose. In some embodiments, a subject (e.g., a subject having an eye disorder, such as, but not limited to wAMD, RVO or DME) who has been administered 3 loading doses (of the anti-VEGF antibody conjugate, e.g., KSI-301), according to some methods of the present disclosure, has a chance of 50% or more, 55% or more, 60% or more, 65% or more, 70% or more, 75% or more, 80% or more, 85% or more, 90% or more, 95% or more, or about 100%, or a percentage within a range defined by any two of the preceding values, of not receiving a retreatment dose (e.g., a retreatment dose that would be administered due to disease activity meeting one or more retreatment criteria) for at least about 20 weeks after the last loading dose, or after the last retreatment dose.

In some embodiments, 50% or more, 55% or more, 60% or more, 65% or more, 70% or more, 75% or more, 80%

or more, 85% or more, 90% or more, 95% or more, or about 100%, or a percentage within a range defined by any two of the preceding values, of subjects (e.g., subjects having an eye disorder, such as, but not limited to wAMD, RVO or DME) administered 1, 2, 3, 4, 5, or 6 loading doses (loading doses of an anti-VEGF antibody conjugate, e.g., KSI-301), according to some methods of the present disclosure, do not receive a retreatment dose (e.g., a retreatment dose that would be administered due to disease activity meeting one or more retreatment criteria) for at least about 16 weeks after the last loading dose, or after the last retreatment dose. In some embodiments, 50% or more, 55% or more, 60% or more, 65% or more, 70% or more, 75% or more, 80% or more, 85% or more, 90% or more, 95% or more, or about 100%, or a percentage within a range defined by any two of the preceding values, of subjects (e.g., subjects having an eye disorder, such as, but not limited to wAMD, RVO or DME) administered 3 loading doses (loading doses of the anti-VEGF antibody conjugate, e.g., KSI-301), according to some methods of the present disclosure, do not receive a retreatment dose (e.g., a retreatment dose that would be administered due to disease activity meeting one or more retreatment criteria) for at least about 16 weeks after the last loading dose, or after the last retreatment dose.

In some embodiments, a subject (e.g., a subject having an eye disorder, such as, but not limited to wAMD, RVO or DME) who has been administered 1, 2, 3, 4, 5, or 6 loading doses (of the anti-VEGF antibody conjugate, e.g., KSI-301), according to some methods of the present disclosure, has a chance of 50% or more, 55% or more, 60% or more, 65% or more, 70% or more, 75% or more, 80% or more, 85% or more, 90% or more, 95% or more, or about 100%, or a percentage within a range defined by any two of the preceding values, of not receiving a retreatment dose (e.g., a retreatment dose that would be administered due to disease activity meeting one or more retreatment criteria) for at least about 16 weeks after the last loading dose, or after the last retreatment dose. In some embodiments, a subject (e.g., a subject having an eye disorder, such as, but not limited to wAMD, RVO or DME) who has been administered 3 loading doses (e.g., of KSI-301), according to some methods of the present disclosure, has a chance of 50% or more, 55% or more, 60% or more, 65% or more, 70% or more, 75% or more, 80% or more, 85% or more, 90% or more, 95% or more, or about 100%, or a percentage within a range defined by any two of the preceding values, of not receiving a retreatment dose (e.g., a retreatment dose that would be administered due to disease activity meeting one or more retreatment criteria) for at least about 16 weeks after the last loading dose, or after the last retreatment dose.

In some embodiments, for any of the eye disorders provided herein (e.g., wAMD, RVO and/or DME), 2 or 3 loading doses will provide for permanent resolution of the disorder, such that no further retreatment or maintenance doses are required. In some embodiments, for any of the eye disorders provided herein (e.g., wAMD, RVO and/or DME), 2 or 3 loading doses will provide for 1, 2, 3, 4, 5, 6, 7, 8, 9, 10 years or longer of effective suppression of the disorder such that no further retreatment of maintenance doses are required during that time period.

In some embodiments, any of the above dosing schedules is from any ocular disorder. In some embodiments, it is for the treatment and/or prevention of RVO, AMD, wAMD, and/or DME, and/or any of the other disorders provided herein.

In some embodiments, wAMD patients achieve 3 to 6 months of durability via the use of an anti-VEGF antibody conjugate, e.g., KSI-301, or anti-VEGF protein conjugate (e.g., an aflibercept biopolymer conjugate).

In some embodiments, DME patients achieve 3 to 5+ months of durability with only 3 loading doses via the use of the anti-VEGF antibody conjugate, e.g., KSI-301, or anti-VEGF protein conjugate (e.g., an aflibercept biopolymer conjugate). In some embodiments, DME patients achieve 3 to 6+ months of durability with only 3 loading doses via the use of the anti-VEGF antibody conjugate, e.g., KSI-301, or anti-VEGF protein conjugate (e.g., an aflibercept biopolymer conjugate).

In some embodiments, RVO patients achieving 2 to 4+ months of durability with only 3 loading doses via the use of the anti-VEGF antibody conjugate, e.g., KSI-301, or anti-VEGF protein conjugate (e.g., an aflibercept biopolymer conjugate). In some embodiments, RVO patients achieve 2 to 5+ months of durability with only 3 loading doses via the use of the anti-VEGF antibody conjugate, e.g., KSI-301, or anti-VEGF protein conjugate (e.g., an aflibercept biopolymer conjugate).

In some embodiments, sustained improvement in PDR with 3 loading doses via the use of the anti-VEGF antibody conjugate, e.g., KSI-301, or anti-VEGF protein conjugate (e.g., an aflibercept biopolymer conjugate).

In some embodiments, a reduced number of loading doses in DME and RVO is achieved via the use of the anti-VEGF antibody conjugate, e.g., KSI-301, or anti-VEGF protein conjugate (e.g., an aflibercept biopolymer conjugate). In some embodiments, a dosing schedule for wet AMD is: every 3 to 5 months (although some wAMD patients may benefit from dosing every 8 weeks). In some embodiments, a dosing schedule for DME is every 3 to 6 months. In some embodiments, a dosing schedule for RVO is every 8 weeks or longer (e.g., 6 months). In some embodiments, a dosing schedule for diabetic retinopathy (without diabetic macular edema) is every 3 months or longer (e.g., every 4 months or every 6 months). In some embodiments, the dosing schedule can be applied to treat diabetic retinopathy (non-proiliferative diabetic retinopathy and proliferative diabetic retinopathy).

In some embodiments, an RVO patient that has received more than one retreatment after the loading dose, the time to the second retreatment is longer than the time to the first retreatment. This is also surprising and unexpected.

In some embodiments, the amount of the antibody administered to the subject will be between 1 and 5 mg, e.g., 1.25 (25 microliters), 2.5 (50 microliters) or 5 mg (100 microliters).

In some embodiments, one applies only two loading doses and then a re-treatment dose every eight weeks or less frequently (e.g., for RVO). In the case of RVO using only two loading doses or even three loading doses and having as good outcome as with monthly is surprising and unexpected (as data shows that Lucentis, Eylea, and Avastin all need monthly dosing for the primary results). In some embodiments, for DME using an anti-VEGF antibody conjugate, e.g., KSI-301, or anti-VEGF protein conjugate (e.g., an aflibercept biopolymer conjugate), it is possible to obtain good results with 3 loading doses whereas other antibody systems require 4 or 5 monthly loading doses.

In some embodiments, for non-proliferative DR, one can avoid any loading dose or only include a single loading dose to obtain the treatment.

In some embodiments, one can decrease the severity of diabetic retinopathy in patients with either non-proliferative or proliferative diabetic retinopathy. One can achieve this using the same treatment regimen as DME (3 loading doses and then q12w or less frequent), or with the 'no loading doses or 2 loading doses and then every 3, 4, or 6 months'). In some embodiments, the outcome measures there are 1) improvement in 2 or more steps of diabetic retinopathy severity status on standard color photos of the retina or 2) prevention of 2 or more steps worsening of DRSS using photo. In some embodiments, retreatment is done less frequently than once every 8 weeks.

In some embodiments, the dosing schedule can be applied to non-proliferative DR with 1 to 2 loading doses, or just 1 loading dose and not more than that. The retreatment can occur no sooner than 12 or 16 or 24 weeks thereafter.

In some embodiments, for proliferative DR, 3 monthly loading doses can be applied followed by every 12 weeks or longer.

In some embodiments, a method of administering an anti-VEGF antibody conjugate or anti-VEGF protein conjugate is provided in which the patient gets a loading phase (for example, 2 loading doses q4 weeks), then a retreatment at 8 weeks, then the time to a second retreatment is longer that the time to the first retreatment (for example, 16+ weeks). In some embodiments, this reflects a 'disease modification'.

In some embodiments, an anti-VEGF antibody conjugate, e.g., KSI-301, or anti-VEGF protein conjugate (e.g., an aflibercept biopolymer conjugate), can be administered to a subject with diabetes for a 'reperfusion' of ischemic areas: giving a dose of the anti-VEGF antibody conjugate, e.g., KSI-301 (or a loading phase of the anti-VEGF antibody conjugate, e.g., KSI-301, for example, 3 loading doses every 4 weeks three times, so day 0, week 4, week 8) then retreatments as need.

In some embodiments, a method of disease modification of an eye disorder is provided. The method comprises: administering an anti-VEGF antibody conjugate, e.g., KSI-301, or anti-VEGF protein conjugate (e.g., an aflibercept biopolymer conjugate), to a subject having an eye disorder at a first loading dose, whereby the eye disorder is thereby modified in a beneficial manner to the subject.

In some embodiments, a method of treating an eye disorder is provided. The method comprises identifying a subject with DME, DR or RVO; administering 1-6 loading doses of an anti-VEGF antibody conjugate, e.g., KSI-301, or anti-VEGF protein conjugate (e.g., an aflibercept biopolymer conjugate), to the subject; providing a first retreatment dose of the anti-VEGF antibody conjugate, e.g., KSI-301, or anti-VEGF protein conjugate (e.g., aflibercept biopolymer conjugate), to the subject following a first amount of time from the last loading dose; and providing a second retreatment dose of the anti-VEGF antibody conjugate, e.g., KSI-301, or anti-VEGF protein conjugate (e.g., aflibercept biopolymer conjugate), to the subject, following a second amount of time from the first retreatment dose of the anti-VEGF antibody conjugate, e.g., KSI-301, or anti-VEGF protein conjugate (e.g., aflibercept biopolymer conjugate), wherein the second amount of time is greater than the first amount of time. In some embodiments, the second amount of time is 10, 20, 30, 40, 50, 60, 70, 80, 90, 100% or greater than the first amount of time. In some embodiments, the second amount of time is 1, 2, 3, 4, 5, 6, 7, 8, 9, 10, 11, 12, 13, 14, 15, 16 or more weeks longer than the first amount of time. In some embodiments, the loading doses include 3 doses of the anti-VEGF antibody conjugate, e.g., KSI-301, or anti-VEGF protein conjugate (e.g., aflibercept biopolymer conjugate), administered to the subject.

In some embodiments, an anti-VEGF antibody conjugate, e.g., KSI-301, or anti-VEGF protein conjugate (e.g., an aflibercept biopolymer conjugate), can be applied in a method of treating DME or proliferative diabetic retinopathy or non-proliferative diabetic retinopathy with an anti-VEGF antibody conjugate, e.g., KSI-301, or anti-VEGF protein conjugate (e.g., an aflibercept biopolymer conjugate), that results in reperfusion (of ischemic tissue), and therefore represents improvement of underlying disease. The terms perfusion and reperfusion are used interchangeably herein.

In some embodiments, following the loading injections and the first retreatment injection, each subsequent retreatment injection will be less frequent than the first retreatment injection. That is, the amount of time between retreatment injections can be increased, give the properties of the present method. In particular, the amount of time can increase by 1, 2, 3, 4, 5, 6, 7, 8, 9, 10, 11, 12, 13, 14, 15, 16, 17, 18, 19, 20 or more weeks for each subsequent retreatment injection. In some embodiments, the amount of time increases by 1, 2, 3, 4, 5, 6, 7, 8, 90, 10, 15, 20, 25, 30, 35, 40, 45, 50, 100, 500, 1000, 5000, 10,000 percent or more between each retreatment.

In some embodiments, a method of treating an eye disorder can include: administering an anti-VEGF antibody conjugate, e.g., KSI-301, or anti-VEGF protein conjugate (e.g., an aflibercept biopolymer conjugate), to a subject in need of treating an eye disorder at a first loading dose, wherein the eye disorder is diabetic macular edema (DME); and repeating

the loading dose at least once, but not more than twice, whereby the subject retains a therapeutic result of the anti-VEGF antibody conjugate, e.g., KSI-301, or anti-VEGF protein conjugate (e.g., aflibercept biopolymer conjugate), therapy for at least 8 weeks after a final loading dose. In some embodiments, the subject retains the therapeutic result of the anti-VEGF antibody conjugate, e.g., KSI-301, therapy, or anti-VEGF protein conjugate (e.g., aflibercept biopolymer conjugate) therapy, for at least 8, 9, 10, 11, 12, 13, 14, 15, 16, 17, 18, 19, 20, 21, 22, 23, 24, 25, 26, 27, 28, 29, 30, 32, 34, 36, 38, 40, 44, 48, 52, 56, or 60 or more weeks after the final loading dose. In some embodiments, the loading dose is administered twice, or three times. In some embodiments, the loading dose is administered monthly or every other month.

In some embodiments, the method further includes administering one or more subsequent doses of the anti-VEGF antibody conjugate, e.g., KSI-301, or anti-VEGF protein conjugate (e.g., an aflibercept biopolymer conjugate), to the subject after the final loading dose. In some embodiments, the subsequent doses of the anti-VEGF antibody conjugate is administered at a dosing schedule of Q8W, Q12W, Q16W, Q20W, or Q24W, or longer. In some embodiments, the dosing schedule is between Q8W and Q24W. In some embodiments, no subsequent dose of the anti-VEGF antibody conjugate is administered to the subject within at least about one year, about two years, or about three years, after the first loading dose. In some embodiments, no more than one subsequent dose of the anti-VEGF antibody conjugate is administered to the subject within at least about one year, about two years, or about three years, after the first loading dose. In some embodiments, no more than two subsequent doses of the anti-VEGF antibody conjugate is administered to the subject within at least about one year, about two years, or about three years, after the first loading dose. In some embodiments, no more than three subsequent dose of the anti-VEGF antibody conjugate is administered to the subject within at least about one year, about two years, or about three years, after the first loading dose.

In some embodiments, a method of treating an eye disorder includes: administering an anti-VEGF antibody conjugate, e.g., KSI-301, or anti-VEGF protein conjugate (e.g., an aflibercept biopolymer conjugate), to a subject in need of treating an eye disorder at a first loading dose, wherein the eye disorder is wet age-related macular degeneration (wAMD); and repeating the loading dose at least once, but not more than twice, whereby the subject retains a therapeutic result of the anti-VEGF antibody conjugate therapy, or anti-VEGF protein conjugate therapy for at least 12 weeks after a final loading dose. In some embodiments, the subject retains the therapeutic result of the anti-VEGF antibody conjugate, e.g., KSI-301, therapy for at least 12, 13, 14, 15, 16, 17, 18, 19, 20, 21, 22, 23, 24, 25, 26, 27, 28, 29, 30, 32, 34, 36, 38, 40, 44, 48, 52, 56, or 60 or more weeks after the final loading dose. In some embodiments, the loading dose is administered twice, or three times. In some embodiments, the loading dose is administered monthly or every other month.

In some embodiments, the method further includes administering one or more subsequent doses of the anti-VEGF antibody conjugate, e.g., KSI-301, or anti-VEGF protein conjugate (e.g., aflibercept biopolymer conjugate), to the subject after the final loading dose. In some embodiments, the subsequent doses of the anti-VEGF antibody conjugate or anti-VEGF protein conjugate is administered at a dosing schedule of Q2W, Q4W, Q8W, Q12W, Q16W, Q20W, or Q24W, or longer. In some embodiments, the dosing schedule is between Q12W and Q20W. In some embodiments, no more than one, two, three, or four subsequent doses, or no subsequent dose of the anti-VEGF antibody conjugate or anti-VEGF protein conjugate is administered to the subject within about one year of the first loading dose. In some embodiments, the dosing schedule is between Q12W and Q20W. In some embodiments, no more than one, two, three, or four subsequent doses, or no subsequent dose of the anti-VEGF antibody conjugate or anti-VEGF protein conjugate is administered to the subject within about two years of the first loading dose. In some embodiments, no more than one, two, three, or four subsequent doses, or no subsequent dose of the anti-VEGF antibody conjugate or anti-VEGF protein conjugate is administered to the subject within about three years of the first loading dose.

In some embodiments, a method of treating an eye disorder includes: administering an anti-VEGF antibody conjugate, e.g., KSI-301, or anti-VEGF protein conjugate (e.g., an aflibercept biopolymer conjugate), to a subject in need of treating an eye disorder at a first loading dose, wherein the eye disorder is retinal vein occlusion (RVO), e.g., CRVO or BRVO; and repeating the loading dose at least once, but not more than twice, whereby the subject retains a therapeutic result of the anti-VEGF antibody conjugate, e.g., KSI-301, therapy, or anti-VEGF protein conjugate (e.g., aflibercept biopolymer conjugate) therapy, for at least 8 weeks after a final loading dose. In some embodiments, the subject retains the therapeutic result of the anti-VEGF antibody conjugate, e.g., KSI-301, therapy, or anti-VEGF protein conjugate (e.g., aflibercept biopolymer conjugate) therapy, for at least 8, 9, 10, 11, 12, 13, 14, 15, 16, 17, 18, 19, 20, 21, 22, 23, 24, 25, 26, 27, 28, 29, 30, 32, 34, 36, 38, 40, 44, 48, 52, 56, or 60 or more weeks after the final loading dose. In some embodiments, the loading dose is administered twice, or three times. In some embodiments, the loading dose is administered monthly or every other month.

In some embodiments, the method further includes administering one or more subsequent doses of the anti-VEGF antibody conjugate, e.g., KSI-301, or anti-VEGF protein conjugate (e.g., aflibercept biopolymer conjugate), to the subject after the final loading dose. In some embodiments, the subsequent doses of the anti-VEGF antibody conjugate is administered at a dosing schedule of Q8W, Q12W, Q16W, Q20W, or Q24W, or longer. In some embodiments, the dosing schedule is Q8W or longer. In some embodiments, no subsequent dose of the anti-VEGF antibody conjugate is administered to the subject within at least about one year, about two years, or about three years, after the first loading dose. In some embodiments, no more than one subsequent dose of the anti-VEGF antibody conjugate is administered to

the subject within at least about one year, about two years, or about three years, after the first loading dose. In some embodiments, no more than two subsequent dose of the anti-VEGF antibody conjugate is administered to the subject within at least about one year, about two years, or about three years, after the first loading dose. In some embodiments, no more than three subsequent dose of the anti-VEGF antibody conjugate is administered to the subject within at least about one year, about two years, or about three years, after the first loading dose.

In some embodiments, a method of treating an eye disorder includes administering to a subject in need of treating an eye disorder a therapeutically effective amount of an anti-VEGF antibody conjugate, e.g., KSI-301, or anti-VEGF protein conjugate (e.g., an aflibercept biopolymer conjugate), wherein the eye disorder is diabetic retinopathy (DR), thereby treating the eye disorder. In some embodiments, the anti-VEGF antibody conjugate is administered according to a dosing schedule of Q12W, Q16W, Q20W, or Q24W, or longer. In some embodiments, the dosing schedule is between Q12W and Q24W. In some embodiments, the method further comprises administering to the subject at least one loading dose, but no more than two loading doses, of the anti-VEGF antibody conjugate. In some embodiments, the time between any two consecutive loading doses is about 4 or 8 weeks (once a month or once every other month).

In some embodiments, a method of treating an eye disorder includes administering to a subject in need of treating an eye disorder a first dose of a plurality of doses of an anti-VEGF antibody conjugate, e.g., KSI-301, or anti-VEGF protein conjugate (e.g., an aflibercept biopolymer conjugate), in a dosing schedule comprising: a loading dosing schedule comprising 1-3 loading doses of the anti-VEGF antibody conjugate, wherein the first dose is a loading dose; followed by a maintenance dosing schedule comprising one or more subsequent doses of the anti-VEGF antibody conjugate after a final loading dose, wherein the maintenance dosing schedule comprises a predetermined dosing schedule of Q8W or longer. In some embodiments, the predetermined dosing schedule is Q8W, Q12W, Q16W, Q20W, or Q24W, or longer. In some embodiments, the eye disorder is wAMD, and the predetermined dosing schedule is Q12W or longer, e.g., Q16W, Q20W, or Q24W, or longer. In some embodiments, the eye disorder is DME, DR, or RVO.

In some embodiments, the method includes an individualized dosing schedule. A method of treating an eye disorder with an individualized dosing schedule can include: evaluating a therapeutic result of the anti-VEGF antibody conjugate, e.g., KSI-301, therapy, or anti-VEGF protein conjugate (e.g., an aflibercept biopolymer conjugate) therapy, in the subject at one or more time points after the first dose (e.g., a loading dose or a maintenance dose); and administering a subsequent dose of the anti-VEGF antibody conjugate, or anti-VEGF protein conjugate, to the subject at a subsequent time point specified by the predetermined dosing schedule, unless the therapeutic result is retained by the subject, in which case extending the time interval until administering the subsequent dose. In some embodiments, a subject's eye health, e.g. retinal health, may be evaluated or assessed at a follow-up visit 4, 8, or 12 weeks, or any time point within a range defined by the above values, after the last dose, e.g., last loading dose, or last maintenance dose. In some embodiments, the therapeutic result of the anti-VEGF antibody conjugate therapy or anti-VEGF protein conjugate therapy may be assessed by any suitable option as disclose herein (e.g., visual acuity, retinal thickness, etc.). In some embodiments, if the therapeutic result of the anti-VEGF antibody conjugate therapy or anti-VEGF protein conjugate therapy is retained, as disclosed herein, no subsequent dose, e.g., maintenance dose, is needed, and the actual dosing schedule may deviate from the predetermined dosing schedule. In some embodiments, the next subsequent dose, e.g., maintenance dose, is postponed as long as the therapeutic result of the anti-VEGF antibody conjugate therapy or anti-VEGF protein conjugate therapy is retained. In some embodiments, for example, an actual dosing schedule may deviate from a predetermined Q8W dosing schedule if at 8 weeks after the last loading dose or the last maintenance dose, the subject's treated eye retains the therapeutic result of the anti-VEGF antibody conjugate therapy, the anti-VEGF antibody conjugate is not administered at the scheduled time. In some embodiments, the next maintenance dose may be postponed indefinitely as long as the therapeutic result of the anti-VEGF antibody conjugate therapy is retained.

In some embodiments, a method of treating an eye disorder includes: identifying a subject in need of treating an eye disorder, wherein the eye disorder is presumed ocular histoplasmosis syndrome; and intravitreally administering to the subject a therapeutically effective amount of the anti-VEGF antibody conjugate, e.g., KSI-301, or anti-VEGF protein conjugate (e.g., an aflibercept biopolymer conjugate), thereby treating the eye disorder. In some embodiments, the therapeutically effective amount comprises about 1 mg to about 5 mg, about 1.25 mg to about 5 mg, or about 2.5 mg to about 5 mg (by weight of the anti-VEGF antibody portion) of the anti-VEGF antibody conjugate. In some embodiments, no more than one injection of the anti-VEGF antibody conjugate or anti-VEGF protein conjugate is required to treat the eye disorder.

In some embodiments, administering to the subject in need of treating an eye disorder no more than three loading doses of the anti-VEGF antibody conjugate (e.g., KSI-301) provides a therapeutic result that lasts at least 24 weeks or longer. In some embodiments, administering to the subject in need of treating an eye disorder no more than two doses of the anti-VEGF antibody conjugate (e.g., KSI-301) provides a therapeutic result that lasts at least 24 weeks or longer. In some embodiments, administering to the subject in need of treating an eye disorder no more than one dose of the anti-VEGF antibody conjugate (e.g., KSI-301) provides a therapeutic result that lasts at least 24 weeks or longer. In some embodiments, after the final loading dose, no additional dose (e.g., maintenance dose) of the anti-VEGF antibody conjugate is administered to the subject for at least 24 weeks (i.e., it is effective for that period of time such that additional

maintenance or retreatment doses are not required during that time). In some embodiments, after administering one loading dose, no additional dose (e.g., maintenance dose) of the anti-VEGF antibody conjugate is administered to the subject for at least 24 weeks. In some embodiments, after administering two loading doses, no additional dose (e.g., maintenance dose) of the anti-VEGF antibody conjugate is administered to the subject for at least 24 weeks. In some embodiments, after administering three loading dose, no additional dose (e.g., maintenance dose) of the anti-VEGF antibody conjugate is administered to the subject for at least 24 weeks. In some embodiments, an interval between loading doses is about one month to about two months. In some embodiments, an interval between loading doses is about one month or about two months. In some embodiments, the anti-VEGF antibody conjugate (e.g., KSI-301) is administered to the subject at a dosing schedule (e.g., maintenance dosing schedule) of Q24W or longer. In some embodiments, the eye disorder is wAMD. In some embodiments, the eye disorder is RVO. In some embodiments, the eye disorder is DME. In some embodiments, the eye disorder is DR.

ANTIBODY CONJUGATES

Provided herein are anti-VEGF antibodies (including anti-VEGF proteins, e.g., afliberccept) and conjugates thereof. In some embodiments, the antibodies themselves are different from other anti-VEGF agents and provide superior results over other anti-VEGF agents. In some embodiments, the anti-VEGF antibody conjugate displays a surprising superiority over other antibodies and/or the expectation of the activity other antibody conjugates.

In some embodiments, the anti-VEGF antibody conjugate is KSI-301, which is an antibody conjugate comprising:

(1) an anti-VEGF-A antibody; and

(2) a phosphorylcholine containing polymer, wherein the polymer is covalently bonded to the anti-VEGF-A antibody at a cysteine outside a variable region of the anti-VEGF-A antibody, and wherein said cysteine replaces a non-cysteine amino acid that occurs in a same position in sequence, wherein the anti-VEGF-A antibody comprises a light chain and heavy chain, said heavy chain comprising an Fc region, wherein the cysteine is in the Fc region of the heavy chain, wherein the sequence of a heavy chain comprises SEQ ID NO 1 (or any of the variants thereof in FIG. 64), and wherein the sequence of a light chain comprises SEQ ID NO. 2, wherein the antibody conjugate has the following structure:

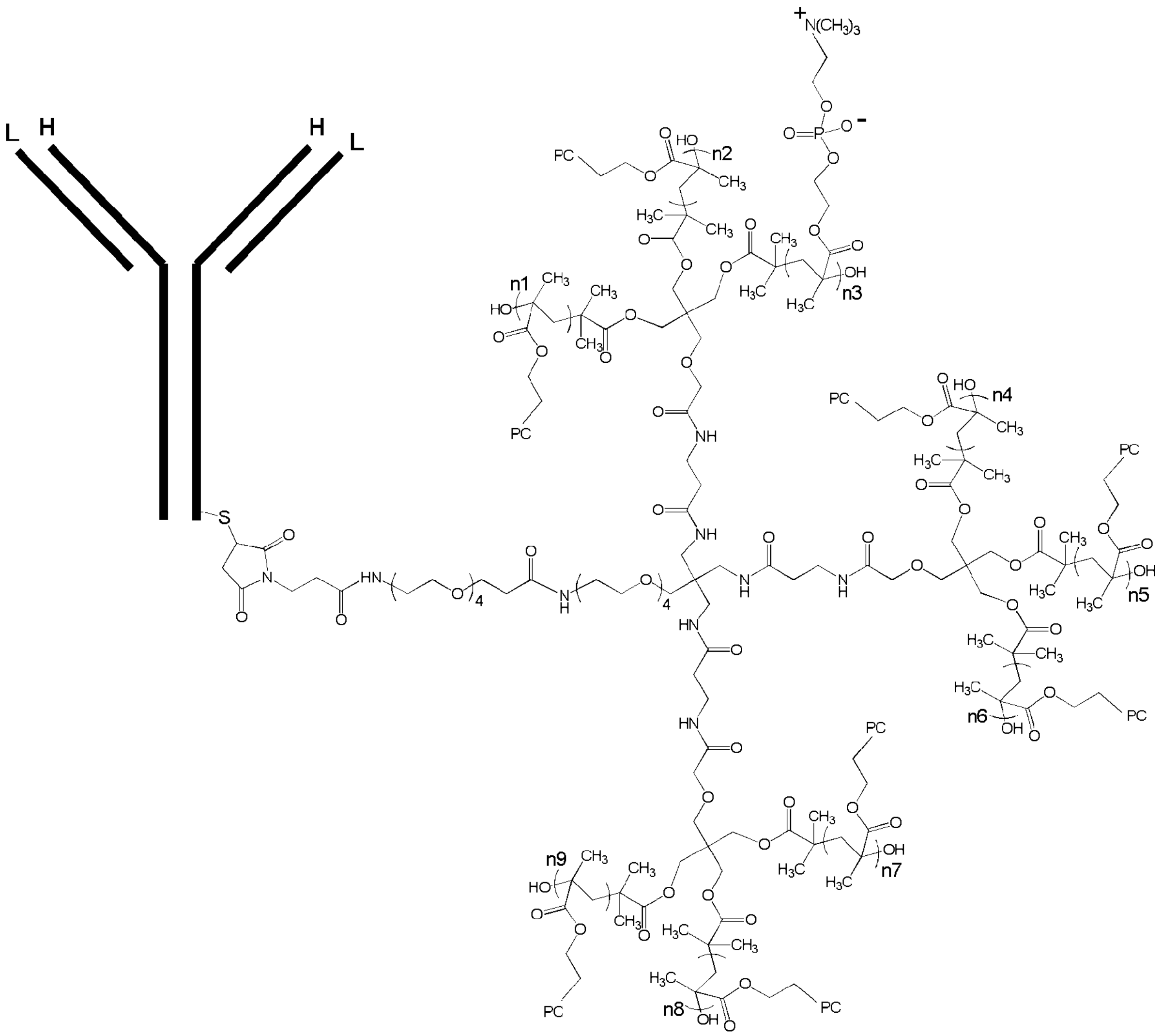

;

wherein:

each heavy chain of the anti-VEGF-A antibody is denoted by the letter H, and each light chain of the anti-VEGF-A antibody is denoted by the letter L;

the polymer is bonded to the anti-VEGF-A antibody through a sulfhydryl at C443 according to EU numbering, which bond is depicted on one of the heavy chains above;

PC is 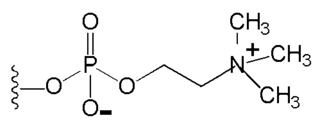 ,

where the curvy line indicates the point of attachment to the rest of the polymer; and

n1, n2, n3, n4, n5, n6, n7, n8 and n9 are the same or different such that the sum of n1, n2, n3, n4, n5, n6, n6, n7, n8 and n9 is 2500 plus or minus 15%.

Historically, conjugating a molecule to a protein often resulted in a decrease in the protein's binding interaction to its intended target. In some embodiments of the present disclosure, when conjugating to a location that is outside of the active site, the same level of decrease as might have been expected is not necessarily observed. The evidence provided herein shows the opposite effect as to what may have been expected. In some embodiments, and without intending to be limited by theory, the conjugate can be superior to the antibody alone. For example, the interaction of a ligand and its

specific receptor is often driven through the stereospecific interaction of the ligand and the receptor, as directed by the interactions of the hydrophilic amino acids on the ligand with the hydrophilic amino acids on the receptor, and water molecules are front and center in those interactions. At the same time, this hydrophilic stereospecificity is further enhanced by de-emphasizing and/or suppressing non-specific hydrophobic interactions that might generally be mediated/created by hydrophobic-to-hydrophobic amino acids.

In some embodiments, an anti-VEGF antibody conjugate is provided that is capable of blocking at least 90% of an interaction between a VEGF ligand ("VEGFL") and a VEGF-receptor ("VEGFR"). For example, it can block at least 90, 91, 92, 93, 94, 95, 96, 97, 98, 99, or effectively all of the interaction between VEGFR and VEGFL. In some embodiments, the noted blocking occurs at saturating concentrations. In some embodiments, an anti-VEGF antibody conjugate is provided that blocks at least 95% of an interaction between a VEGF ligand and a VEGF-receptor. An example of such superiority of blocking is the ability of the anti-VEGF antibody bioconjugate (an antibody conjugate provided herein, e.g., KSI-301) to block to a higher degree than Lucentis®(ranibizumab) or Avastin®(bevacizumab) or even the antibody OG1950 (unconjugated). Indeed, this result was unexpected in that while the addition of a polymer to an antibody (to form an antibody conjugate), could be expected to have some or no detrimental impact on binding/activity of the antibody, it was unexpected that it would actually improve the blocking ability of the antibody in this manner.

In some embodiments, the antibodies or conjugates thereof inhibit at least 70, 80, 85, 86, 87, 88, 89, 90, 91, 92, 93, 94, 95, 96, 97, 98, 99, or 100% of the activity and/or interaction between VEGFR and VEGFL. In some embodiments, the IC50 value can be 0.1, 1, 2, 3, 4, 5, 6, 7 , 8, 9, 10, 20, 30, 40, 50, 100 nM or less than any one or more of the preceding values. In some embodiments, the KD can be 2*10^-13, 1*10^-13, 1*10^-12, 1*10^-11, 1*10^-10M or less than any one of the preceding values. In some embodiments, the IC50 value can be 1, 5, 10, 20, 30, 40, 50, 60, 70 80, 90, 100, 200, 300, 400, 500, 600, 700, 800, 900, 1,000, 1,100, 1,200, 1,300, or less than any one of the preceding values.

In some embodiments, an anti-VEGF antibody is provided that blocks at least 90% of an interaction between a VEGF ligand and a VEGF-receptor. For example, it can block at least 91, 92, 93, 94, 95, 96, 97, 98, 99, or effectively all of the interaction between VEGFR and VEGFL. As example of such superiority of blocking, is the ability of of OG1950 (and antibody provided herein) to block to a higher degree than Lucentis®(ranibizumab) or Avastin®(bevacizumab).

In some embodiments, other antibodies, such as Lucentis®(ranibizumab) or Avastin®(bevacizumab) can be conjugated to one or more of the polymers as described herein, by one or more of the processes described herein. In some embodiments, any antibody, or fragment thereof, can be conjugated to one or more of the polymers as described herein, by one or more of the processes described herein.

In some embodiments the antibody comprises a heavy chain amino acid variable region that comprises SEQ ID NO 1 and a light chain amino acid variable region that comprises SEQ ID NO. 2. In some embodiments, the antibody is conjugated to one or more of the polymers provided herein. In some embodiments, the conjugated antibody is at least 90% identical to SEQ ID NO: 1 and/or 2. In some embodiments, the antibody contains the 6 CDRs within SEQ ID NO:1 and SEQ ID NO: 2, as well as a point mutation of L443C (EU numbering, or 449C in SEQ ID NO: 1). In some embodiments, the conjugated antibody is at least 90% identical to SEQ ID NO: 1 and/or 2 and includes the following mutations: L234A, L235A, and G237A (EU numbering), and at least one of the following mutations: Q347C (EU numbering)or L443C (EU numbering).

In some embodiments an antibody that binds to VEGF-A is provided. The antibody comprises: a $CDR_H1$ that is the $CDR_H1$ in SEQ ID NO: 1, a $CDR_H2$ that is the $CDR_H2$ in SEQ ID NO: 1, a $CDR_H3$ that is the $CDR_H3$ in SEQ ID NO: 1, a $CDR_L1$ that is the $CDR_L1$ in SEQ ID NO: 2, a $CDR_L2$ that is the $CDR_L2$ in SEQ ID NO: 2, a $CDR_L3$ that is the $CDR_L3$ in SEQ ID NO: 2, at least one of the following mutations: L234A, L235A, and G237A (EU numbering), and at least one of the following mutations: Q347C (EU numbering) or L443C (EU numbering).

As will be appreciated by one of skill in the art, in light of the present specification, any of the antibodies provided herein can be conjugated to any of the polymers provided herein and/or any antibody provided herein can have a cysteine added such that it allows for site specific conjugation to a polymer.

"VEGF" or "vascular endothelial growth factor" is a human vascular endothelial growth factor that affects angiogenesis or an angiogenic process. In particular, the term VEGF means any member of the class of growth factors that (i) bind to a VEGF receptor such as VEGFR-1 (Flt-1), VEGFR-2 (KDR/Flk-1), or VEGFR-3 (FLT-4); (ii) activates a tyrosine kinase activity associated with the VEGF receptor; and (iii) thereby affects angiogenesis or an angiogenic process.

The VEGF family of factors is made up of five related glycoproteins: VEGF-A (also known as VPE), -B, -C, -D and PGF (placental growth factor). Of these, VEGF-A is the most well studied and is the target of anti-angiogenic therapy. Ferrara et al, (2003) Nat. Med. 9:669-676. VEGF-A exists as a number of different isotypes which are generated both by alternative splicing and proteolysis: VEGF-$A_{206}$, VEGF-$A_{189}$, VEGF-$A_{165}$, and VEGF-$A_{121}$. The isoforms differ in their ability to bind heparin and non-signaling binding proteins called neuropilins. The isoforms are all biologically active as dimers.

The various effects of VEGF are mediated by the binding of a VEGF, e.g., VEGF-A (P15692), -B (P49766), -C (P49767) and -D (Q43915), to receptor tyrosine kinases (RTKs). The VEGF family receptors belong to class V RTKs and

each carry seven Ig-like domains in the extracellular domain (ECD). In humans, VEGF binds to three types of RTKs: VEGFR-1 (Flt-1) (P17948), VEGFR-2 (KDR, Flk-1) (P935968) and VEGFR-3 (Flt-4) (P35916). Unless otherwise apparent from the context reference to a VEGF means any of VEGF-A, -B, -C , -D, and PGF, in any of the natural isoforms or natural variants or induced variants having at least 90, 95, 98 or 99% or 100% sequence identity to a natural form. In some embodiments, such VEGFs are human VEGFs. Likewise reference to a VEGFR means any of VEGR-1, R-2 or R-3, including any natural isoform or natural variant, or an induced variant having at least 90, 95, 98 or 99% or 100% sequence identity to a natural sequences.

VEGF antagonist therapies have been approved for the treatment of certain cancers and wet AMD. Bevacizumab (AVASTIN, Genentech/Roche) is a humanized mouse monoclonal antibody that binds to and neutralizes human VEGF, in particular to all isoforms of VEGF-A and to bioactive proteolytic fragments of VEGF-A. See, e.g., Ferrara N, Hillan KJ, Gerber HP, Novotny W. 2004. Discovery and development of bevacizumab, an anti-VEGF antibody for treating cancer. Nat Rev Drug Discov. 3(5):391-400. Bevacizumab has been approved for the treatment of certain cancers. The protein sequence of the heavy and light chains of bevacizumab (DrugBank DB00112) are set forth in SEQ ID NO. 3 (heavy) and SEQ ID NO. 4 (light).

Bevacizumab variable light chain CDRs are $CDR_L1$: SASQDISNYLN (SEQ ID NO: 12), $CDR_L2$: FTSSLHS (SEQ ID NO: 13) and $CDR_L3$: QQYSTVPWT (SEQ ID NO: 14). Bevacizumab variable heavy chain CDRs are $CDR_H1$: GYTFTNYGMN, $CDR_H2$: WINTYTGEPTYAADFKR (SEQ ID NO: 10), and $CDR_H3$: YPHYYGSSHWYFDV. CDRs are defined by Kabat except CDRH1 uses the composite Kabat/Chothia definition. In some embodiments, a cysteine can be added to the Bevacizumab sequence and the antibody (and/or a variant that includes the 6 CDRs of Bevacizumab) can be conjugated to any one or more of the polymers provided herein.

Another anti-VEGF molecule, derived from the same mouse monoclonal antibody as bevacizumab has been approved as a treatment for wet AMD: ranibizumab (LUCENTIS®(ranibizumab), Genentech/Roche). Ranibizumab is an antibody fragment or Fab. Ranibizumab was produced by affinity maturation of the variable heavy and light chains of bevacizumab. The sequence of the heavy and light chains of ranibizumab (as published by Novartis) is set forth in SEQ ID NO. 5 and 6 respectively. In some embodiments, a cysteine can be added to the ranibizumab sequence and the antibody (and/or a variant that includes the 6 CDRs of ranibizumab) can be conjugated to any one or more of the polymers provided herein.

The Ranibizumab CDRS are the same as Bevacizumab except where an improvement was added after affinity maturation: Ranibizumab variable light chain CDRs are $CDR_L1$: SASQDISNYLN (SEQ ID NO: 12), $CDR_L2$: FTSSLHS (SEQ ID NO: 13) and $CDR_L3$: QQYSTVPWT (SEQ ID NO: 14). Ranibizumab variable heavy chain CDRs are $CDR_H1$: GYDFTHYGMN (SEQ ID NO: 9), $CDR_H2$: WINTYTGEPTYAADFKR (SEQ ID NO: 10), and $CDR_H3$: YPYYYGTSH-WYFDV (SEQ ID NO: 11).

In some embodiments, an antibody conjugate is presented having an anti-VEGF-A antibody bonded at a cysteine outside a variable region of the antibody to a phosphorylcholine containing polymer, wherein the cysteine has been added via recombinant DNA technology. In some embodiments, the polymer is bonded to a single cysteine. In some embodiments, "added by recombinant DNA technology" means that the cysteine residue replaces a non-cysteine amino acid that occurs in the same position in a known or existing antibody or in a consensus antibody sequence. Thus, for example where the antibody is an IgG1 and the heavy chain possess a leucine at EU position 443, the leucine is replaced via recombinant DNA technology with a cysteine (L443C, EU numbering, or 449C in SEQ ID NO: 1). Correspondingly, the native IgG1 sequence at EU position 347 is Q (glutamine) and the Q is replaced with cysteine via recombinant DNA technology to yield Q347C.

In some embodiments, the anti-VEGF-A antibody comprises a light chain and a heavy chain where the heavy chain has an Fc region. In some embodiments, the cysteine is in the Fc region and the anti-VEGF-A antibody is an immunoglobulin G (IgG). In some embodiments, the anti-VEGF-A heavy chain has $CDR_H1$: GYDFTHYGMN (SEQ ID NO: 9), $CDR_H2$: WINTYTGEPTYAADFKR (SEQ ID NO: 10), and $CDR_H3$: YPYYYGTSHWYFDV (SEQ ID NO: 11), and position 221 (via sequential counting as in SEQ ID NO. 3) is T, and the anti-VEGF-A light chain has $CDR_L1$: SASQDISNYLN (SEQ ID NO: 12), $CDR_L2$: FTSSLHS (SEQ ID NO: 13), and $CDR_L3$: QQYSTVPWT (SEQ ID NO: 14), and Kabat position 4 is L.

In some embodiments, the anti-VEGF-A heavy chain isotype is IgG1. In some embodiments, the IgG1 constant domain has one or more mutations relative to an IgG1 constant domain (e.g. constant region of SEQ ID NO. 3) to modulate effector function. In some embodiments, the effector function mutations are one or more of the following: (EU numbering) E233X, L234X, L235X, G236X, G237X, A327X, A330X, and P331X wherein X is any natural or unnatural amino acid. In some embodiments, the mutations are selected from the group consisting of (EU numbering): E233P, L234V, L234A, L235A, G237A, A327G, A330S, and P331S. In some embodiments, the antibody conjugate has the following mutations (EU numbering): L234A, L235A, and G237A.

In some embodiments, the cysteine residue is in the anti-VEGF-A heavy chain and is Q347C (EU numbering) or L443C (EU numbering). In some embodiments, the cysteine residue is L443C (EU numbering, or 449C in SEQ ID NO: 1). In some embodiments, the sequence of the anti-VEGF-A heavy chain is SEQ ID NO. 1 and the sequence of the anti-VEGF-A light chain is SEQ ID NO. 2.

In some embodiments, the phosphorylcholine containing polymer comprises 2-(methacryloyloxyethyl)-2'-(trimethylammonium)ethyl phosphate (MPC) monomers as set forth below:

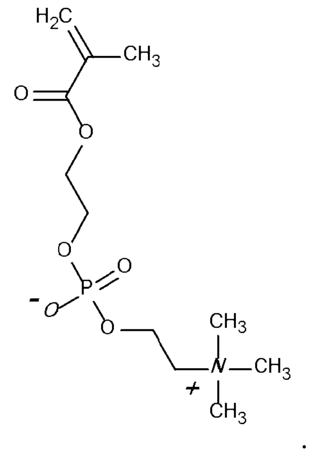

Such that the polymer comprises the following repeating units:

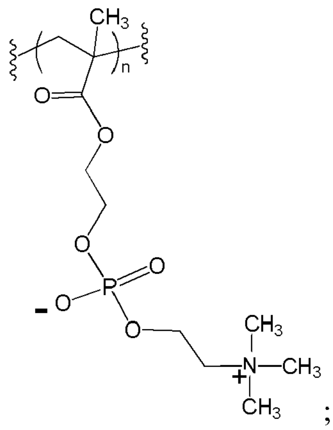

where n is an integer from 1 to 3000 and the wavy lines indicate the points of attachment between monomer units in the polymer.

In some embodiments, the polymer has three or more arms, or is synthesized with an initiator comprising 3 or more polymer initiation sites. In some embodiments, the polymer has 2, 3, 4, 5, 6, 7, 8, 9, 10, 11 or 12 arms, or is synthesized with an initiator comprising 2, 3, 4, 5, 6, 7, 8, 9, 10, 11, or 12 polymer initiation sites. More preferably, the polymer has 3, 6, or 9 arms, or is synthesized with an initiator comprising 3, 6, or 9 polymer initiation sites. In some embodiments, the polymer has 9 arms, or is synthesized with an initiator comprising 9 polymer initiation sites.

In some embodiments, the polymer that is added has a molecular weight between about 300,000 and about 1,750,000 Da (SEC-MALs). In some embodiments, the polymer has a molecular weight between about 500,000 and about 1,000,000 Da. In some embodiments, the polymer has a molecular weight of between about 600,000 to about 900,000 Da. In some embodiments, the polymer has a molecular weight of between about 750,000 to about 850,000 Da. In some embodiments, the polymer has a molecular weight of between about 800,000 to about 850,000 Da. In some embodiments, the polymer has a molecular weight of between about 750,000 to about 800,000 Da.

In some embodiments, any of the antibodies described herein can be further conjugated to a polymer to form a bioconjugate. The molecular weight of the bioconjugate (in total, SEC-MALs) can be between about 350,000 and 2,000,000 Daltons, for example, between about 450,000 and 1,900,000 Daltons, between about 550,000 and 1,800,000 Daltons, between about 650,000 and 1,700,000 Daltons, between about 750,000 and 1,600,000 Daltons, between about 850,000 and 1,500,000 Daltons, between about 900,000 and 1,400,000 Daltons, between about 950,000 and 1,300,000 Daltons, between about 900,000 and 1,000,000 Daltons, between about 1,000,000 and 1,300,000 Daltons, between about 850,000 and 1,300,000 Daltons, between about 850,000 and 1,000,000 Daltons, and between about 1,000,000 and 1,200,000 Daltons.

In some embodiments, the antibody conjugate is purified. In some embodiments, the polymer is aspect of the antibody conjugate is polydisperse, i.e. the polymer PDI is not 1.0. In some embodiments, the PDI is less than 1.5. In some

embodiments, the PDI is less than 1.4. In some embodiments, the PDI is less than 1.3. In some embodiments the PDI is less than 1.2. In some embodiments the PDI is less than 1.1.

In some embodiments, the antibody conjugate has an anti-VEGF-A immunoglobulin G (IgG) bonded to a polymer, which polymer comprises MPC monomers, wherein the sequence of the anti-VEGF-A heavy chain is SEQ ID NO. 1, and the sequence of the anti-VEGF-A light chain is SEQ ID NO. 2, and wherein the antibody is bonded only at C449 in SEQ ID NO. 1 to the polymer. In some embodiments, the polymer has 9 arms and has a molecular weight of between about 600,000 to about 1,000,000 Da.

In some embodiments, the antibody conjugate has an anti-VEGF-A immunoglobulin G (IgG) bonded to a polymer, which polymer comprises MPC monomers, wherein the sequence of the anti-VEGF-A heavy chain is SEQ ID NO. 1, and the sequence of the anti-VEGF-A light chain is SEQ ID NO. 2, and wherein the antibody is bonded only at C443 (EU numbering, or 449C in SEQ ID NO: 1) to the polymer. In some embodiments, the polymer has 9 arms and has a molecular weight of between about 600,000 to about 1,000,000 Da.

In some embodiments, the antibody conjugate has the following structure:

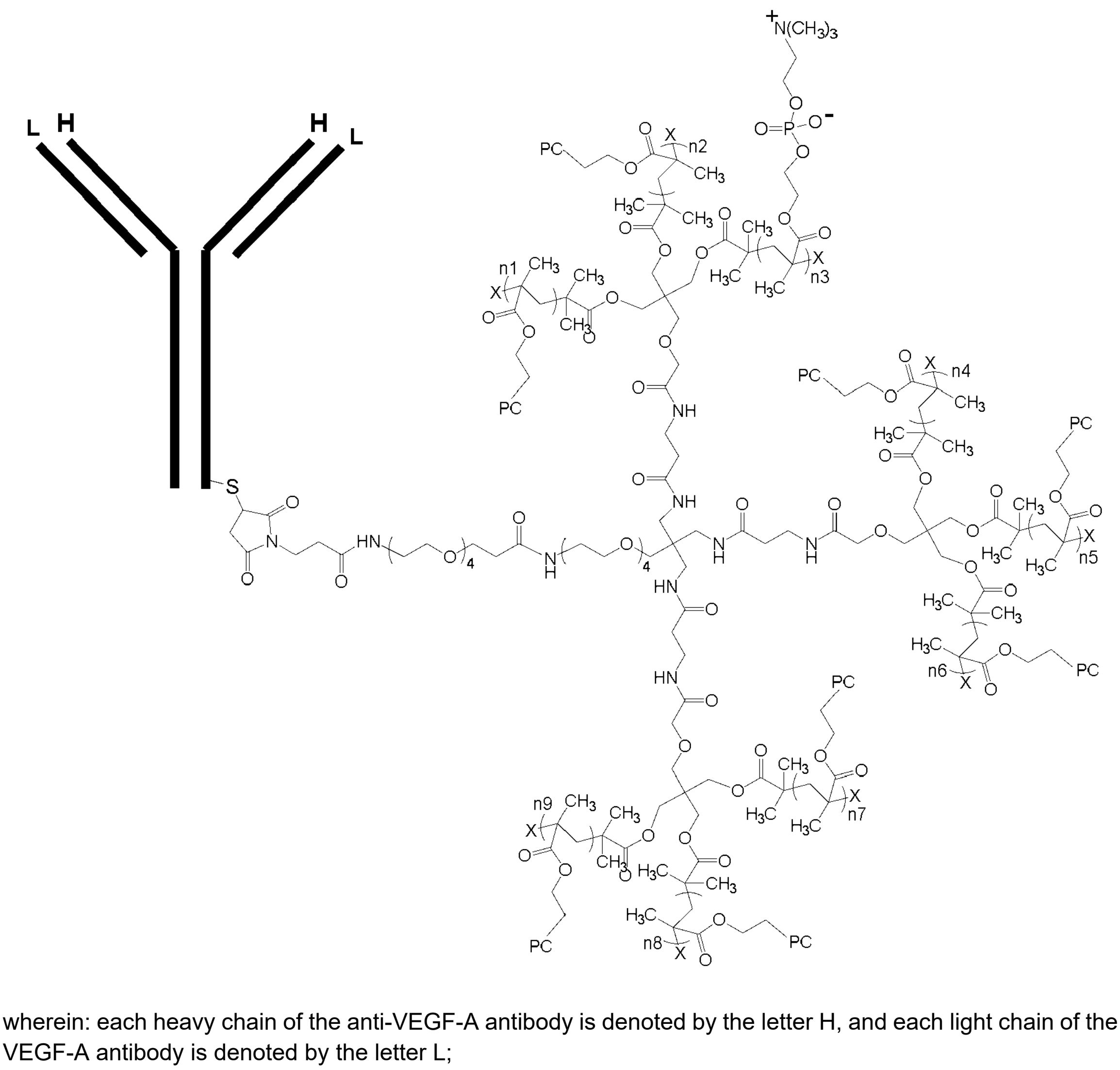

wherein: each heavy chain of the anti-VEGF-A antibody is denoted by the letter H, and each light chain of the anti-VEGF-A antibody is denoted by the letter L;

the polymer is bonded to the anti-VEGF-A antibody through the sulfhydryl of C449 of SEQ ID NO: 1, which bond is depicted on one of the heavy chains; PC is,

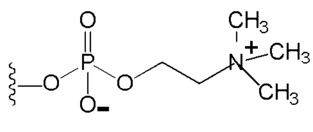

where the curvy line indicates the point of attachment to the rest of the polymer; wherein X is a) -OR where R is H,

methyl, ethyl, propyl, or isopropyl, b) -H, c) any halogen, including -Br, -Cl, or - I, d) -SCN, or e) -NCS; and n1, n2, n3, n4, n5, n6, n7, n8 and n9 are the same or different such that the sum of n1, n2, n3, n4, n5, n6, n6, n7, n8 and n9 is 2500 plus or minus 10%. In some embodiments, n1, n2, n3, n4, n5, n6, n7, n8 and n9 are the same or different and are integers from 0 to 3000. In some embodiments, n1, n2, n3, n4, n5, n6, n7, n8 and n9 are the same or different and are integers from 0 to 500. In some embodiments, X is -OR, where R is a sugar, an aminoalkyl, mono-substituted, poly-substituted or unsubstituted variants of the following residues: saturated $C_1$ -$C_{24}$ alkyl, unsaturated $C_2$ -$C_{24}$ alkenyl or $C_2$ -$C_{24}$ alkynyl, acyl, acyloxy, alkyloxycarbonyloxy, aryloxycarbonyloxy, cycloalkyl, cycloalkenyl, alkoxy, cycloalkoxy, aryl, heteroaryl, arylalkoxy carbonyl, alkoxy carbonylacyl, amino, aminocarbonyl, aminocarboyloxy, nitro, azido, phenyl, hydroxy, alkylthio, arylthio, oxysulfonyl, carboxy, cyano, and halogenated alkyl including polyhalogenated alkyl, --CO--O--$R_7$, carbonyl --CCO--$R_7$, --CO--$NR_8R_9$, --$(CH_2)_n$--$COOR_7$,CO--(CH) $_n$--$COOR_7$, --$(CH_2)$ $_n$--$NR_8R_9$, ester, alkoxycarbonyl, aryloxycarbonyl, wherein n is an integer from 1 to 6, wherein each $R_7$, $R_8$ and $R_9$ is separately selected from the group consisting of a hydrogen atom, halogen atom, mono-substituted, poly-substituted or unsubstituted variants of the following residues: saturated $C_1$- $C_{24}$ alkyl, unsaturated $C_2$ -$C_{24}$ alkenyl or $C_2$- $C_{24}$ alkynyl, acyl, acyloxy, alkyloxycarbonyloxy, aryloxycarbonyloxy, cycloalkyl, cycloalkenyl, alkoxy, cycloalkoxy, aryl, heteroaryl, arylalkoxy carbonyl, alkoxy carbonylacyl, amino, aminocarbonyl, aminocarboyloxy, nitro, azido, phenyl, hydroxy, alkylthio, arylthio, oxysulfonyl, carboxy, cyano, and halogenated alkyl including polyhalogenated alkyl, a 5-membered ring, and a 6-membered ring.

In some embodiments, the antibody conjugate has the following structure:

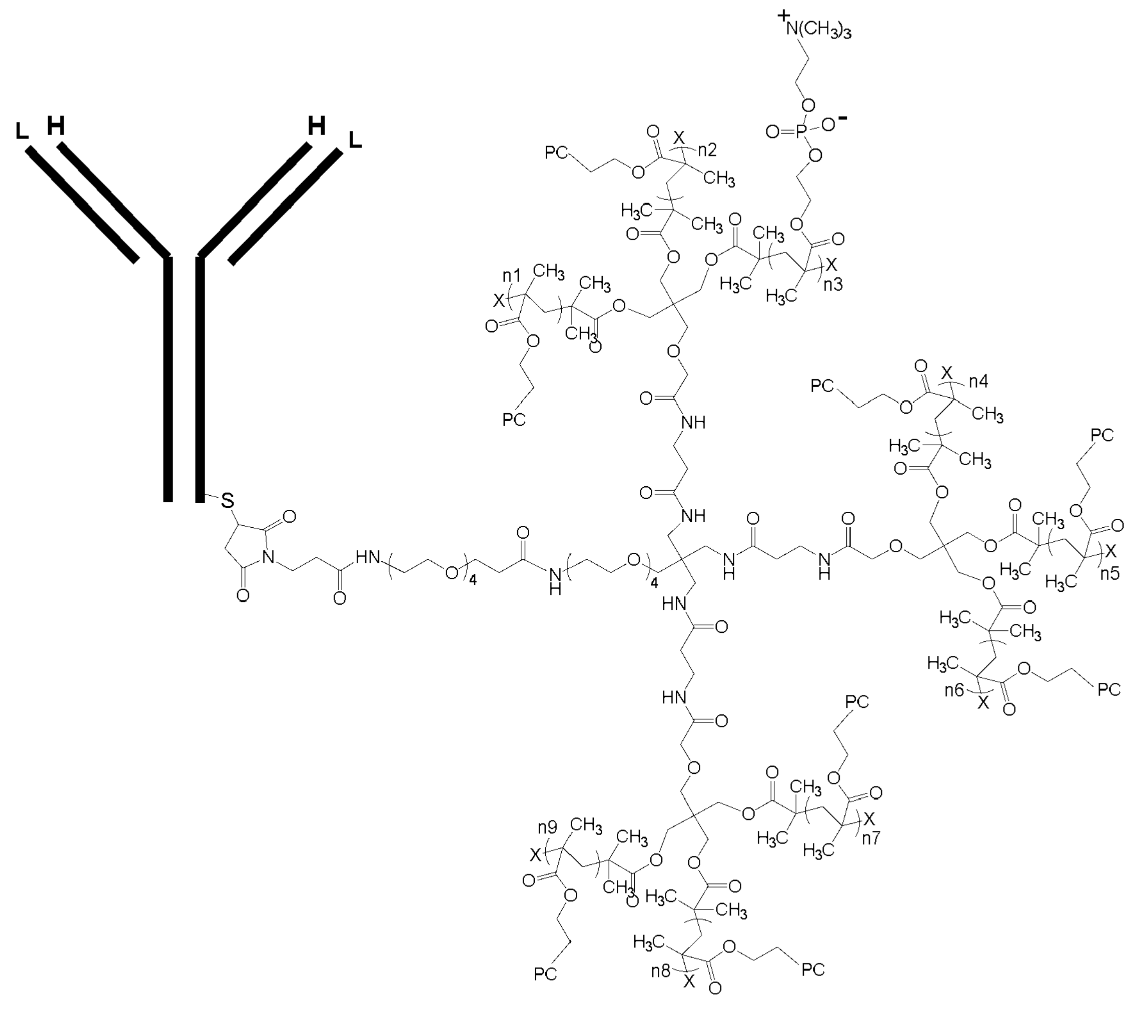

wherein: each heavy chain of the anti-VEGF-A antibody is denoted by the letter H, and each light chain of the anti-VEGF-A antibody is denoted by the letter L;
the polymer is bonded to the anti-VEGF-A antbiody through the sulfhydryl of C443 (EU numbering, or 449C in SEQ ID NO: 1), which bond is depicted on one of the heavy chains; PC is,

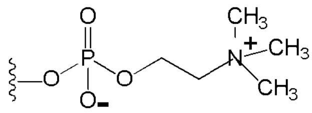

where the curvy line indicates the point of attachment to the rest of the polymer; wherein X is a) -OR where R is H, methyl, ethyl, propyl, or isopropyl, b) -H, c) any halogen, including Br, -Cl, or -I, d) -SCN, or e) -NCS; and n1, n2, n3, n4, n5, n6, n7, n8 and n9 are the same or different such that the sum of n1, n2, n3, n4, n5, n6, n6, n7, n8 and n9 is 2500 plus or minus 10%. In some embodiments, n1, n2, n3, n4, n5, n6, n7, n8 and n9 are the same or different and are integers from 0 to 3000. In some embodiments, n1, n2, n3, n4, n5, n6, n7, n8 and n9 are the same or different and are integers from 0 to 500. In some embodiments, X is -OR, where R is a sugar, an aminoalkyl, mono-substituted, poly-substituted or unsubstituted variants of the following residues: saturated $C_1$ -$C_{24}$ alkyl, unsaturated $C_2$ -$C_{24}$ alkenyl or $C_2$ -$C_{24}$ alkynyl, acyl, acyloxy, alkyloxycarbonyloxy, aryloxycarbonyloxy, cycloalkyl, cycloalkenyl, alkoxy, cycloalkoxy, aryl, heteroaryl, arylalkoxy carbonyl, alkoxy carbonylacyl, amino, aminocarbonyl, aminocarboyloxy, nitro, azido, phenyl, hydroxy, alkylthio, arylthio, oxysulfonyl, carboxy, cyano, and halogenated alkyl including polyhalogenated alkyl, --CO--O--$R_7$, carbonyl --CCO--$R_7$, --CO- -$NR_8R_9$, --$(CH_2)_n$--$COOR_7$, --CO--$(CH)_n$--$COOR_7$, --$(CH_2)_n$--$NR_8R_9$, ester, alkoxycarbonyl, aryloxycarbonyl, wherein n is an integer from 1 to 6, wherein each $R_7$, $R_8$ and $R_9$ is separately selected from the group consisting of a hydrogen atom, halogen atom, mono-substituted, poly-substituted or unsubstituted variants of the following residues: saturated $C_1$- $C_{24}$ alkyl, unsaturated $C_2$ -$C_{24}$ alkenyl or $C_2$- $C_{24}$ alkynyl, acyl, acyloxy, alkyloxycarbonyloxy, aryloxycarbonyloxy, cycloalkyl, cycloalkenyl, alkoxy, cycloalkoxy, aryl, heteroaryl, arylalkoxy carbonyl, alkoxy carbonylacyl, amino, aminocarbonyl, aminocarboyloxy, nitro, azido, phenyl, hydroxy, alkylthio, arylthio, oxysulfonyl, carboxy, cyano, and halogenated alkyl including polyhalogenated alkyl, a 5-membered ring, and a 6-membered ring. In some embodiments, this construct is designated as KSI-301.

In some embodiments, the antibody conjugate is present in a liquid formulation. In some embodiments, the antibody conjugate is combined with a pharmaceutically acceptable carrier. In some embodiments, any of the methods provided herein can use the following drug formulations: a) 12.5 mM sodium phosphate buffer with 0.025% (w/w) polysorbate 20, pH 6.5 at a concentration of 50 mg/mL (based on antibody mass), equivalent to 324 mg/mL of total mass of the antibody-biopolymer conjugate, including 50 mg/mL OG1950 Antibody Intermediate and 274 mg/mL OG1802 Biopolymer Intermediate, b)10-15 mM sodium phosphate buffer with 0.01-0.5% (w/w) polysorbate 20, pH 6.5 at a concentration of about 50 mg/mL (based on antibody mass), equivalent to about 324 mg/mL of total mass of the antibody-biopolymer conjugate, c) 12.5 mM sodium phosphate buffer with optional 0.025% (w/w) polysorbate 20, pH 6.5 at a concentration of 50 mg/mL (based on antibody mass), equivalent to 324 mg/mL of total mass of the antibody-biopolymer conjugate, including 50 mg/mL OG1950 Antibody Intermediate and 274 mg/mL OG1802 Biopolymer Intermediate, d) sodium phosphate buffer with polysorbate 20, pH 6.5 at a concentration of 50 mg/mL (based on antibody mass), equivalent to about 324 mg/mL of total mass of the antibody-biopolymer conjugate, including about 50 mg/mL OG1950 Antibody Intermediate and about 274 mg/mL OG1802 Biopolymer Intermediate, e) 10-15 mM sodium phosphate buffer with 0.01-0.5% (w/w) polysorbate 20, pH 6.5 at a concentration of about 40-55 mg/mL (based on antibody mass), equivalent to about 259-356 mg/mL of total mass of the antibody-biopolymer conjugate f) 10-15 mM sodium phosphate buffer with 0.01-0.5% (w/w) polysorbate 20, pH 6.5 at a concentration of about 45-52.5 mg/mL (based on antibody mass), equivalent to about 292-340 mg/mL of total mass of the antibody-biopolymer conjugate, or g) 10-15 mM sodium phosphate buffer with 0.01-0.5% (w/w) polysorbate 20, pH 6.5 at a concentration of about 40-55 mg/mL (based on antibody mass), equivalent to about 259-356 mg/mL of total mass of the antibody-biopolymer conjugate. In any of these formulations, the antibody can employ the VH and VL (or the 1, 2, 3, 4, 5, or all 6 CDRs within these VH and/or VL sequences) in FIG. 64, e.g., OG1950 (e.g., SEQ ID Nos: 1, 15-18 and 2.

In some embodiments, an anti-VEGF-A antibody is presented. The anti-VEGF-A antibody heavy chain has at least the following CDR sequences: $CDR_H1$: GYDFTHYGMN (SEQ ID NO: 9), $CDR_H2$: WINTYTGEPTYAADFKR (SEQ ID NO: 10), and $CDR_H3$: YPYYYGTSHWYFDV (SEQ ID NO: 11). In some embodiments, the anti-VEGF-A heavy chain has those CDRs and in addition has threonine (T) at position 221 (via sequential counting as in SEQ ID NO. 3). In some embodiments, the anti-VEGF-A light chain has at least the following CDRs: $CDR_L1$: SASQDISNYLN (SEQ ID NO: 12), $CDR_L2$: FTSSLHS (SEQ ID NO: 13) and $CDR_L3$: QQYSTVPWT (SEQ ID NO: 14). In some embodiments, the anti-VEGF-A antibody has those CDRs and in addition has leucine (L) at Kabat position 4. In some embodiments, the isotype of the anti-VEGF-A antibody heavy chain, is IgG1 and has a $CH_1$, hinge, $CH_2$ and $CH_3$ domains. In some embodiments the light chain isotype is kappa. In some embodiments, the anti-VEGF antibody conjugate (e.g., KSI-301) construct will have one or more of these CDRs.

In some embodiments, the IgG1 domain of the anti-VEGF-A antibody has one or more mutations to modulate effector function, such as ADCC, ADCP, and CDC. In some embodiments, the IgG1 mutations reduce effector function. In some embodiments the amino acids to use for effector function mutations include (EU numbering) E233X, L234X, L235X,

G236X, G237X, G236X, D270X, K322X, A327X, P329X, A330X, A330X, P331X, and P331X, in which X is any natural or non-natural amino acid. In some embodiments, the mutations include one or more of the following: E233P, L234V, L234A, L235A, G237A, A327G, A330S and P331S (EU numbering). In some embodiments, the anti-VEGF-A heavy chain has the following mutations (EU numbering): L234A, L235A and G237A. In some embodiments, the number of effector function mutations relative to a natural human IgG1 sequence is no more than 10. In some embodiments the number of effector function mutations relatative to a natural human IgG1 sequence is no more than 5, 4, 3, 2 or 1. In some embodiments, the antibody has decreased Fc gamma binding and/or complement C1q binding, such that the antibody's ability to result in an effector function is decreased. This can be especially advantageous for ophthalmic indications/disorders.

In some embodiments, the anti-VEGF-A antibody comprises one or more of the following amino acid mutations: L234A, L235A, G237A (EU numbering), and L443C (EU numbering, or 449C in SEQ ID NO: 1).

In some embodiments, the anti-VEGF-A antibody is or is part of a human immunoglobulin G (IgG1).

In some embodiments, the VEGF-A antibody comprises a heavy chain constant domain that comprises one or more mutations that reduce an immune-mediated effector function.

In some embodiments an anti-VEGF-A antibody is provided. The anti-VEGF-antibody comprises a heavy chain that comprises a $CDR_H1$ comprising the sequence GYDFTHYGMN (SEQ ID NO: 9), a $CDR_H2$ comprising the sequence WINTYTGEPTYAADFKR (SEQ ID NO: 10), a $CDR_H3$ comprising the sequence YPYYYGTSHWYFDV (SEQ ID NO: 11), a $CDR_L1$ comprising the sequence SASQDISNYLN (SEQ ID NO: 12), a $CDR_L2$ comprising the sequence FTSSLHS (SEQ ID NO: 13), and a $CDR_L3$ comprising the sequence QQYSTVPWT (SEQ ID NO: 14).

Alternatively, the IgG domain can be IgG2, IgG3 or IgG4 or a composite in which a constant regions is formed from more than one of these isotypes (e.g., CH1 region from IgG2 or IgG4, hinge, CH2 and CH3 regions from IgG1). Such domains can contain mutations to reduce and/or modulate effector function at one or more of the EU position mentioned for IgG1. Human IgG2 and IgG4 have reduced effector functions relative to human IgG1 and IgG3.

The anti-VEGF-A heavy chain has a cysteine residue added as a mutation by recombinant DNA technology which can be used to conjugate a half-life extending moiety. In some embodiments, the mutation is (EU numbering) Q347C (EU numbering) and/or L443C (EU numbering, or 449C in SEQ ID NO: 1). In some embodiments, the mutation is L443C (EU numbering, or 449C in SEQ ID NO: 1). In some embodiments, the stoichiometry of antibody to polymer is 1:1; in other words, a conjugate has one molecule of antibody conjugated to one molecule of polymer.

The half-life of the anti-VEGF-A antibodies can be extended by attachment of a "half-life ("half life") extending moieties" or "half-life ("half life") extending groups". Half-life extending moieties include peptides and proteins which can be expressed in frame with the biological drug of issue (or conjugated chemically depending on the situation) and various polymers which can be attached or conjugated to one or more amino acid side chain or end functionalities such as -SH, -OH, -COOH, -CONH2, -NH2, or one or more N- and/or O-glycan structures. Half-life extending moieties generally act to increase the *in vivo* circulatory half-life of biologic drugs.

Examples of peptide/protein half-life extending moieties include Fc fusion (Capon DJ, Chamow SM, Mordenti J, et al. Designing CD4 immunoadhesions for AIDS therapy. Nature. 1989. 337:525-31), human serum albumin (HAS) fusion (Yeh P, Landais D, Lemaitre M, et al. Design of yeast-secreted albumin derivatives for human therapy: biological and antiviral properties of a serum albumin-CD4 genetic conjugate. Proc Natl Acad Sci USA. 1992. 89:1904-08 ), carboxy terminal peptide (CTP) fusion (Fares FA, Suganuma N. Nishimori K, et al. Design of a long-acting follitropin agonist by fusing the C-terminal sequence of the chorionic gonadotropin beta subunit to the follitropin beta subunit. Proc Natl Acad Sci USA. 1992. 89:4304-08), genetic fusion of non-exact repeat peptide sequence (XTEN) fusion (Schellenberger V, Wang CW, Geething NC, et al. A recombinant polypeptide extends the in vivo half-life of peptides and proteins in a tunable manner. Nat Biotechnol. 2009. 27:1186-90), elastin like peptide (ELPylation) (MCpherson DT, Morrow C, Minehan DS, et al. Production and purification of a recombinant elastomeric polypeptide, G(VPGVG19-VPGV, from Escheriachia coli. Biotechnol Prog. 1992. 8:347-52), human transferrin fusion (Prior CP, Lai C-H, Sadehghi H et al. Modified transferrin fusion proteins. Patent WO2004/020405. 2004), proline-alanine-serine (PASylation) (Skerra A, Theobald I, Schlapsky M. Biological active proteins having increased in vivo and/or vitro stability. Patent WO2008/155134 A1. 2008), homo-amino acid polymer (HAPylation) (Schlapschy M, Theobald I, Mack H, et al. Fusion of a recombinant antibody fragment with a homo-amino acid polymer: effects on biophysical properties and prolonged plasma half-life. Protein Eng Des Sel. 2007. 20:273-84) and gelatin like protein (GLK) fusion (Huang Y-S, Wen X-F, Zaro JL, et al. Engineering a pharmacologically superior form of granulocyte-colony-stimulating-factor by fusion with gelatin-like protein polymer. Eur J. Pharm Biopharm. 2010. 72:435-41).

Examples of polymer half-life extending moieties include polyethylene glycol (PEG), branched PEG, PolyPEG® (Warwick Effect Polymers; Coventry, UK), polysialic acid (PSA), starch, hydroxylethyl starch (HES), hydroxyalkyl starch (HAS), carbohydrate, polysaccharides, pullulane, chitosan, hyaluronic acid, chondroitin sulfate, dermatan sulfate, dextran, carboxymethyl-dextran, polyalkylene oxide (PAO), polyalkylene glycol (PAG), polypropylene glycol (PPG), polyoxazoline, polyacryloylmorpholine, polyvinyl alcohol (PVA), polycarboxylate, polyvinylpyrrolidone, polyphosphazene, polyoxazoline, polyethylene-co-maleic acid anyhydride, polystyrene-co-maleic acid anhydride, poly(1-hydroxymethyethylene hydroxymethylformal) (PHF), a zwitterionic polymer, a phosphorylcholine containing polymer and a

polymer comprising MPC, Poly ($Gly_x$-$Ser_y$), Hyaluronic acid (HA), Heparosan polymers (HEP), Fleximers, Dextran, and Poly-sialic acids (PSA).

In one embodiment a half-life extending moiety can be conjugated to an antibody via free amino groups of the protein using N-hydroxysuccinimide (NHS) esters. Reagents targeting conjugation to amine groups can randomly react to ε-amine group of lysines, α-amine group of N-terminal amino acids, and δ-amine group of histidines.

However, the anti-VEGF-A antibodies disclosed herein have many amine groups available for polymer conjugation. Conjugation of polymers to free amino groups, thus, might negatively impact the ability of the antibody proteins to bind to VEGF.

In some embodiments, a half-life extending moiety is coupled to one or more free SH groups using any appropriate thiol-reactive chemistry including, without limitation, maleimide chemistry, or the coupling of polymer hydrazides or polymer amines to carbohydrate moieties of the antibody after prior oxidation. In some embodiments maleimide coupling is used. In some embodiments, coupling occurs at cysteines naturally present or introduced via genetic engineering.

In some embodiments, polymers are covalently attached to cysteine residues introduced into anti-VEGF-A antibodies by site directed mutagenesis. In some embodiments, the cysteine residues are employed in the Fc portion of the antibody. In some embodiments, the sites to introduce cysteine residues into an Fc region are provided in WO 2013/093809, US 7,521,541, WO 2008/020827, US 8,008,453, US 8,455,622 and US2012/0213705, incorporated herein by reference for all purposes. In some embodiments, the cysteine mutations are Q347C (EU numbering) and L443C referring to the human IgG heavy chain by EU numbering.

In some embodiments, conjugates of antibody and high MW polymers serving as half-life extenders are provided. In some embodiments, a conjugate comprises an antibody that is coupled to a zwitterionic polymer wherein the polymer is formed from one or more monomer units and wherein at least one monomer unit has a zwitterionic group is provided. In some embodiments, the zwitterionic group is phosphorylcholine.

In some embodiments, one of the monomer units is HEMA-PC. In some embodiments, a polymer is synthesized from a single monomer which is HEMA-PC.

In some embodiments, some antibody conjugates have 2, 3, or more polymer arms wherein the monomer is HEMA-PC. In some embodiments, the conjugates have 2, 3, 4, 5, 6, 7, 8, 9, 10, 11, or 12 polymer arms wherein the monomer is HEMA-PC. In some embodiments, the conjugates have 3, 6 or 9 arms. In some embodiments, the conjugate has 9 arms.

In some embodiments, polymer-antibody conjugates have a polymer portion with a molecular weight of between 100,000 and 1,500,000 Da. In some embodiments, the conjugate has a polymer portion with a molecular weight between 500,000 and 1,000,000 Da. In some embodiments, the conjugate has a polymer portion with a molecular weight between 600,000 to 800,000 Da. In some embodiments, the conjugate has a polymer portion with a molecular weight between 600,000 and 850,000 Da and has 9 arms. When a molecular weight is given for an antibody conjugated to a polymer, the molecular weight will be the addition of the molecular weight of the protein, including any carbohydrate moieties associated therewith, and the molecular weight of the polymer.

In some embodiments, an anti-VEGF-A antibody has a HEMA-PC polymer which has a molecular weight measured by Mw of between about 100 kDa and 1650 kDa is provided. In some embodiments, the molecular weight of the polymer as measured by Mw is between about 500 kDa and 1000 kDa. In some embodiments, the molecular weight of the polymer as measured by Mw is between about 600 kDa to about 900 kDa. In some embodiments, the polymer molecular weight as measured by Mw is 750 kDa plus or minus 15%.

In some embodiments, the polymer is made from an initiator suitable for ATRP having one or more polymer initiation sites. In some embodiments, the polymer initiation site has a 2-bromoisobutyrate site. In some embodiments, the initiator has 3 or more polymer initiation sites. In some embodiments, the initiator has 3, 4, 5, 6, 7, 8, 9, 10, 11 or 12 polymer initiation sites. In some embodiments, the initiator has 3, 6 or 9 polymer initiation sites. In some embodiments, the initiator has 9 polymer initiation sites. In some embodiments, the initiator is OG1786.

The anti-VEGF-A antibodies can be produced by recombinant expression including (i) the production of recombinant DNA by genetic engineering, (ii) introducing recombinant DNA into prokaryotic or eukaryotic cells by, for example and without limitation, transfection, electroporation or microinjection, (iii) cultivating the transformed cells, (iv) expressing antibody, *e.g.* constitutively or on induction, and (v) isolating the antibody, *e.g.* from the culture medium or by harvesting the transformed cells, in order to (vi) obtain purified antibody.

The anti-VEGF-A antibodies can be produced by expression in a suitable prokaryotic or eukaryotic host system characterized by producing a pharmacologically acceptable antibody molecule. Examples of eukaryotic cells are mammalian cells, such as CHO, COS, HEK 293, BHK, SK-Hip, and HepG2. Other suitable expression systems are prokaryotic (e.g., E. coli with pET/BL21 expression system), yeast (Saccharomyces cerevisiae and/or Pichia pastoris systems), and insect cells.

A wide variety of vectors can be used for the preparation of the antibodies disclosed herein and are selected from eukaryotic and prokaryotic expression vectors. Examples of vectors for prokaryotic expression include plasmids such as,

and without limitation, preset, pet, and pad, wherein the promoters used in prokaryotic expression vectors include one or more of, and without limitation, lac, trc, trp, recA, or araBAD. Examples of vectors for eukaryotic expression include: (i) for expression in yeast, vectors such as, and without limitation, pAO, pPIC, pYES, or pMET, using promoters such as, and without limitation, AOX1, GAP, GAL1, or AUG1; (ii) for expression in insect cells, vectors such as and without limitation, pMT, pAc5, pIB, pMIB, or pBAC, using promoters such as and without limitation PH, p10, MT, Ac5, OpIE2, gp64, or polh, and (iii) for expression in mammalian cells, vectors such as, and without limitation, pSVL, pCMV, pRc/RSV, pcDNA3, or pBPV, and vectors derived from, in one aspect, viral systems such as and without limitation vaccinia virus, adeno-associated viruses, herpes viruses, or retroviruses, using promoters such as and without limitation CMV, SV40, EF-1, UbC, RSV, ADV, BPV, and beta-actin.

Method of Conjugating Proteins to Polymers

In some embodiments, a method is presented of preparing a therapeutic protein-half life extending moiety conjugate having the step of conjugating a therapeutic protein which has a cysteine residue added via recombinant DNA technology to a half-life extending moiety having a sulfhydryl specific reacting group selected from the group consisting of maleimide, vinylsulfones, orthopyridyl-disulfides, and iodoacetamides to provide the therapeutic protein-half life extending moiety conjugate.

In some embodiments a method of preparing the anti-VEGF antibody conjugate, e.g., KSI-301, from OG1950 is provided. The method comprises reducing the OG1950 protein with a 50x molar excess of the TCEP reducing agent. After reduction, the antiobody is oxidized to produce a decapped OG1950 antibody where the inter- and intra- light and heavy chain disulfide bonds naturally occurring in the antibody are formed, but the engineered Cysteine on the heavy chain position L443C (EU numbering, or 449C in SEQ ID NO: 1) remains to be decapped. The OG1950 is then conjugated by adding an excipient and adding 5-10x molar excess of a maleimide biopolymer. The biopolymer links to the OG1950 antibody through a covalent thiolether linkage. After conjugation, the anti-VEGF antibody conjugate, e.g., KSI-301, is purified with both unconjugated antibody and polymer removed.

The protein and process described above can be varied as well. Thus, in some embodiments, a process for preparing a conjugated protein (which need not be an antibody or an anti-VEGF antibody) is provided. The process includes reducing one or more cysteines in a protein to form a decapped protein in a solution. After reducing the one or more cysteines the decapped protein is reoxidized to restore at least one disulfide linkage in the reduced protein while ensuring that an engineered cysteine residue in the protein remains in a free thiol form to form a reoxidized decapped protein in the solution. At least one excipient is then added to the solution. The excipient reduces a polymer induced protein precipitation. After the excipient is added, a polymer is added to the solution, which is conjugated to the reoxidized decapped protein at the engineered cysteine residue to form a conjugated protein.

In some embodiments, the molar excess of the reducing agent can be altered to any amount that functions. In some embodiments 10, 20, 30, 40, 50, 60, 70, 80, 90x molar excess of the reducing agent (which need not be TCEP in all embodiments) can be employed. In some embodiments, any antibody (therapeutic or otherwise) can be employed. In some embodiments, 1, 2, 3, 4, 5, 6, 7, 8, 9, 10, 11, 12, 13, 14, 15x molar excess of a maleimide biopolymer can be employed. In some embodiments, there is an excess of decapped protein to polymer. In some embodiments, the amount of the reduced protein is less than the amount of the polymer. In some embodiments, the amount of the reduced protein is 90%, 80%, 70%, 60%, 50%, 40%, 30%, 20%, 10%, 5%, 4%, 3%, 2%, 1% of the amount of the polymer. In some embodiments, 10-15 times as much polymer is used as protein. In some embodiments the amount of the reduced antibody is greater than the amount of the polymer. In some embodiments the amount of the polymer is greater than the amount of the reduced antibody.

In some embodiments, the purification step is optional.

In some embodiments, the method of making an antibody conjugate comprises conjugating an anti-VEGF-A antibody to a phosphorylcholine containing polymer. In some embodiments the method comprises the steps of conjugating an anti-VEGF-A antibody to a phosphorylcholine containing polymer. The anti-VEGF-A antibody comprises an amino residue added via recombinant DNA technology. In some embodiments, the added amino acid residue is a cysteine residue. In some embodiments, the cysteine residue is added outside a variable region of the antibody. The cysteine residue can be added to either the heavy chain or light chain of the antibody.

In some embodiments, the polymer comprises or consists of a phosphorylcholine containing polymer. In some embodiments, the phosphorylcholine containing polymer comprises a sulfhydryl specific reacting group selected from the group consisting of a maleimide, a vinylsulfone, an orthopyridyl-disulfide, and an iodoacetamide. In some embodiments, the sulfhydryl specific reacting group on the phosphorylcholine containing polymer reacts with the cysteine residue on the anti-VEGF-A antibody to make the antibody conjugate.

In some embodiments, the protein to be conjugated can be an antibody, an antibody protein fusion, or a binding fragment thereof. In some embodiments, the protein is not an antibody but is an enzyme, a ligand, a receptor, or other protein or mutants or variants thereof. In some embodiments, the native protein contains at least one disulfide bond and at

least one non-native cysteine.

In some embodiments, the excipient can be an acid or a base. In some embodiments, the excipient is a detergent, a sugar, or a charged amino acid. In some embodiments, the excipient assists in keeping the protein in solution during the conjugation to the polymer. In some embodiments, the excipient is added to the solution containing the protein, prior to the addition of the polymer to the solution that contains the protein.

In some embodiments, the reaction occurs under aqueous conditions between about pH 5 to about pH 9. In some embodiments, the reaction occurs between 6.0 and 8.5, between 6.5 and 8.0 or between 7.0 and 7.5.

In some embodiments, the polymer is conjugated to the protein at 2-37 degrees Celsius. In some embodiments, the conjugation occurs at 0-40 degrees Celsius, 5-35 degrees Celsius, 10-30 degrees Celsius, and 15-25 degrees Celsius.

In some embodiments, the conjugated proteins described herein can be contacted to an ion exchange medium or hydrophobic interaction chromatography or affinity chromatography medium for purification (to remove the conjugated from the unconjugated). In some embodiments, the ion exchange medium, hydrophobic interaction chromatography, and/or affinity chromatography medium separates the conjugated protein from the free polymer and from the reoxidized decapped protein.

In some embodiments, the processes described herein and outlined in FIG. 18 involves an excipient that is capable of facilitating and/or maintaining a solubility system. In some embodiments, the process allows the solution to maintain the solubility of the two components meant to interact. This can include the solubility of the protein and the polymer and then the end conjugate as well. In some embodiments, without the excipient approach, the issue can be that while the protein is soluble, when the biopolymer is added, the solubility of the solution (e.g., protein) drops and it crashes/precipitates out of solution. Of course, when the protein crashes out, it is not available to conjugate efficiently with the biopolymer. Thus, an excipient can be employed to maintain the solubility of the protein in the presence of the biopolymer so the two can couple to form the protein conjugate (or as depicted in FIG. 18, an antibody conjugate). This also allows for the solubility of the conjugate to be maintained.

In some embodiments, the polymers disclosed herein can comprise one or more of the following: a zwitterion, a phosphorylcholine, or a PEG linker bridging a center of a polymer branching point to the maleimide functional group. In some embodiments, any of the polymers provided herein can be added to a protein via the methods provided herein.

In some embodiments, any of the proteins provided herein can be conjugated to any of the polymers provided herein via one or more of the methods provided herein.

In some embodiments, the process(es) provided herein allow(s) for larger scale processing to make and purify protein and/or antibody conjugates. In some embodiments, the volume employed is at least 1 liter, for example 1, 10, 100, 1,000, 5,000, 10,000, liters or more. In some embodiments, the amount of the antibody conjugate produced and/or purified can be 0.1, 1, 10, 100, 1000, or more grams.

In some embodiments, the therapeutic protein may be any of the anti-VEGF-A antibodies described herein having a cysteine residue added via recombinant DNA technology. In some embodiments, the anti-VEGF antibody heavy chain has the following CDRs: $CDR_H1$: GYDFTHYGMN (SEQ ID NO: 9), $CDR_H2$: WINTYTGEPTYAADFKR (SEQ ID NO: 10), and $CDR_H3$: YPYYYGTSHWYFDV (SEQ ID NO: 11). The heavy chain can also have threonine (T) at position 221 (via sequential counting as in SEQ ID NO. 3). In some embodiments, the anti-VEGF light chain has the following CDRs: $CDR_L1$: SASQDISNYLN (SEQ ID NO: 12), $CDR_L2$: FTSSLHS (SEQ ID NO: 13), and $CDR_L3$: QQYSTVPWT (SEQ ID NO: 14). The anti-VEGF-A light chain can also have leucine (L) at Kabat position 4.

In some embodiments, the anti-VEGF-A antibody is IgG1. In some embodiments, the heavy chain has one or more mutations to modulate effector function. In some embodiments, the mutations are to one or more of the following amino acid positions (EU numbering): E233, L234, L235, G236, G237, A327, A330, and P331. In some embodiments, the mutations are selected from the group consisting of: E233P, L234V, L234A, L235A, G237A, A327G, A330S and P331S (EU numbering). In some embodiments, the mutations are (EU numbering) L234A, L235A and G237A.

In some embodiments, the cysteine residue added to the therapeutic protein via recombinant DNA technology should not be involved in Cys-Cys disulfide bond pairing. In this regard, therapeutic proteins may be dimeric. So for example, an intact anti-VEGF-A antibody has two light chains and two heavy chains. If a Cys residue is introduced into the heavy chain for instance, the intact antibody will have two such introduced cysteines at identical positions and the possibility exists that these cysteine residues will form intra-chain disulfide bonds. If the introduced cysteine residues form Cys-Cys disulfide bonds or have a propensity to do so, that introduced Cys residue will not be useful for conjugation. It is known in the art how to avoid positions in the heavy and light chains that will give rise to intra-chain disulfide pairing. See, e.g., U.S. Patent Application No. 2015/0158952.

In some embodiments, the cysteine residue introduced via recombinant DNA technology is selected from the group consisting of(EU numbering) Q347C and L443C. In some embodiments, the cysteine residue is L443C (EU numbering, or 449C in SEQ ID NO: 1). In some embodiments, the heavy chain the antibody has the amino acid sequence set forth in SEQ ID NO. 1 and the light chain has the amino acid sequence of SEQ ID NO. 2.

In some embodiments, the sulfhydral specific reacting group is maleimide.

In some embodiments, the half-life extending moiety is selected from the group consisting of polyethylene glycol (PEG), branched PEG, PolyPEG® (Warwick Effect Polymers; Coventry, UK), polysialic acid (PSA), starch, hydroxylethyl starch (HES), hydroxyalkyl starch (HAS), carbohydrate, polysaccharides, pullulane, chitosan, hyaluronic acid, chondroitin sulfate, dermatan sulfate, dextran, carboxymethyl-dextran, polyalkylene oxide (PAO), polyalkylene glycol (PAG), polypropylene glycol (PPG), polyoxazoline, polyacryloylmorpholine, polyvinyl alcohol (PVA), polycarboxylate, polyvinylpyrrolidone, polyphosphazene, polyoxazoline, polyethylene-co-maleic acid anyhydride, polystyrene-co-maleic acid anhydride, poly(1-hydroxymethyethylene hydroxymethylformal) (PHF), a zwitterionic polymer, a phosphorylcholine containing polymer and a polymer comprising 2-methacryloyloxy-2'-ethyltrimethylammoniumphosphate (MPC).

In some embodiments, the half-life extending moiety is a zwitterionic polymer. In some embodiments, the zwitterion is phosphorylcholine, i.e. a phosphorylcholine containing polymer. In some embodiments, the polymer is composed of MPC units.

In some embodiments, the MPC polymer has three or more arms. In some embodiments, the MPC polymer has 2, 3, 4, 5, 6, 7, 8, 9, 10, 11 or 12 arms. In some embodiments, the MPC polymer has 3, 6, or 9 arms. In some embodiments, the MPC polymer has 9 arms. In some embodiments, the polymer is synthesized with an initiator comprising 2, 3, 4, 5, 6, 7, 8, 9, 10, 11, 12, or more polymer initiation sites

In some embodiments, the MPC polymer has a molecular weight between about 300,000 and 1,750,000 Da. In some embodiments, the MPC polymer has a molecular weight between about 500,000 and 1,000,000 Da or between about 600,000 to 900,000 Da.

In some embodiments, the method of preparing a therapeutic protein-half life extending moiety conjugate has an additional step of contacting the therapeutic protein with a thiol reductant under conditions that produce a reduced cysteine sulfhydryl group. As discussed above, it is preferable that the cysteine residue added via recombinant DNA technology are unpaired, i.e. are not involved in Cys-Cys intra chain disulfide bonds or are not substantially involved in such bonding. However, Cys residues which are not involved in such Cys-Cys disulfide bonding and are free for conjugation are known to react with with free cysteine in the culture media to form disulfide adducts. See, e.g., WO 2009/052249. A cysteine so derivatized will not be available for conjugation. To free the newly added cysteine from the disulfide adduct, the protein after purification is treated with a reducing agent, e.g., dithiothreitol. However, such treatment with a reducing agent will reduce all of the cysteine residues in the therapeutic protein, including native cysteines many of which are involved in inter and intra chain Cys-Cys disulfides bonds. The native Cys-Cys disulfides are generally crucial to protein stability and activity and they should be reformed. In some embodiments, all native (e.g., inter and intra) Cys-Cys disulfides are reformed.

To reform native inter and intra-chain disulfide residues, after reduction to remove the cysteine disulfide adducts, the therapeutic protein is exposed to oxidizing conditions and/or oxidizing agents for a prescribed period of time, e.g., overnight. In some embodiments, ambient air exposure overnight can be used to achieve reformation of the native disulfide bonds. In some embodiments, an oxidizing agent is employed to restore the native disulfides. In some embodiments, the oxiding agent is selected from the group consisting of acqueous CuSO4 and dehydroascorbic acid (DHAA). In some embodiments, the oxidizing agent is DHAA. In some embodiments, the range of DHAA used is in the range of 5-30 equivalents. In some embodiments, the range is 10-20 equivalents. In some embodiments, the range is 15 equivalents.

In some embodiments, the thiol reductant is selected from the group consisting of: Tris[2-carboxyehtyl]phosphine hydrochloride (TCEP), dithiothreitol (DTT), dithioerythritol (DTE), sodium borohydride ($NaBH_4$), sodium cyanoborohydride (NaCNBH3), β-mercaptoethanol (BME), cysteine hydrochloride and cysteine. In some embodiments, the thiol reductant is TCEP.

In some embodiments, the thiol reductant concentration is between 1 and 100 fold molar excess relative to the therapeutic protein concentration. In some embodiments, the thiol reductant concentration is between 20 to 50 fold molar excess relative to the therapeutic protein concentration. In some embodiments, the thiol reductant is removed following incubation with the therapeutic protein prior to oxidation of the therapeutic protein.

In some embodiments, the method for conjugating a therapeutic protein to a half-life extending moiety has a further step of purifying the therapeutic protein conjugate after conjugation. In some embodiments, the therapeutic protein conjugate is purified using a technique selected from the group consisting of ion exchange chromatography, hydrophobic interaction chromatography, size exclusion chromatography, and affinity chromatography or combinations thereof.

In some embodiments, the therapeutic protein conjugate retains at least 20% biological activity relative to unconjugated therapeutic protein. In some embodiments, the therapeutic protein conjugate retains at least 50% biological activity relative to unconjugated therapeutic protein. In some embodiments, the therapeutic protein conjugate retains at least 90% biological activity relative to native therapeutic protein.

In some embodiments, the therapeutic protein conjugate has an increased half-life relative to unconjugated therapeutic protein. In some embodiments, the therapeutic protein conjugate has at least a 1.5 fold increase in half-life relative to unconjugated therapeutic protein. In some embodiments, the therapeutic protein conjugate has at least a 5 fold increase in half-life relative to unconjugated therapeutic protein.

In some embodiments, the zwitterionic polymer of the method of conjugating a therapeutic protein to a half-life

extending moiety is a radically polymerizable monomer having a zwitterionc group and the method has a further step of polymerizing the free radically polymerizable zwitterionic monomer in a polymerization medium to provide a polymer, the medium comprising: the radically polymerizable zwitterionic monomer; a transition metal catalyst $M_t^{(q-1)+}$ wherein $M_t$ is a transition metal, q is a higher oxidation state of the metal and q-1 is a lower oxidation state of the metal, wherein the metal catalyst is supplied as a salt of the form $Mt^{(q-1)+}X'_{(q-1)}$ wherein X' is a counterion or group or the transition metal catalyst is supplied in situ by providing the inactive metal salt at its higher oxidation state $M_t^{q+}X'_q$ together with a reducing agent that is capable of reducing the transition metal from the oxidized inactive state to the reduced active state; a ligand; and an initiator.

To function as an ATRP transition metal catalyst, the transition metal should have at least two readily accessible oxidation states separated by one electron, a higher oxidation state and a lower oxidation state. In ATRP, a reversible redox reaction results in the transition metal catalyst cycling between the higher oxidation state and the lower oxidation state while the polymer chains cycle between having propagating chain ends and dormant chain ends. See, e.g., U.S. Patent No. 7,893,173.

In some embodiments, the radically polymerizable zwitterionic monomer is selected from the group consisting of

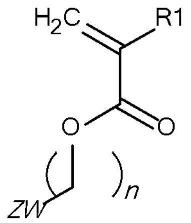 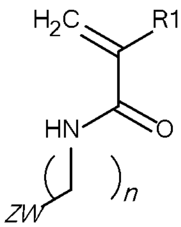 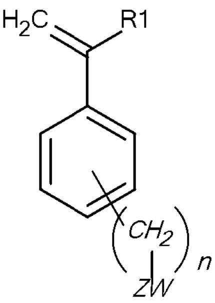 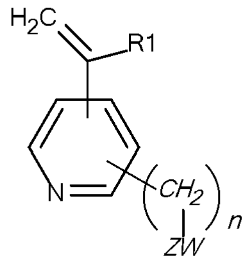

wherein R1 is H or $C_{1-6}$ alkyl, ZW is a zwitterion and n is an integer from 1-6.

In some embodiments, the radically polymerizable monomer is

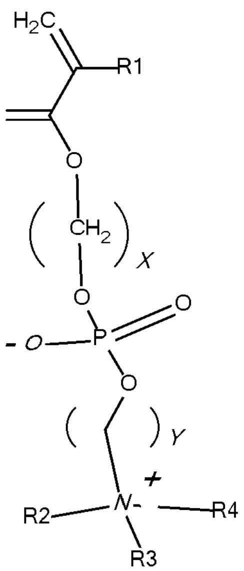

wherein R1 is H or $C_{1-6}$ alkyl, R2, R3, R4 are the same or different and are H or $C_{1-4}$alkyl and X and Y are the same or different and are integers from 1-6. In some embodiments, R1, R2, R3 and R4 are each methyl and X and Y are each 2.

In some embodiments, the radically polymerizable monomer is

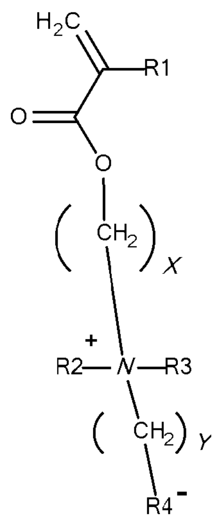

wherein R1 is H or $C_{1-6}$alkyl, R2 and R3 are the same or different and are H or $C_{1-4}$alkyl, R4 is $PO_4$-, $SO_3$- or $CO_2$- and X and Y are the same or different and are integers from 1-6. In some embodiments, R1, R2 and R3 are methyl, R4 is $PO_4$- and X and Y are each 2.

In some embodiments, the monomer is

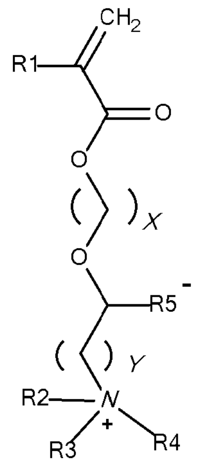

wherein R1 is H or $C_{1-6}$alkyl, R2, R3 and R4 are the same or different and are H or $C_{1-4}$alkyl, R5 is $PO_4$-, $SO_3$- or $CO_2$- and X and Y are the same or different and are integers from 1-6. In some embodiments, R1, R2, R3 and R4 are methyl, R5 is $PO_4$- and X and Y are 2.

In some embodiments, the transition metal Mt is selected from the group consisting of Cu, Fe, Ru, Cr, Mo, W, Mn, Rh, Re, Co, V, Zn, Au, and Ag. In some embodiments, the metal catalyst is supplied as a salt of the form $Mt^{(q-1)+}X'_{(q-1)}$. $M_t^{(q-1)+}$ is selected from the group consisting of $Cu^{1+}$, $Fe^{2+}$, $Ru^{2+}$, $Cr^{2+}$, $Mo^{2+}$, $W^{2+}$, $Mn^{3+}$, $Rh^{3+}$, $Re^{2+}$, $Co^+$, $V^{2+}$, $Zn^+$, $Au^+$, and $Ag^+$ and X' is selected from the group consisting of halogen, $C_{1-6}$ alkoxy, $(SO_4)_{1/2}$, $(PO_4)_{1/3}$, $(R7PO_4)_{1/2}$, $(R7_2PO_4)$, triflate, hexaluorophosphate, methanesulfonate, arylsulfonate, CN and $R7CO_2$, where R7 is H or a straight or branched $C_{1-6}$ alkyl group which may be substituted from 1 to 5 times with a halogen. In some embodiments, $M_t^{(q-1)+}$ is $Cu^{1+}$ and X' is Br.

In some embodiments, $M_t^{(q-1)+}$ is supplied in situ. In some embodiments, $M_t^{q+}X_q$ is $CuBr_2$. In some embodiments, the reducing agent is an inorganic compound. In some embodiments, the reducing agent is selected from the group consisting of a sulfur compound of a low oxidation level, sodium hydrogen sulfite, an inorganic salt comprising a metal ion, a metal, hydrazine hydrate and derivatives of such compounds. In some embodiments, the reducing agent is a metal. In some embodiments, the reducing agent is $Cu^0$.

In some embodiments, the reducing agent is an organic compound. In some embodiments, the organic compound is selected from the group consisting of alkylthiols, mercaptoethanol, or carbonyl compounds that can be easily enolized, ascorbic acid, acetyl acetonate, camphosulfonic acid, hydroxy acetone, reducing sugars, monosaccharides, glucose, aldehydes, and derivatives of such organic compounds.

In some embodiments, the ligand is selected from the group consisting of 2,2'-bipyridine, 4,4'-Di-5-nonyl-2,2'-bipyridine, 4,4-dinonyl-2,2'-dipyridyl, 4,4',4"-tris(5-nonyl)-2,2':6',2"-terpyridine, N,N,N',N',N"-Pentamethyldiethylenetriamine, 1,1,4,7,10,10-Hexamethyltriethylenetetramine, Tris(2-dimethylaminoethyl)amine, N,N-bis(2-pyridylmethyl)octadecylamine, N,N,N',N'-tetra[(2-pyridal)methyl]ethylenediamine, tris[(2-pyridyl)methyl]amine, tris(2-aminoethyl)amine, tris(2-bis(3-butoxy-3-oxopropyl)aminoethyl)amine, tris(2-bis(3-(2-ethylhexoxy)-3-oxopropyl)aminoethyl)amine and Tris(2-bis(3-dodecoxy-3-oxopropyl)aminoethyl)amine. In some embodiments, the ligand is 2,2'-bipyridine.

In some embodiments the initiator has the structure:

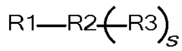

wherein R1 is a nucleophilic reactive group, R2 comprises a linker, and R3 comprises a polymer synthesis initiator moiety having the structure

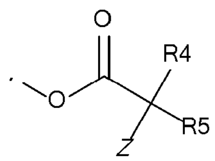

wherein R4 and R5 and are the same or different and are selected from the group consisting of alkyl, substituted alkyl, alkylene, alkoxy, carboxyalkyl, haloalkyl, cycloalkyl, cyclic alkyl ether, alkenyl, alkenylene, alkynyl, alkynylene, cycloalkylene, heterocycloalkyl, heterocycloalkylene, aryl, arylene, arylene-oxy, heteroaryl, amino, amido or any combination thereof; Z is a halogen, - OR (where R is -H, methyl, ethyl, propyl, or isopropyl), -SCN or -NCS; and s is an integer between 1 and 20.

In some embodiments, Z is Br and R4 and R5 are each methyl. In some embodiments, R1 is selected from the group consisting of $-NH_2$, -OH, and -SH.

In some embodiments R2 is alkyl, substituted alkyl, alkylene, alkoxy, carboxyalkyl, haloalkyl, cycloalkyl, cyclic alkyl ether, alkenyl, alkenylene, alkynyl, alkynylene, cycloalkylene, heterocycloalkyl, heterocycloalkylene, aryl, arylene, arylene-oxy, heteroaryl, amino, amido or any combination thereof. In some embodiments, R2 is

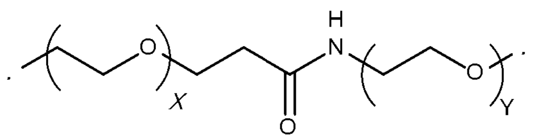

wherein X and Y are the same or different and are integers from 1-20. In some embodiments, X and Y are each 4.

In some embodiments, R3 is

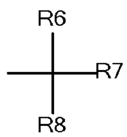

wherein R6, R7 and R8 are the same or different and are selected from the group consisting of

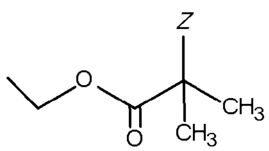

,

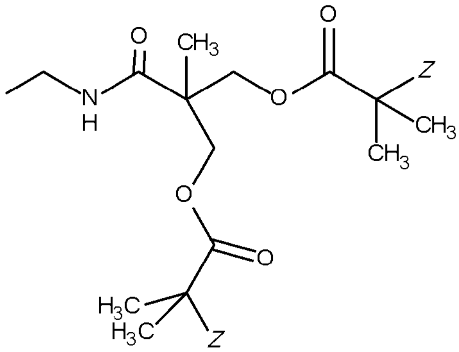

and

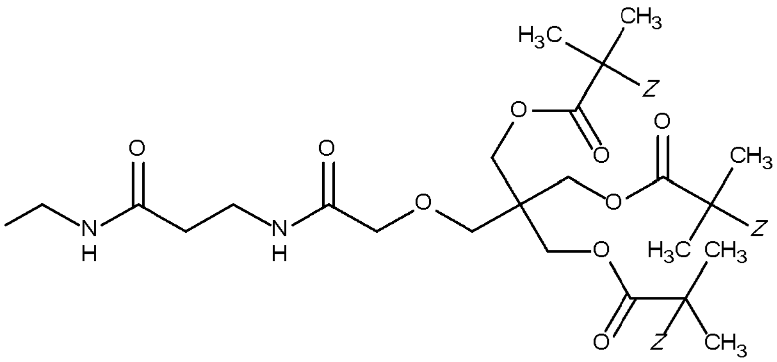

wherein Z is -OR (where R is -H, methyl, ethyl, propyl, or isopropyl), -SCN, -NCS, -F, -Cl, - Br or -I. In some embodiments, Z is -Br and R6, R7 and R8 are each

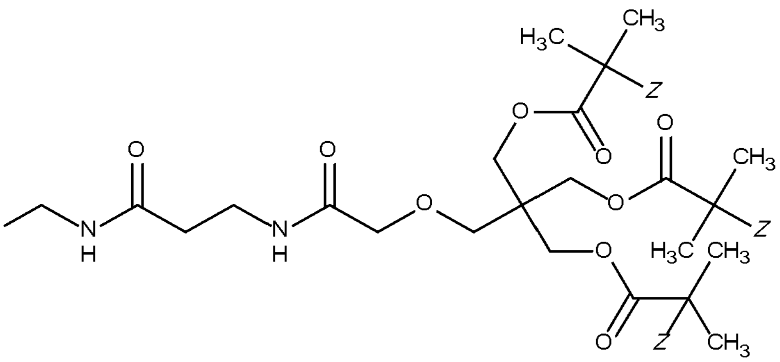

.

In some embodiments, the initiator has the structure:

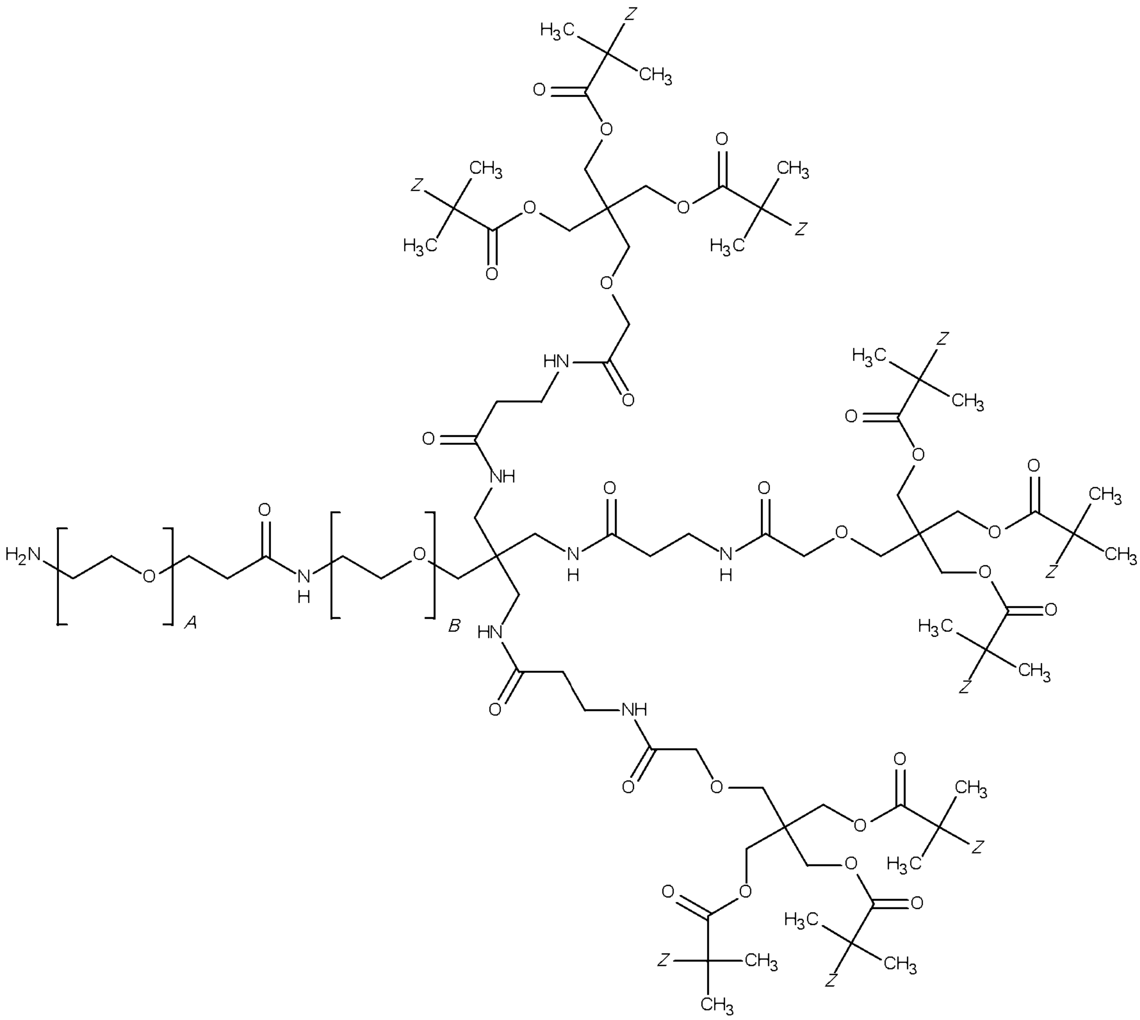

wherein A and B are the same or different and are integers from 2 to 12 and Z is any halide, for example Br. In some embodiments, A and B are each 4.

In some embodiments, the method further has the step of reacting the polymer with a maleimide reagent to provide a polymer having a terminal maleimide. In some embodiments, the maleimide compound is

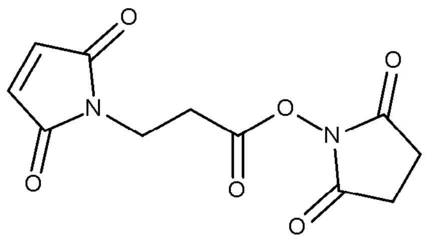

.

Pharmaceutical compositions

Therapeutic proteins can be incorporated into a pharmaceutical composition with a pharmaceutically acceptable excipient. Pharmaceutical compositions adapted for oral administration may be presented as discrete units such as capsules, as solutions, syrups or suspensions (in aqueous or non-aqueous liquids; or as edible foams or whips; or as emulsions). Suitable excipients for tablets or hard gelatine capsules include lactose, maize starch or derivatives thereof, stearic acid or salts thereof. Suitable excipients for use with soft gelatine capsules include for example vegetable oils, waxes, fats, semi-solid, or liquid polyols etc. For the preparation of solutions and syrups, excipients which may be used include for example water, polyols and sugars. For the preparation of suspensions oils (e.g. vegetable oils) may be used to provide oil-in-water or water in oil suspensions.

Pharmaceutical compositions can be adapted for nasal administration wherein the excipient is a solid include a coarse powder having a particle size for example in the range 20 to 500 microns which is administered in the manner in which snuff is taken, i.e. by rapid inhalation through the nasal passage from a container of the powder held close up to the nose. Suitable compositions wherein the excipient is a liquid, for administration as a nasal spray or as nasal drops, include aqueous or oil solutions of the active ingredient. Pharmaceutical compositions adapted for administration by inhalation include fine particle dusts or mists which may be generated by means of various types of metered dose pressurized aerosols, nebulizers or insufflators.

Pharmaceutical compositions adapted for parenteral administration include aqueous and non-aqueous sterile injection solution which may contain anti-oxidants, buffers, bacteriostats and solutes which render the formulation substantially isotonic with the blood of the intended recipient; and aqueous and non-aqueous sterile suspensions which may include suspending agents and thickening agents. Excipients which may be used for injectable solutions include water, alcohols, polyols, glycerine and vegetable oils, for example. The compositions may be presented in unit-dose or multi-dose containers, for example sealed ampoules and vials, and may be stored in a freeze-dried (lyophilized) condition requiring only the addition of the sterile liquid carried, for example water for injections, immediately prior to use. Extemporaneous injection solutions and suspensions may be prepared from sterile powders, granules and tablets. Pharmaceutical compositions can be substantially isotonic, implying an osmolality of about 250-400 mOsm/kg water.

The pharmaceutical compositions may contain preserving agents, solubilizing agents, stabilizing agents, wetting agents, emulsifiers, sweeteners, colorants, odorants, salts (substances may themselves be provided in the form of a pharmaceutically acceptable salt), buffers, coating agents or antioxidants. They may also contain therapeutically active agents in addition to the substance. The pharmaceutical compositions may be employed in combination with one or more pharmaceutically acceptable excipients. Such excipients may include, but are not limited to, saline, buffered saline (such as phosphate buffered saline), dextrose, liposomes, water, glycerol, ethanol and combinations thereof.

Provided herein is a pharmaceutical composition that includes: an anti-VEGF antibody polymer conjugate (e.g., as disclosed herein); 12.5 mM sodium phosphate buffer; and 0.025% (w/w) polysorbate 20, at a pH of about 6.5, wherein approximately 40-55 mg/mL of antibody mass is present and approximately 259-356 mg/mL of total mass of the antibody polymer conjugate is present in the composition. In some embodiments, a pharmaceutical composition is provided that includes: an anti-VEGF antibody polymer conjugate (e.g., as disclosed herein); 12.5 mM sodium phosphate buffer; and 0.025% (w/w) polysorbate 20, at a pH of about 6.5, wherein approximately 50 mg/mL of antibody mass is present and approximately 324 mg/mL of total mass of the antibody polymer conjugate is present in the composition. In some embodiments, the liquid is water for the composition.

Also provided herein is a pharmaceutical composition that includes: a) an anti-VEGF antibody polymer conjugate (e.g., as disclosed herein), 12.5 mM sodium phosphate buffer with 0.025% (w/w) polysorbate 20, pH 6.5, wherein the antibody polymer conjugate is present in the composition at a concentration of 50 mg/mL (based on antibody mass), equivalent to 324 mg/mL of total mass of the antibody polymer conjugate, including 50 mg/mL OG1950 Antibody Intermediate and 274 mg/mL OG1802 Biopolymer Intermediate; b) an anti-VEGF antibody polymer conjugate (e.g., as disclosed herein), 10-15 mM sodium phosphate buffer with 0.01-0.5% (w/w) polysorbate 20, pH 6.5, wherein the antibody polymer conjugate is present in the composition at a concentration of about 50 mg/mL (based on antibody mass), equivalent to about 324 mg/mL of total mass of the antibody polymer conjugate; c) an anti-VEGF antibody polymer conjugate (e.g., as disclosed herein), 12.5 mM sodium phosphate buffer with optional 0.025% (w/w) polysorbate 20, pH 6.5, wherein the antibody polymer conjugate is present in the composition at a concentration of 50 mg/mL (based on antibody mass), equivalent to 324 mg/mL of total mass of the antibody polymer conjugate, including 50 mg/mL OG1950 Antibody Intermediate and 274 mg/mL OG1802 Biopolymer Intermediate; or d) an anti-VEGF antibody polymer conjugate (e.g., as disclosed herein), sodium phosphate buffer with polysorbate 20, pH 6.5, wherein the antibody polymer conjugate is present in the composition at a concentration of 50 mg/mL (based on antibody mass), equivalent to about 324 mg/mL of total mass of the antibody-biopolymer conjugate, including about 50 mg/mL OG1950 Antibody Intermediate and about 274 mg/mL OG1802 Biopolymer Intermediate; e) an anti-VEGF antibody polymer conjugate (e.g., as disclosed herein), 10-15 mM sodium phosphate buffer with 0.01-0.5% (w/w) polysorbate 20, pH 6.5, wherein the antibody polymer conjugate is present in the composition at a concentration of about 40-55 mg/mL (based on antibody mass), equivalent to about 259-356 mg/mL of total mass of the antibody-biopolymer conjugate; f) an anti-VEGF antibody polymer conjugate (e.g., as disclosed herein), 10-15 mM sodium phosphate buffer with 0.01-0.5% (w/w) polysorbate 20, pH 6.5, wherein the antibody polymer conjugate is present in the composition at a concentration of about 45-52.5 mg/mL (based on antibody mass), equivalent to about 292-340 mg/mL of total mass of the antibody-biopolymer conjugate; or g) an anti-VEGF antibody polymer conjugate (e.g., as disclosed herein), 10-15 mM sodium phosphate buffer with 0.01-0.5% (w/w) polysorbate 20, pH 6.5, wherein the antibody polymer conjugate is present in the composition at a concentration of about 40-55 mg/mL (based on antibody mass), equivalent to about 259-356 mg/mL of total mass of the antibody-biopolymer conjugate, wherein the antibody polymer conjugate comprises an antibody comprising i) VH and VL in FIG. 64, or ii) 6 CDRs within the VH and VL of FIG. 64.

The antibodies and pharmaceutical compositions containing them may be administered in an effective regime for

treating or prophylaxis of a patient's disease including, for instance, administration by oral, intravitreal, intravenous, subcutaneous, intramuscular, intraosseous, intranasal, topical, intraperitoneal, and intralesional administration. Parenteral infusions include intramuscular, intravenous, intraarterial, intraperitoneal, or subcutaneous administration or routes among others. In therapy or as a prophylactic, the active agent may be administered to an individual as an injectable composition, for example as a sterile aqueous dispersion In some embodiments the agent is isotonic or substantially isotonic.

For administration to mammals, and particularly humans, it is expected that the dosage of the active agent is from 0.01 mg/kg body weight, typically around 1 mg/kg. The physician can determine the actual dosage most suitable for an individual which depends on factors including the age, weight, sex and response of the individual, the disease or disorder being treated and the age and condition of the individual being treated. The above dosages are exemplary of the average case. There can, of course, be instances where higher or lower dosages are merited. In some embodiments, the dosage can be 0.5 to 20 mg/eye, e.g., 1, 2, 3, 4, 5, 6, 7, 8, 9, 10, 11, 12, 13, 14, 15, 16, 17, 18, or 19 mg.

This dosage may be repeated as often as appropriate (e.g., weekly, fortnightly, monthly, once every two months, quarterly, twice a year, yearly). If side effects develop the amount and/or frequency of the dosage can be reduced, in accordance with normal clinical practice. In one embodiment, the pharmaceutical composition may be administered once every one to thirty days. In one embodiment, the pharmaceutical composition may be administered twice every thirty days. In one embodiment, the pharmaceutical composition may be administered once a week.

The antibodies and pharmaceutical compositions can be employed alone or in conjunction with other compounds, such as therapeutic compounds or molecules, e.g. antiinflammatory drugs, analgesics or antibiotics. Such administration with other compounds may be simultaneous, separate or sequential. The components may be prepared in the form of a kit which may comprise instructions as appropriate.

The antibodies and pharmaceutical compositions disclosed herein can be used for treatment or prophylaxis of disease, particularly the ocular diseases or conditions described herein.

The anti-VEGF antibody conjugates, or anti-VEGF protein conjugates, and pharmaceutical compositions containing them may be formulated for and administered by ocular, intraocular, and/or intravitreal injection, and/or juxtascleral injection, and/or subretinal injection and/or subtenon injection, and/or superchoroidal injection and/or subconjunctival and/or topical administration in the form of eye drops and/or ointment. Such antibodies and compositions can be delivered by a variety of methods, e.g. intravitreally as a device and/or a depot that allows for slow release of the compound into the vitreous, including those described in references such as Intraocular Drug Delivery, Jaffe, Ashton, and Pearson, editors, Taylor & Francis (March 2006). In one example, a device may be in the form of a minipump and/or a matrix and/or a passive diffusion system and/or encapsulated cells that release the compound for a prolonged period of time (Intraocular Drug Delivery, Jaffe, Ashton, and Pearson, editors, Taylor & Francis (March 2006)).

Formulations for ocular, intraocular or intravitreal administration can be prepared by methods and using ingredients known in the art. A main requirement for efficient treatment is proper penetration through the eye. Unlike diseases of the front of the eye, where drugs can be delivered topically, retinal diseases require a more site-specific approach. Eye drops and ointments rarely penetrate the back of the eye, and the blood-ocular barrier hinders penetration of systemically administered drugs into ocular tissue. Accordingly, usually the method of choice for drug delivery to treat retinal disease, such as AMD and CNV, is direct intravitreal injection. Intravitreal injections are usually repeated at intervals which depend on the patient's condition, and the properties and half-life of the drug delivered.

Therapeutic antibodies and related conjugates generally are placed into a container having a sterile access port, for example, an intravenous solution bag or vial having a stopper pierceable by a hypodermic injection needle. Such compositions may also be supplied in the form of pre-filled syringes.

A "stable" formulation is one in which the protein or protein conjugated to a polymer of other half-life extending moiety therein essentially retains its physical stability and/or chemical stability and/or biological activity upon storage. By "stable" is also meant a formulation which exhibits little or no signs of instability, including aggregation and/or deamidation. For example, the formulations provided may remain stable for at least two year, when stored as indicated at a temperature of 5-8°C. Suitable formulations for an anti-VEGF antibody conjugate of the present disclosure is described in e.g., PCT publication number WO2017117464, which is incorporated by reference herein in its entirety.

Various analytical techniques for measuring protein stability are available in the art and are reviewed in Peptide and Protein Drug Delivery, 247-301 (Vincent Lee ed., New York, N.Y., 1991) and Jones, 1993 Adv. Drug Delivery Rev. 10: 29-90, for examples. Stability can be measured at a selected temperature for a selected time period. In some embodiments the storage of the formulations is stable for at least 6 months, 12 months, 12-18 months, or for 2 or more years.

A protein, such as an antibody or fragment thereof, "retains its physical stability" in a pharmaceutical formulation if it shows no signs of aggregation, precipitation, deamidation and/or denaturation upon visual examination of color and/or clarity, or as measured by UV light scattering or by size exclusion chromatography.

A protein "retains its chemical stability" in a pharmaceutical formulation, if the chemical stability at a given time is such that the protein is considered to still retain its biological activity. Chemical stability can be assessed by detecting and quantifying chemically altered forms of the protein. Chemical alteration may involve size modification (e.g., clipping), which

can be evaluated using size exclusion chromatography, SDS-PAGE and/or matrix-assisted laser desorption ionization/-time-of-flight mass spectrometry (MALDI/TOF MS), for examples. Other types of chemical alteration include charge alteration (e.g., occurring as a result of deamidation), which can be evaluated by ion-exchange chromatography, for example. An antibody "retains its biological activity" in a pharmaceutical formulation, if the biological activity of the antibody at a given time is within about 10% (within the errors of the assay) of the biological activity exhibited at the time the pharmaceutical formulation was prepared as determined in an antigen binding assay, for example.

A protein-polymer conjugate "retains its chemical stability" the chemical bond between the protein and the polymer is maintained intact, e.g., it is not hydrolyzed or otherwise disrupted. The protein part of the conjugate retains its chemical stability as described above.

By "isotonic" is meant that the formulation of interest has essentially the same osmotic pressure as human blood or the vitreous for intravitreal injections. Isotonic formulations will generally have an osmotic pressure from about 250 to 400 mOsm. Isotonicity can be measured using a vapor pressure or ice-freezing type osmometer, for example.

As used herein, "buffer" refers to a buffered solution that resists changes in pH by the action of its acid-base conjugate components. In some embodiments, the buffer has a pH from about 3.0 to about 8.0; for example from about 4.5 to 8; or about pH 6 to about 7.5; or about 6.0 to about 7.0, or about 6.5-7.0, or about pH 7.0 to about 7.5; or about 7.1 to about 7.4. A pH of any point in between the above ranges is also contemplated.

In some embodiments, "PBS" phosphate buffered saline, Tris based buffers and histidine based buffers are used.

In some embodiments, the PBS buffer is made up of at least $Na_2HPO_4$, $KH_2PO_4$ and NaCl adjusted so as to provide the appropriate pH. In some embodiments, the buffer may contain other pharmaceutical excipients such as KCl and other salts, detergents and/or preservatives so as to provide a stable storage solution.

A "preservative" is a compound which can be included in the formulation to essentially reduce bacterial action therein, thus facilitating the production of a multi-use formulation, for example. Examples of potential preservatives include octadecyldimethylbenzyl ammonium chloride, hexamethonium chloride, benzalkonium chloride (a mixture of alkylbenzyldimethylammonium chlorides in which the alkyl groups are long-chain compounds), and benzethonium chloride. Other types of preservatives include aromatic alcohols such as phenol, butyl and benzyl alcohol, alkyl parabens such as methyl or propyl paraben, catechol, resorcinol, cyclohexanol, 3-pentanol, and m-cresol.

In some embodiments, formulations, to be safe for human use or for animal testing, should have sufficiently low levels of endotoxin. "Endotoxin" is lipopolysaccharide (LPS) derived from the cell membrane of Gram-negative bacteria. Endotoxin is composed of a hydrophilic polysaccharide moiety covalently linked to a hydrophobic lipid moiety (lipid A). Raetz CR, Ulevitch RJ, Wright SD, Sibley CH, Ding A, Nathan CF. 1991. Gram-negative endotoxin: an extraordinary lipid with profound effects on eukaryotic signal transduction. FASEB J. 5(12):2652-2660. Lipid A is responsible for most of the biological activities of endotoxin, i.e., its toxicity. Endotoxins are shed in large amount upon bacterial cell death as well as during growth and division. They are highly heat-stable and are not destroyed under regular sterilizing conditions. Extreme treatments with heat or pH, e.g., 180-250°C and over 0.1 M of acid or base must be used (Petsch D, Anspach F. 2000. Endotoxin removal from protein solutions. J Biotechnol. 76: 97-119). Such conditions of course would be highly detrimental to biological drugs.

In the biotech and pharmaceutical industries, it is possible to find endotoxin during both production processes and in final products. As bacteria can grow in nutrient poor media, including water, saline and buffers, endotoxins are prevalent unless precautions are taken. Endotoxin injection into an animal or human causes a wide variety of pathophysiological effects, including endotoxin shock, tissue injury and even death. Ogikubo Y, Ogikubo Y, Norimatsu M, Noda K, Takahashi J, Inotsume M, Tsuchiya M, Tamura Y. 2004. Evaluation of the bacterial endotoxin test for quantifications of endotoxin contamination of porcine vaccines. Biologics 32:88-93.

Pyrogenic reactions and shock are induced in mammals upon intravenous injection of endotoxin at low concentrations (1 ng/mL) (Fiske JM, Ross A, VanDerMeid RK, McMichael JC, Arumugham. 2001. Method for reducing endotoxin in Moraxella catarrhalis UspA2 protein preparations. J Chrom B. 753:269-278). The maximum level of endotoxin for intravenous applications of pharmaceutical and biologic product is set to 5 endotoxin units (EU) per kg of body weight per hour by all pharmacopoeias (Daneshiam M, Guenther A, Wendel A, Hartung T, Von Aulock S. 2006. In vitro pyrogen test for toxic or immunomodulatory drugs. J Immunol Method 313:169-175). EU is a measurement of the biological activity of an endotoxin. For example, 100 pg of the standard endotoxin EC-5 and 120 pg of endotoxin from *Escherichia coli* O111:B4 have activity of 1 EU (Hirayama C, Sakata M. 2002. Chromatographic removal of endotoxin from protein solutions by polymer particles. J Chrom B 781:419-432). Meeting this threshold level has always been a challenge in biological research and pharmaceutical industry (Berthold W, Walter J. 1994. Protein Purification: Aspects of Processes for Pharmaceutical Products. Biologicals 22:135-150; Petsch D, Anspach FB. 2000. Endotoxin removal from protein solutions. J Biotech 76:97-119).

The presence of endotoxin in drugs to be delivered via intravitreal injection is of particular concern. Intravitreal injection of drug (penicillin) was first performed in 1945 by Rycroft. Rycroft BW. 1945. Penicillin and the control of deep intra-ocular infection. British J Ophthalmol 29 (2): 57-87. The vitreous is a chamber where high level of drug can be introduced and maintained for relatively long periods of time. The concentration of drug that can be achieved via intravitreal

injection far exceeds what can be generated by topical administration or by systemic administration (e.g. intravenous).

One of the most dangerous complications potentially arising from intravitreal injections is endophthalmitis. Endophthalmitis falls into two classes: infectious and sterile. Infectious endophthalmitis is generally cause by bacteria, fungi or parasites. The symptoms of infectious endophthalmitis include severe pain, loss of vision, and redness of the conjunctiva and the underlying episclera. Infectious endophthalmitis requires urgent diagnosis and treatment. Possible treatments include intravitreal injection of antibiotics and pars plana vitrectomy in some cases. Enucleation may be called for to remove a blind and painful eye. See, e.g., Christy NE, Sommer A. 1979. Antibiotic prophylaxis of postoperative endophthalmitis. Ann Ophthalmol 11 (8): 1261-1265.

Sterile endophthalmitis in contrast does not involve an infectious agent and can be defined as the acute intraocular inflammation of the vitreous cavity that resolves without the need of intravitreal antibiotics and/or vitreoretinal surgery. If a vitreous microbiological study has been done, it needs to be negative culture proven to sustain a diagnosis of sterile endophthalmitis. Marticorena J, Romano V, Gomez-Ulla F. 2012 "Sterile Endophthalmitis after Intravitreal Injections" Med Inflam. 928123.

It has been observed that intravitreal injection of biological drugs contaminated with endotoxin can result in sterile endophthalmitis. Marticorena, et al. Bevacizumab (Avastin) is approved by the Food and Drug Administration for the treatment of glioblastoma and of metastatic colorectal cancer, advanced nonsquamous non-small-cell lung cancer and metastatic kidney cancer. Bevacizumab is also widely used off label as a treatment for wet AMD. Bevacizumab comes from the manufacturer as a 100 mg/4 ml. This solution cannot be directly used for intravitreal injection and should be compounded by a pharmacist. Clusters of sterile endophthalmitis have been observed and are theorized to be cause by inadvertent contamination of bevacizumab by endotoxin by the compounding pharmacist.

Given the dire clinical results of intravitreal injection of endotoxin, the total amount of endotoxin that can be given to a patient via intravitreal dosing is highly limited. In some embodiments, a solution having an antibody or antibody-conjugate is provided having an endotoxin level that does not exceed 5.0 EU/ml. In some embodiments, the endotoxin level does not exceed 1.0 EU/ml. In some embodiments, the endotoxin level does not exceed 0.5 EU/ml. In some embodiments, the endotoxin level does not exceed 0.2 EU/ml. In some embodiments, the endotoxin level does not exceed 2, 1, 0.5, 0.2, 0.1, 0.09, 0.08, 0.07, 0.06, 0.05, 0.04, 0.03, 0.02 or 0.01 EU/ml.

Two commonly used FDA-approved tests for the presence of endotoxin are the rabbit pyrogen test and Limulus Amoebodyte Lysate (LAL) assay (Hoffman S, et al. 2005. International validation of novel pyrogen tests based on human monocytoid cells J. Immunol. Methods 298:161-173; Ding JL, Ho BA. 2001. New era in pyrogen testing. Biotech. 19:277-281). The rabbit pyrogen test was developed in the 1920s and involves monitoring the temperature rise in a rabbit injected with a test solution. However, use of the rabbit pyrogen test has greatly diminished over the years due to expense and long turnaround time. Much more common is the LAL test. LAL is derived from the blood of a horseshoe crab and clots upon exposure to endotoxin.

One of the simplest LAL assays is the LAL gel-clot assay. Essentially, the LAL clotting assay is combined with a serial dilution of the sample in question. Formation of the gel is proportional to the amount of endotoxin in the sample. Serial dilutions are prepared from the sample and each dilution assayed for its ability to form LAL gel. At some point a negative reaction is contained. The amount of endotoxin in the original sample can be estimated from the dilution assay.

Other LAL tests have also been developed, including the turbidimetric LAL assay (Ong KG, Lelan JM, Zeng KF, Barrett G, Aourob M, Grimes CA. 2006. A rapid highly-sensitive endotoxin detection system. Biosensors and Bioelectronics 21:2270-2274) and the chromogenic LAL assay (Haishima Y, Hasegawa C, Yagami T, Tsuchiya T, Matsuda R, Hayashi Y. 2003. Estimation of uncertainty in kinetic-colorimetric assay of bacterial endotoxins. J Pharm Biomed Analysis. 32:495-503). The turbidimetric and chromogenic assays are much more sensitive and quantitative than the simple gel-clot dilution assay.

In some embodiments a method of reducing the amount of endotoxin in a composition having an antibody disclosed herein is provided. The method having the steps of contacting the composition with an affinity chromatography resin that binds to the antibody; eluting the antibody from the affinity chromatography resin to form an affinity chromatography eluent having the antagonist; contacting the affinity chromatography eluent with an ion-exchange resin that binds the antibody; and eluting the antibody from the ion-exchange resin, wherein the antibody eluted from the ion-exchange resin is substantially free from endotoxin.

The above method for reducing the amount of endotoxin, or other method or process recited herein, can be performed in the order described in the steps above or it can optionally be performed by varying the order of the steps or even repeating one or more of the steps. In one embodiment, the method of reducing the amount of endotoxin in a composition is performed in the order of the described steps. In some embodiments, the affinity chromatography resin contacting, washing and eluting steps are repeated in the same order more than one time before contacting the affinity chromatography eluent with the ion exchange resin. The method can also include a filtering step using, for example, a 0.1 micron, 0.22 micron, or 0.44 micron filter, that can be performed on either one or more of the eluents removed after each resin binding step.

In certain instances, the steps of contacting the composition with affinity chromatography resin, washing and

eluting the antibody from the affinity chromatography resin can be repeated more than one time before contacting the first eluent with an ion-exchange resin. In one embodiment, the affinity chromatography resin comprises a recombinant Protein A ("rProteinA") resin. One example of a suitable recombinant Protein A resin is rProteinA Sepharose FF® resin (Amersham, Piscataway, N.J.). In another embodiment, a suitable affinity chromatography resin would comprise a protein G chromatography resin. In other embodiments, a suitable affinity chromatography resin comprises a mixed Protein A/Protein G resin. In other embodiments, a suitable affinity chromatography resin comprises a hydrophobic charge induction resin that comprises a 4-mercaptoethylpyridine ligand such as a MEP HyperCel® resin (BioSepra, Cergy, Saint Christophe, France).

In some embodiments, the ion exchange resin comprises an anion-exchange resin. As will be known by the person skilled in the art, ion exchangers may be based on various materials with respect to the matrix as well as to the attached charged groups. For example, the following matrices may be used, in which the materials mentioned may be more or less crosslinked: MacroCap Q (GE Healthcare Biosciences, Piscataway, NJ), agarose based (such as Sepharose CL-6B®, Sepharose Fast Flow® and Sepharose High Performance ®), cellulose based (such as DEAE Sephacel®), dextran based (such as Sephadex®), silica based and synthetic polymer based. For the anion exchange resin, the charged groups, which are covalently attached to the matrix, may, for example, be diethylaminoethyl, quaternary aminoethyl, and/or quaternary ammonium. In some embodiments the anion-exchange resin comprises a quaternary amine group. An exemplarily anion-exchange resin that has a quaternary amine group for binding the anti-M-CSF antibody is a Q Sepharose® resin (Amersham, Piscataway, N.J.).

In other aspects, if the endotoxin levels are higher than desired after subjecting the composition to the aforementioned anion-exchange chromatography step, the composition may in the alternative be subjected to a cation exchange resin. In some embodiments, any endotoxin in the composition should have a differential binding to the ion-exchange resin than the protein in question to allow purification of the protein from the endotoxin. In this regard, endotoxin is negatively charged and will generally bind to an anion exchange resin. If both the protein and the endotoxin bind to the anion exchange resin, purification of one from the other may be effectuated by using a salt gradient to elute the two into different fractions. The relative binding of the protein to a particular resin may also be effected by changing the pH of the buffer relative to the pI of the protein. In some embodiments, cation-exchange chromatography is the sole ion-exchange chromatography employed.

In some embodiments, if the endotoxin levels are too high after the anion exchange resin, the composition may be further subjected to a second ion-exchange step, for example, by contacting the compositions with a cation exchange resin and followed by a wash step, then elution from the ion-exchange resin. In some embodiments, the cation exchange resin comprises a sulfonic group for binding. Exemplary cation exchange resins are SP Sepharose® resin FF (Amersham, Piscataway, N.J.) Poros XS (CEX) (Life Technology, Grand Island, New York).

In some embodiments, after the solution of antibody protein is produced having the specified level of endotoxin, there are a number of steps prior to final formulation of the protein. In some embodiments, a half-life extending moiety is conjugated to the protein. The conjugate is then formulated into a final drug formulation which is injected into the patients. In some embodiments, the conjugate is again purified on an ion-exchange resin which can be a cation-exchange resin. In other embodiments, the protein is formulated. In all cases, normal laboratory procedures should be employed to prevent the introduction of endotoxin contaminants into the protein sample or into the protein-polymer conjugate.

In some embodiments, any of the following arrangements are contemplated herein:

1. A method of treating diabetic retinopathy, the method comprising:

   identifying a subject with diabetic retinopathy;
   administering a first dose of an anti-VEGF antibody polymer conjugate to the subject;
   administering a second dose of the anti-VEGF antibody polymer conjugate to the subject 8 weeks after the first dose;
   administering a third dose of the anti-VEGF antibody polymer conjugate to the subject 12 weeks after the second dose; and
   administering a subsequent dose of the anti-VEGF antibody polymer conjugate to the subject no more frequently than once every 24 weeks after the third dose.

2. A method of treating diabetic retinopathy, the method comprising:

   identifying a subject with diabetic retinopathy;
   administering a first dose of an anti-VEGF antibody polymer conjugate to the subject;
   administering a second dose of the anti-VEGF antibody polymer conjugate to the subject 8 weeks after the first dose;
   administering a third dose of the anti-VEGF antibody polymer conjugate to the subject 12 weeks after the second

dose; and
administering one or more subsequent doses of the anti-VEGF antibody polymer conjugate to the subject either once every 12 weeks or once every 24 weeks after the third dose.

3. A method of treating diabetic retinopathy, the method comprising:

identifying a subject with diabetic retinopathy;
administering a first dose of an anti-VEGF antibody polymer conjugate to the subject;
administering a second dose of the anti-VEGF antibody polymer conjugate to the subject 4 weeks after the first dose; and
administering one or more subsequent doses to the subject no more frequently than once every 12 weeks, unless, an eye of the subject has a decline in eye health at week 8 from the second loading dose, then administering the anti-VEGF antibody polymer conjugate once every 8 weeks.

4. A method of treating diabetic retinopathy, the method comprising:

identifying a subject with diabetic retinopathy;
administering a first dose of an anti-VEGF antibody polymer conjugate to the subject;
administering a second dose of the anti-VEGF antibody polymer conjugate to the subject 4 weeks after the first dose; and
if the subject has a decline in eye health 8 weeks after the second dose, administering at least one subsequent dose of the anti-VEGF antibody polymer conjugate at a frequency of at least once every 8 weeks, and if the subject maintains eye health or it improves, then administering at least one subsequent dose of the anti-VEGF antibody polymer conjugate at a frequency of no more than once every 12 weeks.

5. A method of treating diabetic retinopathy, the method comprising:

identifying a subject with diabetic retinopathy;
administering a first dose of an anti-VEGF antibody polymer conjugate to the subject;
administering a second dose of the anti-VEGF antibody polymer conjugate to the subject 4 weeks after the first dose; and
administering one or more subsequent doses to the subject no more frequently than once every 12 weeks or once every 8 weeks.

6. The method of arrangement 4, wherein the decline in eye health is based on a vision assessment, wherein the vision assessment measures the decrease of Best Corrected Visual Acuity (BCVA) as greater than 5 letters.
7. The method of arrangement 3, wherein the decline in eye health is that the eye's diabetic retinopathy severity is stable or worsening as determined by physical examination and imaging of the eye.
8. The method of arrangement 7, wherein the decline in eye health is the eye's diabetic retinopathy severity is worsening as determined by physical examination and imaging of the eye.
9. The method of arrangements 1-8, wherein the subject is not retreated with the anti-VEGF antibody conjugate more frequently than once every 12 weeks.
10. A method of treating a subject having wAMD, the method comprising:

identifying a subject having wAMD and receiving a therapy for wAMD,
wherein the therapy is not achieving the goal of treatment; wherein the treatment is not KSI-301; and
administering KSI-301 to the subject in an amount and at a frequency sufficient to treat the wAMD.

11. The method of arrangement 10, wherein the subject having wAMD has experienced wAMD as a recurrent condition.
12. The method of arrangement 10 or 11, wherein sufficient treatment of the subject's wAMD is defined as a gain in Best Corrected Visual Acuity letters after administration of KSI-301.
13. The method of arrangement 12, wherein Best Corrected Visual Acuity is assessed at least 4 weeks after the first administration of KSI-301.
14. The method of arrangement 13, wherein Best Corrected Visual Acuity is assessed at least 8 weeks after the first administration of KSI-301.
15. The method of arrangement 10, wherein the subject is on an approved therapy for wAMD, and wherein the approved therapy is not KSI-301.

16. The method of arrangement 10, wherein the goal of treatment is halting or reversing the loss of vision in the subject.
17. A method of treating wAMD, the method comprising:

identifying a subject with wet AMD;
administering a first loading dose of an anti-VEGF antibody polymer conjugate to the subject;
administering a second loading dose of the anti-VEGF antibody polymer conjugate to the subject 4 weeks after the first loading dose;
administering a third loading dose of the anti-VEGF antibody polymer conjugate to the subject 4 weeks after the second loading dose; and
evaluating an eye of the subject 12 weeks after the third loading dose, wherein if the eye is dry extending maintenance treatments to once every 16 weeks and wherein if the eye is wet providing a maintenance treatment once every 8 weeks.

18. The method of arrangement 17, wherein the eye's status as wet or dry is determined by physical examination and imaging of the eye.
19. The method of arrangement 18, wherein if the eye has excess intraretinal or subretinal fluid affecting the central subfield of the eye, the eye is wet, and wherein the if the eye has no excess intraretinal or subretinal fluid affecting the central subfield of the eye, the eye is dry.
20. A method of treating wAMD, the method comprising:

identifying a subject with wet AMD;
administering a first loading dose of an anti-VEGF antibody polymer conjugate to the subject;
administering a second loading dose of the anti-VEGF antibody polymer conjugate to the subject 4 weeks after the first loading dose;
administering a third loading dose of the anti-VEGF antibody polymer conjugate to the subject 4 weeks after the second loading dose; and
evaluating an eye of the subject using a low-luminance visual acuity assessment (LLVA) at 16 weeks after the third loading dose, wherein if the eye has a LLVA) to BCVA difference of 33 or more letters, the subject will receive an increased dose frequency (e.g. every 4 to 8 weeks) of maintenance doses and if the eye has LLVA to BCVA difference of less than 33 letters, the subject will receive a decreased frequency of maintenance doses (e.g. every 12 to 24 weeks) the subject will receive a decreased frequency of maintenance doses.

21. A method of treating a subject with wet AMD, the method comprising:

administering 1-3 loading doses of an anti-VEGF antibody polymer conjugate to a subject;
determining if an amount of fluid in an eye of the subject 4 weeks after a last loading dose is stabilized in the eye, wherein if the subject has stabilized fluid, administering a maintenance dose at a first frequency, and wherein if the subject has an increase in fluid administering a maintenance dose at a second frequency, wherein the second frequency is more frequent than the first frequency.

22. A method of treating DME, the method comprising:

administering 1-3 loading doses of an anti-VEGF antibody polymer conjugate to a subject having DME;
administering 1-7 maintenance doses of the anti-VEGF antibody polymer conjugate to the subject, wherein 7 or fewer total doses are administered to the subject in a year.

23. A method of treating DME, the method comprising:

administering 2-3 loading doses of an anti-VEGF antibody polymer conjugate to a subject having DME; and
administering a maintenance dose of the anti-VEGF antibody polymer conjugate to the subject every 2-15 months, depending on whether the eye is dry as determined by OCT or OCT-A measurement.

24. A method of treating DME, the method comprising:

administering 2-3 loading doses of an anti-VEGF antibody polymer conjugate to a subject having DME; and
administering a maintenance dose of the anti-VEGF antibody polymer conjugate to the subject every 2-15 months, depending on whether retina thickness in the subject is above 320 microns (or 350 microns).

25. The method of arrangement 24, wherein retina thickness and BCVA is determined every 4 weeks after the administration of the last loading dose for 20 weeks.
26. The method of arrangement 25, wherein the retina thickness is determined by OCT or OCT-A measurements, and wherein the retina thickness is first determined prior to administering the anti-VEGF antibody polymer conjugate.
27. The method of arrangement 26, wherein a second retina thickness is determined 4 weeks after administration of the last loading dose.
28. The method of arrangement 27, wherein when the second retina thickness increases ≥ 75 microns (or ≥ 40 microns) compared to the retina thickness measurement taken prior to administering the anti-VEGF antibody polymer conjugate, then administering the anti-VEGF antibody polymer conjugate and thereafter administering the anti-VEGF antibody polymer conjugate no more than every 8 weeks.
29. The method of arrangement 24, wherein Best Corrected Visual Acuity (BCVA) is measured prior to administration of the anti-VEGF antibody polymer conjugate.
30. The method of arrangement 29 wherein a second BCVA is measured 4 weeks after administration of the last loading dose.
31. The method of arrangement 30, wherein when the second retina thickness increases ≥ 50 microns compared to the retina thickness measurement taken prior to administering the anti-VEGF antibody polymer conjugate and wherein there is a decrease in the second BCVA of ≥ 5 letters compared to the BCVA measured prior to the administration of the anti-VEGF antibody polymer conjugate, then administering the anti-VEGF antibody polymer conjugate and thereafter administering the anti-VEGF antibody polymer conjugate no more than every 8 weeks.
32. The method of arrangement 27 wherein no increase of retina thickness >30 microns is observed compared to the retina thickness measurement taken prior to administering the anti-VEGF antibody polymer conjugate, and not administering a maintenance dose of the anti-VEGF antibody polymer conjugate to the subject for at least 4 weeks.
33. A method of preventing vision complications, the method comprising:

administering 2-3 loading doses of an anti-VEGF antibody polymer conjugate to a subject having DME; and
administering a maintenance dose of the anti-VEGF antibody polymer conjugate to the subject every 3-15 months, depending on:

whether retina thickness in the subject is above 320 microns (or 350 microns); and/or

whether the eye is dry as determined by OCT or OCT-A measurement.

34. A method of treating wAMD, DME, or RVO, the method comprising:

administering 2-3 loading doses of an anti-VEGF antibody polymer conjugate to a subject having wAMD, DME. Or RVO;
obtaining a vision assessment of the subject after 4, 8, 12, 16, 20, 24, 36, 48, or 52 weeks after a last loading dose; and
based on the vision assessment of the patient, placing the patient on a fixed regimen of the anti-VEGF antibody polymer conjugate once every 4, 8, 12, 16, 20, 24, 36, 48, or 52 weeks.

35. The method of arrangement 34, wherein the vision assessment measures the change of Best Corrected Visual Acuity (BCVA) as greater than 3-5 letters.
36. The method of arrangement 34, wherein the vision assessment is based on a change in OCT or OCT-A of at least 30-50 microns.
37. A method of treating a subject with DME, DR or RVO, the method comprising: administering 1-3 loading doses of an anti-VEGF antibody polymer conjugate to a subject with DME, DR or RVO;

not administering more than 3 loading doses to the subject;
providing a follow-on application of the anti-VEGF antibody polymer conjugate at a point in time after a last loading dose or after a last follow-on application of the anti-VEGF antibody polymer conjugate, wherein the loading doses are administered to the subject on a monthly basis, and wherein the subject is above 16 years of age.

38. A method of treating DME, DR, or RVO, the method comprising:

administering 1-3 loading doses of an anti-VEGF antibody polymer conjugate to a subject with DME, DR or RVO;
not administering more than 3 loading doses to the subject;
providing a follow-on application of the anti-VEGF antibody polymer conjugate at a point in time no sooner than 8

or 12 weeks after a last loading dose or after a last follow-on application of the anti-VEGF antibody polymer conjugate, wherein the loading doses are administered to the subject on a monthly basis, and wherein an improvement of Best Corrected Visual Acuity (BCVA) is greater than at least 5 or at least 6.8 to 7.2 letters post treatment.

39. The method of arrangement 38, wherein the subject has DME and the improvement provides a BCVA of at least 5 letters.
40. The method of arrangement 38, wherein the subject has RVO and the improvement provides a BCVA of at least 5 letters.
41. The method of arrangement 38, wherein the subject has DME and the improvement is 1 to 30 letters.
42. The method of arrangement 38, wherein the subject has wAMD and the improvement is 1 to 35 letters.
43. The method of arrangement 38, wherein the subject has RVO and the improvement is 1 to 40 letters.
44. A method of treating a subject with DME, DR, or RVO, the method comprising: administering 1-3 loading doses of an anti-VEGF antibody polymer conjugate to a subject with DME, DR, or RVO;

not administering more than 3 loading doses; and
providing between 0-1 follow-on application of the anti-VEGF antibody polymer conjugate at a point in time no sooner than 8 or 12 weeks after a last loading dose or after a last follow-on application of the anti-VEGF antibody polymer conjugate, wherein the loading doses are administered to the subject on a monthly basis.

45. The method of any one of arrangements 1-44, wherein the anti-VEGF antibody polymer conjugate is part of a formulation, wherein the formulation comprises any one of:

a) 12.5 mM sodium phosphate buffer with 0.025% (w/w) polysorbate 20, pH 6.5 at a concentration of 50 mg/mL (based on antibody mass), equivalent to 324 mg/mL of total mass of the antibody-biopolymer conjugate, including 50 mg/mL OG1950 Antibody Intermediate and 274 mg/mL OG1802 Biopolymer Intermediate,
b)10-15 mM sodium phosphate buffer with 0.01-0.5% (w/w) polysorbate 20, pH 6.5 at a concentration of about 50 mg/mL (based on antibody mass), equivalent to about 324 mg/mL of total mass of the antibody-biopolymer conjugate,
c) 12.5 mM sodium phosphate buffer with optional 0.025% (w/w) polysorbate 20, pH 6.5 at a concentration of 50 mg/mL (based on antibody mass), equivalent to 324 mg/mL of total mass of the antibody-biopolymer conjugate, including 50 mg/mL OG1950 Antibody Intermediate and 274 mg/mL OG1802 Biopolymer Intermediate, or
d) sodium phosphate buffer with polysorbate 20, pH 6.5 at a concentration of 50 mg/mL (based on antibody mass), equivalent to about 324 mg/mL of total mass of the antibody-biopolymer conjugate, including about 50 mg/mL OG1950 Antibody Intermediate and about 274 mg/mL OG1802 Biopolymer Intermediate,
e) 10-15 mM sodium phosphate buffer with 0.01-0.5% (w/w) polysorbate 20, pH 6.5 at a concentration of about 40-55 mg/mL (based on antibody mass), equivalent to about 259-356 mg/mL of total mass of the antibody-biopolymer conjugate,
f) 10-15 mM sodium phosphate buffer with 0.01-0.5% (w/w) polysorbate 20, pH 6.5 at a concentration of about 45-52.5 mg/mL (based on antibody mass), equivalent to about 292-340 mg/mL of total mass of the antibody-biopolymer conjugate, or
g) 10-15 mM sodium phosphate buffer with 0.01-0.5% (w/w) polysorbate 20, pH 6.5 at a concentration of about 40-55 mg/mL (based on antibody mass), equivalent to about 259-356 mg/mL of total mass of the antibody-biopolymer conjugate,

wherein the antibody comprises a) VH and VL in FIG. 64 or 6 CDRs within the VH and VL of FIG. 64.
46. The method of any one of arrangements 1-44, wherein the anti-VEGF antibody polymer conjugate is part of a formulation, wherein the formulation comprises:

12.5 mM sodium phosphate buffer
0.025% (w/w) polysorbate 20,
at a pH of about 6.5, wherein approximately 40-55 mg/mL of antibody mass is present and approximately 259-356 mg/mL of total mass of the antibody-biopolymer conjugate is present in the formulation.

47. The method of arrangement 46, wherein the anti-VEGF antibody polymer conjugate is 45-52.5 mg/ml of antibody and approximately 292-340 mg/mL of total mass of the antibody-biopolymer conjugate, and wherein the antibody comprises a) VH and VL in FIG. 64 or 6 CDRs within the VH and VL of FIG. 64.
48. The method of any one of the preceding arrangements, wherein at least 4 doses of the anti-VEGF antibody

polymer conjugate is administered to the subject in the first year of treatment.

49. A method of treating diabetic retinopathy, comprising:

identifying a subject with diabetic retinopathy;
administering 1-3 loading doses of an anti-VEGF antibody polymer conjugate to the subject;
administering one or more subsequent doses of the anti-VEGF antibody polymer conjugate to the subject no more frequently than once every 24 weeks after the last loading dose,
wherein at least 4 doses of the anti-VEGF antibody polymer conjugate is administered to the subject in the first year of treatment.

50. A method of treating a subject having wAMD, comprising:

identifying a subject having wAMD; and
administering an anti-VEGF antibody polymer conjugate to the subject no more frequently than once every 4 weeks.

51. A method of treating DME, comprising:

identifying a subject having DME;
administering 2-3 monthly loading doses of an anti-VEGF antibody polymer conjugate to the subject; and
administering one or more subsequent doses of the anti-VEGF antibody polymer conjugate to the subject every 2-6 months, depending on:

whether retina thickness in the subject is above 320 microns (or 350 microns); and/or
whether the eye is dry as determined by OCT or OCT-A measurement,

wherein at least 4 doses of the anti-VEGF antibody polymer conjugate is administered to the subject in the first year of treatment.

52. A method of treating RVO, comprising:

identifying a subject having RVO;
administering 2 monthly loading doses of an anti-VEGF antibody polymer conjugate to the subject; and
administering one or more subsequent doses of the anti-VEGF antibody polymer conjugate to the subject no more frequently than once every 2 months,
wherein at least 4 doses of the anti-VEGF antibody polymer conjugate is administered to the subject in the first year of treatment.

53. A method of treating wAMD, comprising:

identifying a subject having wAMD;
administering 3 monthly loading doses of an anti-VEGF antibody polymer conjugate to the subject;
evaluating retina thickness and/or BCVA of the subject at 12 weeks after the third loading dose to thereby determine that the subject has improved visual acuity;
evaluating retina thickness and/or BCVA of the subject at 16 weeks after the third loading dose to thereby determine that the subject has improved visual acuity; and
administering a maintenance dose of the anti-VEGF antibody polymer conjugate to the subject 20 weeks after the third loading dose; and
determining that the subject will receive one or more maintenance doses no more frequently than once every 20 weeks,
wherein the subject has improved visual acuity when at least one of:

retina thickness is reduced by about 50 microns or more compared to retina thickness before administering the first loading dose; and
BCVA is increased by about 3 or more compared to BCVA before administering the first loading dose.

54. A pharmaceutical composition comprising:

an anti-VEGF antibody polymer conjugate;
12.5 mM sodium phosphate buffer; and
0.025% (w/w) polysorbate 20,
at a pH of about 6.5, wherein approximately 40-55 mg/mL of antibody mass is present and approximately 259-356 mg/mL of total mass of the antibody polymer conjugate is present in the composition.

55. A pharmaceutical composition comprising:

an anti-VEGF antibody polymer conjugate;
12.5 mM sodium phosphate buffer; and
0.025% (w/w) polysorbate 20,
at a pH of about 6.5, wherein approximately 50 mg/mL of antibody mass is present and approximately 324 mg/mL of total mass of the antibody polymer conjugate is present in the composition.

56. A pharmaceutical composition comprising:

a) an anti-VEGF antibody polymer conjugate, 12.5 mM sodium phosphate buffer with 0.025% (w/w) polysorbate 20, pH 6.5, wherein the antibody polymer conjugate is present in the composition at a concentration of 50 mg/mL (based on antibody mass), equivalent to 324 mg/mL of total mass of the antibody polymer conjugate, including 50 mg/mL OG1950 Antibody Intermediate and 274 mg/mL OG1802 Biopolymer Intermediate;
b) an anti-VEGF antibody polymer conjugate, 10-15 mM sodium phosphate buffer with 0.01-0.5% (w/w) polysorbate 20, pH 6.5, wherein the antibody polymer conjugate is present in the composition at a concentration of about 50 mg/mL (based on antibody mass), equivalent to about 324 mg/mL of total mass of the antibody polymer conjugate;
c) an anti-VEGF antibody polymer conjugate, 12.5 mM sodium phosphate buffer with optional 0.025% (w/w) polysorbate 20, pH 6.5, wherein the antibody polymer conjugate is present in the composition at a concentration of 50 mg/mL (based on antibody mass), equivalent to 324 mg/mL of total mass of the antibody polymer conjugate, including 50 mg/mL OG1950 Antibody Intermediate and 274 mg/mL OG1802 Biopolymer Intermediate;
d) an anti-VEGF antibody polymer conjugate, sodium phosphate buffer with polysorbate 20, pH 6.5, wherein the antibody polymer conjugate is present in the composition at a concentration of 50 mg/mL (based on antibody mass), equivalent to about 324 mg/mL of total mass of the antibody-biopolymer conjugate, including about 50 mg/mL OG1950 Antibody Intermediate and about 274 mg/mL OG1802 Biopolymer Intermediate;
e) an anti-VEGF antibody polymer conjugate, 10-15 mM sodium phosphate buffer with 0.01-0.5% (w/w) polysorbate 20, pH 6.5, wherein the antibody polymer conjugate is present in the composition at a concentration of about 40-55 mg/mL (based on antibody mass), equivalent to about 259-356 mg/mL of total mass of the antibody-biopolymer conjugate;
f) an anti-VEGF antibody polymer conjugate, 10-15 mM sodium phosphate buffer with 0.01-0.5% (w/w) polysorbate 20, pH 6.5, wherein the antibody polymer conjugate is present in the composition at a concentration of about 45-52.5 mg/mL (based on antibody mass), equivalent to about 292-340 mg/mL of total mass of the antibody-biopolymer conjugate; or
g) an anti-VEGF antibody polymer conjugate, 10-15 mM sodium phosphate buffer with 0.01-0.5% (w/w) polysorbate 20, pH 6.5, wherein the antibody polymer conjugate is present in the composition at a concentration of about 40-55 mg/mL (based on antibody mass), equivalent to about 259-356 mg/mL of total mass of the antibody-biopolymer conjugate,

wherein the antibody polymer conjugate comprises an antibody comprising i) VH and VL in FIG. 64, or ii) 6 CDRs within the VH and VL of FIG. 64.

57. A method of treating diabetic retinopathy, the method comprising:

identifying a subject with diabetic retinopathy;
administering a first dose of an anti-VEGF antibody polymer conjugate to the subject;
administering a second dose of the anti-VEGF antibody polymer conjugate to the subject 8 weeks after the first dose;
administering a third dose of the anti-VEGF antibody polymer conjugate to the subject 12 weeks after the second dose; and
administering a subsequent dose of the anti-VEGF antibody polymer conjugate to the subject no more frequently than once every 24 weeks after the third dose.

58. A method of treating diabetic retinopathy, the method comprising:

identifying a subject with diabetic retinopathy;
administering a first dose of an anti-VEGF antibody polymer conjugate to the subject;
administering a second dose of the anti-VEGF antibody polymer conjugate to the subject 8 weeks after the first dose;
administering a third dose of the anti-VEGF antibody polymer conjugate to the subject 12 weeks after the second dose; and
administering one or more subsequent doses of the anti-VEGF antibody polymer conjugate to the subject either once every 12 weeks or once every 24 weeks after the third dose.

59. A method of treating diabetic retinopathy, the method comprising:

identifying a subject with diabetic retinopathy;
administering a first dose of an anti-VEGF antibody polymer conjugate to the subject;
administering a second dose of the anti-VEGF antibody polymer conjugate to the subject 4 weeks after the first dose; and
administering one or more subsequent doses to the subject no more frequently than once every 12 weeks, unless, an eye of the subject has a decline in eye health at week 8 from the second loading dose, then administering the anti-VEGF antibody polymer conjugate once every 8 weeks.

60. A method of treating diabetic retinopathy, the method comprising:

identifying a subject with diabetic retinopathy;
administering a first dose of an anti-VEGF antibody polymer conjugate to the subject;
administering a second dose of the anti-VEGF antibody polymer conjugate to the subject 4 weeks after the first dose; and
if the subject has a decline in eye health 8 weeks after the second dose, administering at least one subsequent dose of the anti-VEGF antibody polymer conjugate at a frequency of at least once every 8 weeks, and if the subject maintains eye health or it improves, then administering at least one subsequent dose of the anti-VEGF antibody polymer conjugate at a frequency of no more than once every 12 weeks.

61. A method of treating diabetic retinopathy, the method comprising:

identifying a subject with diabetic retinopathy;
administering a first dose of an anti-VEGF antibody polymer conjugate to the subject;
administering a second dose of the anti-VEGF antibody polymer conjugate to the subject 4 weeks after the first dose; and
administering one or more subsequent doses to the subject no more frequently than once every 12 weeks or once every 8 weeks.

62. The method of arrangement 60, wherein the decline in eye health is based on a vision assessment, wherein the vision assessment measures the decrease of Best Corrected Visual Acuity (BCVA) as greater than 5 letters.
63. The method of arrangement 59, wherein the decline in eye health is that the eye's diabetic retinopathy severity is stable or worsening as determined by physical examination and imaging of the eye.
64. The method of arrangement 63, wherein the decline in eye health is the eye's diabetic retinopathy severity is worsening as determined by physical examination and imaging of the eye.
65. The method of arrangements 57-64, wherein the subject is not retreated with the anti-VEGF antibody conjugate more frequently than once every 12 weeks.
66. A method of treating a subject having wAMD, the method comprising:

identifying a subject having wAMD and receiving a therapy for wAMD,
wherein the therapy is not achieving the goal of treatment; wherein the treatment is not KSI-301; and
administering KSI-301 to the subject in an amount and at a frequency sufficient to treat the wAMD.

67. The method of arrangement 66, wherein the subject having wAMD has experienced wAMD as a recurrent condition.
68. The method of arrangements 66 or 67, wherein sufficient treatment of the subject's wAMD is defined as a gain in

Best Corrected Visual Acuity letters after administration of KSI-301.

69. The method of arrangement 68, wherein Best Corrected Visual Acuity is assessed at least 4 weeks after the first administration of KSI-301.

70. The method of arrangement 69, wherein Best Corrected Visual Acuity is assessed at least 8 weeks after the first administration of KSI-301.

71. The method of arrangement 66, wherein the subject is on an approved therapy for wAMD, and wherein the approved therapy is not KSI-301.

72. The method of arrangement 66, wherein the goal of treatment is halting or reversing the loss of vision in the subject.

73. A method of treating wAMD, the method comprising:

identifying a subject with wet AMD;
administering a first loading dose of an anti-VEGF antibody polymer conjugate to the subject;
administering a second loading dose of the anti-VEGF antibody polymer conjugate to the subject 4 weeks after the first loading dose;
administering a third loading dose of the anti-VEGF antibody polymer conjugate to the subject 4 weeks after the second loading dose; and
evaluating an eye of the subject 12 weeks after the third loading dose, wherein if the eye is dry extending maintenance treatments to once every 16 weeks and wherein if the eye is wet providing a maintenance treatment once every 8 weeks.

74. The method of arrangement 73, wherein the eye's status as wet or dry is determined by physical examination and imaging of the eye.

75. The method of arrangement 74, wherein if the eye has excess intraretinal or subretinal fluid affecting the central subfield of the eye, the eye is wet, and wherein the if the eye has no excess intraretinal or subretinal fluid affecting the central subfield of the eye, the eye is dry.

76. A method of treating wAMD, the method comprising:

identifying a subject with wet AMD;
administering a first loading dose of an anti-VEGF antibody polymer conjugate to the subject;
administering a second loading dose of the anti-VEGF antibody polymer conjugate to the subject 4 weeks after the first loading dose;
administering a third loading dose of the anti-VEGF antibody polymer conjugate to the subject 4 weeks after the second loading dose; and
evaluating an eye of the subject using a low-luminance visual acuity assessment (LLVA) at 16 weeks after the third loading dose, wherein if the eye has a LLVA to BCVA difference of 33 or more letters, the subject will receive an increased dose frequency (e.g. every 4 to 8 weeks) of maintenance doses and if the eye has LLVA to BCVA difference of less than 33 letters, the subject will receive a decreased frequency of maintenance doses (e.g. every 12 to 24 weeks) the subject will receive a decreased frequency of maintenance doses.

77. A method of treating a subject with wet AMD, the method comprising:

administering 1-3 loading doses of an anti-VEGF antibody polymer conjugate to a subject;
determining if an amount of fluid in an eye of the subject 4 weeks after a last loading dose is stabilized in the eye, wherein if the subject has stabilized fluid, administering a maintenance dose at a first frequency, and wherein if the subject has an increase in fluid administering a maintenance dose at a second frequency, wherein the second frequency is more frequent than the first frequency.

78. A method of treating DME, the method comprising:

administering 1-3 loading doses of an anti-VEGF antibody polymer conjugate to a subject having DME;
administering 1-7 maintenance doses of the anti-VEGF antibody polymer conjugate to the subject, wherein 7 or fewer total doses are administered to the subject in a year.

79. A method of treating DME, the method comprising:

administering 2-3 loading doses of an anti-VEGF antibody polymer conjugate to a subject having DME; and
administering a maintenance dose of the anti-VEGF antibody polymer conjugate to the subject every 2-15 months, depending on whether the eye is dry as determined by OCT or OCT-A measurement.

80. A method of treating DME, the method comprising:

administering 2-3 loading doses of an anti-VEGF antibody polymer conjugate to a subject having DME; and administering a maintenance dose of the anti-VEGF antibody polymer conjugate to the subject every 2-15 months, depending on whether retina thickness in the subject is above 320 microns (or 350 microns).

81. The method of arrangement 80, wherein retina thickness and BCVA is determined every 4 weeks after the administration of the last loading dose for 20 weeks.
82. The method of arrangement 81, wherein the retina thickness is determined by OCT or OCT-A measurements, and wherein the retina thickness is first determined prior to administering the anti-VEGF antibody polymer conjugate.
83. The method of arrangement 82, wherein a second retina thickness is determined 4 weeks after administration of the last loading dose.
84. The method of arrangement 83, wherein when the second retina thickness increases ≥ 75 microns (or ≥ 40 microns) compared to the retina thickness measurement taken prior to administering the anti-VEGF antibody polymer conjugate, then administering the anti-VEGF antibody polymer conjugate and thereafter administering the anti-VEGF antibody polymer conjugate no more than every 8 weeks.
85. The method of arrangement 80, wherein Best Corrected Visual Acuity (BCVA) is measured prior to administration of the anti-VEGF antibody polymer conjugate.
86. The method of arrangement 85 wherein a second BCVA is measured 4 weeks after administration of the last loading dose.
87. The method of arrangement 86, wherein when the second retina thickness increases ≥ 50 microns compared to the retina thickness measurement taken prior to administering the anti-VEGF antibody polymer conjugate and wherein there is a decrease in the second BCVA of ≥ 5 letters compared to the BCVA measured prior to the administration of the anti-VEGF antibody polymer conjugate, then administering the anti-VEGF antibody polymer conjugate and thereafter administering the anti-VEGF antibody polymer conjugate no more than every 8 weeks.
88. The method of arrangement 83 wherein no increase of retina thickness >30 microns is observed compared to the retina thickness measurement taken prior to administering the anti-VEGF antibody polymer conjugate, and not administering a maintenance dose of the anti-VEGF antibody polymer conjugate to the subject for at least 4 weeks.
89. A method of preventing vision complications, the method comprising:

administering 2-3 loading doses of an anti-VEGF antibody polymer conjugate to a subject having DME; and administering a maintenance dose of the anti-VEGF antibody polymer conjugate to the subject every 3-15 months, depending on:

whether retina thickness in the subject is above 320 microns (or 350 microns); and/or

whether the eye is dry as determined by OCT or OCT-A measurement.

90. A method of treating wAMD, DME, or RVO, the method comprising:

administering 2-3 loading doses of an anti-VEGF antibody polymer conjugate to a subject having wAMD, DME. Or RVO;
obtaining a vision assessment of the subject after 4, 8, 12, 16, 20, 24, 36, 48, or 52 weeks after a last loading dose; and
based on the vision assessment of the patient, placing the patient on a fixed regimen of the anti-VEGF antibody polymer conjugate once every 4, 8, 12, 16, 20, 24, 36, 48, or 52 weeks.

91. The method of Arrangement 90, wherein the vision assessment measures the change of Best Corrected Visual Acuity (BCVA) as greater than 3-5 letters.
92. The method of Arrangement 90, wherein the vision assessment is based on a change in OCT or OCT-A of at least 30-50 microns.
93. A method of treating a subject with DME, DR or RVO, the method comprising: administering 1-3 loading doses of an anti-VEGF antibody polymer conjugate to a subject with DME, DR or RVO;

not administering more than 3 loading doses to the subject;
providing a follow-on application of the anti-VEGF antibody polymer conjugate at a point in time after a last loading dose or after a last follow-on application of the anti-VEGF antibody polymer conjugate, wherein the loading doses are administered to the subject on a monthly basis, and wherein the subject is above 16 years of age.

94. A method of treating DME, DR, or RVO, the method comprising:

administering 1-3 loading doses of an anti-VEGF antibody polymer conjugate to a subject with DME, DR or RVO;
not administering more than 3 loading doses to the subject;
providing a follow-on application of the anti-VEGF antibody polymer conjugate at a point in time no sooner than 8 or 12 weeks after a last loading dose or after a last follow-on application of the anti-VEGF antibody polymer conjugate, wherein the loading doses are administered to the subject on a monthly basis, and wherein an improvement of Best Corrected Visual Acuity (BCVA) is greater than at least 5 or at least 6.8 to 7.2 letters post treatment.

95. The method of arrangement 94, wherein the subject has DME and the improvement provides a BCVA of at least 5 letters.
96. The method of arrangement 94, wherein the subject has RVO and the improvement provides a BCVA of at least 5 letters.
97. The method of arrangement 94, wherein the subject has DME and the improvement is 1 to 30 letters.
98. The method of arrangement 94, wherein the subject has wAMD and the improvement is 1 to 35 letters.
99. The method of arrangement 94, wherein the subject has RVO and the improvement is 1 to 40 letters.
100. A method of treating a subject with DME, DR, or RVO, the method comprising: administering 1-3 loading doses of an anti-VEGF antibody polymer conjugate to a subject with DME, DR, or RVO;

not administering more than 3 loading doses; and
providing between 0-1 follow-on application of the anti-VEGF antibody polymer conjugate at a point in time no sooner than 8 or 12 weeks after a last loading dose or after a last follow-on application of the anti-VEGF antibody polymer conjugate, wherein the loading doses are administered to the subject on a monthly basis.

101. The method of any one of arrangements 57-100, wherein the anti-VEGF antibody polymer conjugate is part of a formulation, wherein the formulation comprises:

12.5 mM sodium phosphate buffer
0.025% (w/w) polysorbate 20,
at a pH of about 6.5, wherein approximately 50 mg/mL of antibody mass is present and approximately 324 mg/mL of total mass of the antibody-biopolymer conjugate is present in the formulation.

102. The method of any one of arrangements 57-100, wherein the anti-VEGF antibody polymer conjugate is part of a formulation, wherein the formulation comprises:

12.5 mM sodium phosphate buffer, and
0.025% (w/w) polysorbate 20,
at a pH of about 6.5, wherein approximately 40-55 mg/mL of antibody mass is present and approximately 259-356 mg/mL of total mass of the antibody-biopolymer conjugate is present in the formulation.

103. The method of arrangement 102, wherein the anti-VEGF antibody polymer conjugate is 45-52.5 mg/ml of antibody and approximately 310-330 mg/mL of total mass of the antibody-biopolymer conjugate, and wherein the antibody polymer conjugate comprises a) VH and VL in FIG. 64 or 6 CDRs within the VH and VL of FIG. 64.
104. The method of any one of the preceding arrangements, wherein at least 4 doses of the anti-VEGF antibody polymer conjugate is administered to the subject in the first year of treatment.
105. A method of treating diabetic retinopathy, comprising:

identifying a subject with diabetic retinopathy;
administering 1-3 loading doses of an anti-VEGF antibody polymer conjugate to the subject;
administering one or more subsequent doses of the anti-VEGF antibody polymer conjugate to the subject no more frequently than once every 24 weeks after the last loading dose,
wherein at least 4 doses of the anti-VEGF antibody polymer conjugate is administered to the subject in the first year of treatment.

106.A method of treating a subject having wAMD, comprising:

identifying a subject having wAMD; and

administering an anti-VEGF antibody polymer conjugate to the subject no more frequently than once every 4 weeks.

107. A method of treating DME, comprising:

identifying a subject having DME;
administering 2-3 monthly loading doses of an anti-VEGF antibody polymer conjugate to the subject; and
administering one or more subsequent doses of the anti-VEGF antibody polymer conjugate to the subject every 2-6 months, depending on:

whether retina thickness in the subject is above 320 microns (or 350 microns); and/or

whether the eye is dry as determined by OCT or OCT-A measurement,

wherein at least 4 doses of the anti-VEGF antibody polymer conjugate is administered to the subject in the first year of treatment.

108. A method of treating RVO, comprising:

identifying a subject having RVO;
administering 2 monthly loading doses of an anti-VEGF antibody polymer conjugate to the subject; and
administering one or more subsequent doses of the anti-VEGF antibody polymer conjugate to the subject no more frequently than once every 2 months,
wherein at least 4 doses of the anti-VEGF antibody polymer conjugate is administered to the subject in the first year of treatment.

109. A method of treating wAMD, comprising:

identifying a subject having wAMD;
administering 3 monthly loading doses of an anti-VEGF antibody polymer conjugate to the subject;
evaluating retina thickness and/or BCVA of the subject at 12 weeks after the third loading dose to thereby determine that the subject has improved visual acuity;
evaluating retina thickness and/or BCVA of the subject at 16 weeks after the third loading dose to thereby determine that the subject has improved visual acuity; and
administering a maintenance dose of the anti-VEGF antibody polymer conjugate to the subject 20 weeks after the third loading dose; and
determining that the subject will receive one or more maintenance doses no more frequently than once every 20 weeks,
wherein the subject has improved visual acuity when at least one of:

retina thickness is reduced by about 50 microns or more compared to retina thickness before administering the first loading dose; and
BCVA is increased by about 3 or more compared to BCVA before administering the first loading dose.

110. A pharmaceutical composition comprising:

an anti-VEGF antibody polymer conjugate;
12.5 mM sodium phosphate buffer; and
0.025% (w/w) polysorbate 20,
at a pH of about 6.5, wherein approximately 40-55 mg/mL of antibody mass is present and approximately 259-356 mg/mL of total mass of the antibody polymer conjugate is present in the composition.

111. A pharmaceutical composition comprising:

an anti-VEGF antibody polymer conjugate;
12.5 mM sodium phosphate buffer; and
0.025% (w/w) polysorbate 20,
at a pH of about 6.5, wherein approximately 50 mg/mL of antibody mass is present and approximately 324 mg/mL

of total mass of the antibody polymer conjugate is present in the composition.

112. A pharmaceutical composition comprising:

a) an anti-VEGF antibody polymer conjugate, 12.5 mM sodium phosphate buffer with 0.025% (w/w) polysorbate 20, pH 6.5, wherein the antibody polymer conjugate is present in the composition at a concentration of 50 mg/mL (based on antibody mass), equivalent to 324 mg/mL of total mass of the antibody polymer conjugate, including 50 mg/mL OG1950 Antibody Intermediate and 274 mg/mL OG1802 Biopolymer Intermediate;
b) an anti-VEGF antibody polymer conjugate, 10-15 mM sodium phosphate buffer with 0.01-0.5% (w/w) polysorbate 20, pH 6.5, wherein the antibody polymer conjugate is present in the composition at a concentration of about 50 mg/mL (based on antibody mass), equivalent to about 324 mg/mL of total mass of the antibody polymer conjugate;
c) an anti-VEGF antibody polymer conjugate, 12.5 mM sodium phosphate buffer with optional 0.025% (w/w) polysorbate 20, pH 6.5, wherein the antibody polymer conjugate is present in the composition at a concentration of 50 mg/mL (based on antibody mass), equivalent to 324 mg/mL of total mass of the antibody polymer conjugate, including 50 mg/mL OG1950 Antibody Intermediate and 274 mg/mL OG1802 Biopolymer Intermediate;
d) an anti-VEGF antibody polymer conjugate, sodium phosphate buffer with polysorbate 20, pH 6.5, wherein the antibody polymer conjugate is present in the composition at a concentration of 50 mg/mL (based on antibody mass), equivalent to about 324 mg/mL of total mass of the antibody-biopolymer conjugate, including about 50 mg/mL OG1950 Antibody Intermediate and about 274 mg/mL OG1802 Biopolymer Intermediate;
e) an anti-VEGF antibody polymer conjugate, 10-15 mM sodium phosphate buffer with 0.01-0.5% (w/w) polysorbate 20, pH 6.5, wherein the antibody polymer conjugate is present in the composition at a concentration of about 40-55 mg/mL (based on antibody mass), equivalent to about 259-356 mg/mL of total mass of the antibody-biopolymer conjugate;
f) an anti-VEGF antibody polymer conjugate, 10-15 mM sodium phosphate buffer with 0.01-0.5% (w/w) polysorbate 20, pH 6.5, wherein the antibody polymer conjugate is present in the composition at a concentration of about 45-52.5 mg/mL (based on antibody mass), equivalent to about 292-340 mg/mL of total mass of the antibody-biopolymer conjugate; or
g) an anti-VEGF antibody polymer conjugate, 10-15 mM sodium phosphate buffer with 0.01-0.5% (w/w) polysorbate 20, pH 6.5, wherein the antibody polymer conjugate is present in the composition at a concentration of about 40-55 mg/mL (based on antibody mass), equivalent to about 259-356 mg/mL of total mass of the antibody-biopolymer conjugate,

wherein the antibody polymer conjugate comprises an antibody comprising i) VH and VL in FIG. 64, or ii) 6 CDRs within the VH and VL of FIG. 64.

## EXAMPLES

Example 1: Retinal Half-Life, Retinal Bioavailability and Systemic Clearance of KSI-301

KSI-301 administered intravitreally showed extended half-life in the retina and choroid/retinal pigment epithelium (RPE) in a rabbit model (Figure 1). Comparison of the KSI-301 data with published results for Aflibercept and Ranibizumab indicated that KSI-301 exhibited superior durability compared to the other anti-VEGF therapeutics. Data are from a rabbit model. Ranibizumab data: Gaudrealt et al (2007) IOVS 46(2) 726, Gaudrealt et al (2007) Retina 27(9) 1260, Bakri et al (2007) Ophthalmol 114(12) 2179; Aflibercept data: EVER Congress Portoroz Slovenia (2008) Struble (Covance) Koehler-Stec (Regeneron). Aflibercept data was adjusted arithmetically to reflect 2,000μg dose administered (based on rabbit in vivo dosing of 500 μg); KSI-301 data was adjusted arithmetically to reflect 5,000 μg dose administered (based on rabbit in vivo dosing of 725 μg). Error bars reflects standard error of the mean.
KSI-301 administered intravitreally showed excellent retinal bioavailability (FIG. 2). Comparison of the KSI-301 data with published results for Aflibercept indicated better bioavailability of KSI-301. The data from FIG. 2 are from a covance rabbit ADME (absorption, distribution, metabolism, elimination) model. Aflibercept data (2008): EVER Congress Portoroz Slovenia Struble (Covance), Koehler-Stec (Regeneron). Error bars reflects standard error of the mean.
Intravenously administered KSI-301 showed rapid systemic clearance (FIG. 3). Comparison of the KSI-301 data with published results for Bevacizumab indicated faster systemic clearance for KSI-301. The Bevacizumab data was from Yeung et al 2010 Cancer Research.

Example 2: Ascending Dose Escalation Study of Intravitreal Administrations of KSI-301 in Subjects with Diabetic Macular Edema (DME)

*Study Design*

An ascending dose escalation study was carried out to assess ocular and systemic safety, tolerability, and establish a maximum tolerated dose (MTD) of KSI-301. One of three doses, 1.25 mg, 2.5 mg, and 5 mg (by weight of protein), were given to each subject as a single injection. Three subjects were enrolled at each dose level. Subjects were followed for a total of 12 weeks at the following time points; Day 2, Week 1, Week 2, Week 4, Week 8, and final follow up at Week 12.At the study onset, the first 3 subjects were enrolled into the 1st dosing cohort (1.25 mg of KSI-301) and treated with a single intravitreal dose of KSI-301. Subjects were sequentially assessed with a waiting period of at least 24 hours between subjects to allow sufficient time for safety review for each subject before approving injection of the next subject.

Dose groups were enrolled in an escalating fashion once the 3rd subject at the preceding dose level completed a safety period of one week after their dose of KSI-301. Subject enrollment and dose escalation were based upon review of safety information and the occurrence of dose limiting toxicities (DLTs).

Eight of the nine enrolled subjects were previously treated with limited to no response despite multiple prior anti-VEGF treatments and severe disease (BCVA median 3, range 0-7 in the year prior)

*Results*

A single injection of KSI-301 resulted in rapid, high-magnitude responses durable to 12 weeks (Figure 4). No intraocular inflammation and no drug-related adverse events were observed. **Figure 4:** Application of 1.25 mg, 2.5 mg, or 5 mg (by weight of protein) of KSI-301 administered intravitreally to subjects with diabetic macular edema (DME). BCVA and OCT CST was measured after intravitreal injection of KSI-301. Median changes from baseline to week 12, pooled across 3 dose groups (n=9 patients total, 3 patients per dose group). Do DV, Angiogenesis 2019; Patel et al., ARVO 2019.

The results show the effectiveness of KSI-301 as a single injection in patients, most of whom had previously been treated for their DME with other anti-VEGF agents (bevacizumab, aflibercept, and/or ranibizumab). The results indicate that KSI-301 when administered intravitreally provides an improvement in visual acuity and retinal thickness (measured on OCT or OCT-A) in a stable fashion through 12 weeks. The experiment examined diabetic macular edema (DME) patients with severe disease (n=9). Some patients were previously treated (8/9) with limited to no response despite multiple prior anti-VEGF treatments and severe disease (median 3, range 0-7 in the year prior). The results demonstrated that a single injection of KSI-301 resulted in rapid, high-magnitude responses durable to 12 weeks. No intraocular inflammation and no drug-related adverse events were observed.

The results were obtained from application of 1.25 mg, 2.5 mg, or 5 mg (by weight of protein) of KSI-301 administered intravitreally to subjects with diabeti macular edema. The results show the effectiveness of KSI-301 as a single injection in patients, most of whom had previously been treated for their DME with other anti-VEGF agents (bevacizumab, aflibercept, and/or ranibizumab). The results indicate that KSI-301 when administered in a single dose, as done here, had unexpected therapeutic effects because the visual acuity and retinal thickness (measured on OCT or OCT-A) improved in a stable fashion through 12 weeks.

<u>Example 3: Open Label, Multi-center Exploratory Study to Investigate Multiple Intravitreal Administrations of KSI-301 in Subjects with wet Age-Related Macular Degeneration (wAMD)</u>

The following study design and methods were followed in Examples 3, 4 and 5, unless indicated otherwise. In general, patients who were anti-VEGF treatment naïve and with either wAMD, DME, or RVO were randomly assigned to receive three monthly loading doses of either 2.5 mg or 5 mg (by weight of antibody) KSI-301 and then were followed every month or more often thereafter, and re-treated when either the physician determined that re-treatment due to disease activity was required or the patient met any re-treatment criteria.

*Overall Study Design*

In this study, patients who were anti-VEGF treatment naïve and with either wAMD, DME, or RVO were randomly assigned to receive three monthly loading doses of either 2.5 mg or 5 mg (by weight of antibody) KSI-301 and then were followed every month or more often thereafter, and retreated when either the physician determined that re-treatment due to disease activity is required or the patient meets any of the re-treatment criteria. These criteria were related to signs of disease recurrence and/or vision loss due to disease recurrence.

Two dose levels of KSI-301, 2.5 mg (50 µL) and 5 mg (100 µL) (by weight of protein) were evaluated in a multiple-dose study. Each subject received 3 initial intravitreal injections of KSI-301, the first at Day 1, the second at Week 4 and the third at Week 8 (Figure 5). All cohorts were randomized 1:3 to the 2.5 mg or 5 mg dose.

**Figure 5:** Study design for a randomized, open label study to evaluate multidose safety, efficacy and durability of intravitreal administration of KSI-301.

Subjects were evaluated every 4 weeks and may have received additional administration of the study drug starting at Week 16, if specific re-treatment criteria were met. There were no mandatory injections at assessment visits, except in the case of the wet AMD cohort, where a mandatory intravitreal injection was given at a visit if it had been 24 weeks since the last injection.

Eligible subjects were selected based on predetermined inclusion and exclusion criteria. Subjects were treatment naïve with respect to the eye disorder to be treated, and had no history of retinal disease other than the condition under investigation.

KSI-301 was formulated in approximately 12.5 mM sodium phosphate and 0.025% polysorbate 20 as an aqueous solution at 50 mg/mL (based on antibody mass) and filled into single-use 2.0 mL vials.

During the study, multiple-dose exposure of KSI-301 was well-tolerated and no intraocular inflammation was observed. 113 subjects were dosed, with 308 total doses given (104 doses at day 1, 96 doses at week 4, 84 doses at week 8). The following were observed:

- No intraocular inflammation or ocular SAEs in the study eye were reported to date;
- No drug-related adverse events (AEs) or drug-related serious adverse events (SAEs) were reported to date;
- Most AEs were assessed as mild and were consistent with profile of intravitreal anti-VEGFs;
- 8 non-ocular SAEs that were not drug-related were reported in 4 subjects:

  One 92 y/o RVO subject with hospitalization related to a pre-existing condition that resulted in death;
  One 66 y/o RVO subject with hospitalization related to dizziness;
  One 43 y/o DME subject with hospitalization related to a pre-existing condition;
  One 56 y/o DME subject with hospitalization related to a pre-existing condition. *wAMD Cohort*

Patients included in the wAMD cohort: were ≥50 years of age; had treatment naïve wet age-related macular degeneration involving the fovea; had a lesion area <30 $mm^2$ (12 disc areas) of any lesion type; had a BCVA ETDRS letter score ≤ 78 and ≥ 23 (~20/25 to ~20/320 Snellen equivalent) in the study eye at Screening and confirmed at Day 1; and had a decrease in vision in the study eye determined to be primarily the result of wAMD.

The average characteristics of the study population is show in Table 1.

Table 1: Study population characteristics

| Variable | wAMD cohort (n = 35) | DME cohort (n = 35) | RVO cohort (n = 35) |
|---|---|---|---|
| Age (years, mean) | 76 | 60 | 64 |
| Gender (Female, %) | 71.4 | 40.7 | 37.1 |
| Race, n (%), White | 32 (91.4) | 28 (82.4) | 31 (88.6) |
| BCVA (ETDRS letters, mean) | 66 | 70 | 59 |
| BCVA, Snellen 20/40 or better, n (%) | 14 (40.0) | 16 (47.1) | 6 (17.1) |
| OCT CST (microns, mean) | 380 | 402 | 630 |

*Trial Assessments*

The following assessments were made every 4 weeks after the 3 initial intravitreal injections of KSI-301: Best Corrected Visual Acuity (BCVA) by the Early treatment diabetic retinopathy study (ETDRS) visual acuity test; Spectral Domain Optical Coherence Tomography (SD-OCT); OCT angiography (OCT-A).

*Re-treatment Criteria (non-loading dose)*

Re-treatment with intravitreal injection of KSI-301 was performed if at least one of the following re-treatment criteria were met. These criteria are related to signs of disease recurrence and/or vision loss due to disease recurrence.

- Increase in OCT or OCT-A central subfield retinal thickness (CST) ≥75 μm with a decrease in BCVA of ≥ 5 letters compared to Week 12;
- Decrease in BCVA of > 5 letters compared to Day 1, due to worsening wAMD disease activity (e.g. increased intraretinal fluid, increased subretinal fluid, new intraretinal hemorrhage, new subretinal hemorrhage);
- Decrease in BCVA of ≥ 10 letters compared to the best prior BCVA, due to worsening wAMD disease activity (e.g.

increased intraretinal fluid, increased subretinal fluid, new intraretinal hemorrhage, new subretinal hemorrhage); or

- 24 weeks / 6 months have elapsed since the previous injection.

*Results*

Improvement in BCVA and OCT CST were observed in patients after the first loading dose administered on Day 1 (Figure 6; Figure 12, left column). The improved BCVA and OCT CST values were comparable to a standard of care anti-VEGF therapy. The therapeutic effect of KSI-301 was sustained during the loading phase, and continued for at least 8 weeks after the final loading dose administered at Week 8 (Figure 6). Reduction in CST was sustained for 7 months without re-treatment in a representative patient (Figure 12, left column).

**Figure 6:** Initial improvements in best corrected vision (BCVA) and retinal thickness (OCT) in patients with wet AMD. BCVA and CST assessment for wAMD cohort. N = 25. Includes randomized patients that reached Week 16 visit by the data cutoff date; 2.5 and 5 mg doses were pooled. BCVA= best corrected visual acuity; OCT= optical coherence tomography; CST= central subfield thickness.

**Figure 12:** Durability of therapy in patients from the wAMD cohort. OCT scan of individual retinas at day 1 (row 1), week 1 (row 2), month 3 (row 3), and month 7 (row 4) of treatment with KSI-301. Left column: wAMD patient; middle column: DME patient; right column: RVO patient.

The results show the durability of therapy in patients from the wAMD cohort to date (Figure 7). The data indicate that KSI-301 provide therapeutic effects that are unexpected relative to the current agents, and may provide better results because only 1 patient has been retreated before week 20 (or about 3 months since the last loading dose), 1 patient has been re-treated at 3 months since the last loading dose, and 11/14 (80%) have reached 4 months or longer until the first re-treatment. This indicates that the target dosing interval of 3 to 5 months in wAMD is possible.

In summary, only 1 patient out of 25 (4%) was re-treated before 3 months (Figure 7). Only 1 patient out of 20 (5%) was re-treated at 3 months. 86% (18/21) of the patients reached 3 months or longer after the last loading dose without re-treatment, and 80% (11/14) of the patients reached 4 months or longer after the last loading dose without re-treatment. This indicates that the target dosing interval in wAMD can be 3 to 5 months.

**Figure 7:** Treatment durability of KSI-301 in the wAMD cohort. Includes randomized patients that reached the first re-treatment opportunity (Week 12 visit) by the data cutoff date. Each bar represents an individual patient. Re-treatment is indicated by (♦). All depicted patients were followed beyond the indicated last assessment time point (indicated by a right arrow). The results can be summarized as follows:

18.5% have received re-treatment (5/27), Among patients reaching week 20, 95%
(20/21) have not received re-treatment for greater than 12-weeks after last loading dose
81.5% have not required re-treatment (22/27)
Among patients reaching week 20, 95% (20/21) have not received re-treatment for at least 12-weeks after the 3$^{rd}$ loading dose
Retreatment up to 6 months after the loading phase has been achieved in the KSI-301 5 mg dose.

<u>Example 4: Open Label, Multi-center Exploratory Study to Investigate Multiple Intravitreal Administrations of KSI-301 in Subjects with Diabetic Macular Edema (DME)</u>

The overall study design and trial assessment were as described in Example 3.

*DME Cohort*

Patients included in the DME cohort had: treatment naïve diabetic macular edema; a BCVA ETDRS letter score ≤ 78 and ≥ 23 (~20/25 to ~20/320 Snellen equivalent) in the study eye at Screening and confirmed at Day 1; Central subfield thickness (CST) of≥300 microns on SD-OCT (Heidelberg Spectralis or equivalent); and a decrease in vision in the study eye determined to be primarily the result of DME.

The average characteristics of the study population is show in Table 1, above.

*Re-treatment Criteria (non-loading dose)*

Re-treatment with intravitreal injection of KSI-301 was performed if at least one of the following re-treatment criteria were met. These criteria are related to signs of disease recurrence and/or vision loss due to disease recurrence.

Increase in OCT central subfield retinal thickness (CST) ≥75 μm with a decrease in BCVA of ≥ 5 letters compared to Week 12 or the prior visit (4-week span between visits); or

Decrease in BCVA of ≥ 10 letters compared to the best prior BCVA, due to worsening DME/RVO disease activity

(e.g. increased intraretinal fluid, increased subretinal fluid, new intraretinal hemorrhage, new exudates).

*Results*

Improvement in BCVA and OCT CST were observed in patients after the first loading dose administered on Day 1 (Figure 8; Figure 12, middle column). Therapeutic effect of KSI-301 was sustained during the loading phase, and continued for at least 8 weeks after the final loading dose administered at Week 8 (Figure 8). Reduction in CST was sustained for 7 months without re-treatment in a representative patient (Figure 12, middle column).

The data demonstrates the effect of KSI-301 after 3 monthly doses in patients with DME (Figure 8). The data are unexpected because current products require either monthly therapy or 5 monthly loading doses, whereas KSI-301 provided high levels of improvement after only 3 loading doses.

**Figure 8:** BCVA and CST assessment for DME cohort. N = 12. Includes randomized patients that reached Week 16 visit by the data cutoff date; 2.5 and 5 mg doses were pooled. BCVA= best corrected visual acuity; OCT= optical coherence tomography; CST= central subfield thickness.

No patient required re-treatment before 3 months after the last loading dose, and no patient required a $4^{th}$ or $5^{th}$ monthly loading dose. (Figure 9). 18% (2/11) of patients were re-treated at 3 months. Among patients reaching week 20, 81.8% (9/11) did not require retreatment for over 12-weeks after the 3rd loading dose. Some patients reached 4, 5, or 6 months without re-treatment.

The data indicate the potential for 3+ month dosing interval in patients with DME after only 3 loading doses (Figure 9). Furthermore, the data are unexpected because no DME patient has yet been re-treated before 3 months. Additionally over 80% have gone longer than 3 months after the last loading dose before they needed to be re-treated. This is unexpected because with conventional therapies, as many as 9-10 injections are administered in the first 12 months of therapy.

**Figure 9:** Treatment durability of KSI-301 in the DME cohort. Includes randomized patients that reached the first re-treatment opportunity (Week 12 visit) by the data cutoff date. Each bar represents an individual patient. Re-treatment is indicated by (♦). All depicted patients were followed beyond the indicated last assessment time point (indicated by a right arrow). The results can be summarized as follows:

11.8% have received re-treatment (2/17)
Among patients reaching week 20, 80% (8/10) have not received re-treatment for greater than 12-weeks after last loading dose
88.2% have not received re-treatment (15/17)
Among patients reaching week 20, 81.8% (9/11) have not received re-treatment for greater than 12-weeks after only 3 loading doses
No patient has required a $4^{th}$ or $5^{th}$ monthly loading dose.

FIG. 16 depicts the Diabetic Retinopathy Severity results (DRSS). The figure shows the proportion of patients with differing levels of diabetic retinopathy severity, measured on a standardized photographic reading scale by an independent expert reading center. DR can be described as different levels of severity, from mild to moderate to severe non-proliferative diabetic retinopathy, for example, or mild to high-risk proliferative DR. In this case, the majority of patients had level 47 disease (moderate NPDR) at baseline. After 12 weeks (3 loading doses and then one month), 27% of the patients had an improvement in DR severity by >=2 steps on the severity scale, 13% had a one-step improvement, and 60% maintained the same level of DR severity. (DR severity is known to improve following the application of anti-VEGF therapy but with other agents it takes 1 to 2 years to reach peak effect, and the effect is lost in many patients, and the disease worsens again, if the anti-VEGF therapy is stopped). The images show a patient with proliferative (level 65) disease who had disease modification and improved to non-proliferative (level 53, 2 steps on the standard grading scale) at week 12. With no additional doses given, the effect on DR severity was maintained for an additional 14 weeks which was the maximal follow-up time available for that patient.
In some embodiments, a patient with non-proliferative DR can be treated using an anti-VEGF antibody conjugate (e.g., KSI-301) with no or potentially only a few loading doses (e.g., 1 dose, 2 doses, or 3 initiating doses) and then retreated every 3 to 6 months for the treatment of non-proliferative DR.

Example 4.5

A patient with non-proliferative DR is identified. The patient is treated with no loading dose, but treated every 3-6 months with a single injection of an anti-VEGF antibody conjugate (e.g., KSI-301) and the non-proliferative DR is treated. In the alternative, the patient is treated with 1, 2, or 3 loading doses first.

Example 5: Open Label, Multi-center Exploratory Study to Investigate Multiple Intravitreal Administrations of KSI-301 in Subjects with Retinal Vein Occlusion (RVO)

The overall study design and trial assessment were as described in Example 3.

*RVO Cohort*

Patients included in the RVO cohort had: treatment naïve retinal vein occlusion with macular edema and secondary visual impairment; a BCVA ETDRS letter score ≤ 78 and ≥ 23 (~20/25 to ~20/320 Snellen equivalent) in the study eye at Screening and confirmed at Day 1; Central subfield thickness (CST) of ≥300 microns on SD-OCT (Heidelberg Spectralis or equivalent); Branch retinal vein occlusion (BRVO) or central retinal vein occlusion (CRVO); and a decrease in vision in the study eye determined by the investigator to be primarily the result of macular edema secondary to RVO.

The average characteristics of the study population is show in Table 1.

*Re-treatment Criteria*

Re-treatment with intravitreal injection of KSI-301 was performed if at least one of the following re-treatment criteria were met. These criteria are related to signs of disease recurrence and/or vision loss due to disease recurrence.

- Increase in OCT central subfield retinal thickness (CST) ≥75 μm with a decrease in BCVA of ≥ 5 letters compared to Week 12 or the prior visit (4-week span between visits); or
- Decrease in BCVA of ≥ 10 letters compared to the best prior BCVA, due to worsening DME/RVO disease activity (e.g. increased intraretinal fluid, increased subretinal fluid, new intraretinal hemorrhage, new exudates).

*Results*

Improvement in BCVA and OCT CST were observed in patients after the first loading dose administered on Day 1 (Figure 10; Figure 12, right column). Therapeutic effect of KSI-301 was sustained during the loading phase, and continued for at least 8 weeks after the final loading dose administered at Week 8 (Figure 10). Reduction in CST was sustained for 7 months without re-treatment in a representative patient (Figure 12, right column).

The data demonstrate the effect of KSI-301 after 3 monthly doses in patients with RVO (Figure 10). The data are unexpected because a continued improvement in visual acuity is observed from 8 weeks to 16 weeks. In studies of ranibizumab, aflibercept, and bevacizumab in RVO, switching from monthly therapy to less than monthly therapy results in worsening of visual acuity and OCT CST.

**Figure 10:** BCVA and CST assessment for RVO cohort. N = 14. Includes randomized patients that reached Week 16 visit by the data cutoff date; 2.5 and 5 mg doses were pooled. BCVA= best corrected visual acuity; OCT= optical coherence tomography; CST= central subfield thickness.

75% (18/24) of patients in the RVO cohort did not require re-treatment (Figure 11). 8% (2/24) of patients had first reetreatment at 1 month; 23% (3/13) of patients had first retreatment at 2 months; and 11% (1/9) of patients had first re-treatment at 3 months. Among patients reaching week 20, 56% (5/9) did not require re-treatment for over 12-weeks after the 3rd loading dose. 100% (18/18) patients in the 5 mg cohort did not require a $4^{th}$ or $5^{th}$ monthly loading dose, and if any, received re-treatment at 9 weeks or later after the last loading doses. Moreover, only 2 of 8 patients on 5 mg had received first re-treatment at 2 months, and both then had a longer time before the next treatment. Thus, of the patients who have received more than one re-treatment, the time between the first and second re-treatment was extended compared to the time between the last loading dose and the first re-treatment.

The data shows that in the 5 mg dose group, the RVO patients were receiving first retreatment at 8 weeks or later (Figure 11). This result is unexpected because conventional therapy requires monthly dosing to treat RVO. Moreover, of the patients who had received more than one retreatment, the time between first and second retreatment was extended, which was also unexpected because typically patients with RVO have disease recurrence on a particular pattern, or regular intervals. Finally, over half the patients (5/9) have gone for longer than 3 months since the last loading dose, which was also unexpected given the high intraocular VEGF load of RVO.

**Figure 11:** Treatment durability of KSI-301 in the RVO cohort. Includes randomized patients that reached the first re-treatment opportunity (Week 12 visit) by the data cutoff date. Each bar represents an individual patient. Re-treatment is indicated by (♦). Patients followed beyond the indicated last assessment time point are indicated by a right arrow. Discontinuation is indicated by a left arrow. Further results shown in FIG. 11 can be summarized as follows:

- 25% have received re-treatment (6/24)
- Among patients reaching week 20, 56% (5/9) have not received re-treatment for greater than 12-weeks after last

loading dose

- 75% have not received re-treatment (18/24)
- Among patients reaching week 20, 56% (5/9) have not received re-treatment for greater than 12-weeks after only 3 loading doses
- 18/18 patients in the KSI-301 5 mg cohort didn't require a 4th monthly loading dose

Example 6

One first identifies a subject in need of having an eye disorder treated (either prophylactically or otherwise). Then one administers between 1 and 5 mg of antibody of an anti-VEGF antibody conjugate (e.g., KSI-301) to the subject in a first loading dose, and optionally a second loading dose and optionally a third loading dose (within about one month of each other). Following the last loading dose, the subject will retain a therapeutic benefit of the anti-VEGF therapy for at least 12 weeks. This can be applied to diabetic macular edema (DME), retinal vein occlusion (RVO), wet age-related macular degeneration (AMD), and/or in the alternative diabetic retinopathy (DR).

Example 6.5

One first identifies a subject in need of having an eye disorder treated (either prophylactically or otherwise). Then one administers between 1 and 5 mg of antibody of an anti-VEGF antibody conjugate (e.g., KSI-301) to the subject in a first loading dose, and optionally a second loading dose and optionally a third loading dose (within about one month of each other). Following the last loading dose, the subject will retain a therapeutic benefit of the anti-VEGF antibody conjugate therapy for at least 16 weeks. Alternatively, following the last loading dose, the subject will retain a therapeutic benefit of the anti-VEGF antibody conjugate therapy for at least 20 weeks. Alternatively, following the last loading dose, the subject will retain a therapeutic benefit of the anti-VEGF antibody conjugate therapy for at least 24 weeks. Alternatively, following the last loading dose, the subject will retain a therapeutic benefit of the anti-VEGF antibody conjugate therapy for at least 30 weeks. Alternatively, following the last loading dose, the subject will retain a therapeutic benefit of the anti-VEGF antibody conjugate therapy for at least 36 weeks. Alternatively, following the last loading dose, the subject will retain a therapeutic benefit of the anti-VEGF antibody conjugate therapy for at least 42 weeks. Alternatively, following the last loading dose, the subject will retain a therapeutic benefit of the anti-VEGF antibody conjugate therapy for at least 48 weeks. Alternatively, following the last loading dose, the subject will retain a therapeutic benefit of the anti-VEGF antibody conjugate therapy for at least 54 weeks. Alternatively, following the last loading dose, the subject will retain a therapeutic benefit of the anti-VEGF antibody conjugate therapy for at least 60 weeks. This can be applied to diabetic macular edema (DME), retinal vein occlusion (RVO), wet age-related macular degeneration (AMD), and/or in the alternative diabetic retinopathy (DR).

Example 7

The present non-limiting example provides a method of treating retinal vein occlusion (RVO). The method comprises administering an anti-VEGF antibody conjugate (e.g., KSI-301) to a subject with RVO at a first loading dose. One can then repeat the loading dose, once or in the alternative two times. This results in the subject retaining a therapeutic result of the anti-VEGF antibody conjugate therapy for at least 8 weeks after a final loading dose. In the alternative, this results in the subject retaining a therapeutic result of the anti-VEGF antibody bioconjugate, e.g., KSI-301, therapy for at least 12 weeks after a final loading dose. In the alternative, this results in the subject retaining a therapeutic result of the anti-VEGF antibody bioconjugate, e.g., KSI-301, therapy for at least 20 weeks after a final loading dose.

Example 7.5

The present non-limiting example provides a method of treating retinal vein occlusion (RVO). The method comprises administering an anti-VEGF antibody conjugate (e.g., KSI-301) to a subject with RVO at a first loading dose. One can then repeat the loading dose, once or in the alternative two times. This results in the subject retaining a therapeutic result of the anti-VEGF antibody conjugate therapy for at least 24 weeks after a final loading dose. In the alternative, this results in the subject retaining a therapeutic result of the anti-VEGF antibody conjugate therapy for at least 30 weeks after a final loading dose. In the alternative, this results in the subject retaining a therapeutic result of the anti-VEGF antibody conjugate therapy for at least 36 weeks after a final loading dose. In the alternative, this results in the subject retaining a therapeutic result of the anti-VEGF antibody conjugate therapy for at least 42 weeks after a final loading dose. In the alternative, this results in the subject retaining a therapeutic result of the anti-VEGF antibody conjugate therapy for at least 48 weeks after a final loading dose. In the alternative, this results in the subject retaining a therapeutic result of the anti-VEGF antibody conjugate therapy for at least 54 weeks after a final loading dose. In the alternative, this results in the subject retaining a therapeutic result of the anti-VEGF antibody conjugate therapy for at least 60 weeks after a final loading

dose.

Example 8

The present non-limiting example provides a method of improving perfusion of an eye, the method comprises identifying a subject with DME, DR or RVO. One then administers at least 2 loading doses of an anti-VEGF antibody conjugate (e.g., KSI-301) to the subject at 1.25-5 mg, with one month between each injection. One does not administer more than 2 injections. One then provides one or more further doses (retreatments) of the anti-VEGF antibody conjugate, e.g., KSI-301, to the subject, until the subject displays improved perfusion in at least one eye.

Example 9

The present non-limiting example provides a method of improving perfusion of an eye. The method involves identifying a subject with non-proliferative DR and administering an initial dose of an anti-VEGF antibody conjugate (e.g., KSI-301) to the subject (between 1 and 5 mg of antibody), to provide improved perfusion in at least one eye. The dose is repeated until perfusion is achieved within the subject's treated eye.

Example 10

The present non-limiting example provides a method of treating a subject with DME, DR or RVO. The method comprises administering 1-3 loading doses of an anti-VEGF antibody conjugate (e.g., KSI-301) (at 1-5 mg of antibody on a once monthly basis) to a subject with DME, DR or RVO. One does not administer more than 3 loading doses to the subject. To the extent required, if at all, one provides a follow-on application of the anti-VEGF antibody conjugate (retreatment) at a point in time no sooner than 12 weeks after a last loading dose.

Example 10.5

The present non-limiting example provides a method of treating a subject with DME, DR or RVO. The method comprises administering 1-3 loading doses of an anti-VEGF antibody conjugate (e.g., KSI-301) (at 1-5 mg of antibody on a once monthly basis) to a subject with DME, DR or RVO. One does not administer more than 3 loading doses to the subject. To the extent required, if at all, one provides a follow-on application of the anti-VEGF antibody conjugate (retreatment) at a point in time no sooner than 16 weeks after a last loading dose. Alternatively, one provides a follow-on application of the anti-VEGF antibody conjugate (retreatment) at a point in time no sooner than 20 weeks after a last loading dose. Alternatively, one provides a follow-on application of the anti-VEGF antibody conjugate (retreatment) at a point in time no sooner than 24 weeks after a last loading dose. Alternatively, one provides a follow-on application of the anti-VEGF antibody conjugate (retreatment) at a point in time no sooner than 30 weeks after a last loading dose. Alternatively, one provides a follow-on application of the anti-VEGF antibody conjugate (retreatment) at a point in time no sooner than 36 weeks after a last loading dose. Alternatively, one provides a follow-on application of the anti-VEGF antibody conjugate (retreatment) at a point in time no sooner than 42 weeks after a last loading dose. Alternatively, one provides a follow-on application of the anti-VEGF antibody conjugate (retreatment) at a point in time no sooner than 48 weeks after a last loading dose. Alternatively, one provides a follow-on application of the anti-VEGF antibody conjugate (retreatment) at a point in time no sooner than 54 weeks after a last loading dose. Alternatively, one provides a follow-on application of the anti-VEGF antibody conjugate (retreatment) at a point in time no sooner than 60 weeks after a last loading dose.

Example 11

The present non-limiting example provides a method of treating a subject with non-proliferative DR. One identifies a patient in need of therapy and administers 1 or 2 loading doses of an anti-VEGF antibody conjugate(e.g., KSI-301) (1-5 mg, once a month) to a subject with non-proliferative DR. One does not administer more than 2 loading doses to the subject. Optionally, one can provide a follow-on administration of the anti-VEGF antibody conjugate, e.g., KSI-301, (retreatment) at a point in time no sooner than 12 weeks after a last loading dose.

Example 11.5

The present non-limiting example provides a method of treating a subject with non-proliferative DR. One identifies a patient in need of therapy and administers 1 or 2 loading doses of an anti-VEGF antibody conjugate (e.g., KSI-301) (1-5 mg, once a month) to a subject with non-proliferative DR. One does not administer more than 2 loading doses to the subject. Optionally, one can provide a follow-on administration of the anti-VEGF antibody conjugate

(retreatment) at a point in time no sooner than 16 weeks after a last loading dose. Alternatively, one can provide a follow-on administration of the anti-VEGF antibody conjugate (retreatment) at a point in time no sooner than 20 weeks after a last loading dose. Alternatively, one can provide a follow-on administration of the anti-VEGF antibody conjugate (retreatment) at a point in time no sooner than 24 weeks after a last loading dose. Alternatively, one can provide a follow-on administration of the anti-VEGF antibody conjugate (retreatment) at a point in time no sooner than 30 weeks after a last loading dose. Alternatively, one can provide a follow-on administration of the anti-VEGF antibody conjugate (retreatment) at a point in time no sooner than 36 weeks after a last loading dose. Alternatively, one can provide a follow-on administration of the anti-VEGF antibody conjugate (retreatment) at a point in time no sooner than 42 weeks after a last loading dose. Alternatively, one can provide a follow-on administration of the anti-VEGF antibody conjugate (retreatment) at a point in time no sooner than 48 weeks after a last loading dose. Alternatively, one can provide a follow-on administration of the anti-VEGF antibody conjugate (retreatment) at a point in time no sooner than 54 weeks after a last loading dose. Alternatively, one can provide a follow-on administration of the anti-VEGF antibody conjugate (retreatment) at a point in time no sooner than 60 weeks after a last loading dose.

Example 12

The present non-limiting example provides a method of treating a subject with RVO. The method comprises administering 1 or 2 loading doses of an anti-VEGF antibody conjugate(e.g., KSI-301) (1-5 mg, once monthly) to a subject with RVO. No additional loading doses are administered to the3 subject. One then, optionally, provides a follow-on administration of the anti-VEGF antibody conjugate (retreatment) at a point in time no sooner than 8 weeks after a last loading dose. In the alternative, the retreatment occurs no sooner than 12 weeks. In the alternative, the retreatment occurs no sooner than 16 weeks. In the alternative, the retreatment occurs no sooner than 20 weeks. In the alternative, the retreatment occurs no sooner than 24 weeks.

Example 12.5

The present non-limiting example provides a method of treating a subject with RVO. The method comprises administering 1 or 2 loading doses of an anti-VEGF antibody conjugate (e.g., KSI-301) (1-5 mg, once monthly) to a subject with RVO. No additional loading doses are administered to the3 subject. One then, optionally, provides a follow-on administration of the anti-VEGF antibody conjugate (retreatment) at a point in time no sooner than 30 weeks after a last loading dose. In the alternative, the retreatment occurs no sooner than 36 weeks. In the alternative, the retreatment occurs no sooner than 42 weeks. In the alternative, the retreatment occurs no sooner than 48 weeks. In the alternative, the retreatment occurs no sooner than 54 weeks. In the alternative, the retreatment occurs no sooner than 60 weeks.

Example 13

The present non-limiting example provides a method of treating RVO. One administers an anti-VEGF antibody conjugate(e.g., KSI-301) (1-5 mg, once a month) to a subject in need of treating RVO at 1-3 loading doses. The subject thereby retains a therapeutic result of the anti-VEGF antibody conjugate therapy for RVO for at least 8 weeks after a final loading dose. The subject will retain at least one, if not most or all of the therapeutic benefits over this period of time, such that the subject will not substantially benefit from another retreatment for at least 14 weeks following the last application of the anti-VEGF antibody conjugate.

Example 13.5

The present non-limiting example provides a method of treating RVO. One administers an anti-VEGF antibody conjugate (e.g., KSI-301) (1-5 mg, once a month) to a subject in need of treating RVO at 1-3 loading doses. The subject thereby retains a therapeutic result of the anti-VEGF antibody conjugate therapy for RVO for at least 12 weeks after a final loading dose. The subject will retain at least one, if not most or all of the therapeutic benefits over this period of time, such that the subject will not substantially benefit from another retreatment for at least 18 weeks following the last application of the anti-VEGF antibody bioconjugate. Alternatively, the subject retains at least one, if not most or all of the therapeutic benefits over this period of time, such that the subject will not substantially benefit from another retreatment for at least 24 weeks following the last application of the anti-VEGF antibody bioconjugate. Alternatively, the subject retains at least one, if not most or all of the therapeutic benefits over this period of time, such that the subject will not substantially benefit from another retreatment for at least 30 weeks following the last application of the anti-VEGF antibody bioconjugate. Alternatively, the subject retains at least one, if not most or all of the therapeutic benefits over this period of time, such that the subject will not substantially benefit from another retreatment for at least 36 weeks following the last application of the anti-VEGF antibody bioconjugate. Alternatively, the subject retains at least one, if not most or all of the therapeutic

benefits over this period of time, such that the subject will not substantially benefit from another retreatment for at least42 weeks following the last application of the anti-VEGF antibody bioconjugate. Alternatively, the subject retains at least one, if not most or all of the therapeutic benefits over this period of time, such that the subject will not substantially benefit from another retreatment for at least 48 weeks following the last application of the anti-VEGF antibody bioconjugate. Alternatively, the subject retains at least one, if not most or all of the therapeutic benefits over this period of time, such that the subject will not substantially benefit from another retreatment for at least 54 weeks following the last application of the anti-VEGF antibody bioconjugate. Alternatively, the subject retains at least one, if not most or all of the therapeutic benefits over this period of time, such that the subject will not substantially benefit from another retreatment for at least 60 weeks following the last application of the anti-VEGF antibody bioconjugate.

EXAMPLE 14

One first identifies a subject in need of having an eye disorder treated (either prophylactically or otherwise). Then one administers between 1 and 5 mg of antibody of an anti-VEGF antibody conjugate (e.g., KSI-301) to the subject in a first loading dose, and optionally a second loading dose and optionally a third loading dose (within about one month of each other). Following the last loading dose, the subject will retain a therapeutic benefit of the anti-VEGF antibody conjugate therapy for at least 12 weeks. This can be applied to diabetic macular edema (DME), retinal vein occlusion (RVO), wet age-related macular degeneration (AMD), and/or in the alternative diabetic retinopathy (DR). Following any retreatment application, the duration between any subsequent retreatment events will increase, as the subject will need less and less treatment for each retreatment administered.

EXAMPLE 14.5

One first identifies a subject in need of having an eye disorder treated (either prophylactically or otherwise). Then one administers between 1 and 5 mg of antibody of an anti-VEGF antibody conjugate (e.g., KSI-301) to the subject in a first loading dose, and optionally a second loading dose and optionally a third loading dose (within about one month of each other). Following the last loading dose, the subject will retain a therapeutic benefit of the anti-VEGF antibody conjugate therapy for at least 18 weeks. Alternatively following the last loading dose, the subject will retain a therapeutic benefit of the anti-VEGF antibody conjugate therapy for at least 24 weeks. Alternatively following the last loading dose, the subject will retain a therapeutic benefit of the anti-VEGF antibody conjugate therapy for at least 30 weeks. Alternatively following the last loading dose, the subject will retain a therapeutic benefit of the anti-VEGF antibody conjugate therapy for at least 36 weeks. Alternatively following the last loading dose, the subject will retain a therapeutic benefit of the anti-VEGF antibody conjugate therapy for at least 42 weeks. Alternatively following the last loading dose, the subject will retain a therapeutic benefit of the anti-VEGF antibody conjugate therapy for at least 48 weeks. Alternatively following the last loading dose, the subject will retain a therapeutic benefit of the anti-VEGF antibody conjugate therapy for at least 54 weeks. Alternatively following the last loading dose, the subject will retain a therapeutic benefit of the anti-VEGF antibody conjugate therapy for at least 60 weeks.

This can be applied to diabetic macular edema (DME), retinal vein occlusion (RVO), wet age-related macular degeneration (AMD), and/or in the alternative diabetic retinopathy (DR). Following any retreatment application, the duration between any subsequent retreatment events will increase, as the subject will need less and less treatment for each retreatment administered.

Example 15

The present non-limiting example summarizes the safety and characteristics observed for the use of KSI-301 through repeated administration. 113 subjects were dosed over phase 1a and 1b. 308 total doses were administered. The following was observed with respect to safety:

- No intraocular inflammation or ocular SAEs in the study eye reported to date
- No drug-related AEs or drug-related SAEs reported to date
- Most AEs were assessed as mild and are consistent with profile of intravitreal anti-VEGFs
- 8 non-ocular SAEs that were not drug-related have been reported in 4 subjects:

  - One 92 y/o RVO subject with hospitalization related to a pre-existing condition that resulted in death
  - One 66 y/o RVO subject with hospitalization related to dizziness
  - One 43 y/o DME subject with hospitalization related to a pre-existing condition
  - One 56 y/o DME subject with hospitalization related to a pre-existing condition.

The above examples demonstrate that Antibody Biopolymer Conjugate (ABC) constructs are a new design platform for long durability intravitreal medicines. KSI-301 has achieved important development results, including:

**Excellent Safety:** zero cases of intraocular inflammation after 300+ doses
**Strong Efficacy:** across 3 major phenotypically variable retinal diseases wet AMD, DME/DR & RVO
**Remarkable Biological Durability:** majority of treated eyes extended to 4 months or beyond without retreatment after 3 loading doses. It is anticipated that potential is being demonstrated for:

3 to 5+ month interval in wAMD
3 to 5+ month interval in DME
2 to 3+ month interval in RVO

EXAMPLE 16

FIGs. 17A and 17B depict OCT and OCT angiography of a wet AMD patient that has been treated with 3 loading doses of 5mg KSI-301 at baseline, week 4 and week 8. In additional to diminishing fluid on the OCT images, there's a direct effect on the choroidal neovascular membrane in both flow and size as represented with the spot reduction in the center of the panels. The choroidal neovascular membrane is the core feature of wet AMD, and having a direct effect in this membrane is believed to be a sign of disease modification.

EXAMPLE 17

**FIGs. 18A-18D** show an example of a DME with disease modification post 3 loading doses, with significant DRSS improvement and reperfusion representing disease modification. FIG. 18A shows a time course revealing that it is possible to get both fast and long lasting effect in DME with only 3 loading doses (no retreatment required for at least 5 months). FIG. 18B demonstrates that the effectiveness of only three loading doses of KSI-301 is present in proliferative diabetic retinopathy. As shown, there was a fast and substantial (2 steep) improvement, sustained 14 weeks after 3 loading doses of KSI-301. In addition to the conversion from PDR to NPDR, the subject also displayed signs of peripheral vascular reperfusion (FIGs. 18C and 18D).

Thus, use of KSI-301 in the manner provided herein can be used to achieve fast and prolonged results and reperfusion.

EXAMPLE 18

FIG. 19 displays the results in an RVO patient, that after 3 loading doses, no additional doses were required for at least 5 months, representing what is believed to be disease modification. Thus, use of KSI-301 in the manner provided herein can be used to achieve disease modification.

EXAMPLE 19

FIG. 20 displays a set of OCT images of a patient showing the effect of 3 loading doses lasting 8 weeks until diseases recurs and the patient receives retreatment. The effect of that 4th dose lasts 16 weeks until the patient requires retreatment, effectively doubling the retreatment interval from 8 to 16 weeks, which could be a sign of disease modification. Thus, this demonstrates that additional effectiveness is achieved through the retreatment process as well, allowing for more time between subsequent required retreatments.

EXAMPLE 20

FIG. 21A-21C Show the results of a single injection of KSI-301. Phase 1 Single Dose Study - Summary:

- Rapid high-magnitude and durable treatment responses were seen at all dose levels tested.
- Twelve weeks after a single dose, median BCVA improvement from baseline of +9 ETDRS chart letters and median improvement in retinal edema of -121 microns (OCT CST) were observed.
- No dose-limiting toxicities, drug-related adverse events, or intraocular inflammation were observed through each patients' last visit at 12 weeks.

FIG. 21A show a graph of the median changes from baseline to week 12. It demonstrates a rapid, high response that is durable for KSI-301 administration. Case study 1 results are shown in FIG. 21B, which demonstrates the

effectiveness of KSI-301 administration on chronic macular edema in a subject with prior suboptimal response. FIG. 21C displays the results from Case study 2, which shows the resolution of subretinal fluid through 12 weeks in a subject with chronic edema and extensive foveal lipid exudates.

These results provide two cases where after a single injection, in previously treated and failed patients, a single injection of KSI-301 has long lasting benefit. In case 2, the effect slow manifested and increased over time after KSI-301 injection, demonstrating a form of disease modification.

EXAMPLE 21

Follow-up assessments of patients enrolled in the study described in Example 3 were performed. The results show durability of KSI-301 treatment in patients from the wAMD cohort that extended to 3 to 5 or more months (Figure 22). 83% (20/24) of patients reached 4 months or longer before first retreatment. 85% (22/26) of patients did not receive retreatment for longer than 3 months after the last loading dose. 6% (2/31) were retreated before 3 months after the last loading dose, and 8% (2/25) were retreated at 3 months after the last loading dose. These results indicate that the target dosing interval in wAMD can be 3 to 5 or more months after the loading dose.

**Figure 22:** Treatment durability of KSI-301 in the wAMD cohort. Includes randomized patients that reached the first re-treatment opportunity (Week 12 visit) by the data cutoff date. Each bar represents an individual patient. Re-treatment is indicated by (♦). Patients followed beyond the indicated last assessment time point (indicated by a right arrow). One discontinued patient is indicated by left arrow.

Improvement in BCVA and OCT CST continued to be observed in patients at week 20, 12 weeks after the last loading dose (Figure 23). The improved BCVA and OCT CST values were comparable to a standard of care anti-VEGF therapy. The therapeutic effect of KSI-301 was sustained during the loading phase, and continued for at least 12 weeks after the final loading dose administered at Week 8 (Figure 23). Improvement in BCVA and OCT CST was observed in a larger cohort of patients that reached Week 12 (Figure 25).

**Figure 23:** Improvements in best corrected vision (BCVA) and retinal thickness (OCT) in patients with wet AMD. BCVA and CST assessment for wAMD cohort. N = 25. Includes randomized patients that reached Week 20 visit by the data cutoff date; 2.5 and 5 mg doses were pooled. BCVA= best corrected visual acuity; OCT= optical coherence tomography; CST= central subfield thickness. Error bars represent standard error of the mean.

**Figure 25:** Improvements in BCVA and OCT in wet AMD patients treated with KSI-301. N = 31. Includes randomized patients that reached Week 12 visit by the data cutoff date; 2.5 and 5 mg doses were pooled. BCVA= best corrected visual acuity; OCT= optical coherence tomography; CST= central subfield thickness. Error bars represent standard error of the mean.

Improvement in BCVA and OCT CST of a subset of patients from the wAMD cohort that did not have high pigment epithelial detachment (PED) were analyzed. These patients also showed sustained improvement in BCVA and OCT CST values, and the therapeutic effect of KSI-301 was sustained during the loading phase, and continued for at least 12 weeks after the final loading dose administered at Week 8 (Figure 24). Improvement in BCVA and OCT CST was observed in a larger cohort of wAMD patients without high PED that reached Week 12 (Figure 26).

**Figure 24:** Improvements in best corrected vision (BCVA) and retinal thickness (OCT) in wet AMD patients without high PED. BCVA and CST assessment for wAMD cohort. N = 23. High PED defined as presence of a PED with baseline CST ≥500 microns. Includes randomized patients that reached Week 20 visit by the data cutoff date; 2.5 and 5 mg doses were pooled. BCVA= best corrected visual acuity; OCT= optical coherence tomography; CST= central subfield thickness. Error bars represent standard error of the mean.

**Figure 26:** Improvements in BCVA and OCT in wet AMD patients with high PED, treated with KSI-301. N = 29. High PED defined as presence of a PED with baseline CST ≥500 microns. Includes randomized patients that reached Week 12 visit by the data cutoff date; 2.5 and 5 mg doses were pooled. BCVA= best corrected visual acuity; OCT= optical coherence tomography; CST= central subfield thickness. Error bars represent standard error of the mean.

EXAMPLE 22

Follow-up assessments of patients enrolled in the study described in Example 4 were performed. The results show durability of KSI-301 treatment in patients from the DME cohort that extended more than 4 months (Figure 27). 72% (8/11) of patients reached 4 months or longer without retreatment. 81% (13/16) did not require retreatment more than 3 months after the last loading dose. One patient was retreated before 3 months, and 13% (2/15) of patients were retreated at 3 months. These results indicate that the target dosing interval in DME can be 3 or more months after the loading dose.

**Figure 27:** Treatment durability of KSI-301 in the DME cohort. Includes randomized patients that reached the first re-treatment opportunity (Week 12 visit) by the data cutoff date. Each bar represents an individual patient. Re-treatment is indicated by (♦). All depicted patients were followed beyond the indicated last assessment time point (indicated by a right arrow).

Improvement in BCVA and OCT CST were observed in DME patients at Week 20, 12 weeks after the last loading dose (Figure 28). Therapeutic effect of KSI-301 was sustained during the loading phase, and continued for at least 12 weeks after the final loading dose administered at Week 8. Improvement in BCVA and OCT CST was observed in a larger cohort of DME patients that reached Week 12 (Figure 29).

**Figure 28:** BCVA and CST assessment for DME cohort. N = 15. Includes randomized patients that reached Week 20 visit by the data cutoff date; 2.5 and 5 mg doses were pooled. BCVA= best corrected visual acuity; OCT= optical coherence tomography; CST= central subfield thickness. Error bars represent standard error of the mean.

**Figure 29:** BCVA and CST assessment for DME cohort. N = 19. Includes randomized patients that reached Week 12 visit by the data cutoff date; 2.5 and 5 mg doses were pooled. BCVA= best corrected visual acuity; OCT= optical coherence tomography; CST= central subfield thickness. Error bars represent standard error of the mean.

EXAMPLE 23

Follow-up assessments of patients enrolled in the study described in Example 5 were performed. The results show durability of KSI-301 treatment in patients from the RVO cohort that extended more than 3 months (Figure 30). Only 3 patients received more than 1 retreatment, and in those 3 patients, each retreatment occurred at a longer interval than the first interval until retreatment. 50% (9/18) of patients reached 3 months or longer without retreatment. 6% (2/32), 30% (7/23) and 14% (2/14) of patients received first retreatment at 1, 2 and 3 months, respectively. These results indicate that the target dosing interval in RVO can be 2 to 3 months or longer.

**Figure 30:** Treatment durability of KSI-301 in the RVO cohort. Includes randomized patients that reached the first re-treatment opportunity (Week 12 visit) by the data cutoff date. Each bar represents an individual patient. Re-treatment is indicated by (♦). Patients followed beyond the indicated last assessment time point are indicated by a right arrow. Discontinuation is indicated by a left arrow.

Improvement in BCVA and OCT CST were observed in RVO patients at Week 20, 12 weeks after the last loading dose (Figure 31). Therapeutic effect of KSI-301 was sustained during the loading phase, and continued for at least 12 weeks after the final loading dose administered at Week 8 (Figure 31). Improvement in BCVA and OCT CST was observed in a larger cohort of RVO patients that reached Week 12 (Figure 32).

**Figure 31:** BCVA and CST assessment for RVO cohort. N = 15. Includes randomized patients that reached Week 20 visit by the data cutoff date; 2.5 and 5 mg doses were pooled. BCVA= best corrected visual acuity; OCT= optical coherence tomography; CST= central subfield thickness. Error bars represent standard error of the mean.

**Figure 32:** BCVA and CST assessment for RVO cohort. N = 32. Includes randomized patients that reached Week 12 visit by the data cutoff date; 2.5 and 5 mg doses were pooled. BCVA= best corrected visual acuity; OCT= optical coherence tomography; CST= central subfield thickness. Error bars represent standard error of the mean.

EXAMPLE 24A

During the study described in Examples 22-23, multiple-dose exposure of KSI-301 was well-tolerated and no intraocular inflammation was observed. 116 subjects were dosed, with 338 total doses given (107 doses at day 1, 103 doses at week 4, 96 doses at week 8). The following were observed:

- No intraocular inflammation or ocular SAEs in the study eye were reported to date;
- No drug-related adverse events (AEs) or drug-related serious adverse events (SAEs) were reported to date;
- Most AEs were assessed as mild and were consistent with profile of intravitreal anti-VEGFs;
- 12 non-ocular SAEs that were not drug-related were reported in 7 subjects:
    - One 92 y/o RVO subject with hospitalization related to a pre-existing condition that resulted in death;
    - One 66 y/o RVO subject with hospitalization related to dizziness;
    - One 43 y/o RVO subject with a broken leg related to a motorcycle accident;
    - One 85 y/o RVO subject with hospitalization related to a pre-existing condition.

EXAMPLE 24B

The study described in Examples 3-5, 15-23 was further extended to up to 72 weeks, as shown in Figure 33.

**Figure 33:** Updated study design for a randomized, open label study to evaluate multidose safety, efficacy and durability of intravitreal administration of KSI-301.

The updated average characteristics of the study population is show in Table 2.

Table 2: Updated study population characteristics

| Variable | wAMD cohort | DME cohort | RVO cohort |
|---|---|---|---|
| | (n = 51) | (n = 35) | (n = 35) |
| Age, mean (SD), years | 77.9 (10.5) | 59.7 (11.7) | 63.6 (12.6) |
| Gender, n (%), female | 32 (62.7) | 14 (40.0) | 13 (37.1) |
| Race, n (%), White | 48 (94.1) | 28 (80.0) | 31 (88.6) |
| BCVA, mean (SD), ETDRS letters | 63.3 (13.3) | 66.8 (10.2) | 54.9 (15.4) |
| Snellen equivalent | ~20/50 | ~20/50 | 20/80 |
| BCVA, Snellen 20/40 or better, n (%) | 20 (39.2) | 16 (45.7) | 6 (17.1) |
| OCT CST, mean (SD), microns | 430 (162) | 453 (110) | 675 (237) |
| SD= standard deviation; BCVA= best corrected visual acuity; OCT= optical coherence tomography; CST= central subfield thickness | | | |

Further follow-up assessments of patients enrolled in the study described in Example 21 were performed. The results show durability of KSI-301 treatment in patients from the wAMD cohort that extended to 3 to 6 or more months (Figure 34). In 55% (16/29) of patients, the first retreatment was at 6 months after the last loading dose, which was a mandatory retreatment in the study design for the wAMD cohort. 72% (21/29) of patients did not receive retreatment for 5 months or longer after the last loading dose. 84% (27/32) of patients did not receive retreatment for 4 months or longer after the last loading dose. 86% (30/35) of patients did not receive retreatment for 3 months or longer after the last loading dose. 14% (5/35) were retreated at or before 3 months after the last loading dose. These results indicate that the target dosing interval in wAMD can be 3 to 6 or more months after the last loading dose.

**Figure 34:** Treatment durability of KSI-301 in the wAMD cohort. Includes randomized patients that reached the first re-treatment opportunity (Week 12 visit) by the data cutoff date. Each bar represents an individual patient. Re-treatment is indicated by (♦). Patients followed beyond the indicated last assessment time point are indicated by a right arrow. One discontinued patient is indicated by the left arrow.

Improved BCVA and OCT CST was maintained in patients at week 24, 16 weeks after the last loading dose (Figure 35). The baseline (pre-treatment) BCVA for this cohort was 64.1 ETDRS letters. The average improvement in BCVA at Week 24 was +5.9 letters (corresponding to 20/40 Snellen VA). The improved BCVA and OCT CST values were comparable to reported improvements in a standard of care anti-VEGF therapy (baseline of 60.8 letters, gain of ~6 letters; or baseline of 61.5, gain of ~5.2 letters at Week 20). The average improvement in OCT CST was -58 microns at Week 24. The therapeutic effect of KSI-301 was sustained during the loading phase, and continued for at least 16 weeks after the final loading dose administered at Week 8 (Figure 35).

Over the 16 weeks after the final loading dose, a patient received on average 0.16 injections of KSI-301 for retreatment. (Figure 35). This is in comparison to the standard of care treatments, aflibercept or brolucizumab, which require a mean number of injections of 1.0 over the same time period, per the respective labels.

**Figure 35:** Improvements in best corrected vision (BCVA) and retinal thickness (OCT) in patients with wet AMD. BCVA and CST assessment for wAMD cohort. N = 31. Includes randomized patients that reached Week 24 visit by the data cutoff date; 2.5 and 5 mg doses were pooled. BCVA= best corrected visual acuity; OCT= optical coherence tomography; CST= central subfield thickness. Error bars represent standard error of the mean.

## EXAMPLE 25

Further follow-up assessments of patients enrolled in the study described in Example 22 were performed. The DME cohort population characteristics were as described in Table 2. The results show durability of KSI-301 treatment in patients from the DME cohort that extended more than 6 months (Figure 36). 69% (9/13) of patients reached 6 months or longer without retreatment. 73% (11/15) did not require retreatment more than 5 months after the last loading dose. 80% (16/20) did not require retreatment more than 4 months after the last loading dose. 83% (20/24) did not require retreatment more than 3 months after the last loading dose. 17% (4/24) of patients were retreated during the first 3 months. These results indicate that the target dosing interval in DME can be 3 to 6 or more months after the last loading dose.

**Figure 36:** Treatment durability of KSI-301 in the DME cohort. Includes randomized patients that reached the first re-treatment opportunity (Week 12 visit) by the data cutoff date. Each bar represents an individual patient. Re-treatment is indicated by (♦). All depicted patients were followed beyond the indicated last assessment time point (indicated by a right arrow).

Improvement in BCVA and OCT CST were observed in DME patients at Week 24, 16 weeks after the last loading dose (Figure 37). The average improvement in BCVA at Week 24 was +6.8 letters (corresponding to ~20/32$^{+2}$ Snellen VA). The average improvement in OCT CST was -133 microns at Week 24. Therapeutic effect of KSI-301 was sustained during the loading phase, and continued for at least 16 weeks after the final loading dose administered at Week 8.

Over the 16 weeks after the loading dose a patient on average received 0.21 injections of KSI-301. (Figure 37). This is in comparison to the standard of care treatment, aflibercept or brolucizumab, which requires a mean number of injections of 2.0 over the same time period, per the label (aflibercept), or based on a pivotal study design (brolucizumab).

**Figure 37:** BCVA and CST assessment for DME cohort. N = 19. Includes randomized patients that reached Week 24 visit by the data cutoff date; 2.5 and 5 mg doses were pooled. BCVA= best corrected visual acuity; OCT= optical coherence tomography; CST= central subfield thickness. Error bars represent standard error of the mean.

EXAMPLE 26

Follow-up assessments of patients enrolled in the study described in Example 23 were performed. The RVO cohort population characteristics were as described in Table 2. The results show durability of KSI-301 treatment in patients from the RVO cohort that extended more than 4 months (Figure 38). 53% (16/30) of patients reached 4 months or longer without retreatment. 55% (17/31) of patients reached 3 months or longer without retreatment. 34% (11/32) of patients received retreatment during the first 2 months after the last loading dose, while 45% (14/31) received retreatment during the first 3 months after the last loading dose. These results indicate that the target dosing interval in RVO can be 2 to 4 months or longer.

**Figure 38:** Treatment durability of KSI-301 in the RVO cohort. Includes randomized patients that reached the first re-treatment opportunity (Week 12 visit) by the data cutoff date. Each bar represents an individual patient. Re-treatment is indicated by (♦). Patients followed beyond the indicated last assessment time point are indicated by a right arrow. Discontinuation is indicated by a left arrow.

Improvement in BCVA and OCT CST continued to be observed in RVO patients at Week 24, 16 weeks after the last loading dose (Figure 39). The average improvement in BCVA at Week 24 was +22.2 letters (corresponding to 20/32 Snellen VA). The average improvement in OCT CST was -350 microns at Week 24. The therapeutic effect of KSI-301 was sustained during the loading phase, and continued for at least 16 weeks after the final loading dose administered at Week 8 (Figure 39).

Over the 16 weeks after the final loading dose a patient received on average 0.46 injections of KSI-301 for retreatment. (Figure 39). This is in comparison to the standard of care treatments, aflibercept or brolucizumab, which require a mean number of injections of 3.0 over the same time period, per the label (aflibercept), or based on a pivotal study design (brolucizumab).

**Figure 39:** BCVA and CST assessment for RVO cohort. N = 30. Includes randomized patients that reached Week 24 visit by the data cutoff date; 2.5 and 5 mg doses were pooled. BCVA= best corrected visual acuity; OCT= optical coherence tomography; CST= central subfield thickness. Error bars represent standard error of the mean.

EXAMPLE 27

During the study described in Examples 3-5, and 15-26, multiple-dose exposure of KSI-301 was well-tolerated and no intraocular inflammation was observed. 130 subjects were dosed, with 420 total doses given (121 doses at day 1, 112 doses at week 4, 105 doses at week 8). The following were noted:

- No intraocular inflammation or ocular SAEs in the study eye were reported to date;
- No drug-related adverse events (AEs) or drug-related serious adverse events (SAEs) were reported to date;
- Most AEs were assessed as mild and were consistent with profile of intravitreal anti-VEGFs;
- 16 non-ocular SAEs that were not drug-related were reported in 10 subjects:

  One 92 y/o RVO subject with hospitalization related to a pre-existing condition that resulted in death;
  Six (43, 56, 62, 66, 70 and 72 y/o, respectively) DME subjects with hospitalization related to a pre-existing condition;
  One 66 y/o RVO subject with hospitalization related to dizziness;
  One 43 y/o RVO subject with a broken leg related to a motorcycle accident;
  One 85 y/o RVO subject with hospitalization related to a pre-existing condition.

EXAMPLE 28: Phase 2 Randomized Study of KSI-301 and a Standard of Care Treatment in wAMD

About 550 treatment naïve wAMD patients participate in a randomized study comparing treatment with KSI-301

with treatment with a standard of care therapeutic (aflibercept). The standard of care treatment includes 3 loading doses of the therapeutic (at 2 mg per administration) administered at 4-week intervals, followed by alternating administration of a maintenance dose or sham injection every 4 weeks, starting at 16 weeks after initial treatment (Figure 40). Thus, the standard of care therapeutic is administered to the patient every 8 weeks.

KSI-301 is administered (at 5 mg per administration) to another patient cohort with 3 loading doses at 4-week intervals. After the final loading dose (Week 8), patients receive at least a sham injection every 4 weeks, starting at 16 weeks after initial treatment (Figure 40). One cohort of KSI-301-treated patients are assessed for disease activity every 12 weeks (Q12W) after the last loading dose (Week 8). Another cohort of KSI-301-treated patients are assessed for disease activity every 16 weeks (Q16W) after the last loading dose (Week 8). A patient is retreated with KSI-301 if the assessed disease activity meets pre-specified retreatment criteria. A third cohort of KSI-301-treated patients are administered KSI-301 every 20 weeks (Q20W) after the last loading dose (Week 8). All patients are administered KSI-301 at 20 weeks after the last loading dose (Week 8).

**Figure. 40:** Study design for randomized study to evaluate KSI-301 against Aflibercept in treating treatment naïve wAMD patients. KSI-301 is dosed as infrequently as every 20 weeks.

*Re-treatment Criteria (non-loading dose)*

Re-treatment with intravitreal injection of KSI-301 is performed if at least one of the following re-treatment criteria is met. These criteria are related to signs of disease recurrence and/or vision loss due to disease recurrence.

- Increase in OCT central subfield retinal thickness (CST) ≥50 μm with a decrease in BCVA of ≥ 5 letters compared to Week 12;
- Decrease in BCVA of ≥ 10 letters compared to the best prior BCVA, due to worsening wAMD disease activity (e.g. increased intraretinal fluid, increased subretinal fluid, new intraretinal hemorrhage, new subretinal hemorrhage);
- Increase in OCT central subfield retinal thickness (CST) ≥ 75 μm;
- New macular hemorrhage.

The disease activity observed during assessments of wAMD patients treated with KSI-301 in the Phase 1b study, as described in Examples 3, 15, 21 and 24, was used to construct a hypothetical schedule of retreatment for each patient, under the stricter retreatment criteria specified in the Phase 2 clinical trial (Figure 41A). Out of 32 total patients, 24 patients (or 75%) would have reached the 5-month cap for retreatment without any earlier need for retreatment (Figures 41A and 41B). Only 12.5 % of the patients would have required a first retreatment at 12 weeks, and another 12.5% would have required a first retreatment at 16 weeks (Figures 41A and 41B).

**Figures 41A and 41B:** Treatment durability of KSI-301 in the wAMD cohort under hypothetical retreatment criteria in a Phase 2 clinical trial (Figure 41), and hypothetical probability of a patient remaining on Q20W dosing (Figure 41B). Includes randomized patients that would have met retreatment criteria before or at Week 28 by the data cutoff date. Each bar represents an individual patient.

EXAMPLE 29

The study described in Examples 3-5, 15-24 was further extended to Weeks 76 to 148 (Months 19 to 36) (Figure 43). Figure 43 shows an updated study design for a randomized, open label study to evaluate multidose safety, efficacy and durability of intravitreal administration of KSI-301. The number of patients for the wAMD, DME and RVO arms of the study were 51, 35 and 35, respectively.

EXAMPLE 30

This non-limiting example shows further follow-up assessments of the clinical study of KSI-301 for wAMD described in Examples 21 and 24. The results showed improved average BCVA and OCT CST was maintained in patients at week 44, 36 weeks after the last loading dose (Figure 44). The therapeutic effect of the anti-VEGF antibody conjugate was sustained during the loading phase, and continued for at least 36 weeks after the final loading dose administered at Week 8. 58% of patients received their first retreatment dose at Week 32 (Q6M dosing), which was a mandated retreatment dose for any patient who had not been retreated since the last loading dose. Further, a patient received on average 1.32 injections of the anti-VEGF antibody conjugate between Week 12 and 40, of which 44% were mandated Q6M doses. In contrast, the mean number of injections according to the label for a conventional treatment (aflibercept) over the same time period would be 4.

Figure 45A shows the individual break down of the follow-up assessment. Durability of the anti-VEGF antibody conjugate treatment in patients from the wAMD cohort extended to 3 to 6 or more months. Figure 45B summarizes the

results shown in Figure 45A. In 72% of patients, the patient achieved at least one treatment interval of 6 months (Q6M - the mandated cap for the interval between treatment doses) sometime after the last loading dose (Figure 45B). 49% (20/41) of patients did not receive retreatment for 6 months after the last loading dose. 66% (27/41) of patients did not receive retreatment for 5 months or longer after the last loading dose. 82% (40/49) of patients did not receive retreatment for 4 months or longer after the last loading dose. 92% (45/49) of patients did not receive retreatment for 3 months or longer after the last loading dose. Only 8% (4/49) were retreated at or before 2 months after the last loading dose. These results indicate that the target dosing interval of the anti-VEGF antibody conjugate in wAMD can be 3 to 6 or more months after the last loading dose.

Thus, nearly half of wAMD patients were on time to first retreatment of 6 months, and >60% wAMD patients achieved a 6 month interval at least once during follow up. These results were unexpected on the basis of the half-life of KSI-301 alone.

Figure 46 shows a case example of a wAMD patient in the Phase 1b study described above, treated with KSI-301 with 6-month dosing through 1 year. OCT images were taken at the indicated time points. BCVA was also assessed (indicated by the change in ETDRS Letters value under the Week label). OCT and BCVA improvement was observed as early as 1 month (Week 12) after the last loading dose, and was sustained for at least 6 months (Week 32) after the last loading dose. At 6 months (Week 32), a mandatory retreatment dose was administered. 6 months (Week 56) after retreatment, the patient maintained the OCT and BCVA improvement. In total, the patient received 4 intravitreal injections in the first year of treatment.

In some embodiments, a wAMD patient is administered intravitreally an effective amount of an anti-VEGF antibody conjugate, e.g., KSI-301, at a dosing interval of about 3 months, about 4 months, about 5 months, or about 6 months, or any time interval between any two of the above values, at any time period after the patient is administered the last loading dose. In some embodiments, a wAMD patient is not administered a maintenance dose of the anti-VEGF antibody conjugate until at least about 3 months, at least about 4 months, at least about 5 months, or at least about 6 months or more, or until any time point between any two of the above values, after the last loading dose. In some embodiments, a wAMD patient has about 40%, about 50%, about 55%, about 60%, or about 65% or higher chance of not requiring a maintenance dose of the anti-VEGF antibody conjugate until about 6 months or more after the last loading dose or the last maintenance dose. In some embodiments, a wAMD patient has about 50%, about 55%, about 60%, about 65%, or about 70% or higher chance of not requiring a maintenance dose of the anti-VEGF antibody conjugate until about 5 months or more after the last loading dose or the last maintenance dose. In some embodiments, a wAMD patient has about 50%, about 60%, about 70%, or about 80% or higher chance of not requiring a maintenance dose of the anti-VEGF antibody conjugate until about 4 months or more after the last loading dose or the last maintenance dose. In some embodiments, a wAMD patient has about 60%, about 70%, about 80%, or about 90% or higher chance of not requiring a maintenance dose of the anti-VEGF antibody conjugate until about 3 months or more after the last loading dose or the last maintenance dose. In some embodiments, a subject receives 8 or fewer, 7 or fewer, 6 or fewer, 5 or fewer, 4 or fewer intravitreal injections of the anti-VEGF antibody conjugate in the first year of treatment for wAMD, including the loading doses. In some embodiments, a subject receives 4 or fewer, 3 or fewer, 2 or fewer, or 1 maintenance doses of the anti-VEGF antibody conjugate per year for treatment of wAMD.

## EXAMPLE 31

This non-limiting example shows benchmarking of KSI-301 durability compared to aflibercept (Eylea®) long-interval RCT data for treatment-naive wAMD (Figure 47) and Eylea real-world data for wAMD (Figure 48). Figure 47 shows the distribution of treatment intervals among treatment-naive wAMD patients in the KSI-301 Phase 1b study described above was compared to that in a treat-and-extend randomized clinical trial for Ranibizumab and Aflibercept, a combination of two conventional anti-VEGF treatments. Almost 50% of patients receiving the conventional anti-VEGF treatments had treatment interval of 4 weeks, and the treatment interval extended to a maximum interval of 12 weeks (3 months) for about 15% of the patients. In contrast, about 50% of patients treated with KSI-301 had treatment interval of 24 weeks (6 months) - the maximum interval permitted under the study. Over 80% of the patient receiving KSI-301 treatment had a treatment interval of 16 weeks or more, and exceeded the maximum treatment interval achieved by the conventional anti-VEGF treatments.

Figure 48 shows a comparison of the mean treatment interval for patients treated with KSI-301 in the Phase 1b study described above and the mean treatment interval from real-world data for aflibercept (Eylea®) treat-and-extend. Patients treated with aflibercept had a mean treatment interval of 8 weeks, and a mean maximum treatment interval of 9.6 weeks. In contrast, patients on KSI-301 had a mean first interval (mean interval to first retreatment after last loading dose) of 19.3 weeks, and a mean maximum interval of 20.6 weeks. These results demonstrate the superior duration of the therapeutic effect of the anti-VEGF antibody conjugate compared to conventional anti-VEGF treatments for treating wAMD.

In some embodiments, the mean maximum interval for treating wAMD with the anti-VEGF antibody conjugate,

e.g., KSI-301, is at least about 30%, at least about 40%, at least about 50%, at least about 60%, at least about 70%, at least about 80%, at least about 90%, at least about 100%, at least about 110%, at least about 120%, at least about 130%, at least about 140%, at least about 150%, at least about 170% or at least about 200% longer than the mean maximum interval for a conventional anti-VEGF treatment. In some embodiments, the anti-VEGF antibody conjugate is administered to a wAMD patient at a frequency that is at most about 80%, at most about 70%, at most about 60%, at most about 50%, at most about 40%, at most about 30%, at most about 20%, at most about 10% or less than the frequency of administration of a conventional anti-VEGF treatment to treat wAMD.

EXAMPLE 32

This non-limiting example shows further follow-up assessments of the clinical study of KSI-301 for DME described in Examples 22 and 25. Figure 49 shows that the therapeutic effect of KSI-301 was sustained during the loading phase, and continued for at least 36 weeks after the final loading dose administered at Week 8. A patient received on average 0.61 injections of the anti-VEGF antibody conjugate between Week 12 and 40, and 67% of patients required no retreatment injections. In contrast, the mean number of injections according to the label for a conventional treatment (aflibercept) over the same time period would be 5.

Figure 50A shows the individual break down of the follow-up assessment. Durability of the KSI-301 treatment in patients from the DME cohort extended to 3 to 6 or more months. Figure 50B summarizes the results shown in Figure 50A. 45% (15/33) of the patients to date (more than 6 months since the last loading dose) have not required a retreatment dose after the loading doses. In 79% of patients, the patient achieved at least one treatment interval of 6 months or longer sometime after the last loading dose. In 67% (22/33) of patients time to retreatment was 6 months or longer after the last loading dose. 70% (23/33) of patients did not receive retreatment for 5 months or longer after the last loading dose. 76% (25/33) of patients did not receive retreatment for 4 months or longer after the last loading dose. 97% (32/33) of patients did not receive retreatment for 3 months or longer after the last loading dose. Only 3% (1/33) of patients were retreated at 2 months after the last loading dose. No patients were retreated before 2 months after the last loading dose.

These results indicate that the target dosing interval of the anti-VEGF antibody conjugate, e.g., KSI-301, in DME can be 3 to 6 or more months after the last loading dose.

In summary, 2/3 of DME patients have required no additional treatment more than 6 months after the 3 loading doses. These results would not have been expected based on the high treatment need in DME with marketed anti-VEGF treatments (e.g., the median number of injections in the first year based on DRCR.net treatment algorithm is 9-10).

Figure 51 shows a case example of a DME patient in the Phase 1b study described above, treated with KSI-301 with no treatment after the loading phase. OCT images were taken at the indicated time points. BCVA was also assessed (indicated by the change in ETDRS Letters value under the Week label). OCT and BCVA improvement was observed as early as 1 month (Week 12) after the last loading dose, and was sustained for at least 12 months (Week 56) after the last loading dose. No retreatment doses were required for over 12 months after the last loading dose. In total, the patient received 3 intravitreal injections in the first year of treatment.

In some embodiments, a DME patient is administered intravitreally an effective amount of an anti-VEGF antibody conjugate, e.g., KSI-301, at a dosing interval of about 3 months, about 4 months, about 5 months, about 6 months, about 7 months, about 8 months, about 9 months, or about 10 months, about 11 months, about 12 months, about 13 months, or about 14 months or more, or any time interval between any two of the above values, at any time period after the patient is administered the last loading dose. In some embodiments, a DME patient is not administered a maintenance dose of the anti-VEGF antibody conjugate until at least about 3 months, at least about 4 months, at least about 5 months, at least about 6 months, at least about 7 months, at least about 8 months, at least about 9 months, at least about 10 months, at least about 11 months, at least about 12 months, at least about 13 months, or at least about 14 months or more, or until any time point between any two of the above values, after the last loading dose. In some embodiments, a DME patient has about 30%, about 35%, about 40%, about 45%, or about 50% or higher chance of not requiring a maintenance dose of the anti-VEGF antibody conjugate until about 7 months or more after the last loading dose or the last maintenance dose. In some embodiments, a DME patient has at least about 40%, at least about 50%, at least about 55%, at least about 60%, or at least about 65% chance of not requiring a maintenance dose of the anti-VEGF antibody conjugate until about 6 months or more after the last loading dose or the last maintenance dose. In some embodiments, a DME patient has at least about 50%, at least about 55%, at least about 60%, at least about 65%, or at least about 70% chance of not requiring a maintenance dose of the anti-VEGF antibody conjugate until about 5 months or more after the last loading dose or the last maintenance dose. In some embodiments, a DME patient has at least about 50%, at least about 60%, at least about 70%, or at least about 75% chance of not requiring a maintenance dose of the anti-VEGF antibody conjugate until about 4 months or more after the last loading dose or the last maintenance dose. In some embodiments, a DME patient has at least about 60%, at least about 70%, at least about 80%, at least about 90%, or at least about 95% chance of not requiring a maintenance dose of the anti-VEGF antibody conjugate until about 3 months or more after the last loading dose or the last maintenance dose. In some embodiments, a subject receives 8 or fewer, 7 or fewer, 6 or fewer, 5 or fewer, 4 or fewer, or 3 or fewer, intravitreal injections

of the anti-VEGF antibody conjugate in the first year of treatment for DME, including the loading doses. In some embodiments, a subject receives 4 or fewer, 3 or fewer, 2 or fewer, or 1 or fewer, or no maintenance doses of the anti-VEGF antibody conjugate per year for treatment of DME.

## EXAMPLE 33

This non-limiting example shows benchmarking of KSI-301 durability compared to Eylea in DME. Figure 52 shows the mean number of injections required in one-year interval for patients with DME treated with KSI-301 (right) in the Phase 1b study described above, or with Eylea (aflibercept) (left), a conventional anti-VEGF treatment. Patients on conventional anti-VEGF treatment required on average 6 monthly loading doses, and 3.2 maintenance doses, for a total of 9.2 injections in the first year of treatment. In contrast, patients treated with KSI-301 required only on average 3 monthly loading doses, and 1 maintenance dose, for a total of 4 injections in the first year of treatment. Thus, KSI-301 allowed for treatment of DME at half the number of loading and maintenance doses compared to a conventional anti-VEGF treatment. These results demonstrate the superior duration of the therapeutic effect of the anti-VEGF antibody conjugate compared to conventional anti-VEGF treatments for treating DME.

In some embodiments, the number of loading doses (e.g., monthly loading doses) required for treatment of DME with the anti-VEGF antibody conjugate, e.g., KSI-301, is at most about 75%, at most about 70%, at most about 65%, at most about 60%, at most about 55%, at most about 50%, at most about 45%, or at most about 40% of the number of loading doses required for treatment with a conventional anti-VEGF treatment. In some embodiments, the number of maintenance doses required for treatment of DME with the anti-VEGF antibody conjugate is at most about 75%, at most about 70%, at most about 65%, at most about 60%, at most about 55%, at most about 50%, at most about 45%, or at most about 40% of the number of maintenance doses required for treatment with a conventional anti-VEGF treatment. In some embodiments, the total number of doses per year required for treatment of DME with the anti-VEGF antibody conjugate is at most about 75%, at most about 70%, at most about 65%, at most about 60%, at most about 55%, at most about 50%, at most about 45%, or at most about 40% of the total number of doses required per year for treatment with a conventional anti-VEGF treatment.

## EXAMPLE 34

This non-limiting example shows further follow-up assessments of the clinical study of KSI-301 for RVO described in Examples 23 and 26. Figure 53 shows that the therapeutic effect of the anti-VEGF antibody conjugate was sustained during the loading phase, and continued for at least 36 weeks after the final loading dose administered at Week 8. A patient received on average 1.33 injections of the anti-VEGF antibody conjugate between Week 12 and 40, and only 36% of patients required more than one retreatment dose. In contrast, the mean number of injections according to the label for a conventional treatment (aflibercept) over the same time period would be 8.

Figure 54A shows the individual break down of the follow-up assessment. Durability of KSI-301 treatment in patients from the RVO cohort extended to 2 to 4 or more months after 3 monthly loading doses. Figure 54B summarizes the results shown in Figure 54A. 81% of the patients achieved at least one treatment interval of 4 months or longer sometime after the last loading dose. In 56% (18/32) of patients time to retreatment was 4 months or longer after the last loading dose. 66% (21/32) of patients did not receive retreatment for 3 months or longer after the last loading dose. 94% (31/33) of patients did not receive retreatment for 2 months or longer after the last loading dose. Only 6% (2/34) of patients were retreated at 1 month after the last loading dose.

These results indicate that the target dosing interval of the anti-VEGF antibody conjugate, e.g., KSI-301, in RVO can be 2 to 4 months or more after the last loading dose.

Figure 56 shows a case example of a CRVO patient in the Phase 1b study described above, treated with KSI-301. OCT images were taken at the indicated time points and thickness measurement is indicated below the Week labels on the left. BCVA was also assessed (indicated by the change in ETDRS Letters value on the right). The OCT measurement before treatment indicated the patient had the most severe CRVO. OCT and BCVA improvement was observed as early as 1 week after the first loading dose, and continued to improve over the following 3 weeks until the next monthly loading dose. OCT continued to improve and BCVA was maintained one month after the second loading dose. These results indicated that it may be possible to control the most severe CRVO cases with only 2 loading doses of the anti-VEGF antibody conjugate.

In some embodiments, a RVO (e.g., BRVO, CRVO) patient is administered intravitreally an effective amount of an anti-VEGF antibody conjugate, e.g., KSI-301, at a dosing interval of about 2 months, about 3 months, about 4 months, about 5 months, about 6 months, about 7 months, about 8 months, about 9 months, about 10 months, about 11 months, about 12 months, about 13 months, about 14 months, or about 15 months or more, or any time interval between any two of the above values, at any time period after the last loading dose. In some embodiments, a RVO patient is not administered a maintenance dose of the anti-VEGF antibody conjugate until at least about 2 months, at least about 3 months, at least

about 4 months, at least about 5 months, at least about 6 months, at least about 7 months, at least about 8 months, at least about 9 months, at least about 10 months, at least about 11 months, at least about 12 months, at least about 13 months, at least about 14 months, or at least about 15 months or more, or any time point between any two of the above values, after the last loading dose. In some embodiments, a RVO patient has about 45%, about 50%, about 55%, about 60%, about 65%, or about 70% or higher chance of not requiring a maintenance dose of the anti-VEGF antibody conjugate until about 4 months or more after the last loading dose or the last maintenance dose. In some embodiments, a RVO patient has about 50% or higher, about 55% or higher, about 60% or higher, about 65% or higher, or about 70% or higher chance of not requiring a maintenance dose of the anti-VEGF antibody conjugate until about 3 months or more after the last loading dose or the last maintenance dose. In some embodiments, a RVO patient has about 60%, about 70%, about 80%, about 85%, about 90%, or about 95% or higher chance of not requiring a maintenance dose of the anti-VEGF antibody conjugate until about 2 months or more after the last loading dose or the last maintenance dose. In some embodiments, a subject receives 8 or fewer, 7 or fewer, 6 or fewer, 5 or fewer, 4 or fewer intravitreal injections of the anti-VEGF antibody conjugate in the first year of treatment for RVO, including the loading doses. In some embodiments, a subject receives 5 or fewer, 4 or fewer, 3 or fewer, 2 or fewer, or 1 maintenance doses of the anti-VEGF antibody conjugate per year for treatment of RVO. In some embodiments, an RVO patient (e.g., a CRVO patient) receives 2 or 3 loading doses of the anti-VEGF antibody conjugate.

EXAMPLE 35

This non-limiting example shows benchmarking of KSI-301 durability compared to Eylea in RVO. Figure 55 shows the mean number of injections required in one-year interval for patients with RVO treated with KSI-301 (right) in the Phase 1b study described above, or with Eylea (aflibercept) (left), a conventional anti-VEGF treatment. Patients on conventional anti-VEGF treatment required on average 6 monthly loading doses, and 2.6 maintenance doses, for a total of 8.6 injections in the first year of treatment. In contrast, patients treated with KSI-301 required only on average 3 monthly loading doses, and 1.7 maintenance doses, for a total of 4.7 injections in the first year of treatment. Thus, KSI-301 allowed for treatment of RVO at half the number of loading doses, and about 2/3 the number of maintenance doses compared to a conventional anti-VEGF treatment. These results demonstrate the superior duration of the therapeutic effect of the anti-VEGF antibody conjugate compared to conventional anti-VEGF treatments for treating RVO.

In some embodiments, the number of loading doses (e.g., monthly loading doses) required for treatment of RVO with the anti-VEGF antibody conjugate, e.g., KSI-301, is at most about 75%, at most about 70%, at most about 65%, at most about 60%, at most about 55%, at most about 50%, at most about 45%, or at most about 40% of the number of loading doses required for treatment with a conventional anti-VEGF treatment. In some embodiments, the number of maintenance doses required for treatment of RVO with the anti-VEGF antibody conjugate is at most about 80%, at most about 75%, at most about 70%, at most about 65%, or at most about 60% of the number of maintenance doses required for treatment with a conventional anti-VEGF treatment. In some embodiments, the total number of doses per year required for treatment of RVO with the anti-VEGF antibody conjugate is at most about 80%, at most about 75%, at most about 70%, at most about 65%, at most about 60%, at most about 55%, or at most about 50% of the total number of doses required per year for treatment with a conventional anti-VEGF treatment.

EXAMPLE 36

This non-limiting example provides a Phase 2b/3 wAMD study with KSI-301 dosed as infrequently as once every 20 weeks (after the loading doses). Figure 57 shows a schematic view of the study design. Patients in the KSI-301 treatment arm of the study are on Q12W, Q16W or Q20W dosing schedule, unless a disease activity assessment criteria (below) are met at any follow up assessment, at which time the patient is administered a maintenance dose.

*Disease Activity Assessment Criteria (non-loading dose)*

These criteria are related to signs of disease recurrence and/or vision loss due to disease recurrence.

- Increase in OCT central subfield retinal thickness (CST) ≥50 μm with a decrease in BCVA of ≥ 5 letters compared to Week 12;
- Decrease in BCVA of ≥ 10 letters compared to the best prior BCVA, due to worsening wAMD activity (e.g. increased intraretinal fluid, increased subretinal fluid, new intraretinal hemorrhage, new subretinal hemorrhage);
- Increase in OCT central subfield retinal thickness (CST) ≥ 75 μm compared to Week 12;
- New macular hemorrhage.

EXAMPLE 37

This non-limiting example provides a Phase 3 DME studies with KSI-301 that is dosed as infrequently as once every 24 weeks (after the loading doses). Figure 58 shows a schematic view of the study design. Patients in the KSI-301 treatment arm of the study are on Q8W, Q12W, Q16W, Q20W, Q24W dosing schedule, unless a disease activity assessment criteria (below) are met at any follow up assessment, at which time the patient is administered a maintenance dose.

*Disease Activity Assessment Criteria (non-loading dose)*

These criteria are related to signs of disease recurrence and/or vision loss due to disease recurrence.

- Increase in OCT central subfield retinal thickness (CST) ≥50 μm compared to lowest previous measurement with a decrease in BCVA of ≥ 5 letters compared to the average of the 2 best previous BCVA assessments due to worsening of DME disease activity;
- Increase in OCT CST ≥ 75 μm compared to lowest previous measurement due to worsening of DME disease activity;
- New or worsening proliferative DR (PDR).

EXAMPLE 38

This non-limiting example provides Phase 3 RVO studies with KSI-301 that is dosed once 8 weeks (after the loading doses). Figure 58 shows a schematic view of the study design. Patients in the KSI-301 treatment arm of the study receive two monthly loading doses followed by two every 8-week dosing, then every 8 week dosing with disease activity assessment to individualize dosing. If disease activity assessment criteria (below) are met at any follow up assessment, the patient is administered a maintenance dose.

*Disease Activity Assessment Criteria (non-loading dose)*

These criteria are related to signs of disease recurrence and/or vision loss due to disease recurrence.

- Increase in OCT central subfield retinal thickness (CST) ≥75 μm compared to lowest previous measurement due to worsening of RVO disease activity;
- Increase in OCT CST ≥ 75 μm compared to lowest previous measurement due to worsening of RVO disease activity.

EXAMPLE 39

This non-limiting example shows a clinical study for Non-proliferative diabetic retinopathy (NPDR) with KSI-301 dosed as infrequently as once every 6 months (after the loading doses). Patients in the KSI-301 treatment arm of the study receive two loading doses 8 weeks apart. Then patients receive maintenance doses every 4 or 6 months. The endpoint is not only perfusion but using the ETDRS DRSS (Diabetic retinopathy severity score) % patients with >= 2 or 3 step improvement and 2 or 3 step worsening.

EXAMPLE 40

During the study described in Examples 3-5, 15-26, and 29-35, multiple-dose exposure of KSI-301 was well-tolerated and no intraocular inflammation was observed. 130 subjects were dosed, with 546 total doses given. 121 subjects completed the loading phase in Phase 1b, and 81 received at least one additional retreatment at Week 12 or later. The following were noted:

- Most adverse events (AEs) were assessed as mild and are consistent with profile of conventional intravitreal anti-VEGF treatment;
- To date, 29 serious adverse events (SAEs) have been reported in 16 subjects - none drug related;
- One ocular SAE in the study eye (worsening DME secondary to systemic fluid overload, not drug related);
- Only two AEs of intraocular inflammation, both trace to 1+ vitreous cells, with complete resolution
    - Rate of 0.37% on per-injection basis (2/546 injections), 1.5% on per-patient basis (2/130 patients)
    - No vasculitis or retinitis in either patient.

Example 41: Safety and Efficacy of KSI-301 for wAMD compared to Standard of Care

The present study will assess both ocular and systemic safety and efficacy of administration of 5 mg KSI-301 for wAMD as compared to current standard of care aflibercept at 2 mg. The study is divided into a 21-day screening period, a 36 week treatment period, a 4 week follow period for safety and efficacy assessments at week 40, and a final follow-up for safety assessments at week 44. At the study onset, approximately 500 participants are split into two arms at a 1:1 ratio, to receive either KSI-301 5 mg or aflibercept 2 mg, with participants in the KSI-301 cohort receiving injections every 4 weeks via intravitreal injection. Participants in the aflibercept arm are treated with aflibercept 2 mg once every 4 weeks for 3 months, followed by a sham injection at week 12, and starting at week 16, aflibercept 2 mg once every 8 weeks until week 36 - sham injections are administered every month where the dosing schedule indicates no aflibercept is to be administered.

Eligible subjects are to be selected on predetermined inclusion and exclusion criteria. Subjects should be treatment naive with respect to choroidal neovascularization (CNV) secondary to wAMD, including subfoveal, juxtafoveal, and extrafoveal lesions or retinal angiomatous proliferations (RAP) lesions with a CNV component that affect the central subfield as evidenced by FA or OCT in the study eye at screening. Moreover, participants must have BCVA ETDRS scores between 83 and 25 letters (approximately 20/20 to 20/320 Snellen equivalent), inclusive.

Efficacy Evaluation

Efficacy analysis will be performed on the intent to treat (ITT) population. Primary endpoints are mean change in ETDRS BCVA from Day 1 to Week 40. Primary assessment of efficacy is pairwise comparison in mean change in BCVA between treatment arm A (KSI-301 5 mg) and B (aflibercept 2 mg). Secondary outcomes include ophthalmic assessments (including tonometry), fundus photography, spectral domain (SD)-OCT, and fluorescein angiography (FA).

Pharmacokinetic/Biomarker Evaluation:

Plasma concentrations of study intervention (KSI-301) will also be measured. Blood samples will be analyzed for anti-drug antibodies (ADAs), as well as plasma VEGF levels and other blood factors.

Example 41.5: Dosage Schedule to Treat wAMD

One first identifies a subject in need of having an eye disorder treated wherein the eye disorder is neovascular (wet) age related macular degeneration (wAMD). Optionally, the subject may have had prior treatment for wAMD that was ineffective and in need of an intensive treatment regimen. Then, one administers 5 mg of antibody or of an anti-VEGF antibody conjugate (e.g. KSI-301) to the subject at a schedule of Q4W to treat the disease.

Example 42: Longitudinal Safety and Efficacy of KSI-301 for NPDR

A study will be carried out to assess both ocular and systemic safety and efficacy of administration of 5 mg KSI-301 for NPDR at a Q24W dosing schedule. Study is planned to be divided into a 21 day screening period, with participants given KSI-301 5 mg or sham on Day 1, week 8, week 20, and then Q24W through week 92. Participants who develop DME, PDR, or ASNV will be treated with KSI-301 5 mg with 2 loading doses at Q4W, then at Q12W intervals thereafter. Additional doses of KSI-301 and/or subsequent PRP are to be ordered at the discretion of the clinical Investigator. At the trial outset, approximately 240 participants split 1:1 into two clinical arms, to receive either KSI-301 5 mg, or sham. Eligible subjects are to be selected on predetermined inclusion and exclusion criteria. Participants additionally will have diagnosed type 1 or type 2 diabetes mellitus with HbAlc of ≤12.0%.

Efficacy Evaluation

Participants are to be assessed at week 48 for the proportion of eyes improving greater than 2 steps on DRSS from baseline. Secondary endpoints for efficacy were assessed at week 96 and at the investigator's discretion, and include proportion of eyes developing proliferative DR, diabetic macular edema, or anterior segment neovascularization (ASNV) from baseline, as well as proportion of eyes with greater than two steps worsening DRSS from baseline, BCVA using the ETDRS method, and spectral domain optical coherent tomography (SD-OCT).

Pharmacokinetic/Biomarker Evaluation:

Plasma concentrations of study intervention (KSI-301) will also be measured. Blood samples will be analyzed for

anti-drug antibodies (ADAs), as well as plasma VEGF levels and other blood factors.

Example 42.5: Dosage Schedule to treat NPDR

a. One first identifies a subject in need of having an eye disorder treated, wherein the eye disorder is non-proliferative diabetic retinopathy. Then one administers 5 mg of antibody or of an anti-VEGF antibody conjugate (e.g. KSI-301) to the subject up to 3 loading doses, within about 6 weeks of each other. Or alternatively, subject will be treated 3 loading doses on day 1, week 8 and week 20. Following the last loading dose, subject will retain the therapeutic benefit of the anti-VEGF antibody conjugate therapy for at least 24 weeks.

b. The present non-limiting example provides a method of treating DME, PDR, or ASNV. The method comprises administering an anti-VEGF antibody conjugate (e.g., KSI-301) to a subject developing DME, PDR, or ASNV at a first loading dose. One can repeat the loading dose once after 4 weeks. Thereafter, the subject retains the therapeutic result of the anti-VEGF antibody conjugate therapy for at least 12 weeks after the final loading dose, where subsequent administration of the anti-VEGF antibody conjugate occurs at a schedule of Q12W.

Example 43: Phase 3 Randomized Study of KSI-301 and a Standard of Care Treatment in DME

About 450 treatment naive DME patients will participate in each of the two randomized studies comparing treatment with KSI-301 with treatment with a standard of care therapeutic (aflibercept). The standard of care treatment involves doses of the therapeutic (2 mg per administration) at Q4W for 5 months, followed by a sham injection at week 20, followed by a Q8W dosing schedule with a sham injection every month there is no scheduled standard of care therapeutic scheduled. KSI-301 treatment patients receive the anti-VEGF antibody conjugate at a schedule of Q4W for 3 months, following by a sham injection at week 12, followed by a custom dosing regimen according to investigator discretion, with the minimum interval between treatments being 8 weeks, and the maximum interval being 24 weeks, with sham injections provided every month where the anti-VEGF antibody conjugate is not administered.

Efficacy Evaluation

Primary efficacy endpoints are defined as the mean change in ETDRS BCVA from baseline to year 1 (average of weeks 48 and 52), with pairwise comparison in mean change in BCVA between treatment arm A (KSI-301 5 mg) and treatment arm B (aflibercept 2 mg). Secondary efficacy outcomes will be evaluated using ophthalmic assessments, including tonometry, fundus photography, spectral domain optical coherence tomography (SD-OCT), and fluorescein angiography (FA). Secondary endpoints for efficacy are assessed at weeks 52 and weeks 104.

Re-treatment Criteria

Re-treatment with intravitreal injection of KSI-301 is performed if at least one of the following re-treatment criteria is met. These criteria are related to signs of disease recurrence and/or vision loss due to disease recurrence.

- Increase in OCT CST ≥ 50 μm compared to lowest previous measurement and a decrease in BCVA of ≥5 letters compared to the average of the 2 best previous BCVA assessments, due to worsening of DME disease activity, or
- Increase in OCT CST ≥ 75 μm compared to lowest previous measurement due to worsening of DME disease activity; or
- New or worsening proliferative DR (PDR): progression from NPDR to PDR, vitreous hemorrhage, iris neovascularization and/or new or worsening retinal neovascularization.

Disease Stability Criteria

Extension of base treatment interval is determined if at least one of the following criterion, as well as the absence of any of the re-treatment criteria are met.

- No increase of CST of > 30 μm compared to the lowest pervious measurement.

Pharmacokinetic/Biomarker Evaluation:

Plasma concentrations of study intervention (KSI-301) will also be measured. Blood samples will be analyzed for

anti-drug antibodies (ADAs), as well as plasma VEGF levels and other blood factors.

Example 43.5: Therapeutic Use in treating DME

One first identifies a subject in need of having an eye disorder treated, wherein the eye disorder is DME. Then one administers 5 mg of antibody or of an anti-VEGF antibody conjugate (e.g. KSI-301) to the subject up to 3 loading doses, within about 4 weeks of each other. Following the last loading dose, subject will retain the therapeutic benefit of the anti-VEGF antibody conjugate therapy for at least 8 or at least 12 weeks, and up to 24 weeks before re-treatment. Wherein treatment interval is determined by OCT CST metrics, BCVA increase or decrease of letters compared to previous assessments, or new or worsening proliferative DR (PDR) or progression from NPDR to PDR, vitreous hemorrhage, iris neovascularization, and/or new or worsening retinal neovascularization.

Example 44

One first identifies a subject having diabetic retinopathy (DR). Then one administers an anti-VEGF antibody conjugate (e.g., KSI-301) to the subject in a first dose, a second dose, and a third dose, where the second dose is administered 8 weeks after the first dose, and the third dose is administered 12 weeks after the second dose. Following the third loading dose, administration of subsequent doses occur no more frequently than once every 24 weeks.

Example 45

One first identifies a subject having diabetic retinopathy (DR). Then one administers an anti-VEGF antibody conjugate (e.g., KSI-301) to the subject in a first dose, a second dose, and a third dose, where the second dose is administered 12 weeks after the first dose, and the third dose is administered 24 weeks after the second dose. Following the third dose, subsequent doses of the anti-VEGF therapy are administered once every 12 or 24 weeks.

Example 46

One first identifies a subject having diabetic retinopathy (DR). Then one administers an anti-VEGF antibody conjugate (e.g., KSI-301) to the subject in a first dose and a second dose, the second loading dose is administered 4 weeks following the first loading dose. Following the last dose, administering to the subject one or more subsequent doses of the anti-VEGF antibody conjugate no more frequently than once every 8 or 12 weeks unless the diabetic retinopathy severity is worsening at week 8 from the second loading dose. The treated eye is examined at least 8 weeks after the last loading dose, and administering subsequent doses of the anti-VEGF therapy once every 12 weeks past the last loading dose if the diabetic retinopathy severity is stable or improved, and every 8 weeks if the retinopathy severity is worsening.

Example 47

One first identifies a subject having diabetic retinopathy (DR). Then one administers an anti-VEGF antibody conjugate (e.g., KSI-301) to the subject in a first dose and a second dose, the second dose is administered 4 weeks following the first loading dose. Following the second dose, if the subject has a decline in eye health 8 weeks after the second dose, administering at least one subsequent dose of the anti-VEGF antibody polymer conjugate at a frequency of at least once every 8 weeks, and if the subject maintains eye health or it improves, then administering at least one subsequent dose of the anti-VEGF antibody polymer conjugate at a frequency of no more than once every 12 weeks.

Example 48

One first identifies a subject having diabetic retinopathy (DR). Then one administers an anti-VEGF antibody conjugate (e.g., KSI-301) to the subject in a first dose and a second dose, where the second loading dose is administered 4 weeks after the first dose. Then, one administers one or more subsequent doses of the anti-VEGF therapy once every 8 or 12 weeks.

Example 49

One first identifies a subject having wAMD and currently receiving therapy for wAMD, where the current therapy is not achieving the goal of treatment, where the approved therapy is not KSI-301. Then one administers KSI-301 to the subject in an amount and a dosing schedule sufficient to treat subject's wAMD.

Example 50

One first identifies a subject having wAMD. Then one administers an anti-VEGF antibody conjugate (e.g., KSI-301) to the subject in a first loading dose, a second loading dose, and a third loading dose, where the second loading dose is administered 4 weeks after the first loading dose, and the third loading dose is administered 4 weeks after the second loading dose. Following the third loading dose, evaluating the eye of the subject 12 weeks after the last loading dose, and wherein the eye is dry, extending maintenance treatments of the anti-VEGF therapy once every 16 weeks, and wherein the eye is wet providing maintenance treatment once every 8 weeks.

Example 51

One first identifies a subject having wAMD. Then one administers an anti-VEGF antibody conjugate (e.g., KSI-301) to the subject in a first loading dose, a second loading dose, and a third loading dose, where the second loading dose is administered 4 weeks after the first loading dose, and the third loading dose is administered 4 weeks after the second loading dose. Following the last loading dose, the treated eye is examined and evaluated 8 weeks after the after the third loading dose, , wherein if the eye has a low-luminance visual acuity (LLVA) to BCVA difference of 33 or more letters, the subject will receive an increased dose frequency (e.g. every 4 to 8 weeks) of maintenance doses and if the eye has a LLVA to BCVA difference of less than 33 letters, the subject will receive a decreased frequency of maintenance doses (e.g. every 12 to 24 weeks).

Example 52

One first identifies a subject having wAMD. Then one administers between 1-3 loading doses of an anti-VEGF antibody conjugate (e.g., KSI-301). Following the last loading dose, the treated eye is examined at least 4 weeks after the last loading dose, to determine if the amount of fluid within the eye is stabilized. Thereafter if the subject has stabilized fluid, a maintenance dose is administered at a first frequency, and if the subject has an increase in fluid a maintenance dose is administered at a second frequency, the second frequency being more frequent than the first frequency.

Example 53

The present non-limiting example provides a method of treating DME. The method comprises administering an anti-VEGF antibody polymer conjugate (e.g., KSI-301) to a subject with DME at 1-3 loading doses. The method further comprises administering between 1-7 maintenance doses of the anti-VEGF antibody polymer conjugate to the subject, where the total doses of anti-VEGF antibody polymer conjugate administered within a year is 7 or fewer.

Example 54

The present non-limiting example provides a method of treating DME. The method comprises administering an anti-VEGF antibody polymer conjugate (e.g., KSI-301) to a subject with DME at 2-3 loading doses. The method further comprises administering a maintenance dose of the anti-VEGF antibody polymer conjugate to the subject every 2-15 months, with dosing schedule determined by ophthalmic evaluation of the eye's wetness or dryness using OCT or OCT-A measurement.

Example 55

The present non-limiting example provides a method of treating DME. The method comprises administering an anti-VEGF antibody polymer conjugate (e.g., KSI-301) to a subject with DME at 2-3 loading doses. The method further comprises administering a maintenance dose of the anti-VEGF antibody polymer conjugate to the subject every 2-15 months, with dosing schedule depending on whether the retinal thickness in the subject is above 320 (or 350) microns, determined by ophthalmic evaluation of the eye's retinal thickness using OCT or OCT-A measurement.

Example 56

The present non-limiting example provides a method of preventing vision complications. The method comprises administering an anti-VEGF antibody polymer conjugate (e.g., KSI-301) to a subject with DME at 2-3 loading doses. The method further comprises administering a maintenance dose of the anti-VEGF antibody polymer conjugate to the subject every 2-15 months, with dosing schedule depending on whether the retinal thickness in the subject is above 320 microns (or 350 microns) determined by ophthalmic evaluation of the eye's retinal thickness using OCT or OCT-A measurement

and/or by ophthalmic evaluation of the eye's wetness or dryness using OCT or OCT-A measurement.

Example 57

The present non-limiting example provides a method of treating wAMD, DME, or RVO, the method administering an anti-VEGF antibody polymer conjugate (e.g., KSI-301) to a subject with DME, wAMD, or RVO at 2-3 loading doses. The method further comprises a vision assessment or ophthalmic exam to assess vision of the subject after 4, 8, 12, 16, 20, 24, 36, 48, or 52 weeks after the last loading dose, and based on the vision assessment of the patient, placing the patient on a fixed regimen of the anti-VEGF antibody polymer conjugate once every 4, 8, 12, 16, 20, 24, 36, 48, or 52 weeks.

Example 58

The present non-limiting example provides a method of threating wAMD, DME, or RVO, the method compring identifying a subject above 16 years of age with DME, wAMD or RVO. The method comprises administering an anti-VEGF antibody polymer conjugate (e.g., KSI-301) to a subject with DME, wAMD, or RVO at 1-3 loading doses, not administering more than 3 loading doses to the subject where the loading doses are administered to the subject on a monthly basis. The method further comprises providing a follow-on application of the anti-VEGF antibody polymer conjugate at a point in time no sooner than 8 weeks after a last loading dose or after a last follow-on application of the anti-VEGF antibody polymer conjugate.

Example 59

The present non-limiting example provides a method of treating DME, DR, or RVO, the method comprises administering an anti-VEGF antibody polymer conjugate (e.g., KSI-301) to a subject with DME, wAMD, or RVO at 1-3 loading doses on a monthly basis, and not administering more than 3 loading doses to the subject. The method further comprises providing a follow-on application of the anti-VEGF antibody polymer conjugate no sooner than 8 or 12 weeks after a last loading dose or after a last follow-on application of the anti-VEGF antibody polymer conjugate. Following this, the visual improvement of Best Corrected Visual Acuity (BCVA) is greater than at least 5, or at least 6.8 or at least 7.2 letters post treatment, or:

Where the subject has DME, the improvement provides a BCVA of at least 5 letters or 6.8 to 7.2

Where the subject has wAMD, the improvement provides a BCVA of at least 5 letters or 6.8 to 7.2

Where the subject has RVO, the improvement provides a BCVA of at least 5 letters or 6.8 to 7.2

Where the subject has DME, the improvement is 1 to 30 letters.

Where the subject has wAMD, the improvement is 1 to 35 letters.

Where the subject has RVO, the improvement is 1 to 40 letters.

Example 60

The present non-limiting example provides a method of treating DME, DR, or RVO, the method comprises administering an anti-VEGF antibody polymer conjugate (e.g., KSI-301) to a subject with DME, wAMD, or RVO at 1-3 loading doses, not administering more than 3 loading doses to the subject, wherein each loading dose is administered every 4 weeks. The method further comprises providing between 0-1 follow-on applications of the anti-VEGF antibody polymer conjugate no sooner than 8 or 12 weeks after a last loading dose or a last follow-on application of the anti-VEGF antibody polymer conjugate.

Example 61

This non-limiting example provides a Phase 1b DME and RVO study with KSI-301 wherein subjects are given 2-3 loading doses, waiting one month between each loading dose. 121 patients with wAMD, DME, or RVO were enrolled in the study. For all three indications, (wAMD, DME, or RVO, approximately two-thirds of patients are currently in a ≥6-month treatment free interval. This is accompanied by clinically significant improvements in functional (best-corrected visual acuity (BCVA)) and anatomic (changes in central subfield thickness (CST) using optical coherence tomography (OCT)) parameters.

Example 61.1

Figure 60 shows a mean change in BCVA and CST from baseline to week 52 in patients with DME (n=32). Rapid-onset, high magnitude improvements in both function (BCVA) and retinal anatomy (CST) were observed as early as one

week after the first KSI-301 injection for patients with DME. The initial treatment effects were maintained or continued to improve through Week 52 (n=32), as follows:

- Improved (increased) BCVA: mean BCVA improvement at Week 52 (KSI 301 2.5 mg and KSI 301 5 mg results pooled) was 7.6 ETDRS letters to an approximate Snellen equivalent of 20/40.
- Improved (reduced) Retinal Thickness: mean reduction in retinal thickness by OCT CST at Week 52 (KSI 301 2.5 mg and KSI 301 5 mg results pooled) was 136 microns.
- Reduced treatment burden: after only three loading doses, a mean of 1.0 individualized doses were given over the period between Weeks 12 and 48, inclusive. A mean total of 4.0 injections total were given in Year 1. Figure 61 shows KSI-301 durability for DME patients (n=32) requiring additional doses after the administration of initial loading doses and within 52 weeks of the administration of the first loading dose. Figure 61 further demonstrates the percentage of patients who did require additional doses having treatment intervals of every month, every 2 months, every 3 months, every 4 months, every 5 months, or every 6 months or longer. As shown in Figure 61 (top), 90% of patients with DME required two or fewer retreatments in Year 1 and 50% did not require any retreatment after the initial loading doses until Year 1. As shown in Figure 61 (bottom), 69% of patients were on a 6-month or longer treatment-free interval at Year 1.

Example 61.2

Figure 62 shows a mean change in BCVA and CST from baseline to week 52 in patients with RVO (n=34; BRVO n=19, CRVO n=15). Rapid-onset, high magnitude improvements in both function (BCVA) and retinal anatomy (CST) were observed as early as one week after the first KSI-301 injection for patients with RVO. The initial treatment effects were maintained or continued to improve through Week 52 (n=32), as follows:

- Improved (increased) BCVA: mean BCVA improvement at Week 52 (KSI 301 2.5 mg and KSI 301 5 mg results pooled) was 22.2 ETDRS letters to an approximate Snellen equivalent of 20/32.
- Improved (reduced) Retinal Thickness: mean reduction in retinal thickness by OCT CST at Week 52 (KSI 301 2.5 mg and KSI 301 5 mg results pooled) was 357 μm.
- Reduced treatment burden: after only three loading doses, a mean of 1.7 individualized doses were given over the period between Weeks 12 and 48, inclusive. A mean total of 4.7 injections total were given in Year 1.

Figure 63 shows KSI-301 durability for RVO patients (n=34) requiring additional doses after the administration of initial loading doses and within 52 weeks of the administration of the first loading dose. Figure 63 further demonstrates the percentage of patients who did require additional doses having treatment intervals of every month, every 2 months, every 3 months, every 4 months, every 5 months, or every 6 months or longer. As shown in Figure 63 (top), 72% of patients with RVO required two or fewer retreatments in Year 1 and most (56%) patients required only one (25%) or zero (31%) individualized doses in Year 1. As shown in Figure 63 (bottom), 66% of patients were on a 6-month or longer treatment-free interval at Year 1.

Example 62

This non-limiting example shows the effects of administering an anti-VEGF antibody conjugate on visual acuity gains in patients with wet age-related macular degeneration (wAMD).

A randomized, multicenter study of KSI-301 in wAMD patients was carried out essentially as characterized in Example 28. Patients in the standard of care arm were administered 2 mg aflibercept every 2 months after 3 monthly loading doses (Fig. 40). KSI-301 was administered intravitreally to wAMD patients in the test arm every 3-to-5 months. Patients in the test arm received 3 monthly loading doses of KSI-301 at 5 mg (by weight of protein), followed by additional treatment doses every 3-to-5 months. Disease activity was assessed 3 months after the last loading dose. Patients having disease activity that met pre-specified retreatment criteria were administered a treatment dose. These patients were subsequently retreated every 3 months.

Patients showing sustained visual acuity gains (i.e., patients that did not meet the pre-specified retreatment criteria) at 3 months after the last loading dose were not administered a treatment dose, and were assessed for disease activity 4 months after the last loading dose. Patients showing sustained visual acuity gains (i.e., patients that did not meet the pre-specified retreatment criteria) at 4 months after the last loading dose were not administered a treatment dose, and were treated 5 months after the last loading dose. These patients were then assessed for disease activity at 3 or 4 months after the last treatment dose, and were treated with KSI-301 if disease activity met pre-specified retreatment criteria at either time point, or at 5 months after the last treatment dose if visual acuity gains were sustained.

Patients having disease activity that met pre-specified retreatment criteria at 4 months after the last loading dose were administered a treatment dose. These patients were subsequently assessed for disease activity 3 months after the

last treatment dose. Patients having disease activity that met pre-specified retreatment criteria were administered a second treatment dose. These patients were subsequently retreated after 3 months. Patients showing sustained visual acuity gains (i.e., patients that did not meet the pre-specified retreatment criteria) at 3 months after the last treatment dose were administered KSI-301 4 months after the last treatment dose.

Of a total of 785 patients screened, 559 were chosen to be randomly assigned to a treatment arm (A or B) (Fig. 65). Patients in arm A were treated with KSI-301 at 5 mg according to the dosing schedule discussed above (Q12W, Q16W, or Q20W), and patients in arm B were treated with 2 mg aflibercept every 2 months (Q8W) after 3 monthly loading doses. There was a slightly larger number of discontinuations in the test arm (Arm A) compared to the standard of care arm (Arm B), mainly driven by events associated with undertreatment. Patient demographic were well balanced between the two study arms (Fig. 66). Ocular baseline characteristics were also well balanced between the two study arms and were typical of patients with treatment-naive wAMD (Fig. 67).

A majority of KSI-301-treated patients achieved durable visual gains (Figs. 68A, 68B). The adjusted mean BCVA change from baseline for all patients within each treatment arm showed patients treated with KSI-301 on average showed similar improvement during the initial, monthly dosing phase compared to patients treated with aflibercept, and showed a smaller change during the subsequent disease activity adjustment phase (Fig. 68A). Similar trends were observed in the adjusted mean CST change from baseline (Fig. 68B). This may be in large part due to undertreatment in some patients.

The visual acuity assessment results averaged for the subgroup of patients who achieved a 20-week interval (Q20W) at Year 1 are comparable to the aflibercept group (Fig. 69). In contrast, for some other patients, treatment with KSI-301 no more often than every 12 weeks after the loading phase was insufficient to maintain the visual acuity gains (Fig. 70).

Similarly, improvement in retinal anatomy was seen in all KSI-301 durability subgroups, and was comparable to aflibercept in patients that achieved Q20W dosing with KSI-301 (Fig. 71). In contrast, for other patients who met the criteria for adjustment to Q12W dosing with KSI-301, the clinical effect deteriorated (Fig. 72). Thus, some patients may benefit from treatment with KSI-301 that is more frequent than Q12W. Durability with KSI-301 was observed in 59.4% of patients at Q20W dosing, and 69.7% of patients at Q16W or less frequent dosing. Patients on Q20W dosing achieved meaningful reductions in CST and improvements in vision comparable to aflibercept Q8W.

KSI-301 was safe and well tolerated, and the rate of intraocular inflammation with KSI-301 was low (3.2%) and within the range reported with aflibercept in other wAMD studies (1-4.5%). No cases of intraocular inflammation with vascular occlusion were observed.

All patent filings, websites, other publications, accession numbers and the like cited above or below are incorporated by reference in their entirety for all purposes to the same extent as if each individual item were specifically and individually indicated to be so incorporated by reference. If different versions of a sequence are associated with an accession number at different times, the version associated with the accession number at the effective filing date of this application is meant. The effective filing date means the earlier of the actual filing date or filing date of a priority application referring to the accession number if applicable. Likewise if different versions of a publication, website or the like are published at different times, the version most recently published at the effective filing date of the application is meant unless otherwise indicated. Any feature, step, element, embodiment, or aspect disclosed herein can be used in combination with any other unless specifically indicated otherwise. Although some embodiments have been described in some detail by way of illustration and example for purposes of clarity and understanding, it will be apparent that certain changes and modifications may be practiced within the scope of the appended claims.

## Claims

1. An anti-VEGF antibody polymer conjugate for use in the treatment of diabetic retinopathy, the method of treatment comprising:

   identifying a subject with diabetic retinopathy;
   administering a first dose of an anti-VEGF antibody polymer conjugate to the subject;
   administering a second dose of the anti-VEGF antibody polymer conjugate to the subject 8 weeks after the first dose;
   administering a third dose of the anti-VEGF antibody polymer conjugate to the subject 12 weeks after the second dose; and
   administering one or more subsequent doses of the anti-VEGF antibody polymer conjugate to the subject either once every 12 weeks or once every 24 weeks after the third dose.

2. An anti-VEGF antibody polymer conjugate for use in the treatment of diabetic retinopathy, the method of treatment comprising:

identifying a subject with diabetic retinopathy;
administering a first dose of an anti-VEGF antibody polymer conjugate to the subject;
administering a second dose of the anti-VEGF antibody polymer conjugate to the subject 8 weeks after the first dose;
administering a third dose of the anti-VEGF antibody polymer conjugate to the subject 12 weeks after the second dose; and
administering a subsequent dose of the anti-VEGF antibody polymer conjugate to the subject no more frequently than once every 24 weeks after the third dose.

**3.** The anti-VEGF antibody polymer conjugate for use of any one of claims 1-2, wherein the anti-VEGF antibody polymer conjugate is part of a formulation, wherein the formulation comprises:

12.5 mM sodium phosphate buffer, and
0.025% (w/w) polysorbate 20,
at a pH of about 6.5, wherein approximately 40-55 mg/mL of antibody mass is present and approximately 259-356 mg/mL of total mass of the antibody-biopolymer conjugate is present in the formulation.

**4.** The anti-VEGF antibody polymer conjugate for use of claim 3, wherein the anti-VEGF antibody polymer conjugate is 45-52.5 mg/ml of antibody and approximately 310-330 mg/mL of total mass of the antibody-biopolymer conjugate, and wherein the antibody polymer conjugate comprises a) VH and VL in FIG. 64 or 6 CDRs within the VH and VL of FIG. 64.

**5.** The anti-VEGF antibody polymer conjugate for use of any one of the preceding claims, wherein at least 4 doses of the anti-VEGF antibody polymer conjugate is administered to the subject in the first year of treatment.

**6.** The anti-VEGF antibody polymer conjugate for use of any one of the preceding claims, the anti-VEGF antibody polymer conjugate comprises an anti-VEGF-A antibody comprising:
a heavy chain comprising a complementarity determining region ($CDR_H$) 1 that is the $CDR_H1$ in SEQ ID NO: 1, a $CDR_H2$ that is the $CDR_H2$ in SEQ ID NO: 1, a $CDR_H3$ that is the $CDR_H3$ in SEQ ID NO: 1; and
a light chain comprising a complementarity determining region ($CDR_L$) 1 that is that is the $CDR_L1$ in SEQ ID NO: 2, a $CDR_L2$ that is the $CDR_L2$ in SEQ ID NO: 2, a $CDR_L3$ that is the $CDR_L3$ in SEQ ID NO: 2.

**7.** The anti-VEGF antibody polymer conjugate for use of any one of the preceding claims, wherein the anti-VEGF-A antibody comprises a heavy chain comprising an amino acid sequence at least 90% identical to SEQ ID NO:1.

**8.** The anti-VEGF antibody polymer conjugate for use of any one of the preceding claims, wherein the anti-VEGF-A antibody comprises a light chain comprising an amino acid sequence at least 90% identical to SEQ ID NO:2.

**9.** The anti-VEGF antibody polymer conjugate for use of any one of the preceding claims, wherein the anti-VEGF antibody polymer conjugate comprises a phosphorylcholine-containing polymer conjugated to a non-native cysteine outside a variable region of the antibody, wherein the phosphorylcholine-containing polymer is composed of 2-(methacryloyloxyethyl)-2'-(trimethylammonium)ethyl phosphate (MPC) units.

**10.** The anti-VEGF antibody polymer conjugate for use of any one of the preceding claims, wherein the polymer has 9 arms.

**11.** The anti-VEGF antibody polymer conjugate for use of any one of the preceding claims, wherein the polymer has a molecular weight from about 300,000 to about 1,750,000 Da.

**12.** The anti-VEGF antibody polymer conjugate for use of any one of the preceding claims, wherein the anti-VEGF antibody polymer conjugate has the following structure:

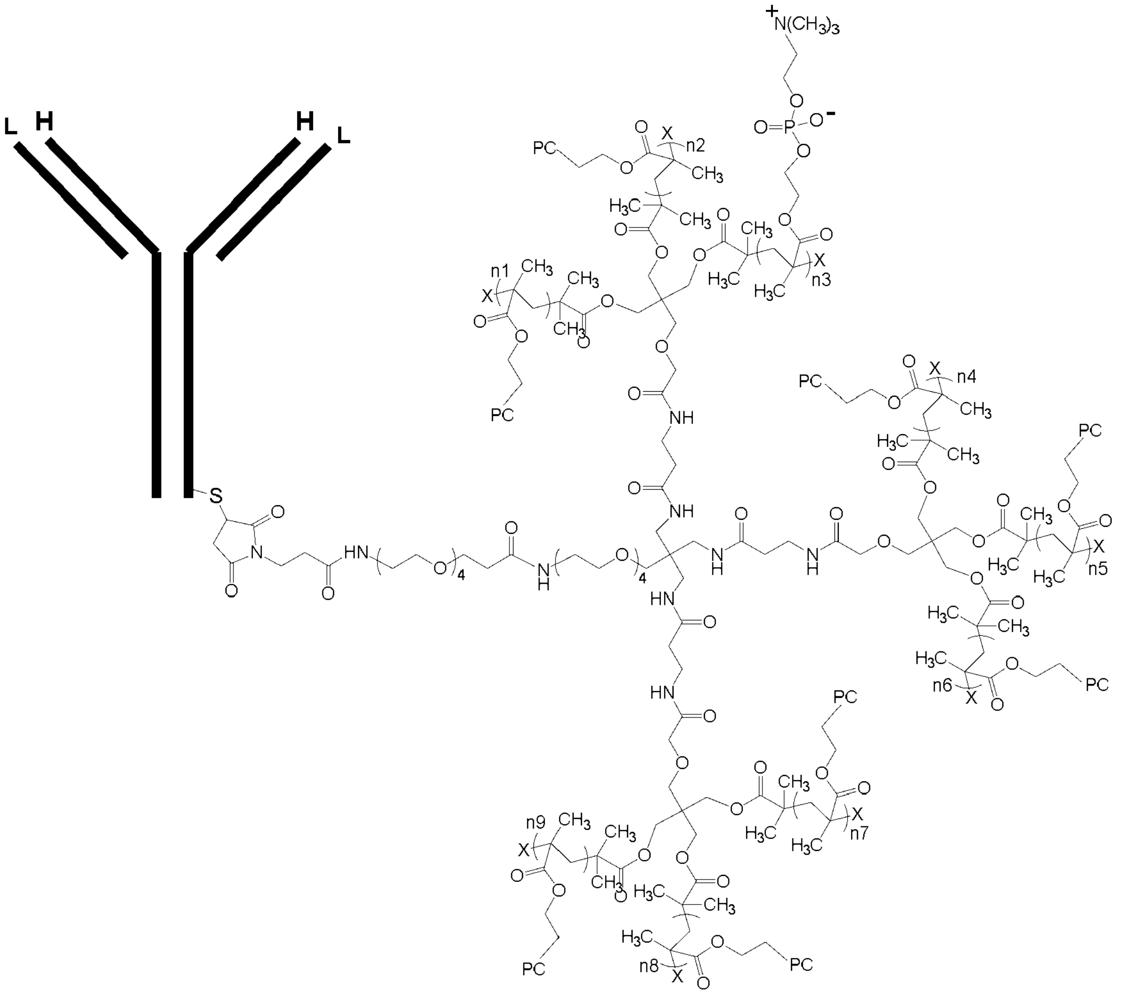

wherein:

each heavy chain of the anti-VEGF-A antibody is denoted by the letter H, and each light chain of the anti-VEGF-A antibody is denoted by the letter L;
the polymer is bonded to the anti-VEGF-A antibody through the sulfhydryl of C443 (EU numbering), which bond is depicted on one of the heavy chains;
PC is,

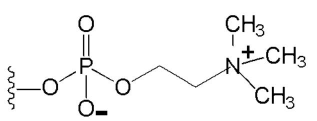

where the curvy line indicates the point of attachment to the rest of the polymer; wherein X is a) -OR where R is H, methyl, ethyl, propyl, or isopropyl, b) -H, c) any halogen, d) -SCN, or e) -NCS; and

n1, n2, n3, n4, n5, n6, n7, n8 and n9 are the same or different such that the sum of n1, n2, n3, n4, n5, n6, n7, n8 and n9 is 2500 plus or minus 15%.

**13.** The anti-VEGF antibody polymer conjugate for use of any one of the preceding claims, wherein

(a) the diabetic retinopathy is non-proliferative diabetic retinopathy;
(b) the subject has type 1 or type 2 diabetes with HbA1 of ≤ 12.0%,
(c) each dose comprises about 5 mg of the anti-VEGF antibody polymer conjugate by weight of the anti-VEGF antibody portion, and/or

(d) the anti-VEGF antibody polymer conjugate is KSI-301, which is supplied as a preservative free, sterile, aqueous solution in a single-use glass vial at a concentration of 50 mg/mL, based on antibody mass.

**14.** A pharmaceutical composition comprising:

an anti-VEGF antibody polymer conjugate;
12.5 mM sodium phosphate buffer; and
0.025% (w/w) polysorbate 20,
at a pH of about 6.5, wherein approximately 50 mg/mL of antibody mass is present and approximately 324 mg/mL of total mass of the antibody polymer conjugate is present in the composition.

**15.** A pharmaceutical composition comprising:

a) an anti-VEGF antibody polymer conjugate, 12.5 mM sodium phosphate buffer with 0.025% (w/w) polysorbate 20, pH 6.5, wherein the antibody polymer conjugate is present in the composition at a concentration of 50 mg/mL (based on antibody mass), equivalent to 324 mg/mL of total mass of the antibody polymer conjugate, including 50 mg/mL OG1950 Antibody Intermediate and 274 mg/mL OG1802 Biopolymer Intermediate;
b) an anti-VEGF antibody polymer conjugate, 10-15 mM sodium phosphate buffer with 0.01-0.5% (w/w) polysorbate 20, pH 6.5, wherein the antibody polymer conjugate is present in the composition at a concentration of about 50 mg/mL (based on antibody mass), equivalent to about 324 mg/mL of total mass of the antibody polymer conjugate;
c) an anti-VEGF antibody polymer conjugate, 12.5 mM sodium phosphate buffer with optional 0.025% (w/w) polysorbate 20, pH 6.5, wherein the antibody polymer conjugate is present in the composition at a concentration of 50 mg/mL (based on antibody mass), equivalent to 324 mg/mL of total mass of the antibody polymer conjugate, including 50 mg/mL OG1950 Antibody Intermediate and 274 mg/mL OG1802 Biopolymer Intermediate;
d) an anti-VEGF antibody polymer conjugate, sodium phosphate buffer with polysorbate 20, pH 6.5, wherein the antibody polymer conjugate is present in the composition at a concentration of 50 mg/mL (based on antibody mass), equivalent to about 324 mg/mL of total mass of the antibody-biopolymer conjugate, including about 50 mg/mL OG1950 Antibody Intermediate and about 274 mg/mL OG1802 Biopolymer Intermediate;
e) an anti-VEGF antibody polymer conjugate, 10-15 mM sodium phosphate buffer with 0.01-0.5% (w/w) polysorbate 20, pH 6.5, wherein the antibody polymer conjugate is present in the composition at a concentration of about 40-55 mg/mL (based on antibody mass), equivalent to about 259-356 mg/mL of total mass of the antibody-biopolymer conjugate;
f) an anti-VEGF antibody polymer conjugate, 10-15 mM sodium phosphate buffer with 0.01-0.5% (w/w) polysorbate 20, pH 6.5, wherein the antibody polymer conjugate is present in the composition at a concentration of about 45-52.5 mg/mL (based on antibody mass), equivalent to about 292-340 mg/mL of total mass of the antibody-biopolymer conjugate; or
g) an anti-VEGF antibody polymer conjugate, 10-15 mM sodium phosphate buffer with 0.01-0.5% (w/w) polysorbate 20, pH 6.5, wherein the antibody polymer conjugate is present in the composition at a concentration of about 40-55 mg/mL (based on antibody mass), equivalent to about 259-356 mg/mL of total mass of the antibody-biopolymer conjugate,

wherein the antibody polymer conjugate comprises an antibody comprising i) VH and VL in FIG. 64, or ii) 6 CDRs within the VH and VL of FIG. 64.

FIG. 1

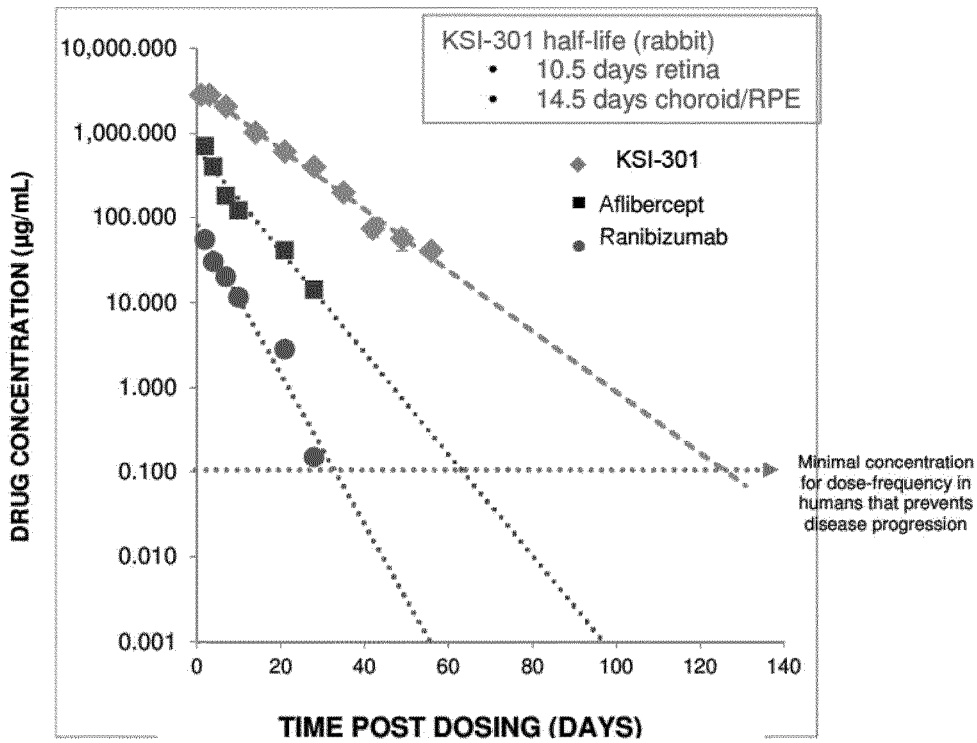
KSI-301 half-life (rabbit)
• 10.5 days retina
• 14.5 days choroid/RPE
KSI-301
Aflibercept
Ranibizumab
DRUG CONCENTRATION (µg/mL)
10,000.000
1,000.000
100.000
10.000
1.000
0.100
0.010
0.001
0
20
40
60
80
100
120
140
TIME POST DOSING (DAYS)
Minimal concentration for dose-frequency in humans that prevents disease progression

FIG. 2

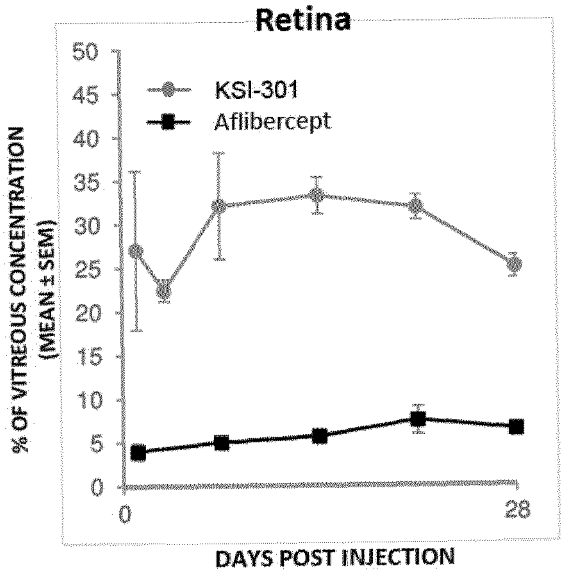
Retina
50
45
40
35
30
25
20
15
10
5
0
% OF VITREOUS CONCENTRATION
(MEAN ± SEM)
KSI-301
Aflibercept
0
28
DAYS POST INJECTION

FIG. 3

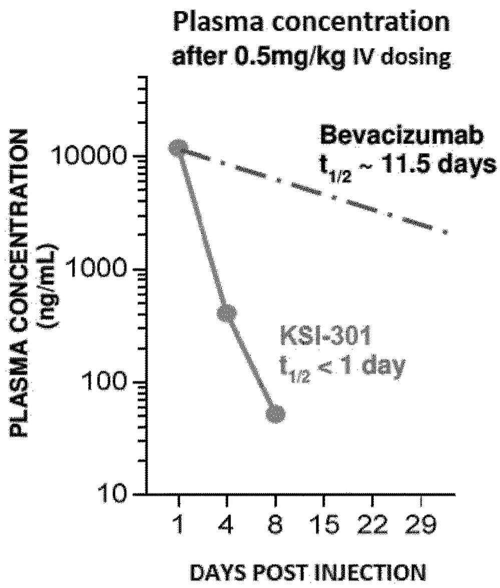
Plasma concentration
after 0.5mg/kg IV dosing
PLASMA CONCENTRATION (ng/mL)
10000
1000
100
10
Bevacizumab
$t_{1/2}$ ~ 11.5 days
KSI-301
$t_{1/2}$ < 1 day
1
4
8
15
22
29
DAYS POST INJECTION

FIG. 4

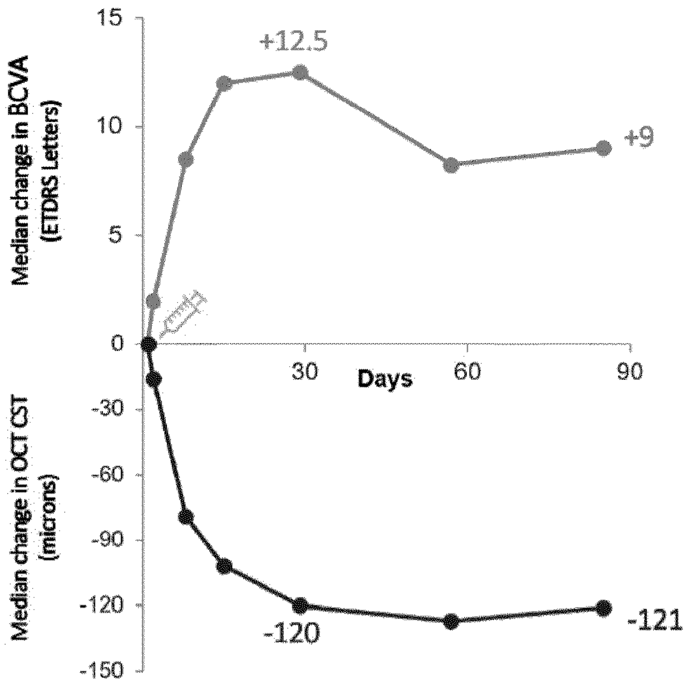

Median change in BCVA (ETDRS Letters)
15
10
5
0
+12.5
+9
30
Days
60
90
Median change in OCT CST (microns)
-30
-60
-90
-120
-150
-120
-121

FIG. 5

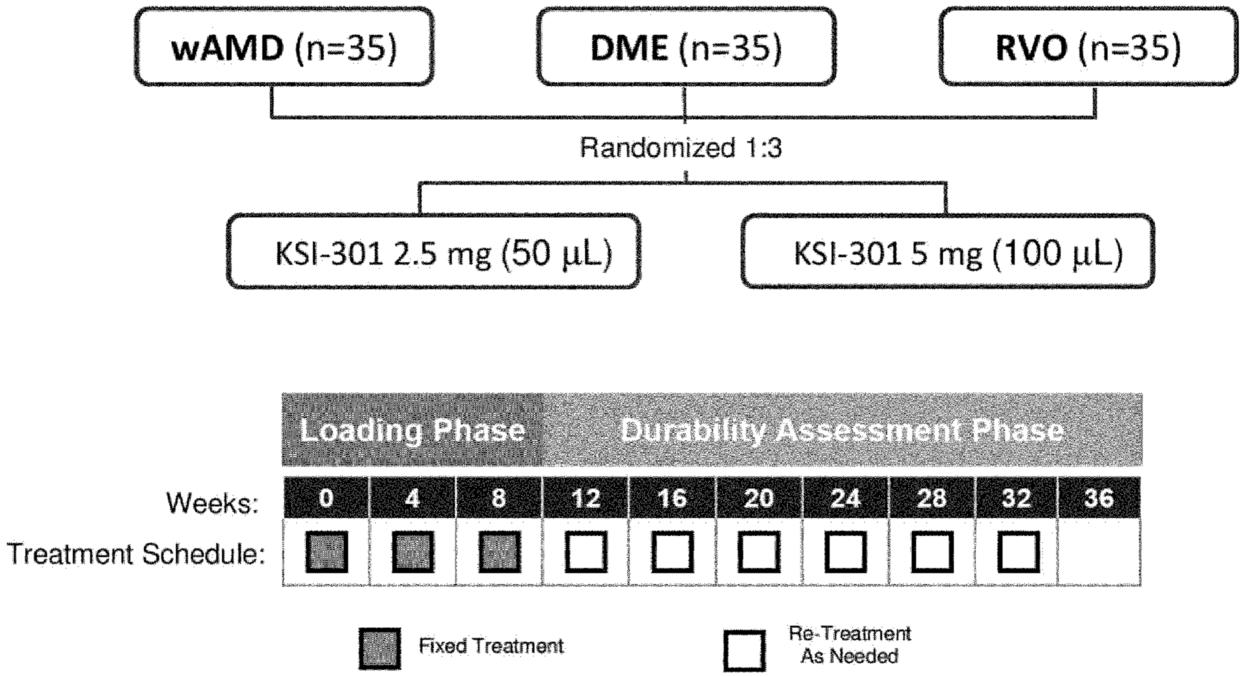
wAMD (n=35)
DME (n=35)
RVO (n=35)
Randomized 1:3
KSI-301 2.5 mg (50 μL)
KSI-301 5 mg (100 μL)
Loading Phase
Durability Assessment Phase
Weeks:
0
4
8
12
16
20
24
28
32
36
Treatment Schedule:
Fixed Treatment
Re-Treatment
As Needed

FIG. 6

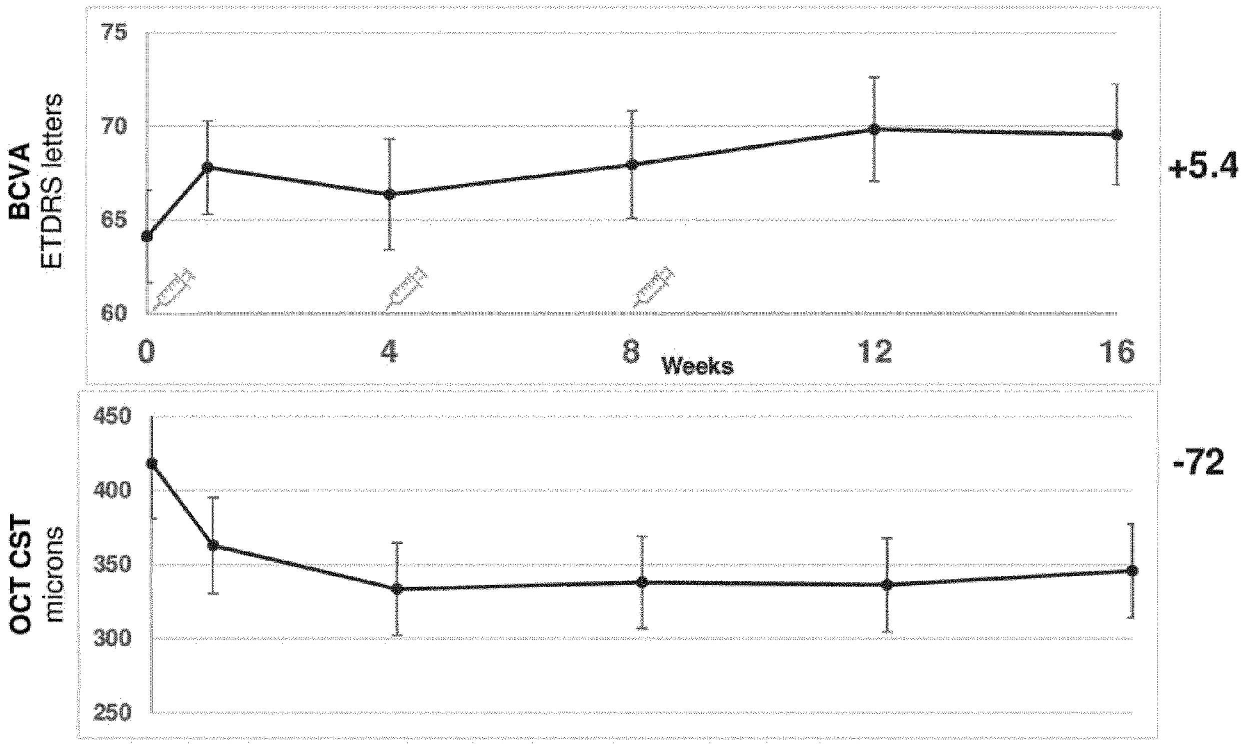
75
70
65
60
BCVA
ETDRS letters
+5.4
0
4
8
Weeks
12
16
450
400
350
300
250
OCT CST
microns
-72

FIG. 7

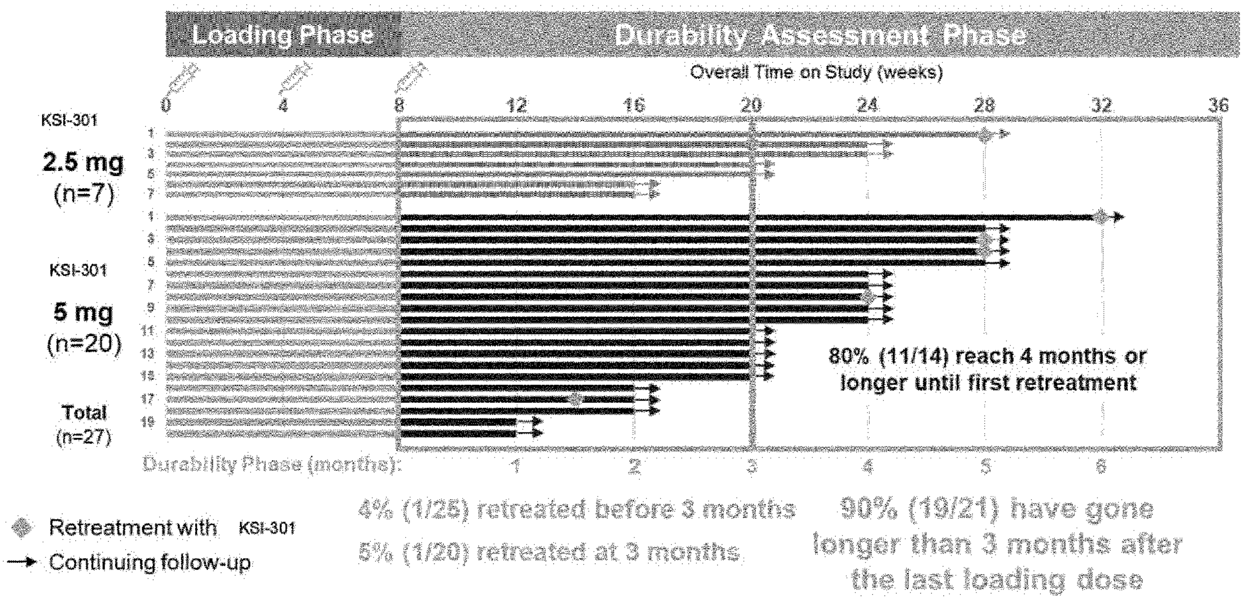
Loading Phase
Durability Assessment Phase
Overall Time on Study (weeks)
0
4
8
12
16
20
24
28
32
36
KSI-301
2.5 mg
(n=7)
KSI-301
5 mg
(n=20)
Total
(n=27)
80% (11/14) reach 4 months or longer until first retreatment
Durability Phase (months):
1
2
3
4
5
6
Retreatment with KSI-301
Continuing follow-up
4% (1/25) retreated before 3 months
5% (1/20) retreated at 3 months
90% (19/21) have gone longer than 3 months after the last loading dose

FIG. 8

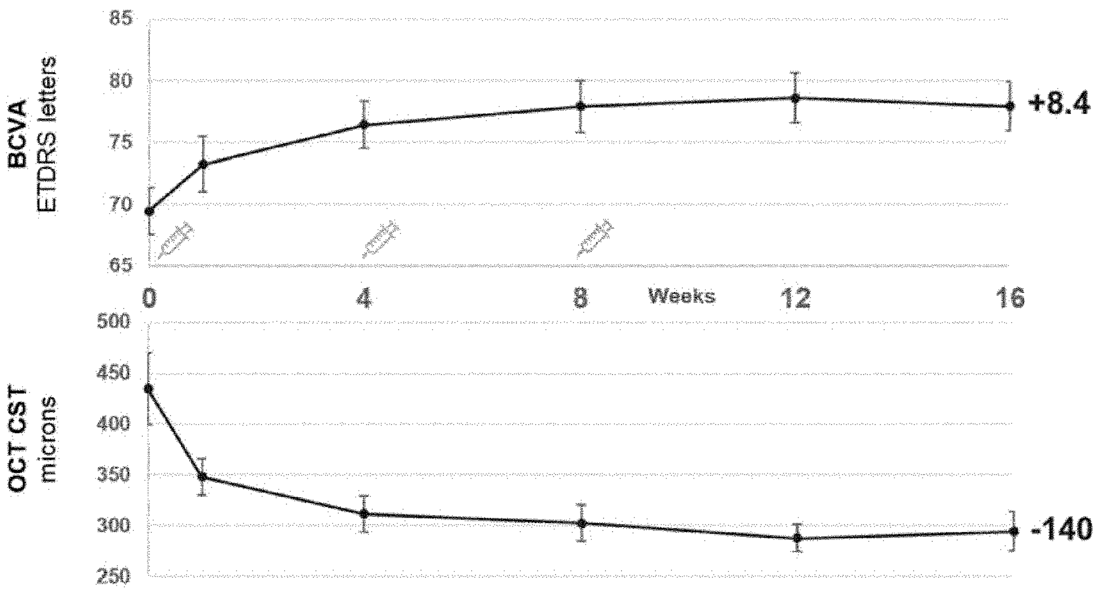


Includes only randomized patients that reached Week 16 visit. 2.5 & 5 mg doses pooled. Error bars represent standard error of the mean. OCT CST values are site reported. BCVA= best corrected visual acuity; OCT= optical coherence tomography; CST= central subfield thickness

n= 12 Patients reaching Week 16 visit by data cutoff

FIG. 9

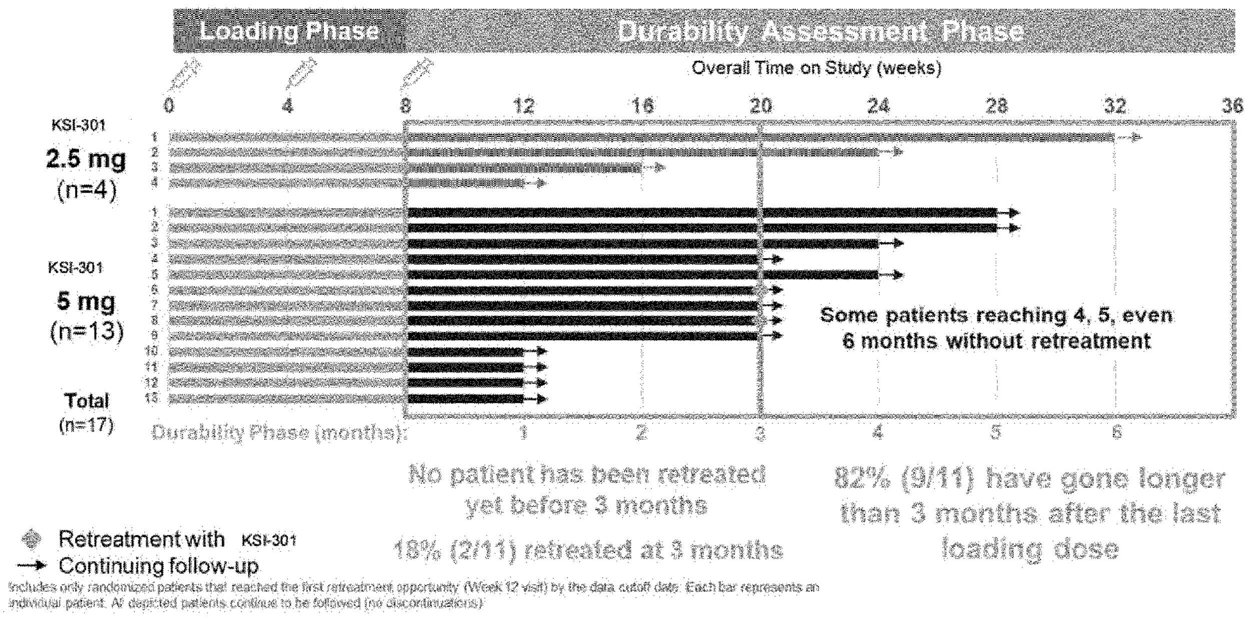
Loading Phase
Durability Assessment Phase
Overall Time on Study (weeks)
0
4
8
12
16
20
24
28
32
36
KSI-301
2.5 mg
(n=4)
KSI-301
5 mg
(n=13)
Total
(n=17)
Some patients reaching 4, 5, even 6 months without retreatment
Durability Phase (months):
1
2
3
4
5
6
No patient has been retreated yet before 3 months
18% (2/11) retreated at 3 months
82% (9/11) have gone longer than 3 months after the last loading dose
Retreatment with KSI-301
Continuing follow-up

FIG. 10

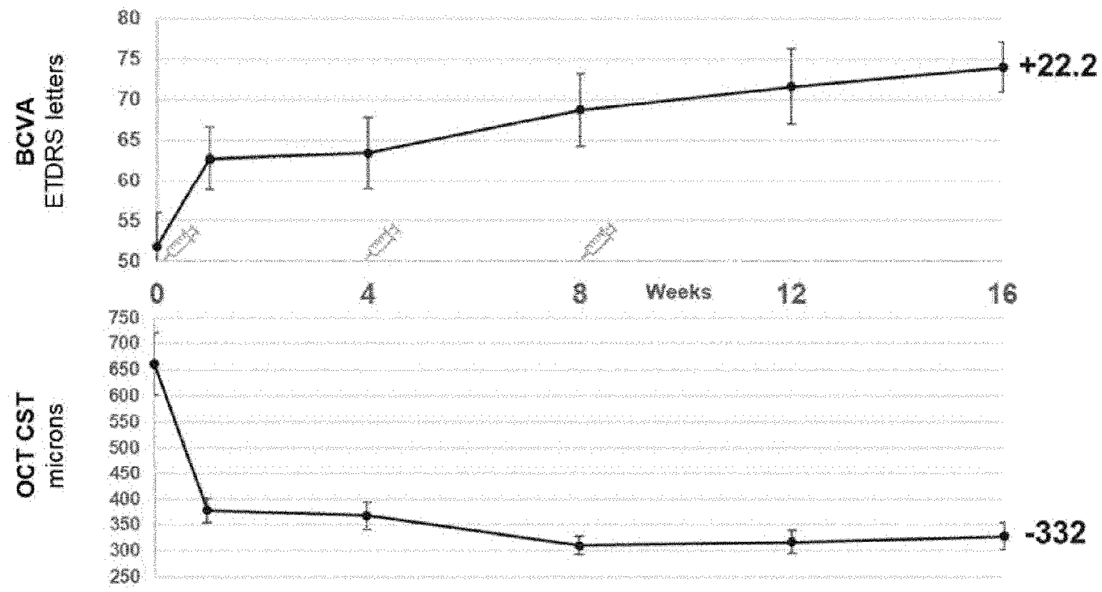


Includes only randomized patients that reached Week 16 visit by the data cutoff date; 2.5 & 5 mg doses pooled. Datapoints include one subject that discontinued after Week 12. Error bars represent standard error of the mean. OCT CST values are site reported. BCVA= best corrected visual acuity; OCT= optical coherence tomography; CST= central subfield thickness

n= 14 Patients reaching Week 16 visit by data cutoff

FIG. 11

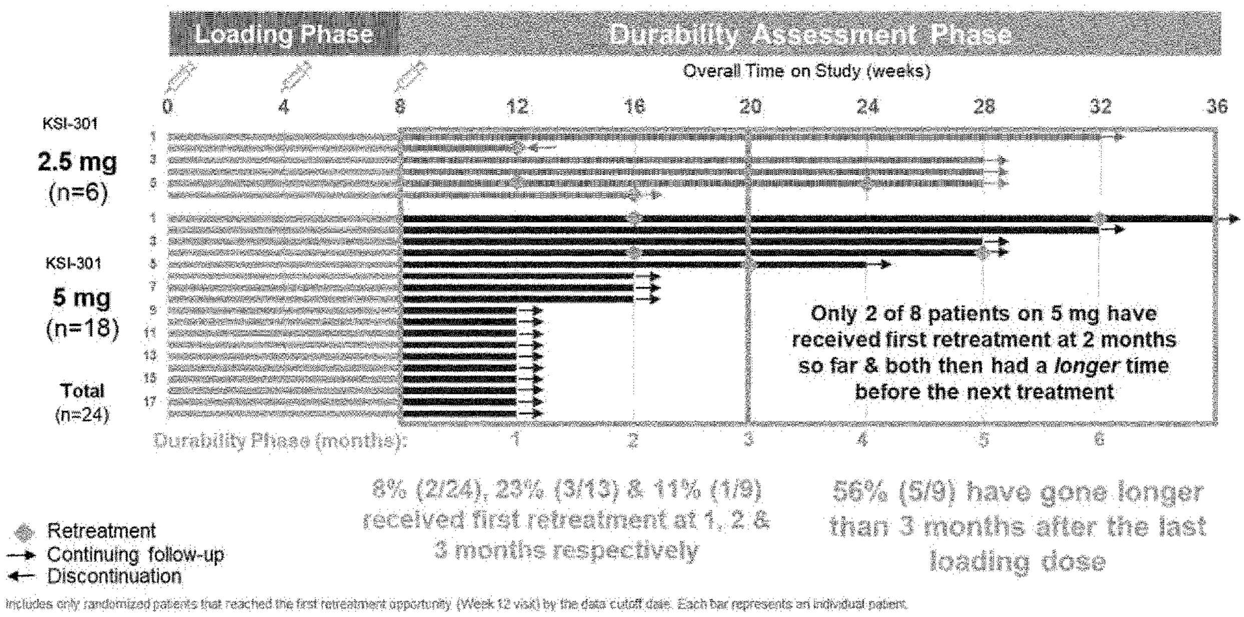
Loading Phase
Durability Assessment Phase
Overall Time on Study (weeks)
0 4 8 12 16 20 24 28 32 36
KSI-301
2.5 mg
(n=6)
KSI-301
5 mg
(n=18)
Total
(n=24)
Only 2 of 8 patients on 5 mg have received first retreatment at 2 months so far & both then had a *longer* time before the next treatment
Durability Phase (months): 1 2 3 4 5 6
8% (2/24), 23% (3/13) & 11% (1/9) received first retreatment at 1, 2 & 3 months respectively
56% (5/9) have gone longer than 3 months after the last loading dose
Retreatment
Continuing follow-up
Discontinuation
Includes only randomized patients that reached the first retreatment opportunity (Week 12 visit) by the data cutoff date. Each bar represents an individual patient.

FIG. 12

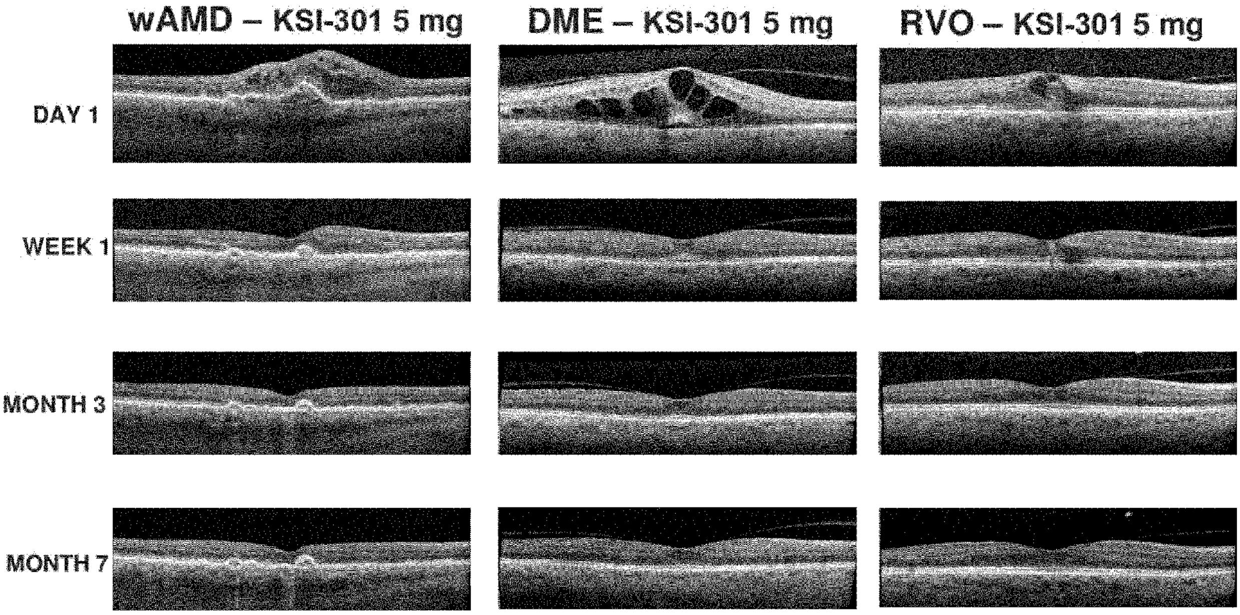
wAMD – KSI-301 5 mg
DME – KSI-301 5 mg
RVO – KSI-301 5 mg
DAY 1
WEEK 1
MONTH 3
MONTH 7

FIG. 13

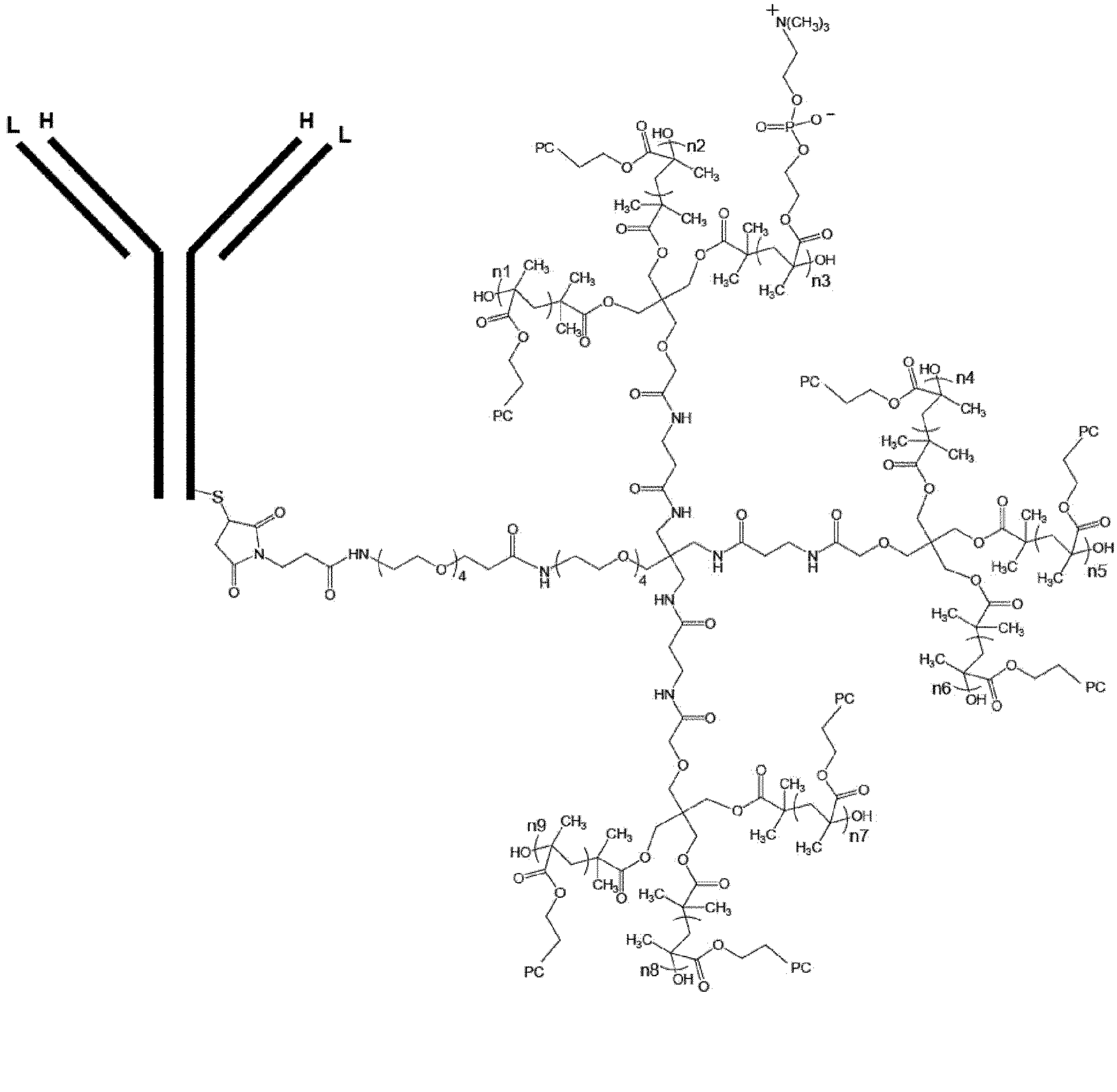

# FIG. 14

Heavy chain amino acid sequence of the anti-VEGF-A antibody component of KSI-301

EVQLVESGGG LVQPGGSLRL SCAASGYDFT HYGMNWVRQA PGKGLEWVGW
**CDRH1 (SEQ ID NO: 9)**
INTYTGEPTY AADFKRRFTF SLDTSKSTAY LQMNSLRAED TAVYYCAKYP
**CDRH2 (SEQ ID NO: 10)**
YYYGTSHWYF DVWGQGTLVT VSSASTKGPS VFPLAPSSKS TSGGTAALGC
**CDRH3 (SEQ ID NO: 11)**
LVKDYFPEPV TVSWNSGALT SGVHTFPAVL QSSGLYSLSS VVTVPSSSLG

TQTYICNVNH KPSNTKVDKK VEPKSCDKTH TCPPCPAPEA AGAPSVFLFP

PKPKDTLMIS RTPEVTCVVV DVSHEDPEVK FNWYVDGVEV HNAKTKPREE

QYNSTYRVVS VLTVLHQDWL NGKEYKCKVS NKALPAPIEK TISKAKGQPR

EPQVYTLPPS REEMTKNQVS LTCLVKGFYP SDIAVEWESN GQPENNYKTT

PPVLDSDGSF FLYSKLTVDK SRWQQGNVFS CSVMHEALHN HYTQKSLSCS

PGK (SEQ ID NO: 1)

Light chain amino acid sequence of the anti-VEGF-A antibody component of KSI-301

DIQLTQSPSS LSASVGDRVT ITCSASQDIS NYLNWYQQKP GKAPKVLIYF
**CDRL1 (SEQ ID NO: 12)**
TSSLHSGVPS RFSGSGSGTD FTLTISSLQP EDFATYYCQQ YSTVPWTFGQ
**CDRL2 (SEQ ID NO: 13)** **CDRL3 (SEQ ID NO: 14)**
GTKVEIKRTV AAPSVFIFPP SDEQLKSGTA SVVCLLNNFY PREAKVQWKV

DNALQSGNSQ ESVTEQDSKD STYSLSSTLT LSKADYEKHK VYACEVTHQG

LSSPVTKSFN RGEC (SEQ ID NO: 2)

FIG. 15

Heavy chain amino acid sequence of bevacizumab

EVQLVESGGG LVQPGGSLRL SCAASGYTFT NYGMNWVRQA PGKGLEWVGW
INTYTGEPTY AADFKRRFTF SLDTSKSTAY LQMNSLRAED TAVYYCAKYP
HYYGSSHWYF DVWGQGTLVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKK VEPKSCDKTH TCPPCPAPEL LGGPSVFLFP
PKPKDTLMIS RTPEVTCVVV DVSHEDPEVK FNWYVDGVEV HNAKTKPREE
QYNSTYRVVS VLTVLHQDWL NGKEYKCKVS NKALPAPIEK TISKAKGQPR
EPQVYTLPPS REEMTKNQVS LTCLVKGFYP SDIAVEWESN GQPENNYKTT
PPVLDSDGSF FLYSKLTVDK SRWQQGNVFS CSVMHEALHN HYTQKSLSLS
PGK (SEQ ID NO: 3)

Light chain amino acid sequence of bevacizumab

DIQMTQSPSS LSASVGDRVT ITCSASQDIS NYLNWYQQKP GKAPKVLIYF
TSSLHSGVPS RFSGSGSGTD FTLTISSLQP EDFATYYCQQ YSTVPWTFGQ
GTKVEIKRTV AAPSVFIFPP SDEQLKSGTA SVVCLLNNFY PREAKVQWKV
DNALQSGNSQ ESVTEQDSKD STYSLSSTLT LSKADYEKHK VYACEVTHQG
LSSPVTKSFN RGEC (SEQ ID NO: 4)

Heavy chain amino acid sequence of ranibizumab

EVQLVESGGG LVQPGGSLRL SCAASGYDFT HYGMNWVRQA PGKGLEWVGW
INTYTGEPTY AADFKRRFTF SLDTSKSTAY LQMNSLRAED TAVYYCAKYP
YYYGTSHWYF DVWGQGTLVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKK VEPKSCDKTH L (SEQ ID NO: 5)

Light chain amino acid sequence of ranibizumab

DIQLTQSPSS LSASVGDRVT ITCSASQDIS NYLNWYQQKP GKAPKVLIYF
TSSLHSGVPS RFSGSGSGTD FTLTISSLQP EDFATYYCQQ YSTVPWTFGQ
GTKVEIKRTV AAPSVFIFPP SDEQLKSGTA SVVCLLNNFY PREAKVQWKV
DNALQSGNSQ ESVTEQDSKD STYSLSSTLT LSKADYEKHK VYACEVTHQG
LSSPVTKSFN RGEC (SEQ ID NO: 6)

FIG. 16

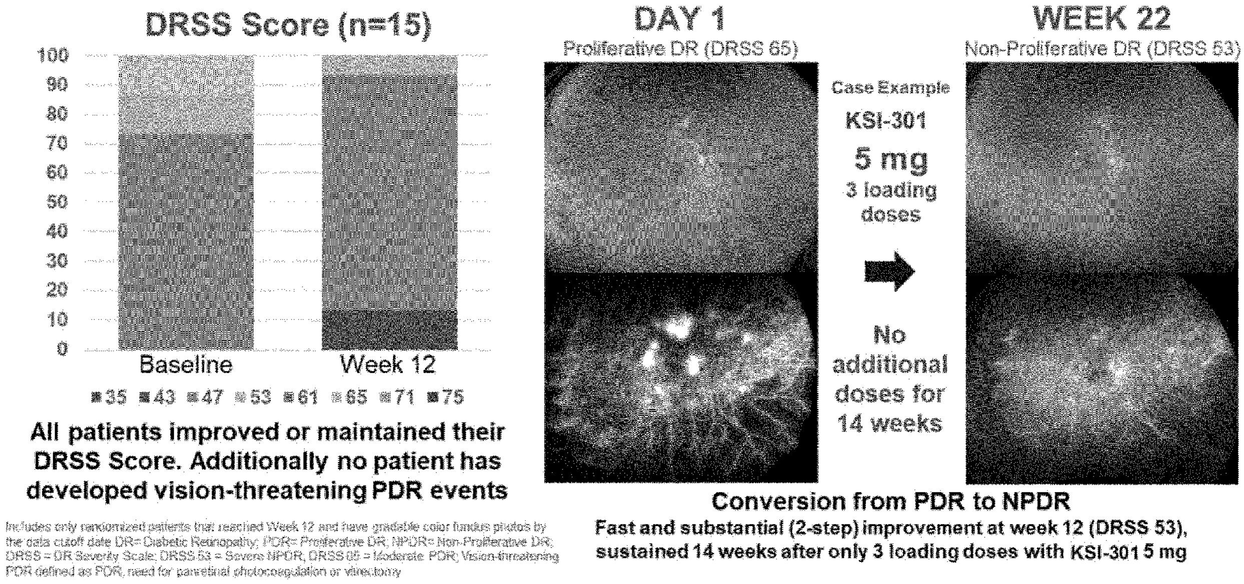
DRSS Score (n=15)
100
90
80
70
60
50
40
30
20
10
0
Baseline
Week 12
35 43 47 53 61 65 71 75
All patients improved or maintained their DRSS Score. Additionally no patient has developed vision-threatening PDR events
Includes only randomized patients that reached Week 12 and have gradable color fundus photos by the data cutoff date DR= Diabetic Retinopathy; PDR= Proliferative DR; NPDR= Non-Proliferative DR; DRSS = DR Severity Scale; DRSS 53 = Severe NPDR; DRSS 65 = Moderate PDR; Vision-threatening PDR defined as PDR, need for panretinal photocoagulation or vitrectomy
DAY 1
Proliferative DR (DRSS 65)
Case Example
KSI-301
5 mg
3 loading doses
No additional doses for 14 weeks
WEEK 22
Non-Proliferative DR (DRSS 53)
Conversion from PDR to NPDR
Fast and substantial (2-step) improvement at week 12 (DRSS 53), sustained 14 weeks after only 3 loading doses with KSI-301 5 mg

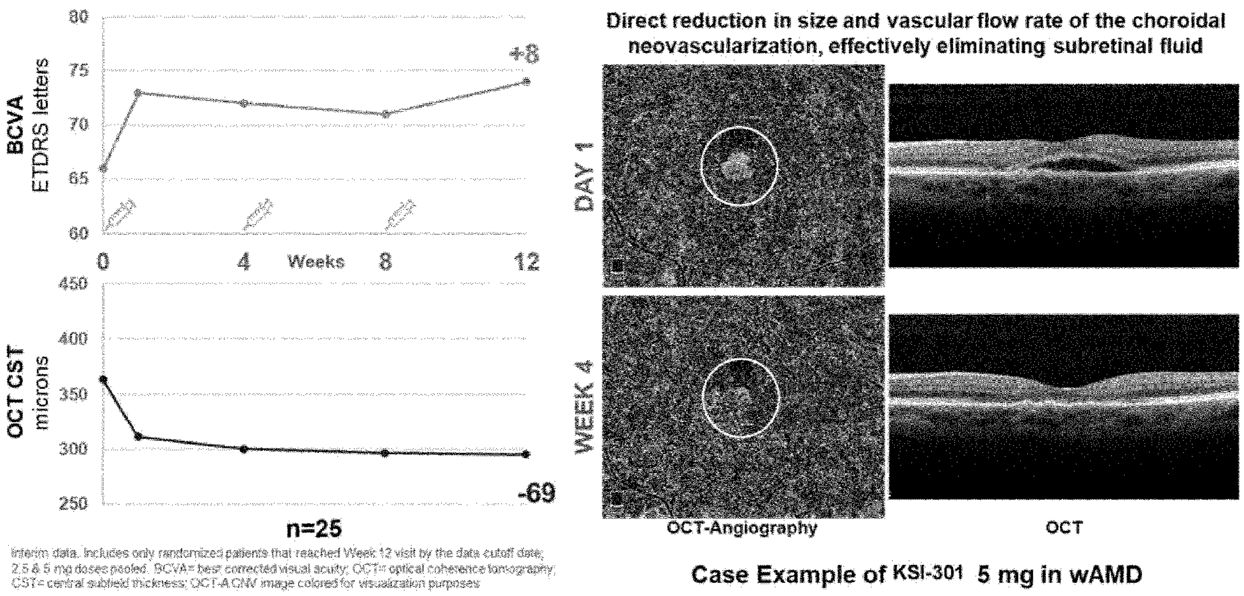
80
75
70
65
60
BCVA
ETDRS letters
+8
0
4
Weeks
8
12
450
400
350
300
250
OCT CST
microns
-69
n=25
Interim data. Includes only randomized patients that reached Week 12 visit by the data cutoff date; 2.5 & 5 mg doses pooled. BCVA= best corrected visual acuity; OCT= optical coherence tomography; CST= central subfield thickness; OCT-A CNV image colored for visualization purposes
Direct reduction in size and vascular flow rate of the choroidal neovascularization, effectively eliminating subretinal fluid
DAY 1
WEEK 4
OCT-Angiography
OCT
Case Example of KSI-301 5 mg in wAMD

FIG. 17A

FIG. 17B

## Direct effect on the choroidal neovascularization

**Example of KSI-301 5 mg in Wet AMD**

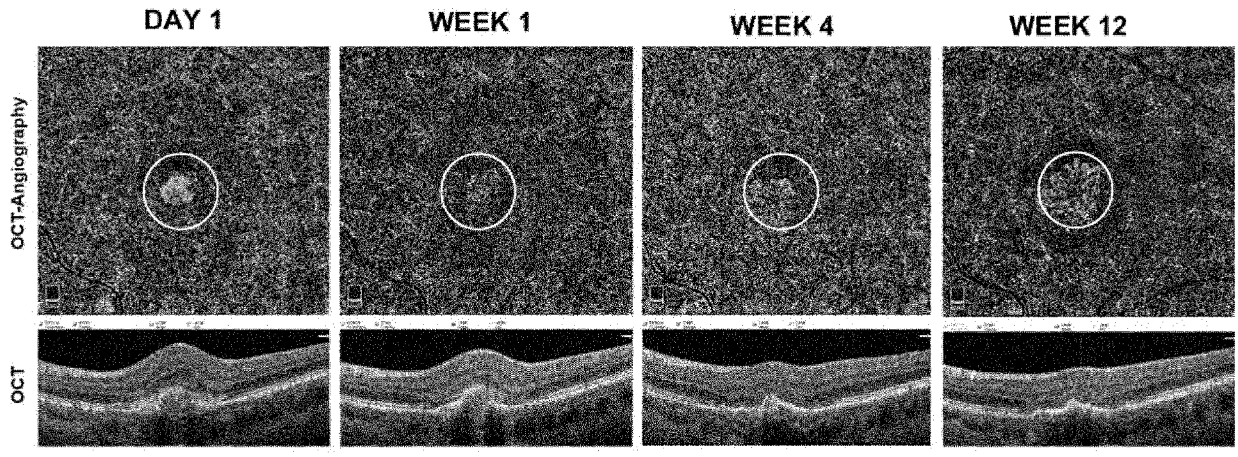


**Fast and direct reduction in size and vascular flow rate of the choroidal neovascularization seen at Week 1, and sustained through the loading phase to Week 12**

OCT = optical coherence tomography; OCT-A CNV image colored for visualization purposes

FIG. 18A

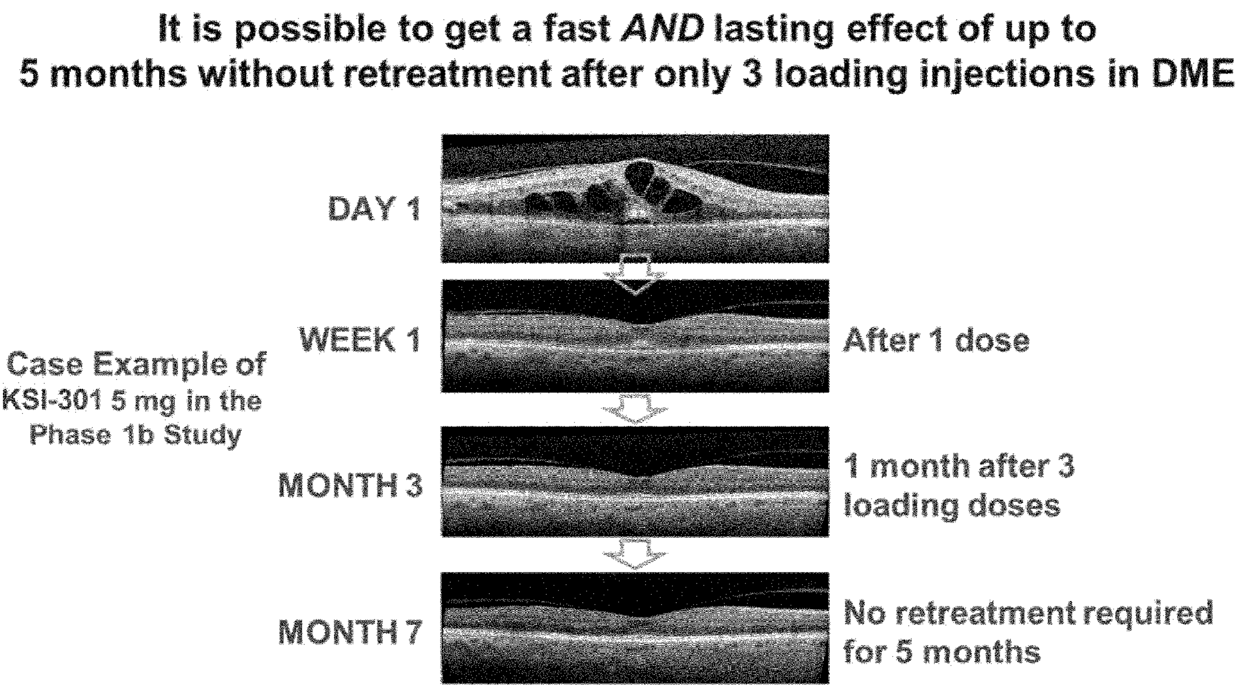
It is possible to get a fast AND lasting effect of up to
5 months without retreatment after only 3 loading injections in DME
DAY 1
WEEK 1
After 1 dose
Case Example of
KSI-301 5 mg in the
Phase 1b Study
MONTH 3
1 month after 3
loading doses
MONTH 7
No retreatment required
for 5 months

FIG. 18B

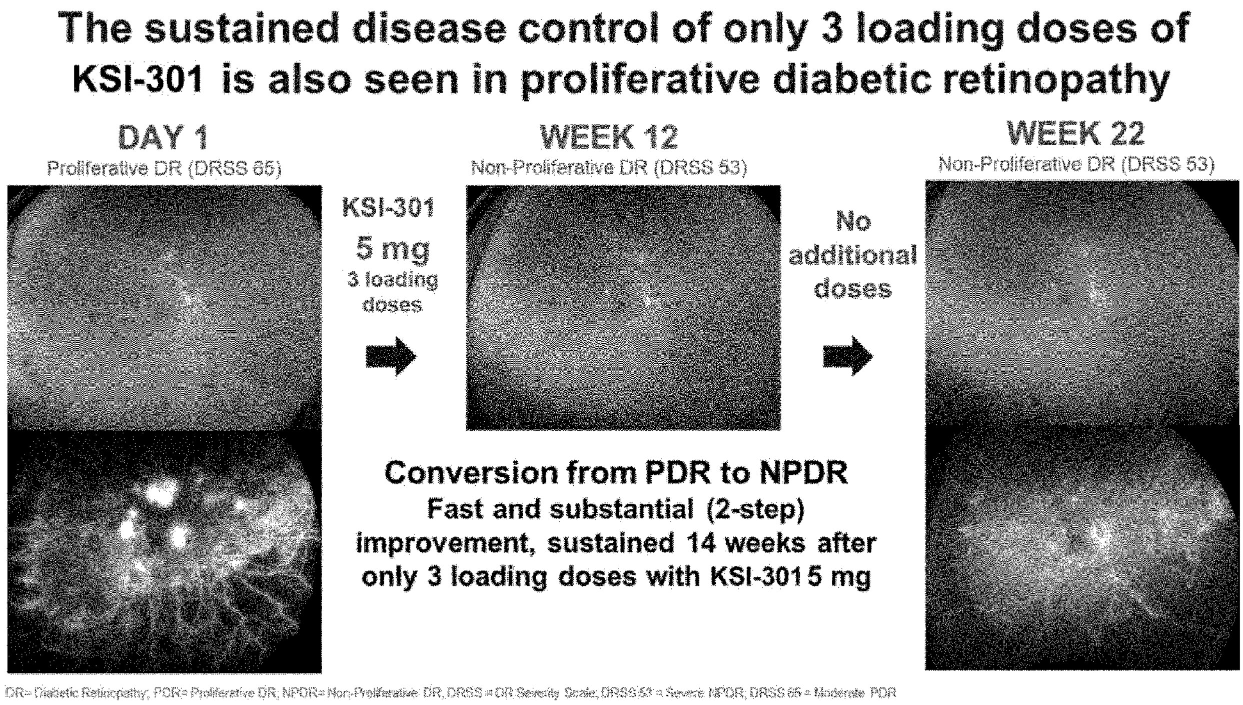
The sustained disease control of only 3 loading doses of KSI-301 is also seen in proliferative diabetic retinopathy
DAY 1
Proliferative DR (DRSS 65)
KSI-301
5 mg
3 loading doses
WEEK 12
Non-Proliferative DR (DRSS 53)
No additional doses
WEEK 22
Non-Proliferative DR (DRSS 53)
Conversion from PDR to NPDR
Fast and substantial (2-step) improvement, sustained 14 weeks after only 3 loading doses with KSI-301 5 mg
DR= Diabetic Retinopathy; PDR= Proliferative DR; NPDR= Non-Proliferative DR, DRSS = DR Severity Scale, DRSS 53 = Severe NPDR; DRSS 65 = Moderate PDR

FIG. 18C

## In addition to the conversion from PDR to NPDR, this patient exhibited signs of peripheral vascular reperfusion

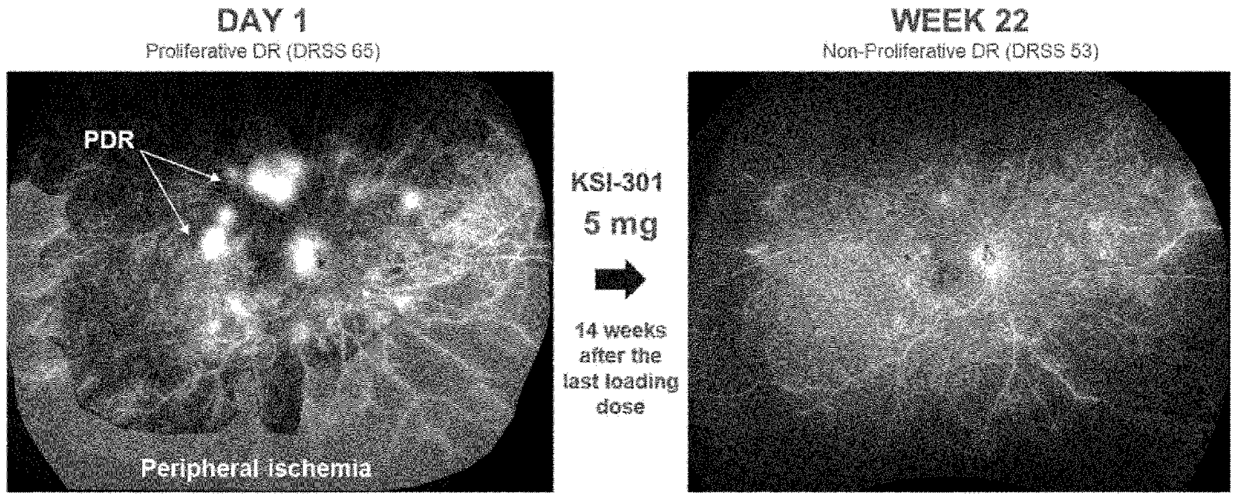


DR= Diabetic Retinopathy; PDR= Proliferative DR; NPDR= Non-Proliferative DR; DRSS = DR Severity Scale; DRSS 53 = Severe NPDR; DRSS 65 = Moderate PDR

FIG. 18D

**In addition to the conversion from PDR to NPDR, this patient exhibited signs of peripheral vascular reperfusion**

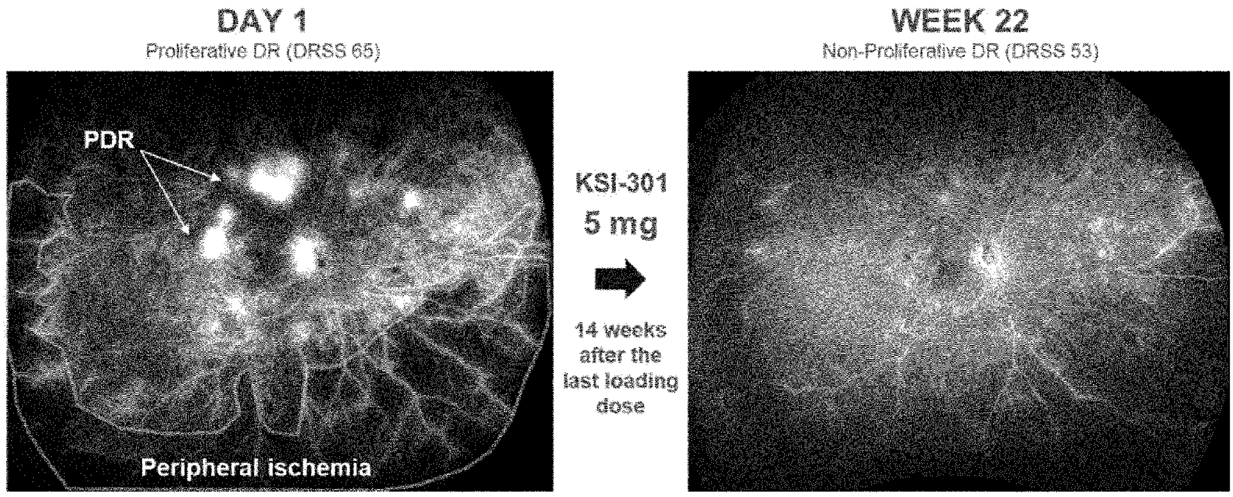


PDR= Proliferative DR; NPDR= Non-Proliferative DR; DRSS = DR Severity Scale; DRSS 53 = Severe NPDR; DRSS 65 = Moderate PDR

FIG. 19

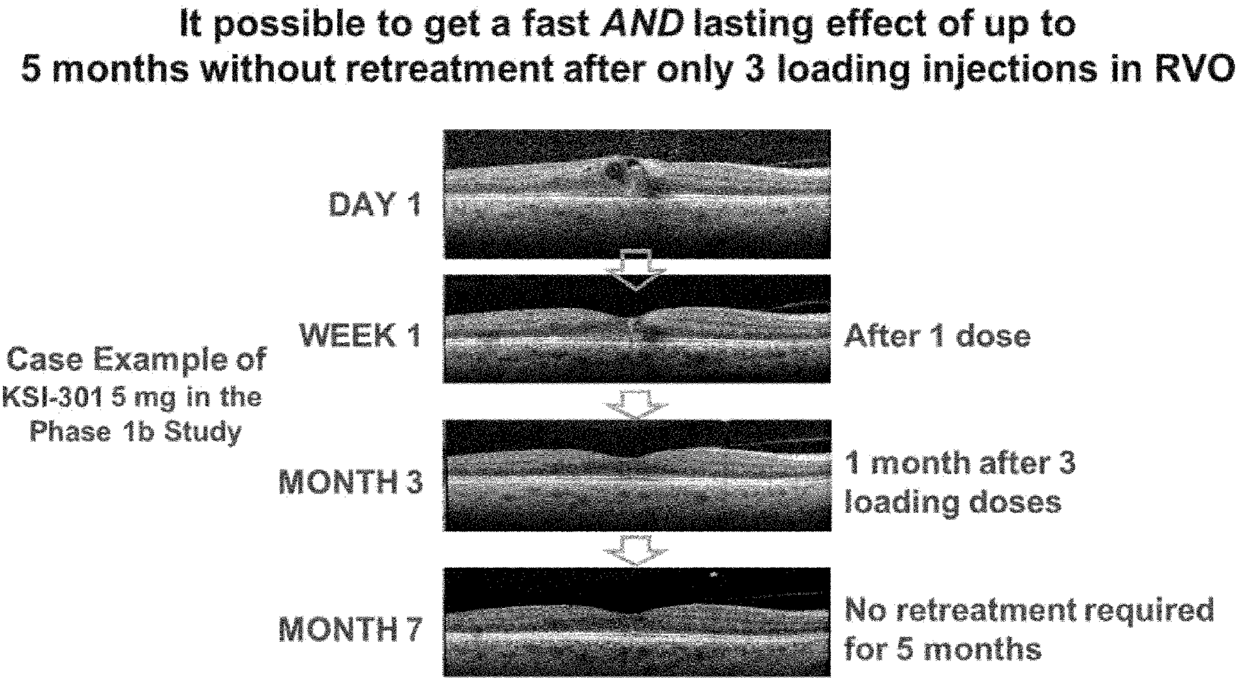
It possible to get a fast *AND* lasting effect of up to
5 months without retreatment after only 3 loading injections in RVO
Case Example of
KSI-301 5 mg in the
Phase 1b Study
DAY 1
WEEK 1
After 1 dose
MONTH 3
1 month after 3 loading doses
MONTH 7
No retreatment required for 5 months

FIG. 20

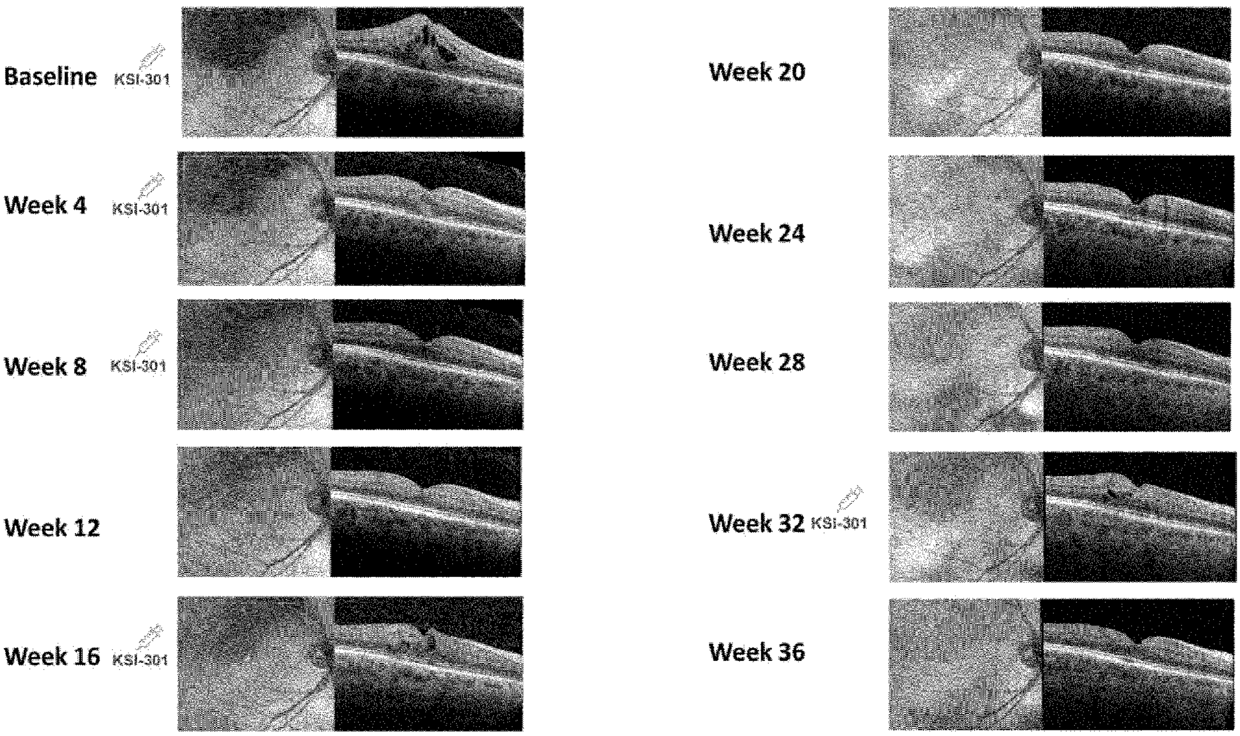
Baseline KSI-301
Week 4 KSI-301
Week 8 KSI-301
Week 12
Week 16 KSI-301
Week 20
Week 24
Week 28
Week 32 KSI-301
Week 36

FIG. 21A

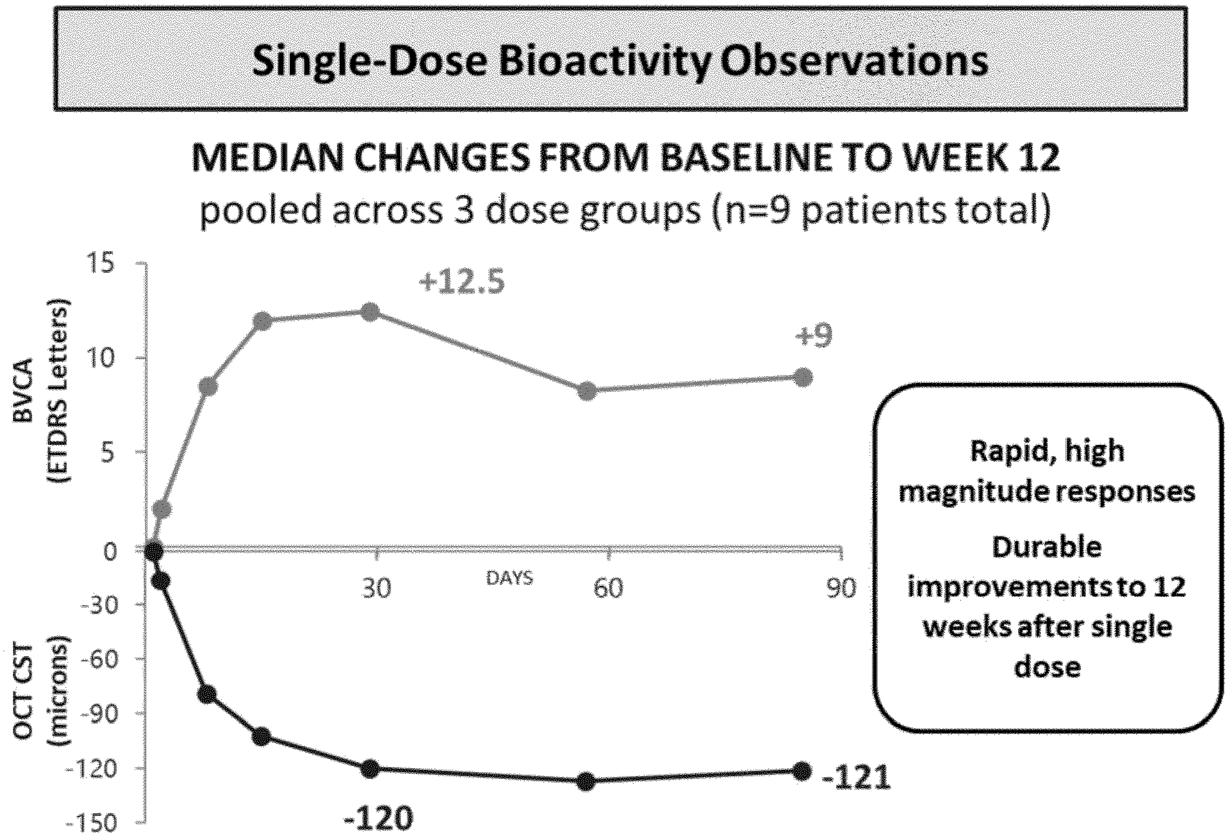
Single-Dose Bioactivity Observations
MEDIAN CHANGES FROM BASELINE TO WEEK 12
pooled across 3 dose groups (n=9 patients total)
BVCA (ETDRS Letters)
15
10
5
0
+12.5
+9
OCT CST (microns)
-30
-60
-90
-120
-150
30
DAYS
60
90
-120
-121
Rapid, high magnitude responses
Durable improvements to 12 weeks after single dose

FIG. 21B

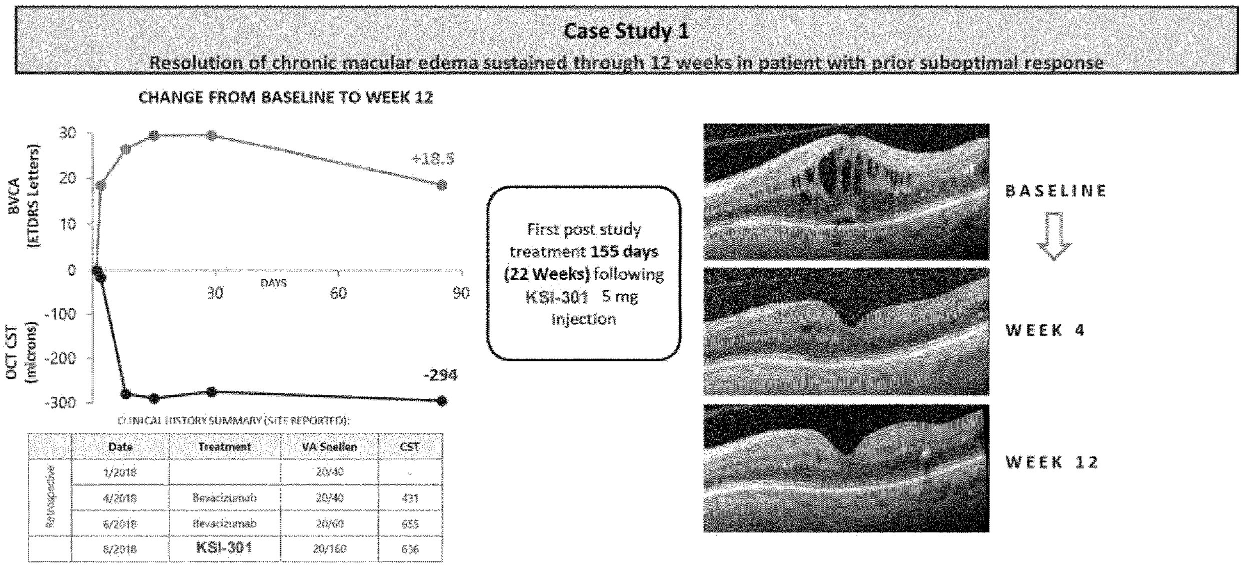
Case Study 1
Resolution of chronic macular edema sustained through 12 weeks in patient with prior suboptimal response
CHANGE FROM BASELINE TO WEEK 12
BVCA (ETDRS Letters)
30
20
10
0
+18.5
DAYS
30
60
90
OCT CST (microns)
-100
-200
-300
-294
CLINICAL HISTORY SUMMARY (SITE REPORTED):
Date | Treatment | VA Snellen | CST
Retrospective
1/2018 | | 20/40 | -
4/2018 | Bevacizumab | 20/40 | 431
6/2018 | Bevacizumab | 20/60 | 655
8/2018 | KSI-301 | 20/160 | 636
First post study treatment 155 days (22 Weeks) following KSI-301 5 mg injection
BASELINE
WEEK 4
WEEK 12

FIG. 21C

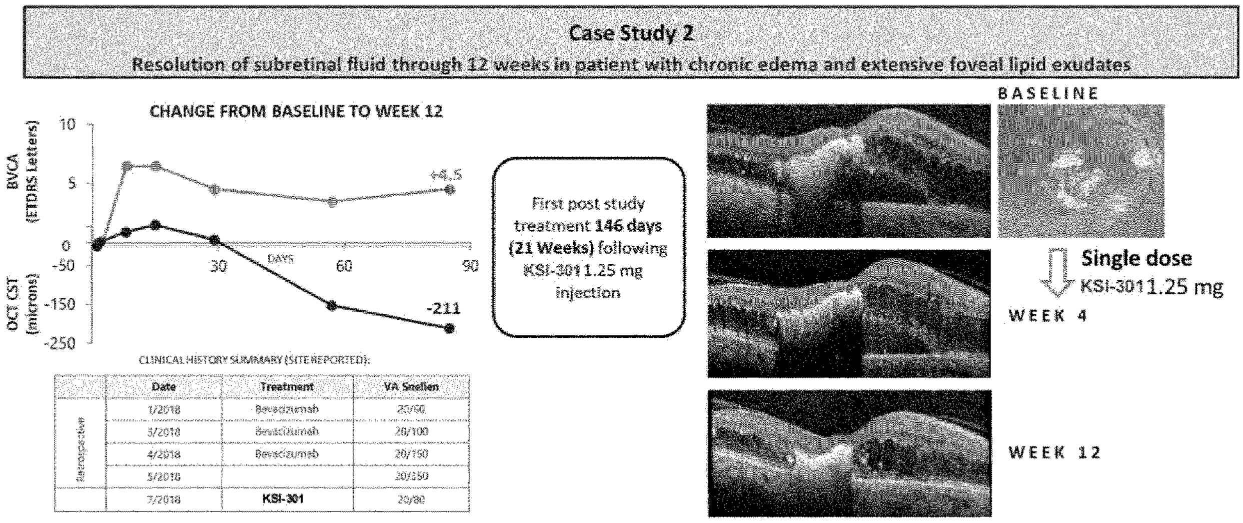
Case Study 2
Resolution of subretinal fluid through 12 weeks in patient with chronic edema and extensive foveal lipid exudates
CHANGE FROM BASELINE TO WEEK 12
BVCA (ETDRS Letters)
10
5
0
+4.5
OCT CST (microns)
-50
-150
-250
DAYS
30
60
90
-211
First post study treatment 146 days (21 Weeks) following KSI-301 1.25 mg injection
BASELINE
Single dose
KSI-301 1.25 mg
WEEK 4
WEEK 12
CLINICAL HISTORY SUMMARY (SITE REPORTED):
Date | Treatment | VA Snellen
Retrospective
1/2018 | Bevacizumab | 20/60
3/2018 | Bevacizumab | 20/100
4/2018 | Bevacizumab | 20/150
5/2018 | | 20/250
7/2018 | KSI-301 | 20/80

FIG. 22

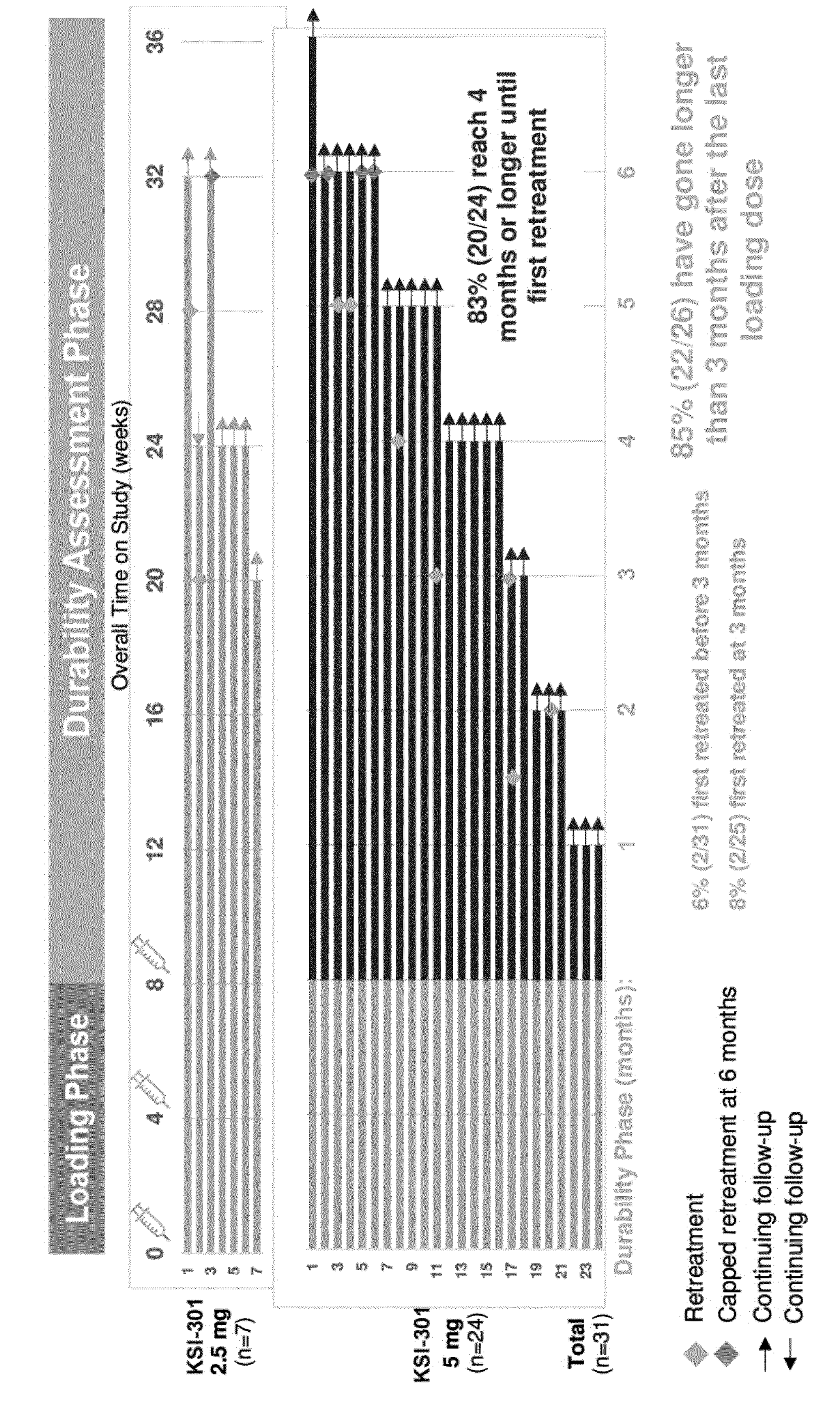


Interim data. Includes only randomized patients that reached the first retreatment opportunity (Week 12 visit) by the data cutoff date. Each bar represents an individual patient.

FIG. 23

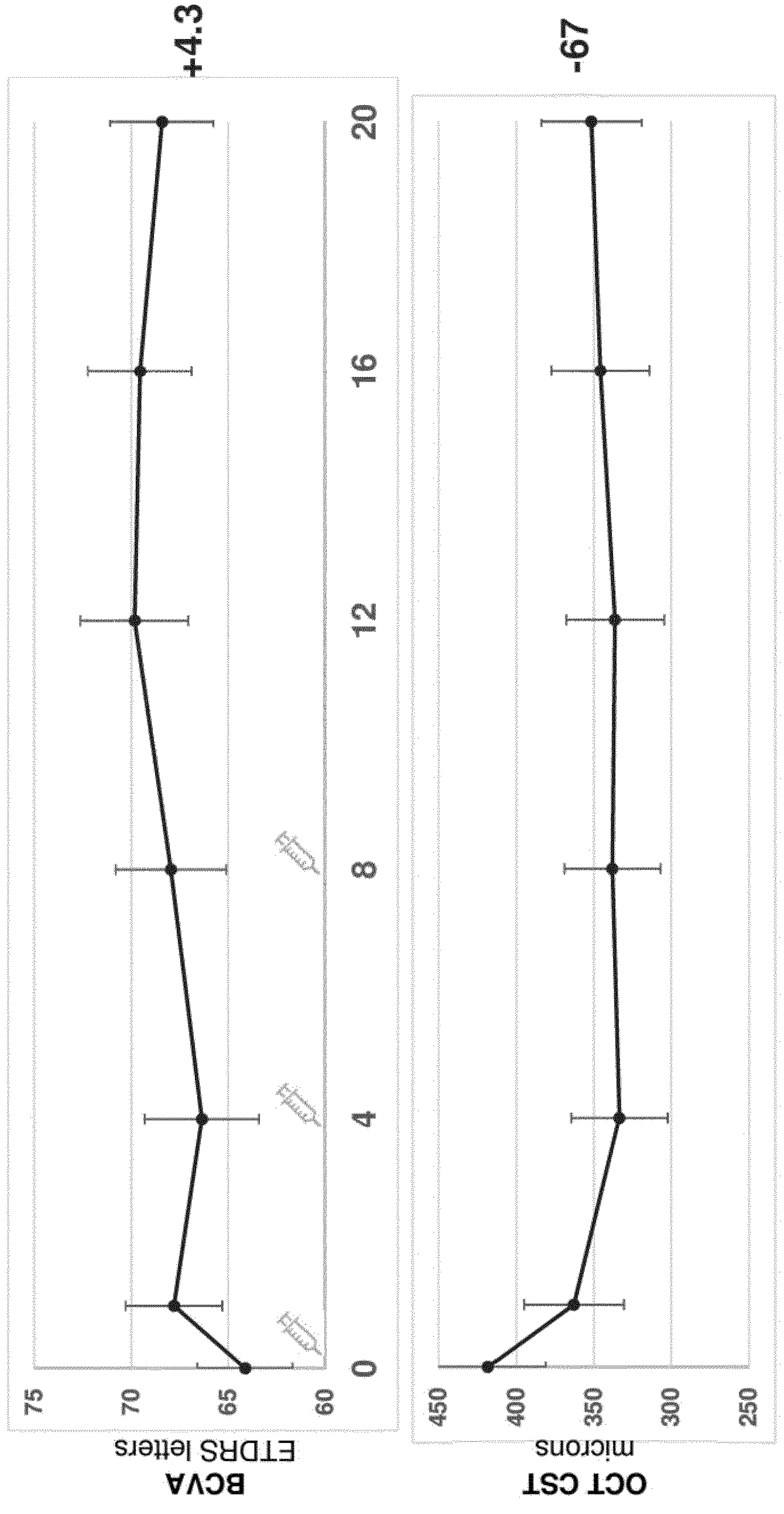


n= 25 Patients reaching Week 20 visit by data cutoff

Interim data. Includes only randomized patients that reached Week 20 visit by the data cutoff date; 2.5 & 5 mg doses pooled. Error bars represent standard error of the mean. OCT CST values are site reported. BCVA= best corrected visual acuity; OCT= optical coherence tomography; CST= central subfield thickness

FIG. 24

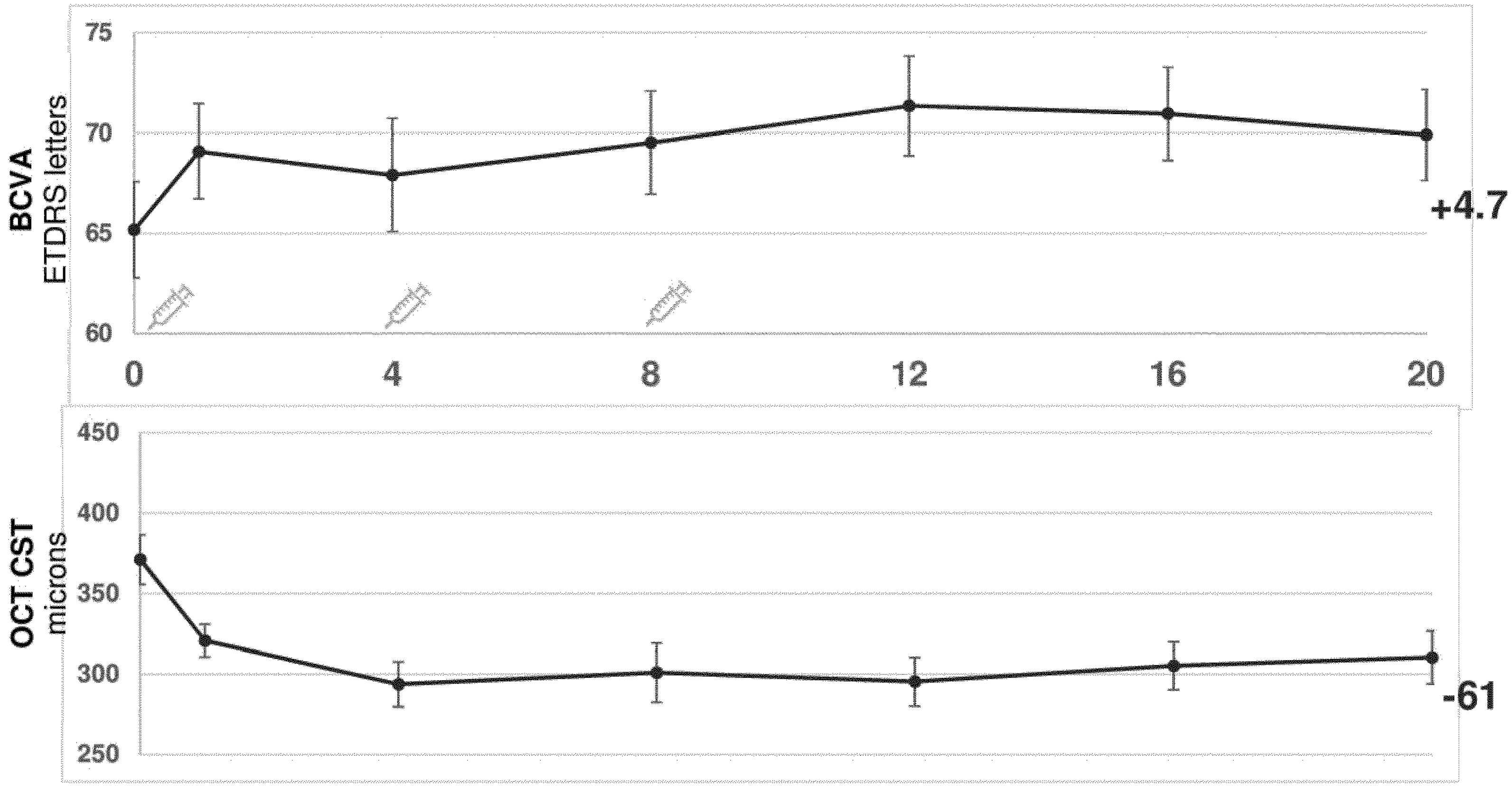


n= 23 Patients without high PEDs reaching Week 20 visit by data cutoff

Interim data. Includes only randomized patients that reached Week 20 visit by the data cutoff date; 2.5 & 5 mg doses pooled. Error bars represent standard error of the mean. OCT CST values are site reported. BCVA= best corrected visual acuity; OCT= optical coherence tomography; CST= central subfield thickness.
High PED defined as presence of a PED with baseline CST ≥500 microns.

FIG. 25

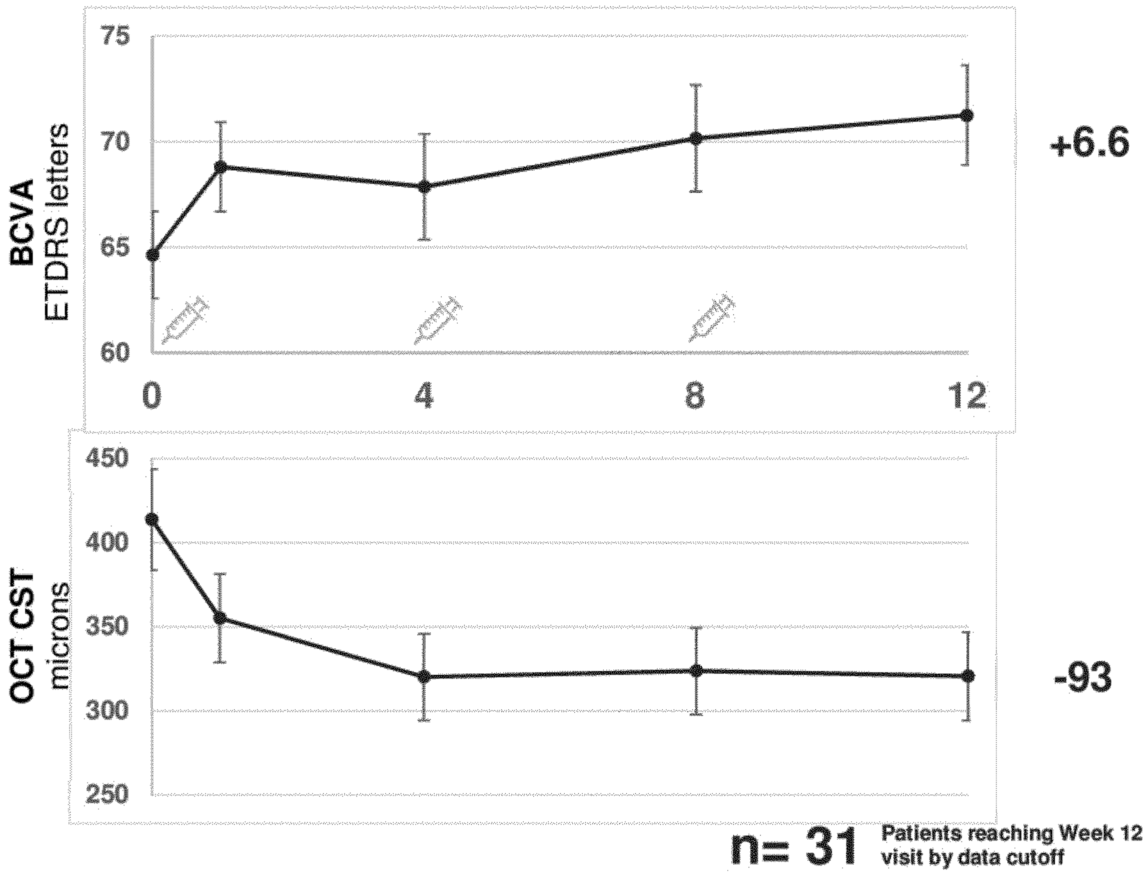


Interim data. Includes only randomized patients that reached Week 12 visit by the data cutoff date; 2.5 & 5 mg doses pooled. Error bars represent standard error of the mean. OCT CST values are site reported. BCVA= best corrected visual acuity; OCT= optical coherence tomography; CST= central subfield thickness

FIG. 26

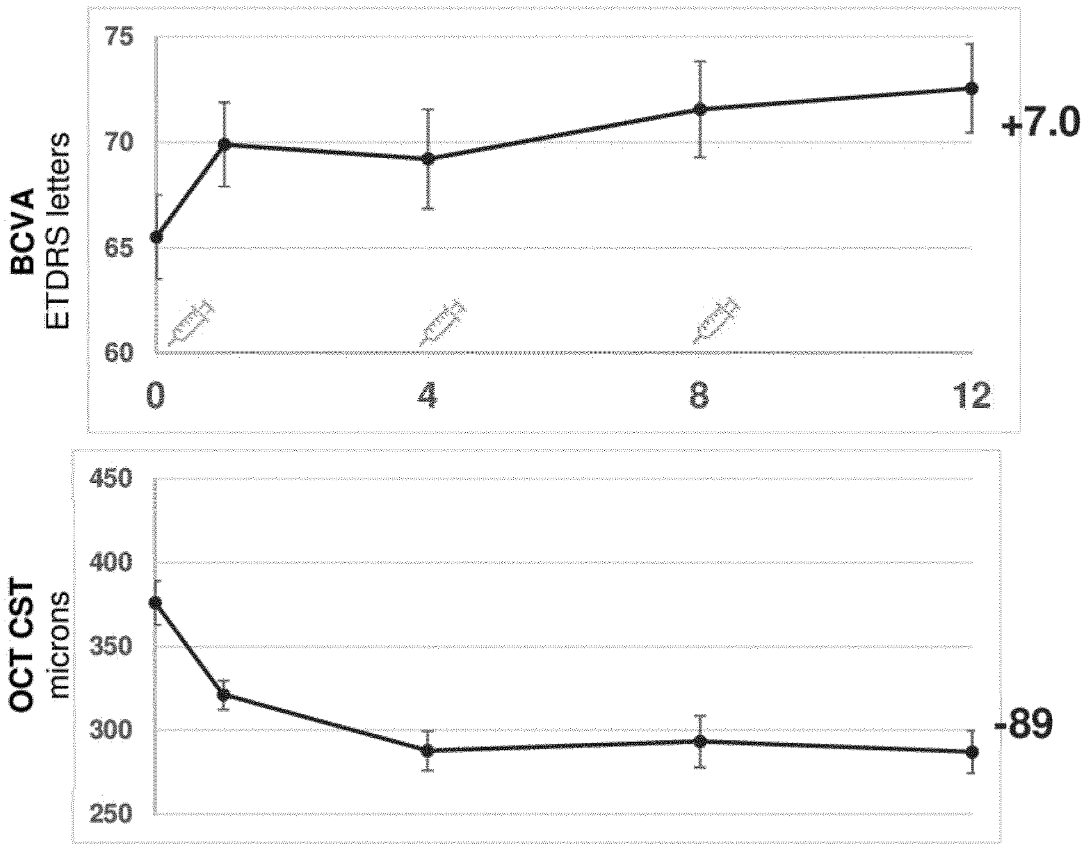


n= 29 Patients without high PEDs reaching Week 12 visit by data cutoff

Interim data. Includes only randomized patients that reached Week 12 visit by the data cutoff date; 2.5 & 5 mg doses pooled. Error bars represent standard error of the mean. OCT CST values are site reported. BCVA= best corrected visual acuity; OCT= optical coherence tomography; CST= central subfield thickness.
High PED defined as presence of a PED with baseline CST ≥500 microns.

FIG. 27

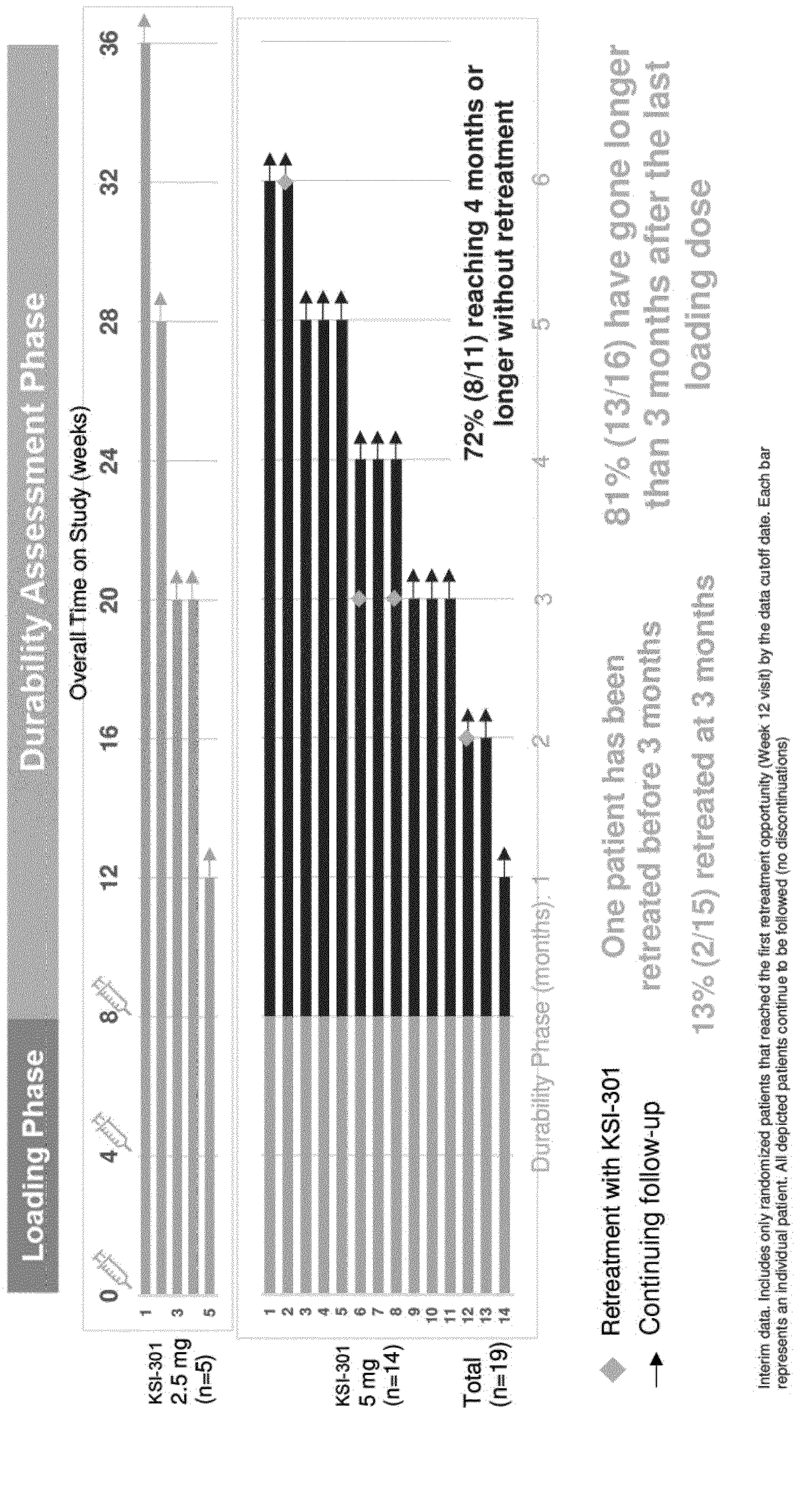
Loading Phase
Durability Assessment Phase
Overall Time on Study (weeks)
0
4
8
12
16
20
24
28
32
36
KSI-301
2.5 mg
(n=5)
KSI-301
5 mg
(n=14)
Total
(n=19)
72% (8/11) reaching 4 months or longer without retreatment
Durability Phase (months): 1
2
3
4
5
6
Retreatment with KSI-301
Continuing follow-up
One patient has been retreated before 3 months
13% (2/15) retreated at 3 months
81% (13/16) have gone longer than 3 months after the last loading dose
Interim data. Includes only randomized patients that reached the first retreatment opportunity (Week 12 visit) by the data cutoff date. Each bar represents an individual patient. All depicted patients continue to be followed (no discontinuations)

FIG. 28

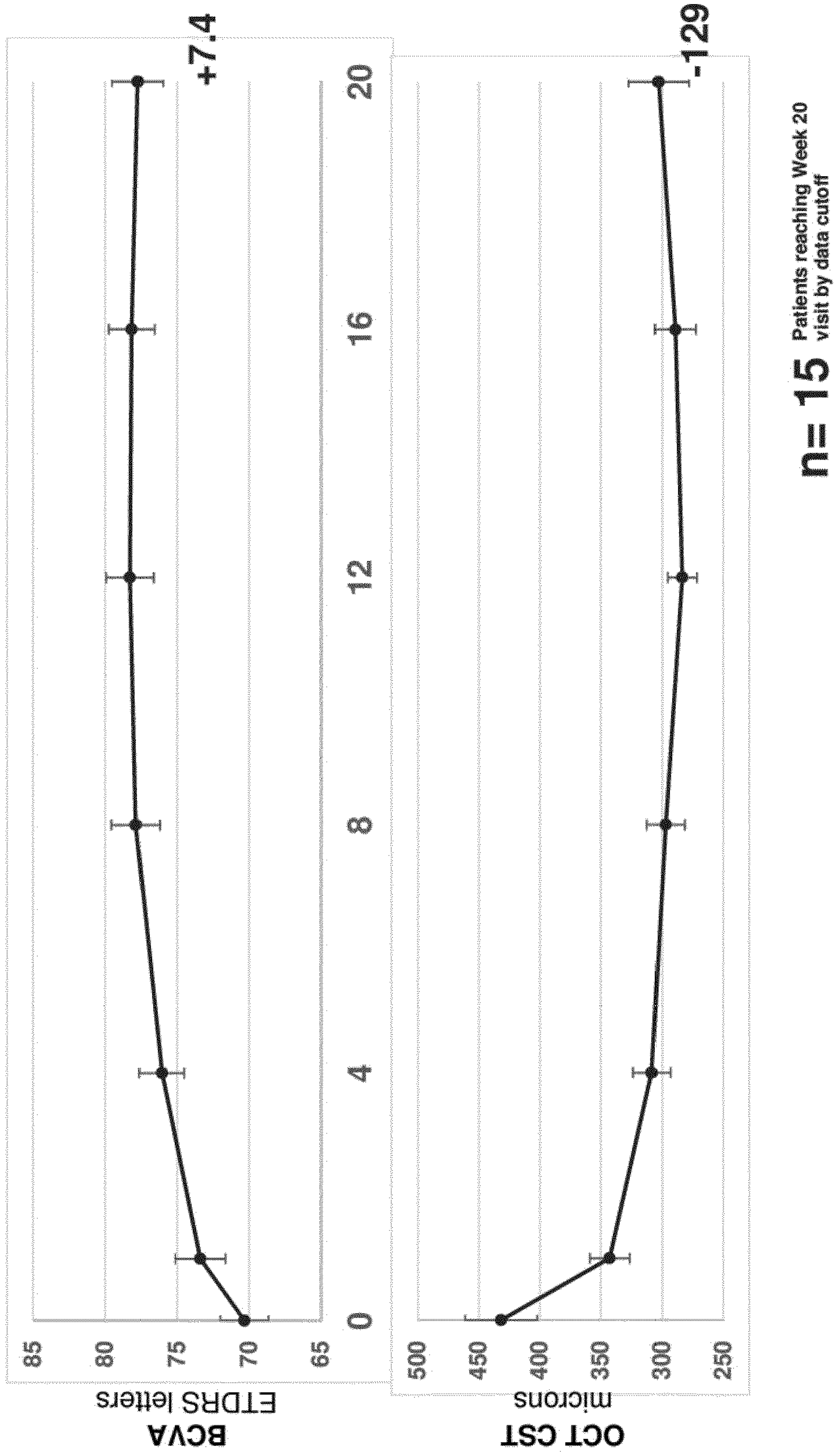


Interim data. Includes only randomized patients that reached Week 20 visit by the data cutoff date; 2.5 & 5 mg doses pooled. Error bars represent standard error of the mean. OCT CST values are site reported. BCVA= best corrected visual acuity; OCT= optical coherence tomography; CST= central subfield thickness

FIG. 29

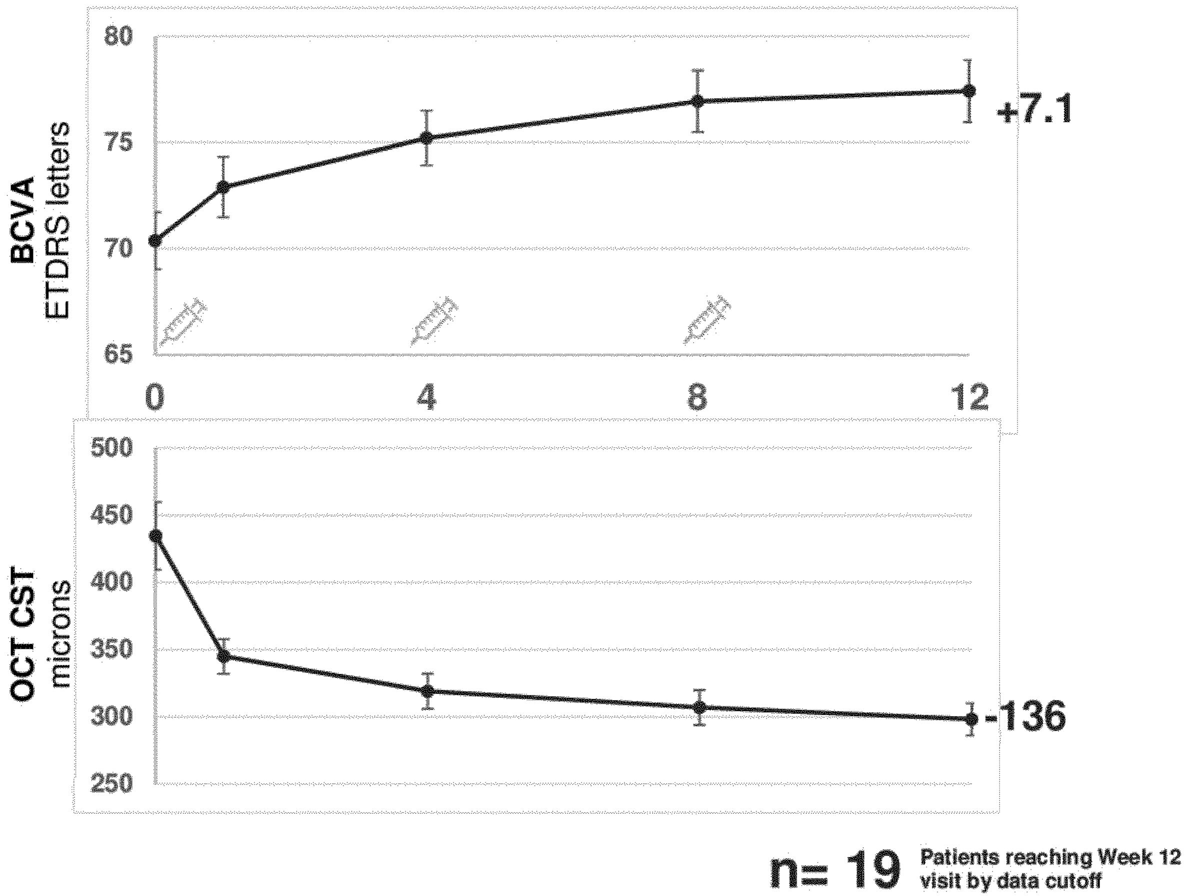


Interim data. Includes only randomized patients that reached Week 12 visit by the data cutoff date; 2.5 & 5 mg doses pooled. Error bars represent standard error of the mean. OCT CST values are site reported. BCVA= best corrected visual acuity; OCT= optical coherence tomography; CST= central subfield thickness

FIG. 30

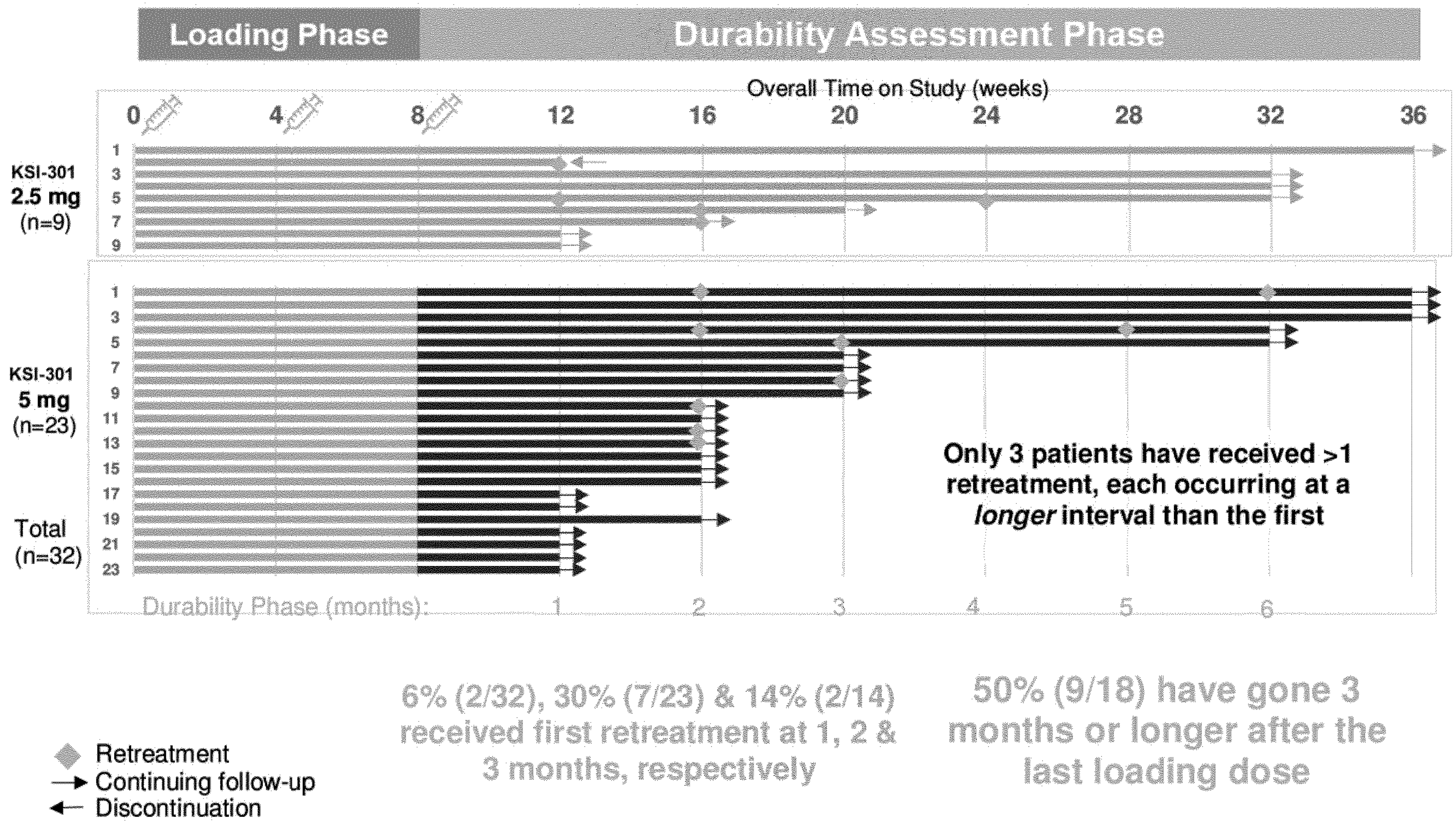


Interim data. Includes only randomized patients that reached the first retreatment opportunity (Week 12 visit) by the data cutoff date. Each bar represents an individual patient.

FIG. 31

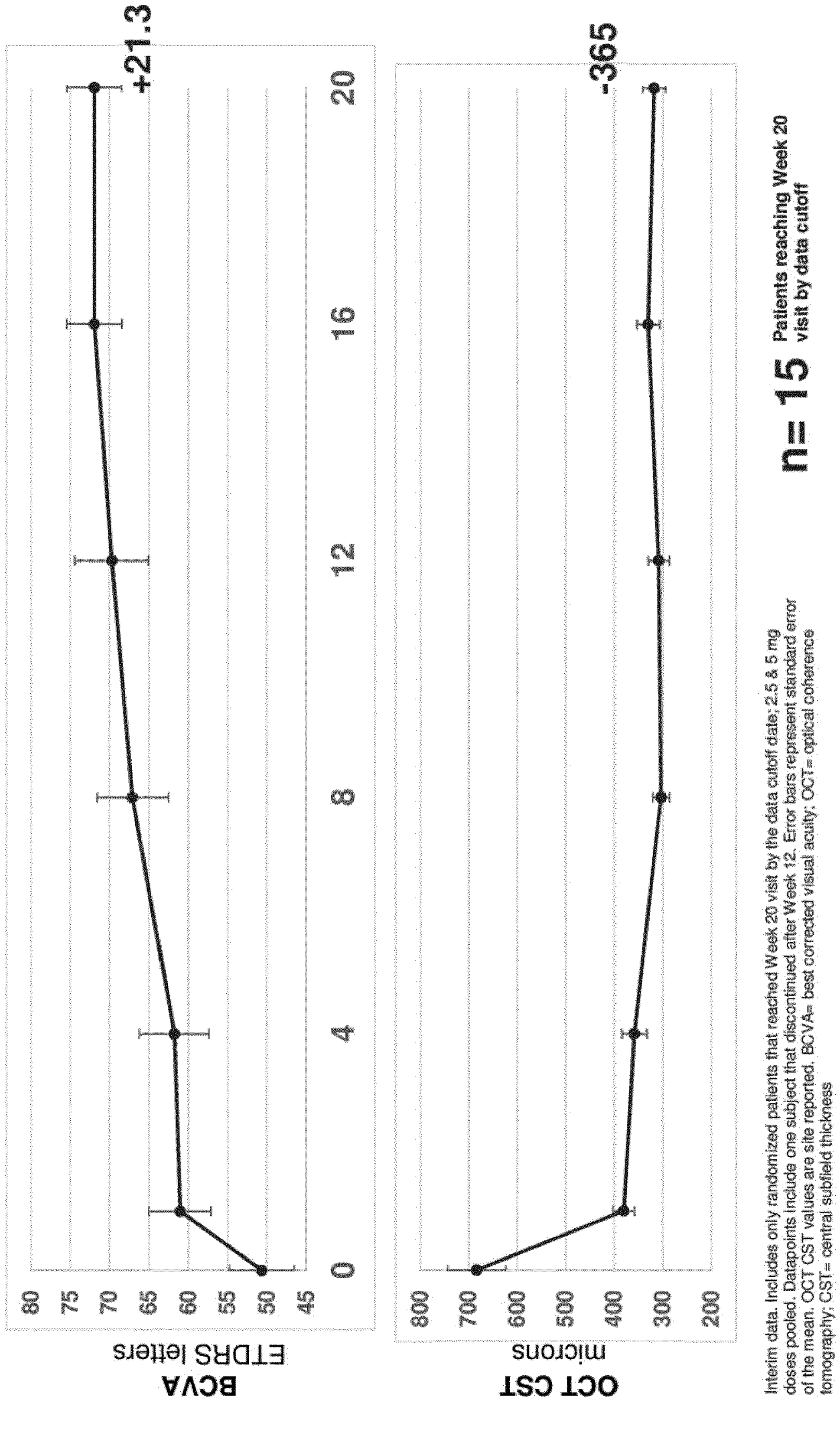


Interim data. Includes only randomized patients that reached Week 20 visit by the data cutoff date; 2.5 & 5 mg doses pooled. Datapoints include one subject that discontinued after Week 12. Error bars represent standard error of the mean. OCT CST values are site reported. BCVA= best corrected visual acuity; OCT= optical coherence tomography; CST= central subfield thickness

FIG. 32

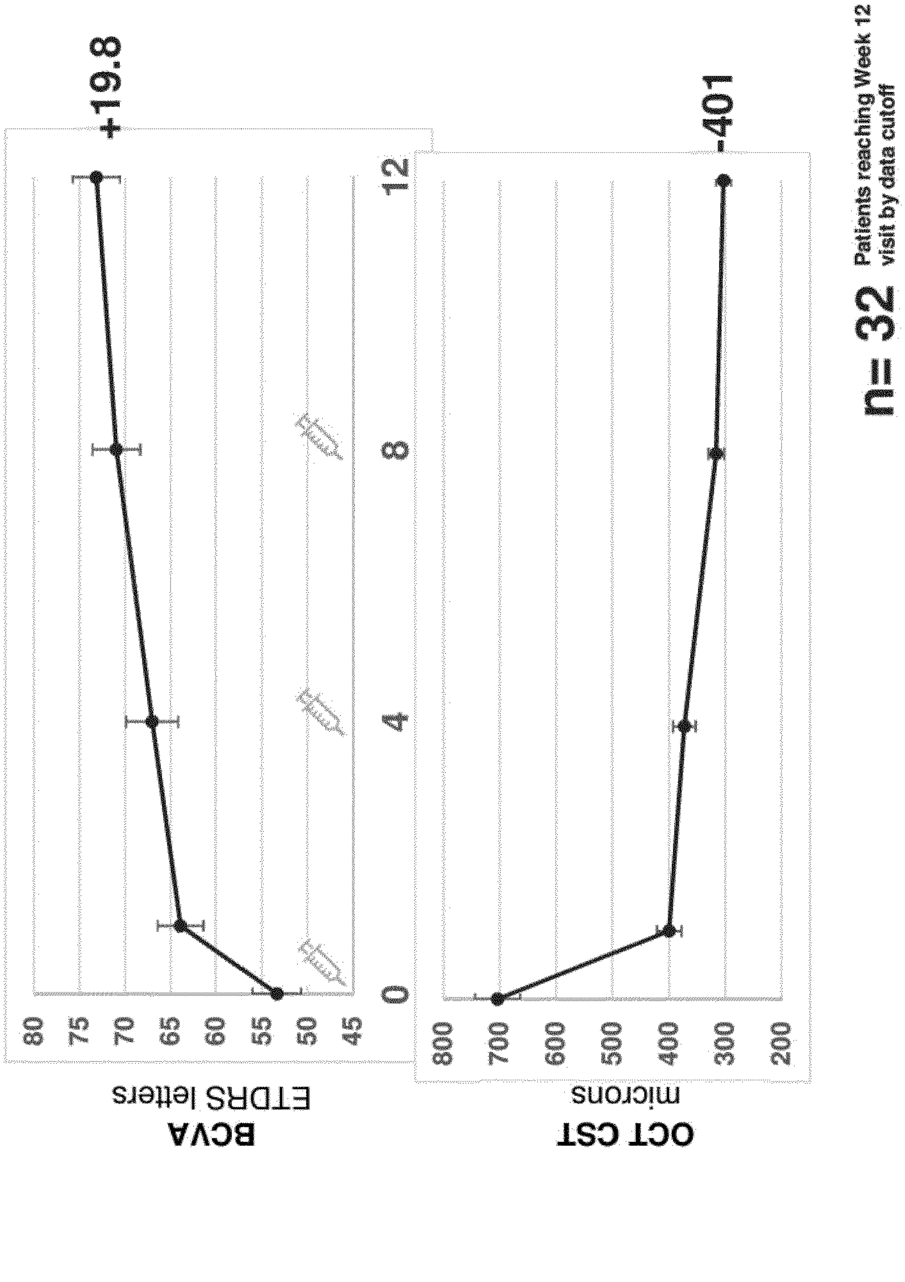


Interim data. Includes only randomized patients that reached Week 12 visit by the data cutoff date; 2.5 & 5 mg doses pooled. Error bars represent standard error of the mean. OCT CST values are site reported. BCVA= best corrected visual acuity; OCT= optical coherence tomography; CST= central subfield thickness

FIG. 33

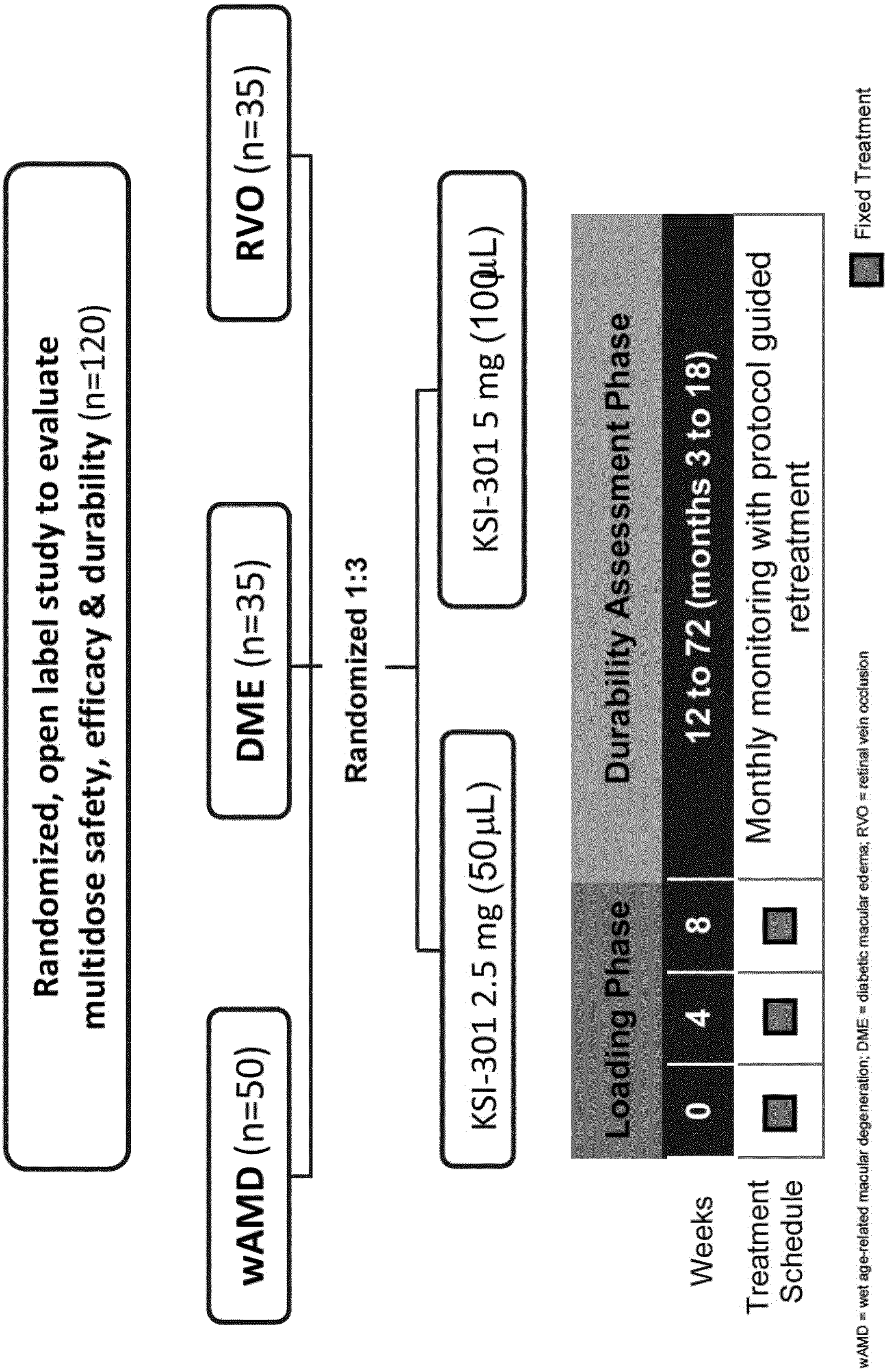
Randomized, open label study to evaluate multidose safety, efficacy & durability (n=120)
wAMD (n=50)
DME (n=35)
RVO (n=35)
Randomized 1:3
KSI-301 2.5 mg (50µL)
KSI-301 5 mg (100µL)
Weeks
Treatment Schedule
Loading Phase
0
4
8
Durability Assessment Phase
12 to 72 (months 3 to 18)
Monthly monitoring with protocol guided retreatment
Fixed Treatment
wAMD = wet age-related macular degeneration; DME = diabetic macular edema; RVO = retinal vein occlusion

FIG. 34

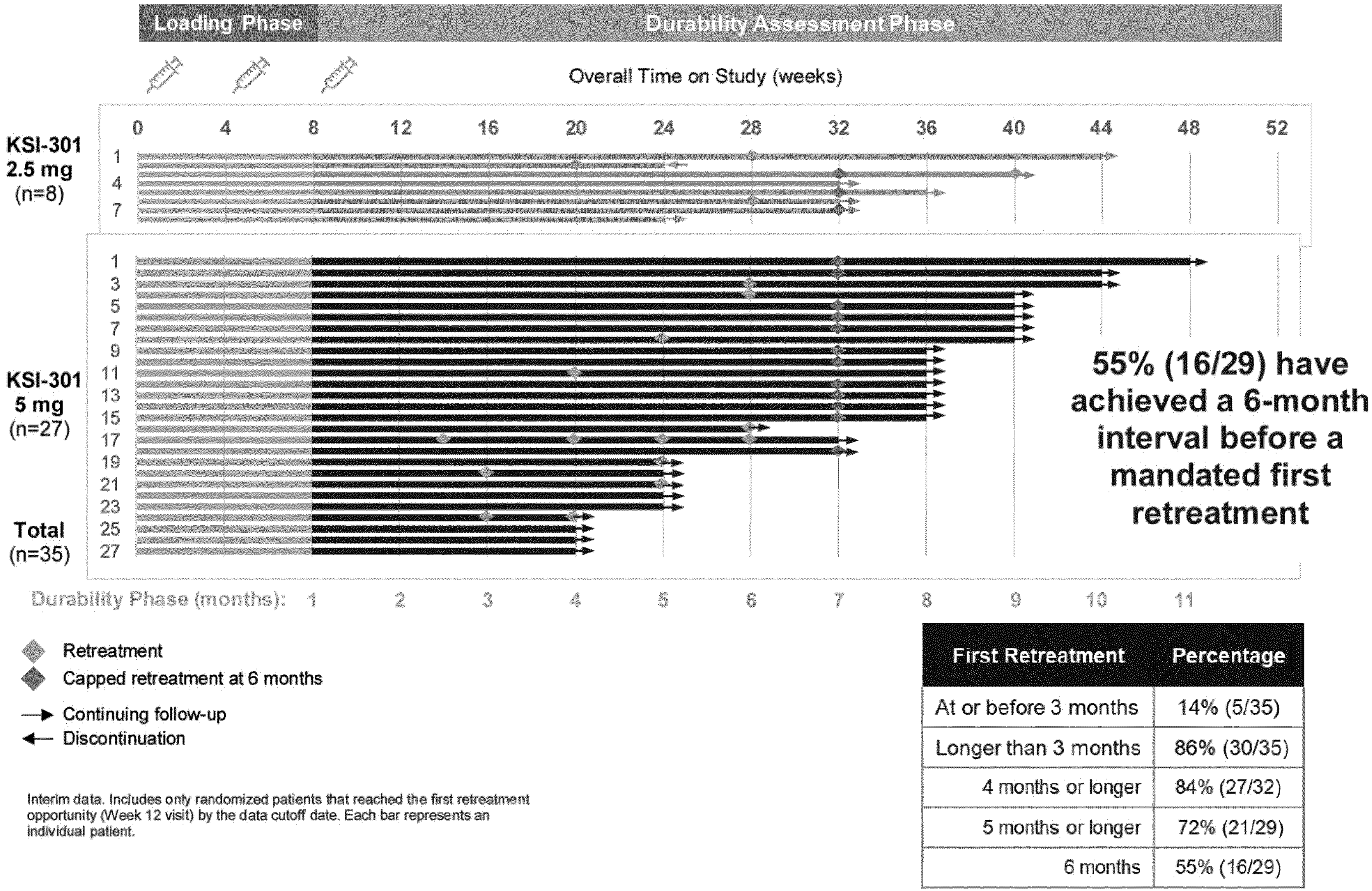


Interim data. Includes only randomized patients that reached the first retreatment opportunity (Week 12 visit) by the data cutoff date. Each bar represents an individual patient.

| First Retreatment | Percentage |
|---|---|
| At or before 3 months | 14% (5/35) |
| Longer than 3 months | 86% (30/35) |
| 4 months or longer | 84% (27/32) |
| 5 months or longer | 72% (21/29) |
| 6 months | 55% (16/29) |

FIG. 35

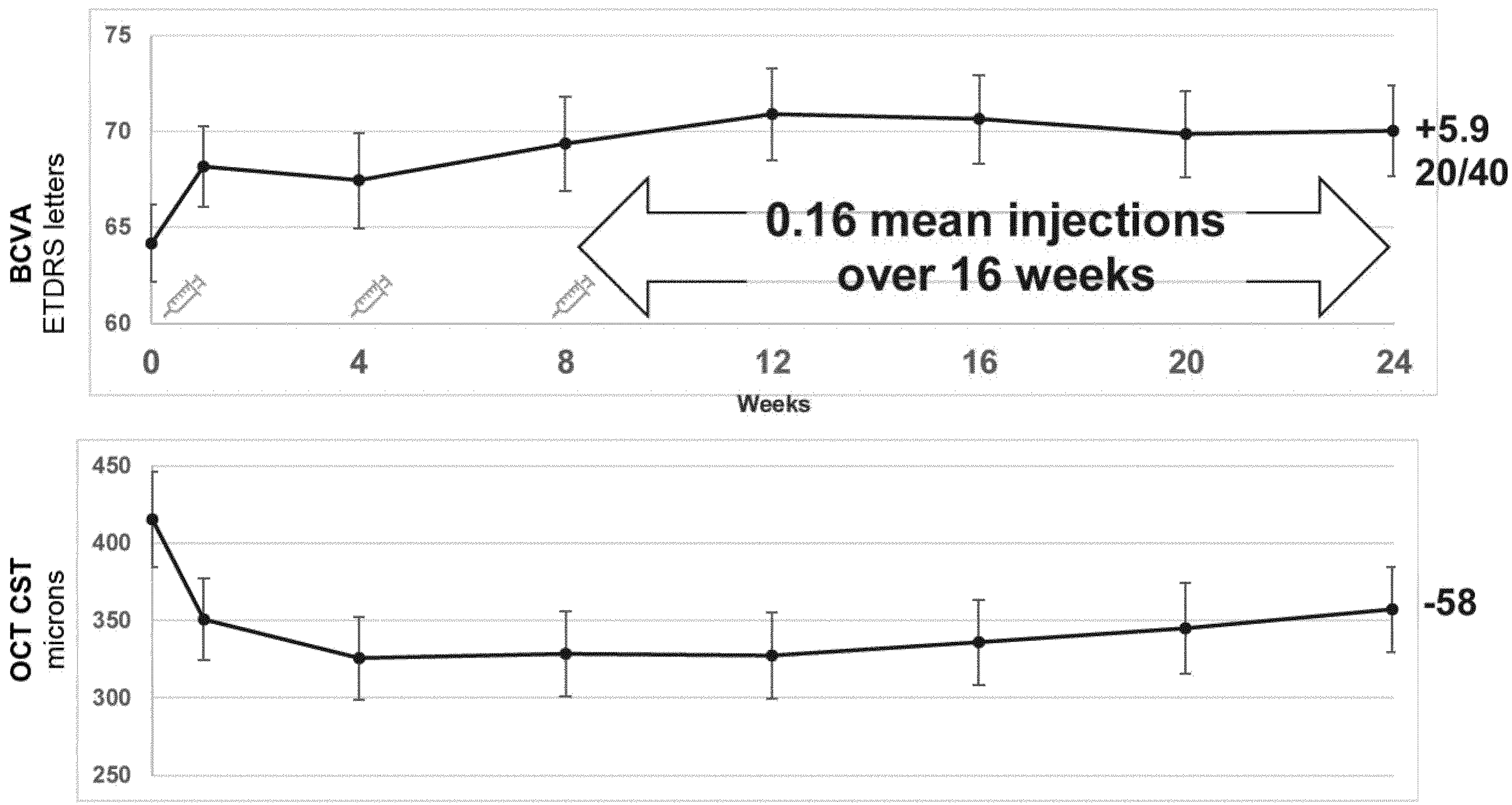


n= 31 Patients reaching Week 24 visit by data cutoff

Interim data. Includes only randomized patients that reached Week 24 visit by the data cutoff date; 2.5 & 5 mg doses pooled. Error bars represent standard error of the mean. OCT CST values are site reported and include PED height. BCVA= best corrected visual acuity; OCT= optical coherence tomography; CST= central subfield thickness. Mean injections reflect the average number of injections received per patient between Week 8 and 24 (compare to aflibercept and brolucizumab per label has mean number of injections 1.0).

FIG. 36

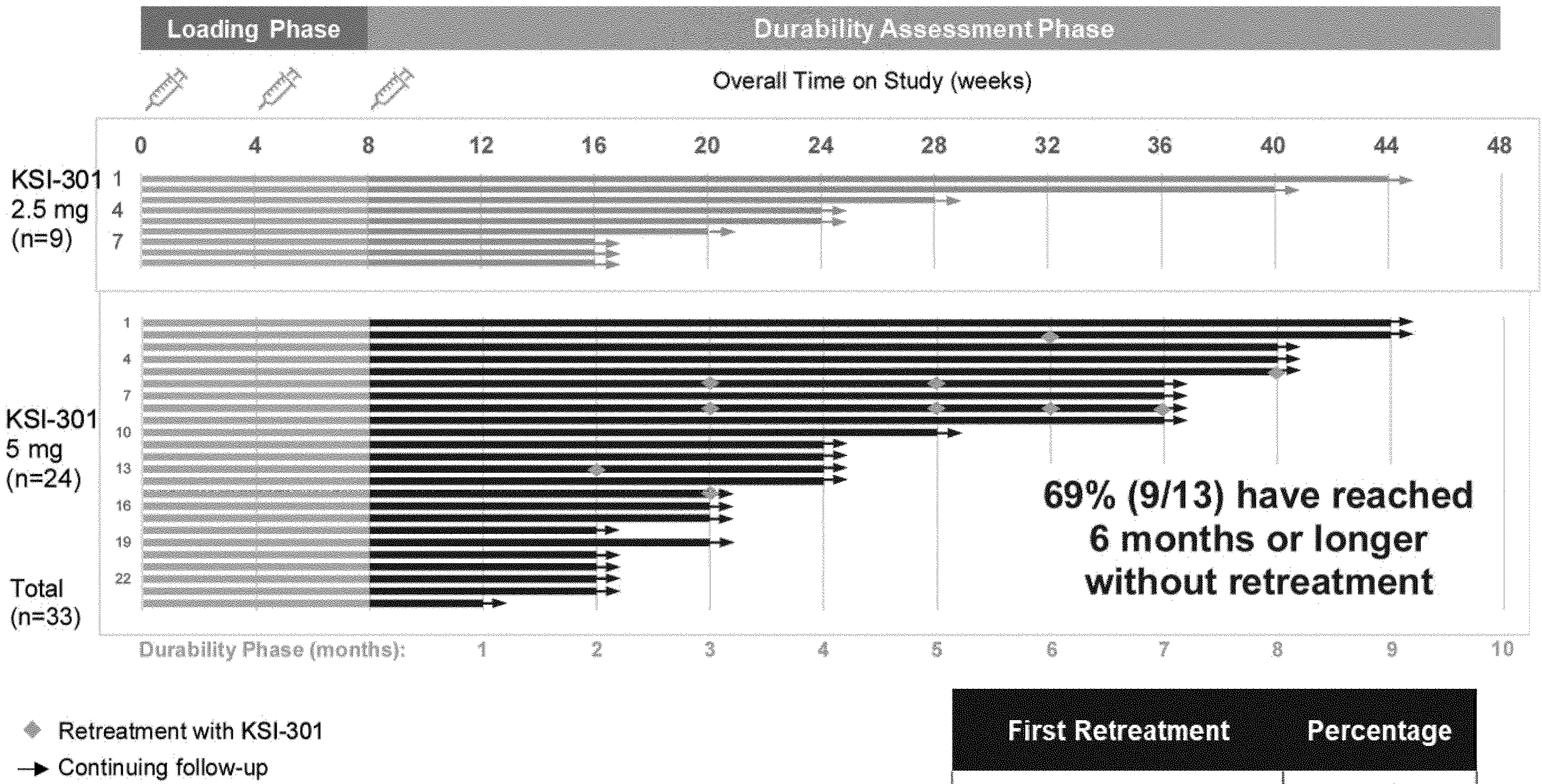


Interim data. Includes only randomized patients that reached the first retreatment opportunity (Week 12 visit) by the data cutoff date. Each bar represents an individual patient. All depicted patients continue to be followed (no discontinuations)

| First Retreatment | Percentage |
|---|---|
| At or before 3 months | 17% (4/24) |
| Longer than 3 months | 83% (20/24) |
| 4 months or longer | 80% (16/20) |
| 5 months or longer | 73% (11/15) |
| 6 months or longer | 69% (9/13) |

FIG. 37

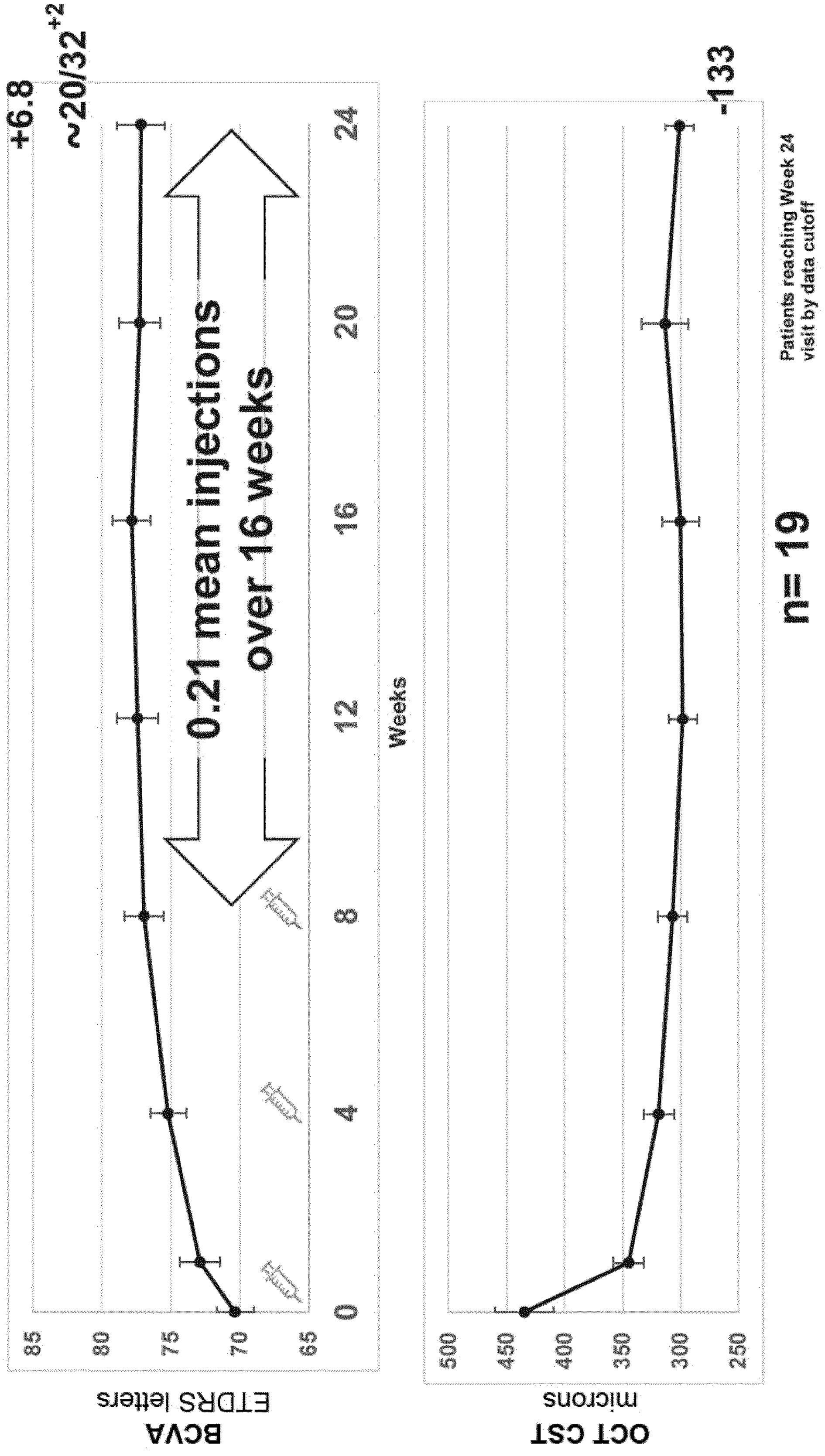


Interim data. Includes only randomized patients that reached Week 24 visit by the data cutoff date; 2.5 & 5 mg doses pooled. Error bars represent standard error of the mean. OCT CST values are site reported. BCVA= best corrected visual acuity; OCT= optical coherence tomography; CST= central subfield thickness. Mean injections reflect the average number of injections received per patient between Week 8 and 24 (compare to aflibercept per label and brolucizumab per pivotal study design has mean number of injections 2.0).

FIG. 38

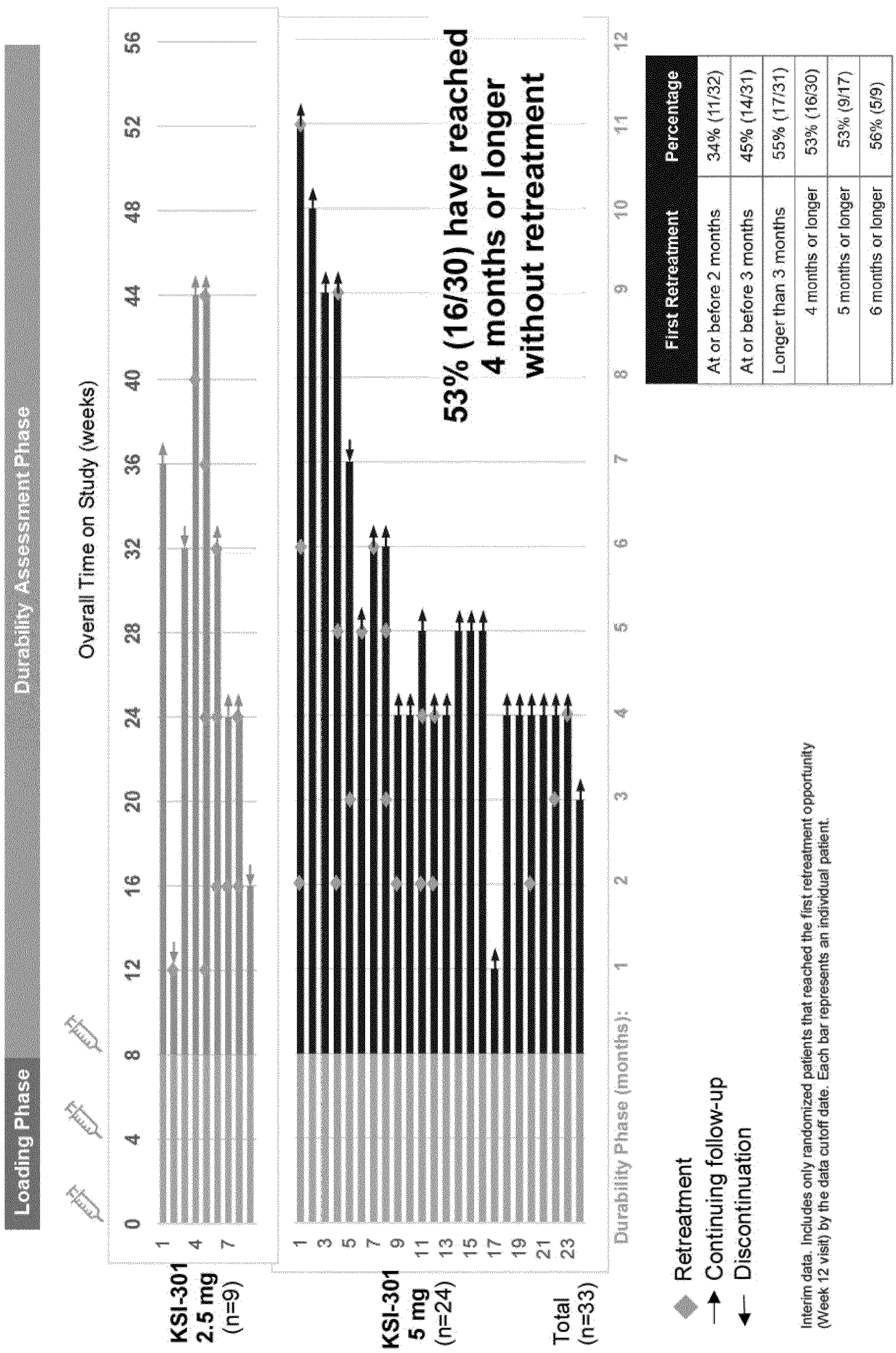


| First Retreatment | Percentage |
|---|---|
| At or before 2 months | 34% (11/32) |
| At or before 3 months | 45% (14/31) |
| Longer than 3 months | 55% (17/31) |
| 4 months or longer | 53% (16/30) |
| 5 months or longer | 53% (9/17) |
| 6 months or longer | 56% (5/9) |

FIG. 39

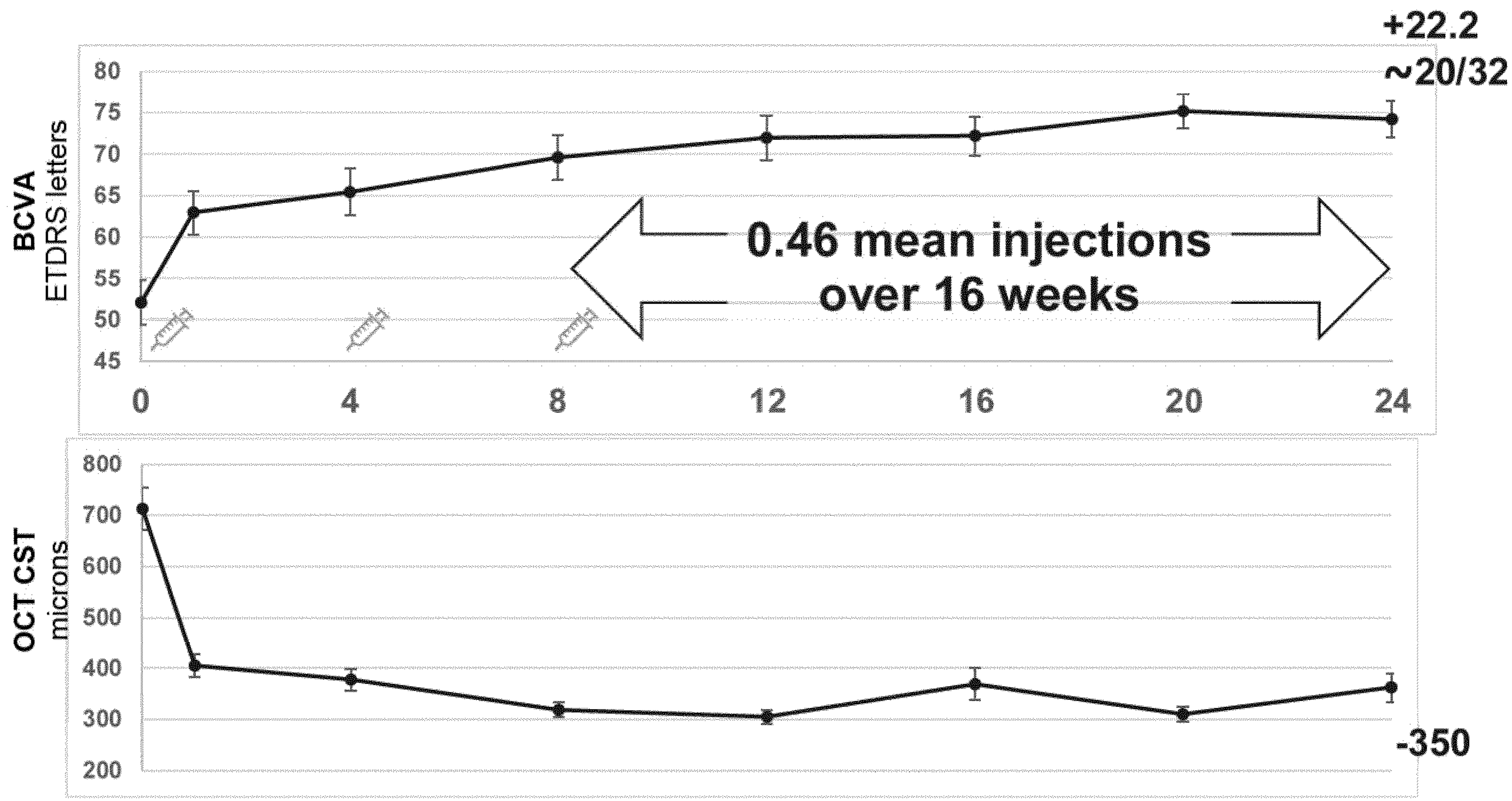


Interim data. Includes only randomized patients that reached Week 24 visit by the data cutoff date; 2.5 & 5 mg doses pooled. Datapoints include two subjects that discontinued after Week 12 – Observed data. Error bars represent standard error of the mean. OCT CST values are site reported. BCVA= best corrected visual acuity; OCT= optical coherence tomography; CST= central subfield thickness. Mean injections reflect the average number of injections received per patient between Week 8 and 24 (compare to aflibercept per label and brolucizumab per pivotal study design have mean number of injections 3.0).

**n= 30** Patients reaching Week 24 visit by data cutoff

**BRVO n= 17**
**CRVO n= 13**

FIG. 40

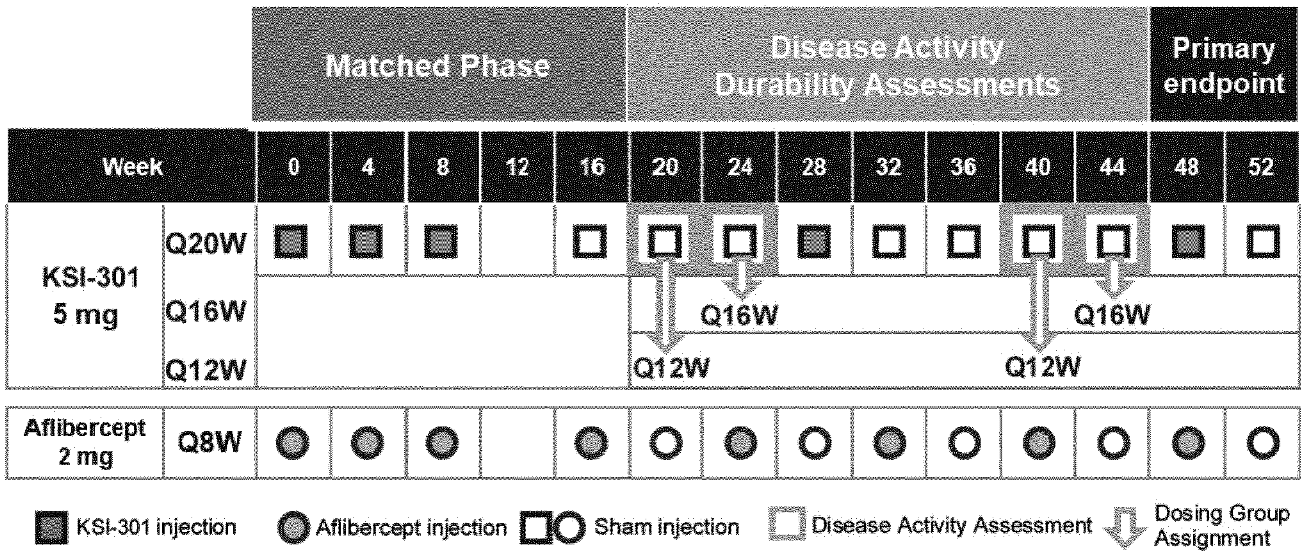


- ~550 treatment naïve wAMD patients
- Randomized study vs aflibercept
- US & EU study sites
- KSI-301dosing: every 12, 16, or 20 weeks depending on pre-specified disease activity assessments*

*After the loading phase

FIG. 41A

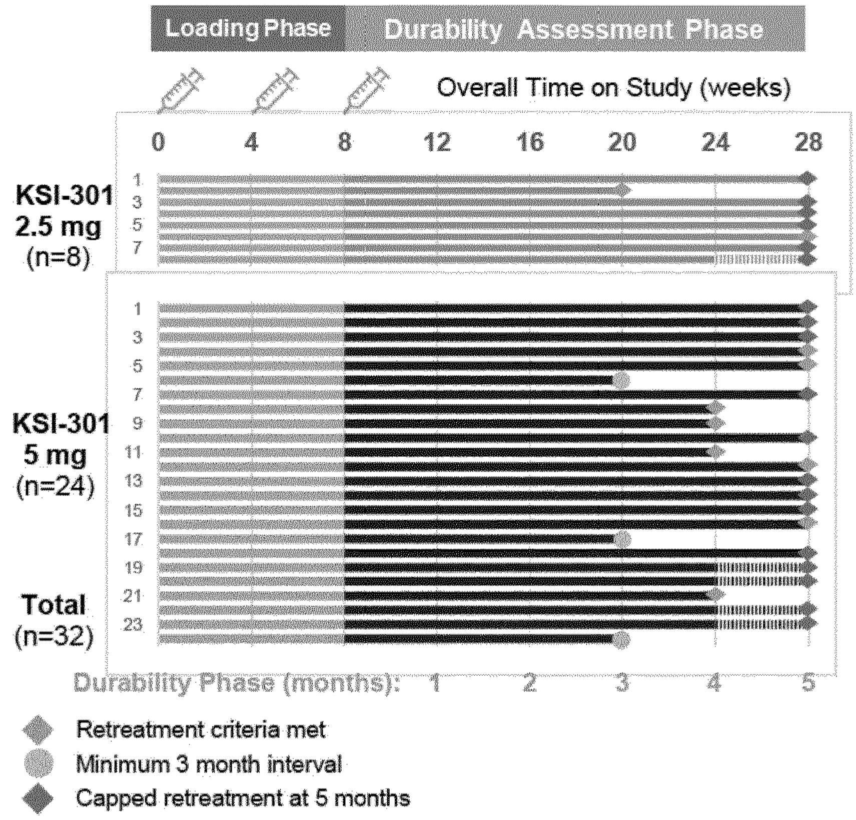


Interim data. Includes only randomized patients that would have met retreatment criteria before or at Week 28 by the data cutoff date. Each bar represents an individual patient.

FIG. 41B

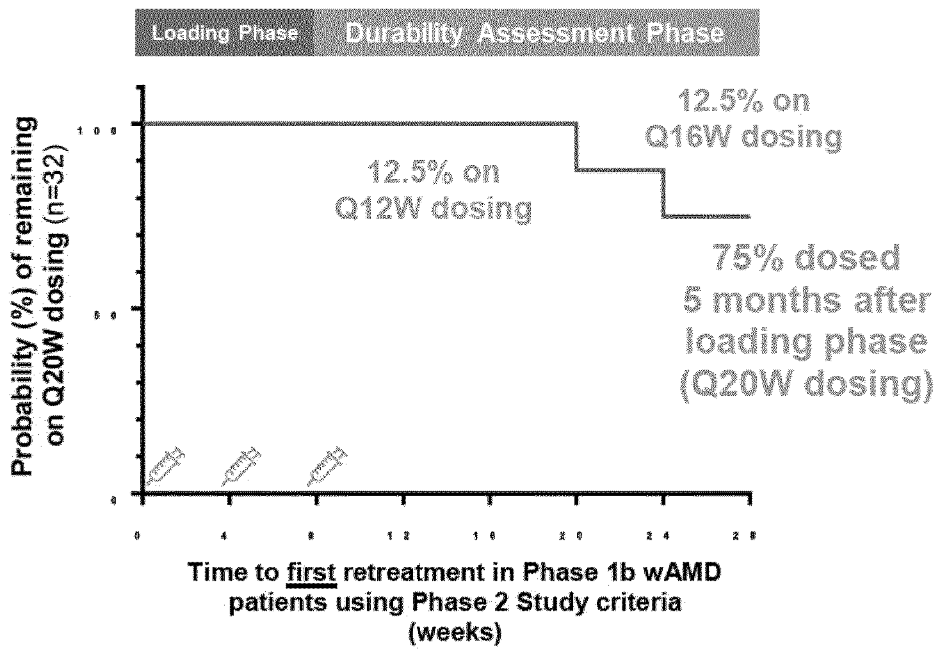


Interim data. Includes only randomized patients that would have met retreatment criteria before or at Week 28 by the data cutoff date. Each bar represents an individual patient.

FIG. 42

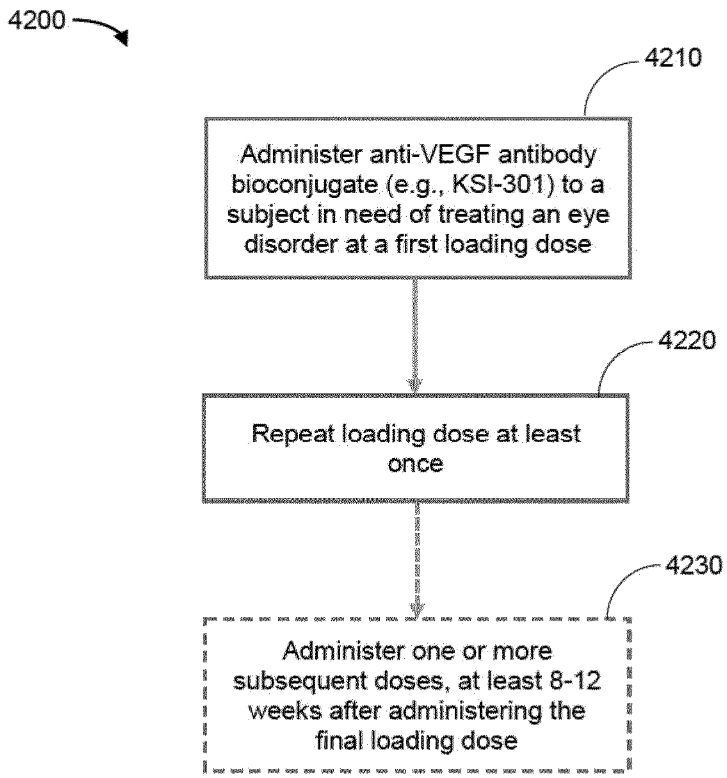
4200
4210
Administer anti-VEGF antibody bioconjugate (e.g., KSI-301) to a subject in need of treating an eye disorder at a first loading dose
4220
Repeat loading dose at least once
4230
Administer one or more subsequent doses, at least 8-12 weeks after administering the final loading dose

FIG. 43

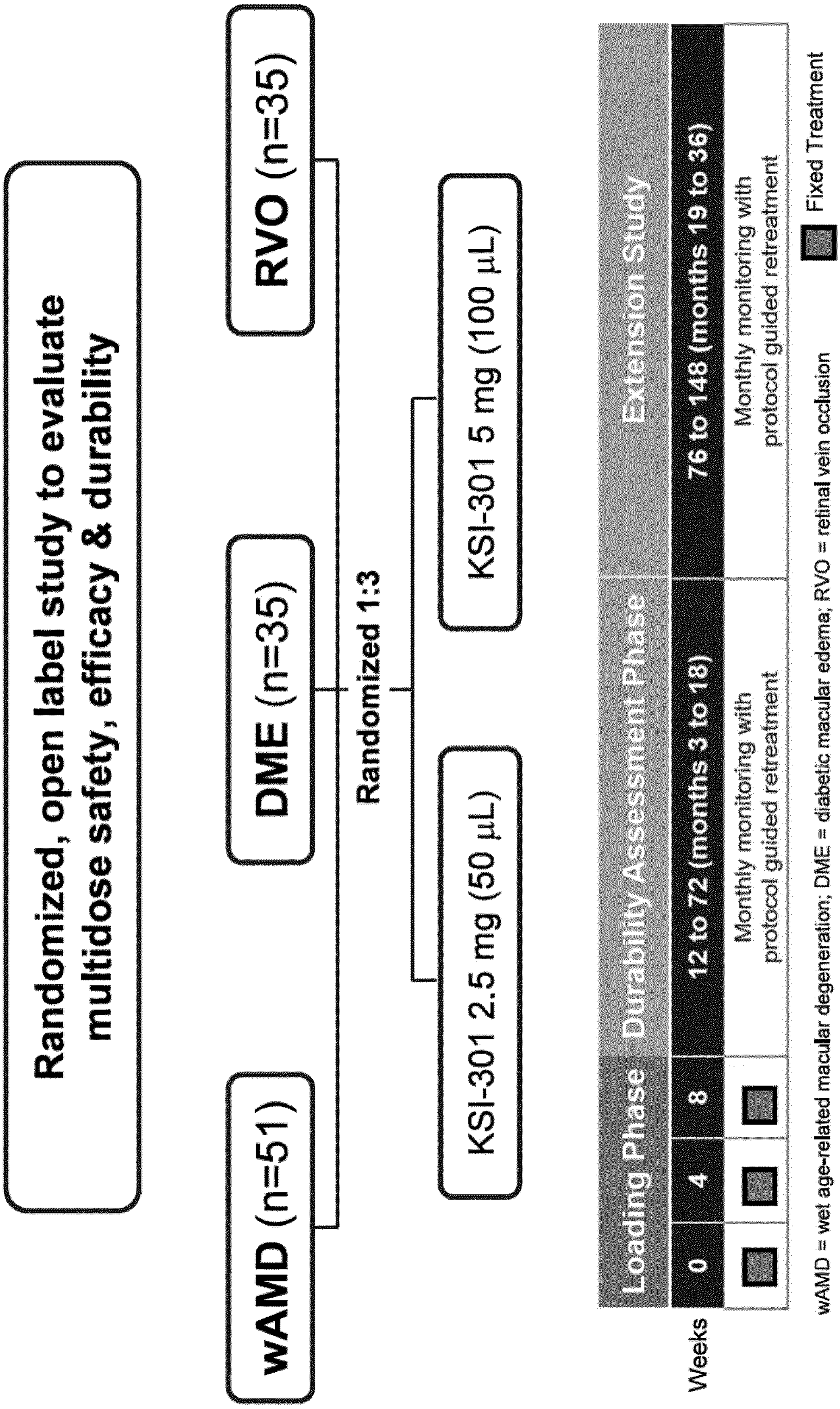

Randomized, open label study to evaluate multidose safety, efficacy & durability
wAMD (n=51)
DME (n=35)
RVO (n=35)
Randomized 1:3
KSI-301 2.5 mg (50 μL)
KSI-301 5 mg (100 μL)
Weeks
Loading Phase
0
4
8
Durability Assessment Phase
12 to 72 (months 3 to 18)
Monthly monitoring with protocol guided retreatment
Extension Study
76 to 148 (months 19 to 36)
Monthly monitoring with protocol guided retreatment
wAMD = wet age-related macular degeneration; DME = diabetic macular edema; RVO = retinal vein occlusion
Fixed Treatment

FIG. 44

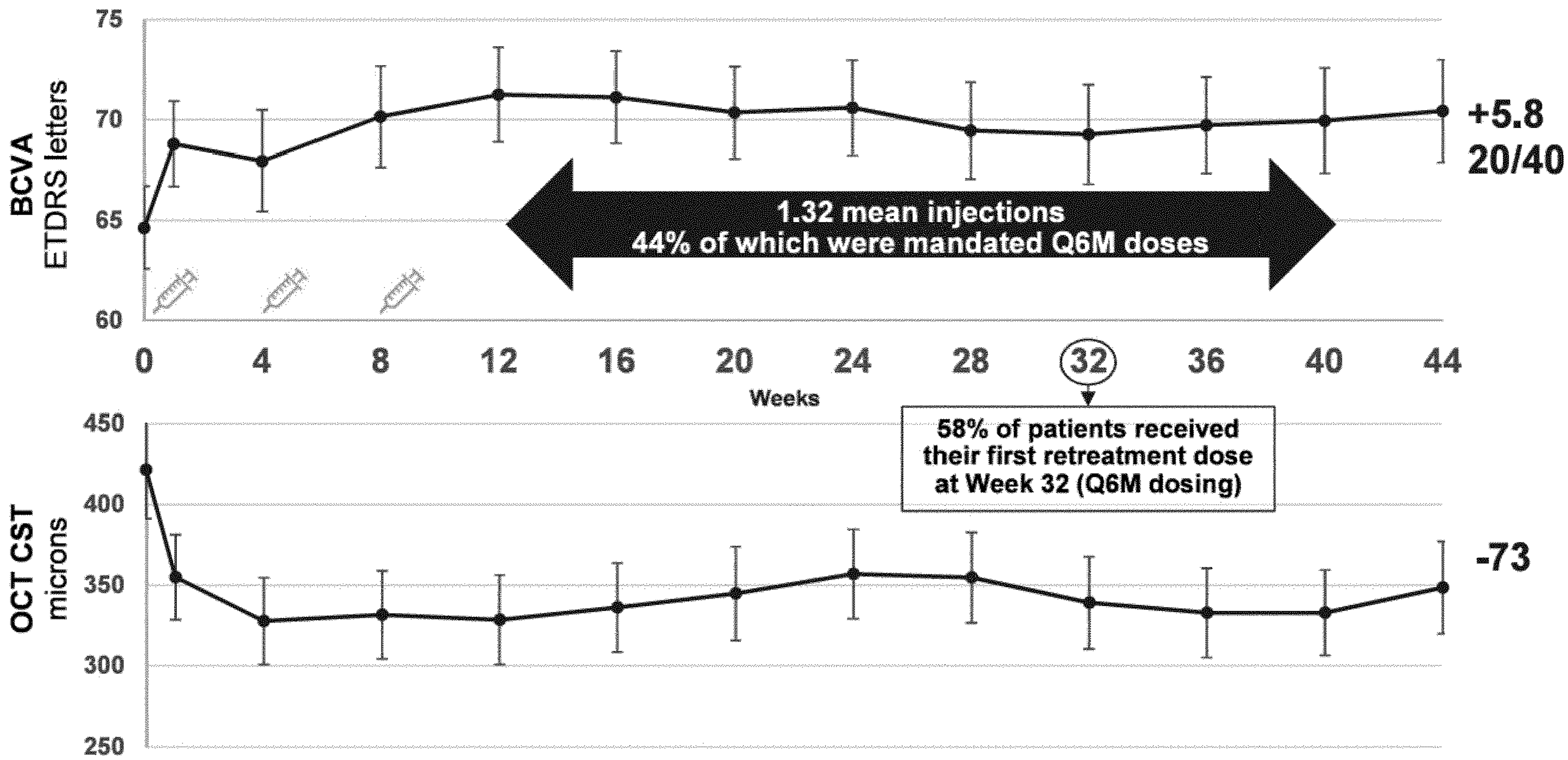


Interim data. Includes only randomized patients that reached Week 44 visit by the data cutoff date; 2.5 & 5 mg doses pooled. Observed data. Error bars represent standard error of the mean. OCT CST values are site reported and include PED height. BCVA= best corrected visual acuity; OCT= optical coherence tomography; CST= central subfield thickness. Mean injections reflect the average number of injections received per patient between Week 12 and 40 (aflibercept per label mean number of injections 4.0).

**n= 31** Patients reaching Week 44 visit by data cutoff

FIG. 45A

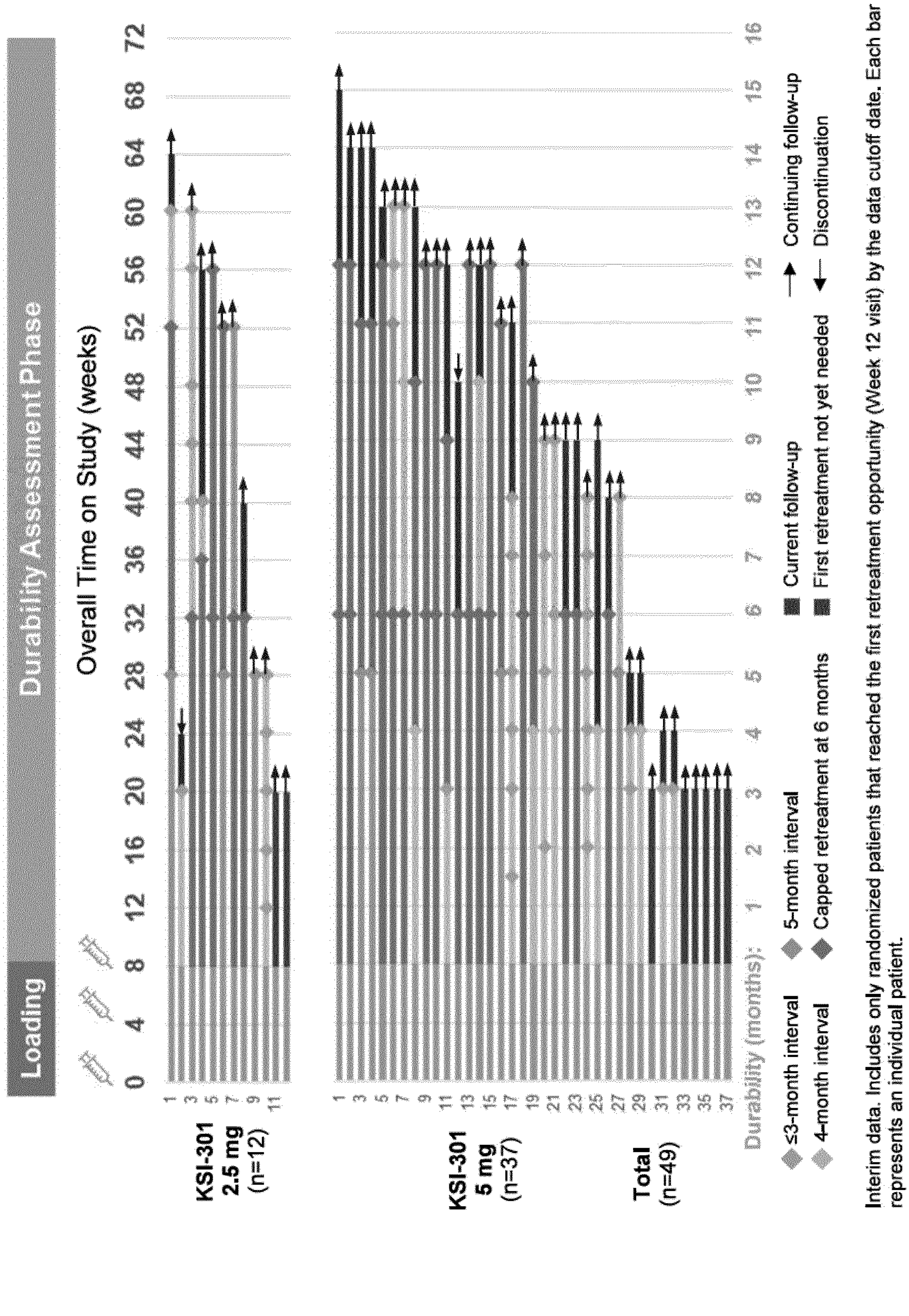
Loading
Durability Assessment Phase
Overall Time on Study (weeks)
0
4
8
12
16
20
24
28
32
36
40
44
48
52
56
60
64
68
72
KSI-301
2.5 mg
(n=12)
KSI-301
5 mg
(n=37)
Total
(n=49)
Durability (months):
≤3-month interval
4-month interval
5-month interval
Capped retreatment at 6 months
Current follow-up
First retreatment not yet needed
Continuing follow-up
Discontinuation
Interim data. Includes only randomized patients that reached the first retreatment opportunity (Week 12 visit) by the data cutoff date. Each bar represents an individual patient.

FIG. 45B

| First Retreatment | Percentage |
|---|---|
| At or before 2 months | 8% (4/49) |
| 3 months or longer | 92% (45/49) |
| 4 months or longer | 82% (40/49) |
| 5 months or longer | 66% (27/41) |
| 6 months | 49% (20/41) |

**72% have achieved a 6-month treatment interval at least once during follow-up**

FIG. 46

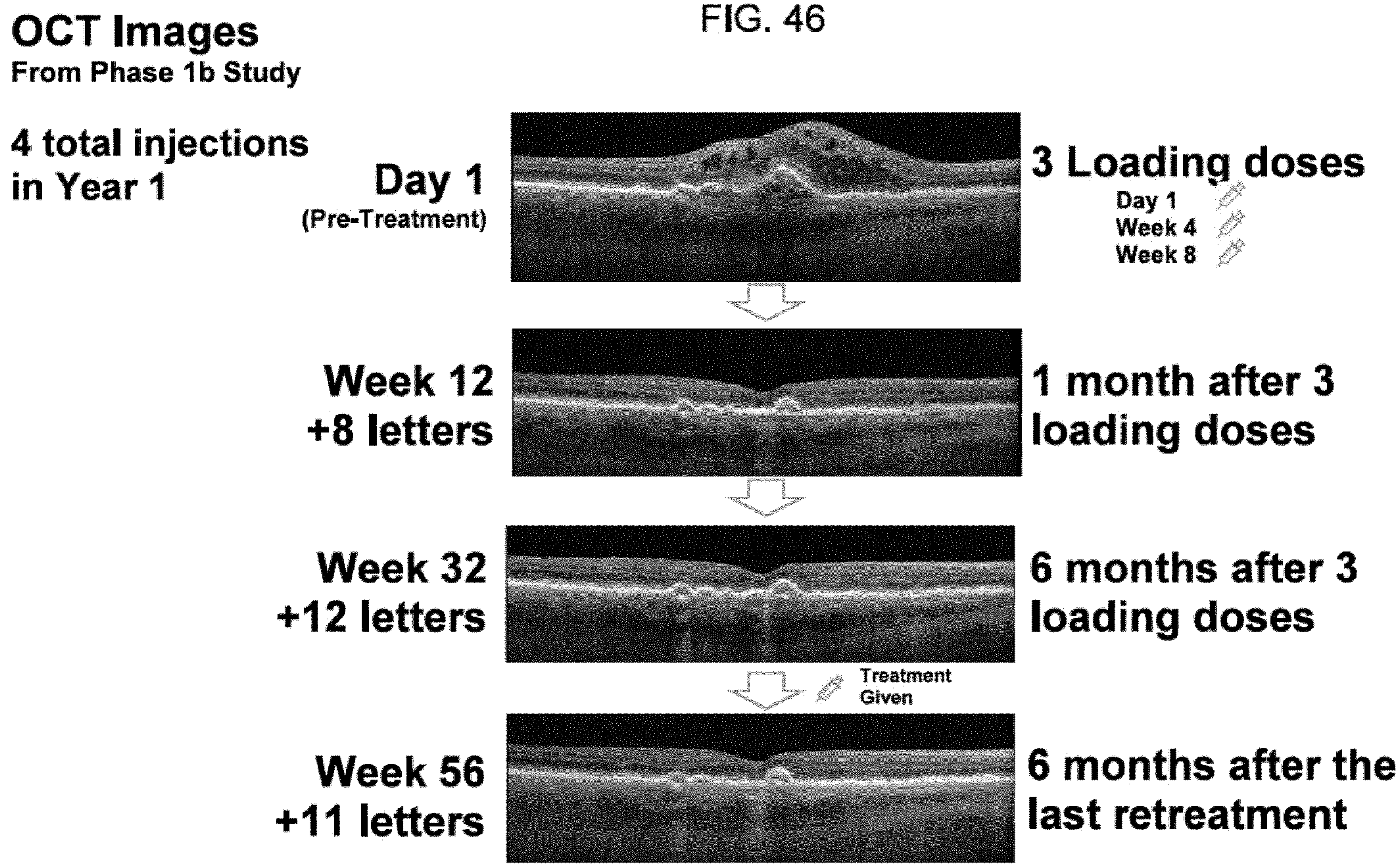
OCT Images
From Phase 1b Study
4 total injections
in Year 1
Day 1
(Pre-Treatment)
3 Loading doses
Day 1
Week 4
Week 8
Week 12
+8 letters
1 month after 3
loading doses
Week 32
+12 letters
6 months after 3
loading doses
Treatment
Given
Week 56
+11 letters
6 months after the
last retreatment

FIG. 47

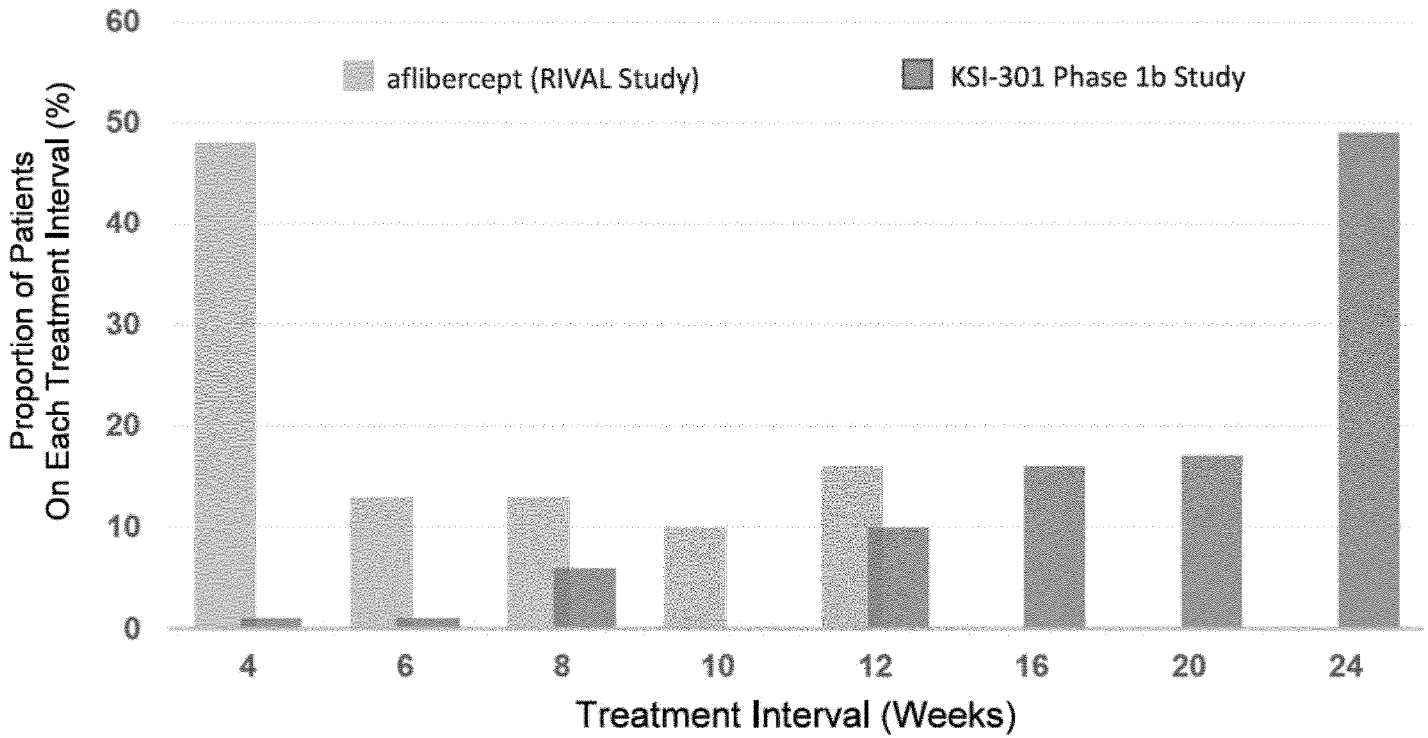


1. Gillies MC, et al. Effect of Ranibizumab and Aflibercept on Best-Corrected Visual Acuity in Treat-and-Extend for Neovascular Age-Related Macular Degeneration: A Randomized Clinical Trial. JAMA Ophthalmol. 2019;137(4):372–379. doi:10.1001/jamaophthalmol.2018.6776
2. For KSI-301: Includes randomized patients that reached the first retreatment opportunity (Week 12 visit) by the data cutoff date.

FIG. 48

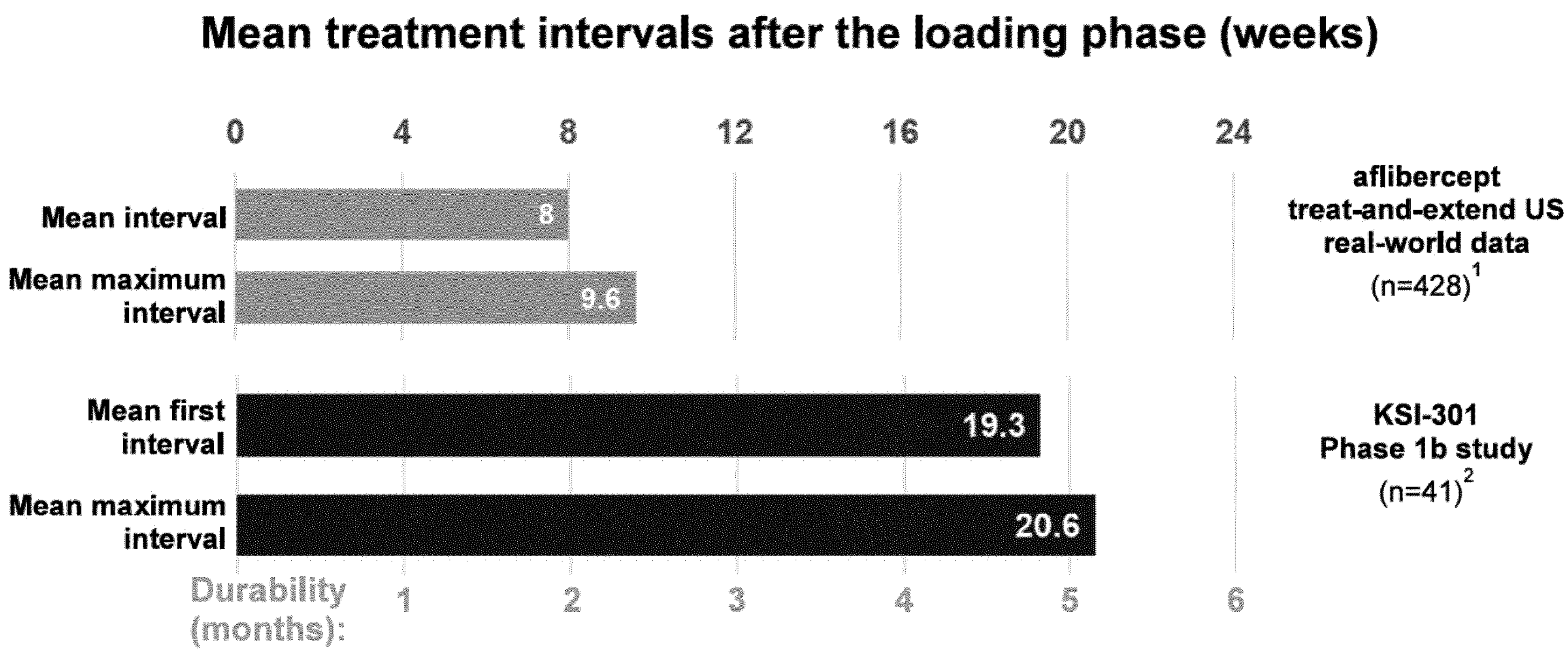


1. Singer MA, et al. Two-Year Real-World Treat and Extend Patterns and Fluid Outcomes Among Neovascular Age-Related Macular Degeneration Patients Treated With Anti-VEGFs. ASRS 2020 virtual meeting. Available at asrs.org. 2. Includes all randomized patients that received all three loading doses and a first retreatment by the data cutoff date. For Eylea data set, mean interval is the average interval per patient over two years, and mean maximum interval is the average of the longest interval achieved per patient at any point during follow-up. For KSI-301 data set, first interval refers to the first retreatment, and mean maximum interval is the average of the longest interval per patient at any point during follow-up.

FIG. 49

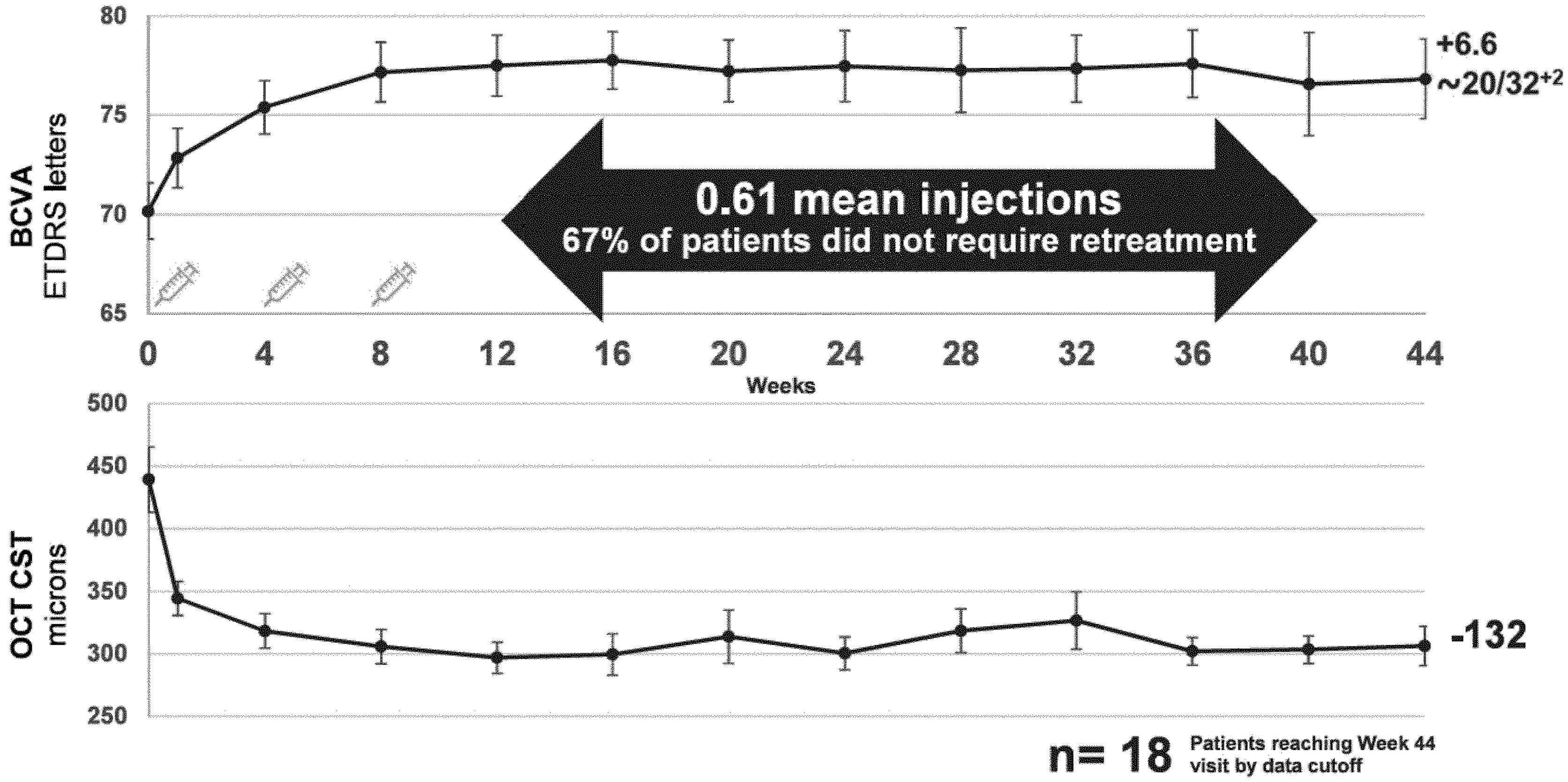


Interim data. Includes only randomized patients that reached Week 44 visit by the data cutoff date; 2.5 & 5 mg doses pooled. Observed data. Error bars represent standard error of the mean. OCT CST values are site reported. BCVA= best corrected visual acuity; OCT= optical coherence tomography; CST= central subfield thickness. Mean injections reflect the average number of injections received per patient between Week 12 and 40 (aflibercept per label mean number of injections 5.0).

FIG. 50A

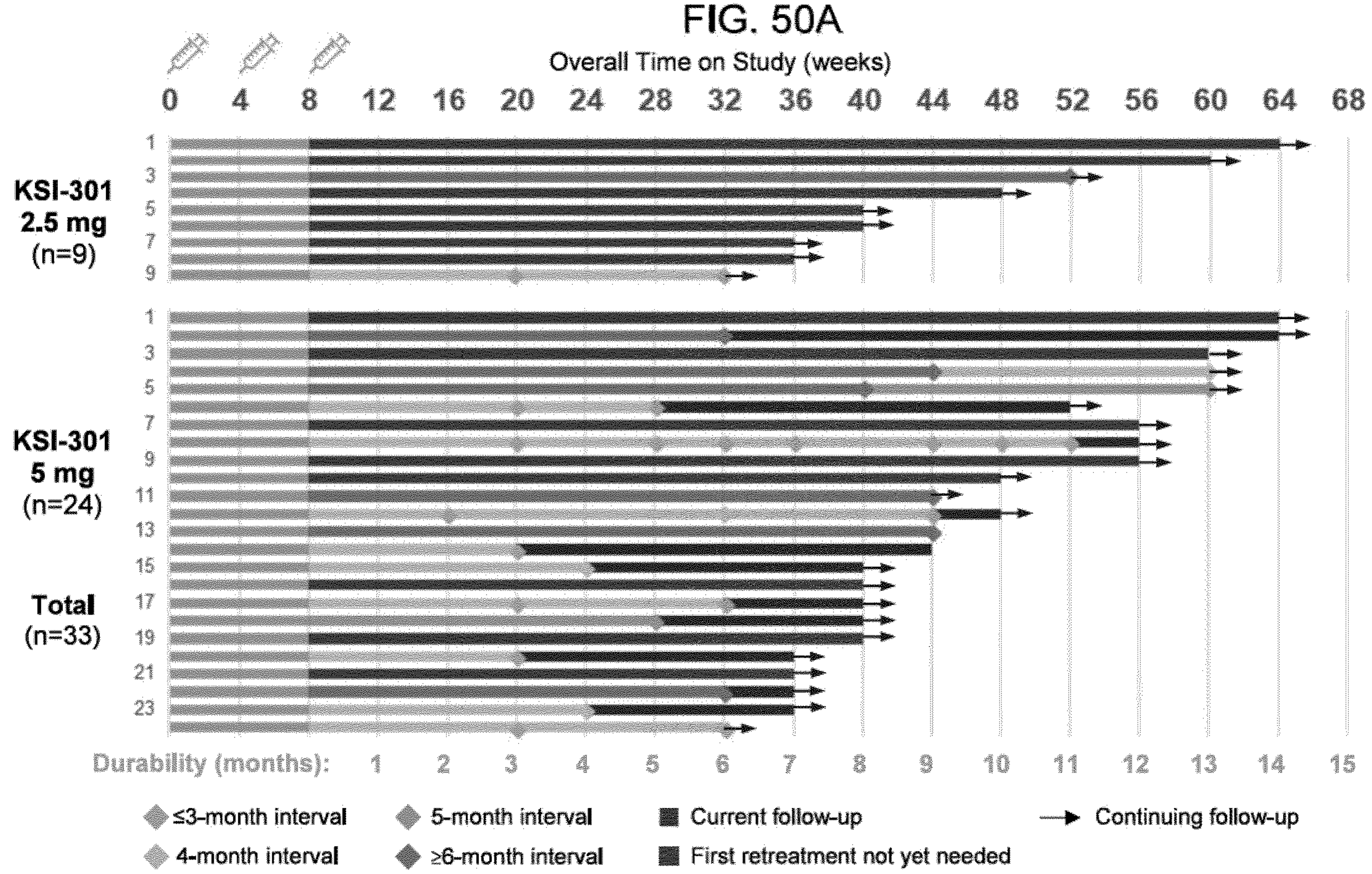


Interim data. Includes only randomized patients that reached the first retreatment opportunity (Week 12 visit) by the data cutoff date. Each bar represents an individual patient. All depicted patients continue to be followed (no discontinuations)

FIG. 50B

| First Retreatment | Percentage |
|---|---|
| Before 2 months | 0% (0/33) |
| At 2 months | 3% (1/33) |
| 3 months or longer | 97% (32/33) |
| 4 months or longer | 76% (25/33) |
| 5 months or longer | 70% (23/33) |
| 6 months or longer | 67% (22/33) |

**45% (15/33) have not yet required a single retreatment; 79% have achieved a 6-month treatment interval at least once during follow-up**

FIG. 51

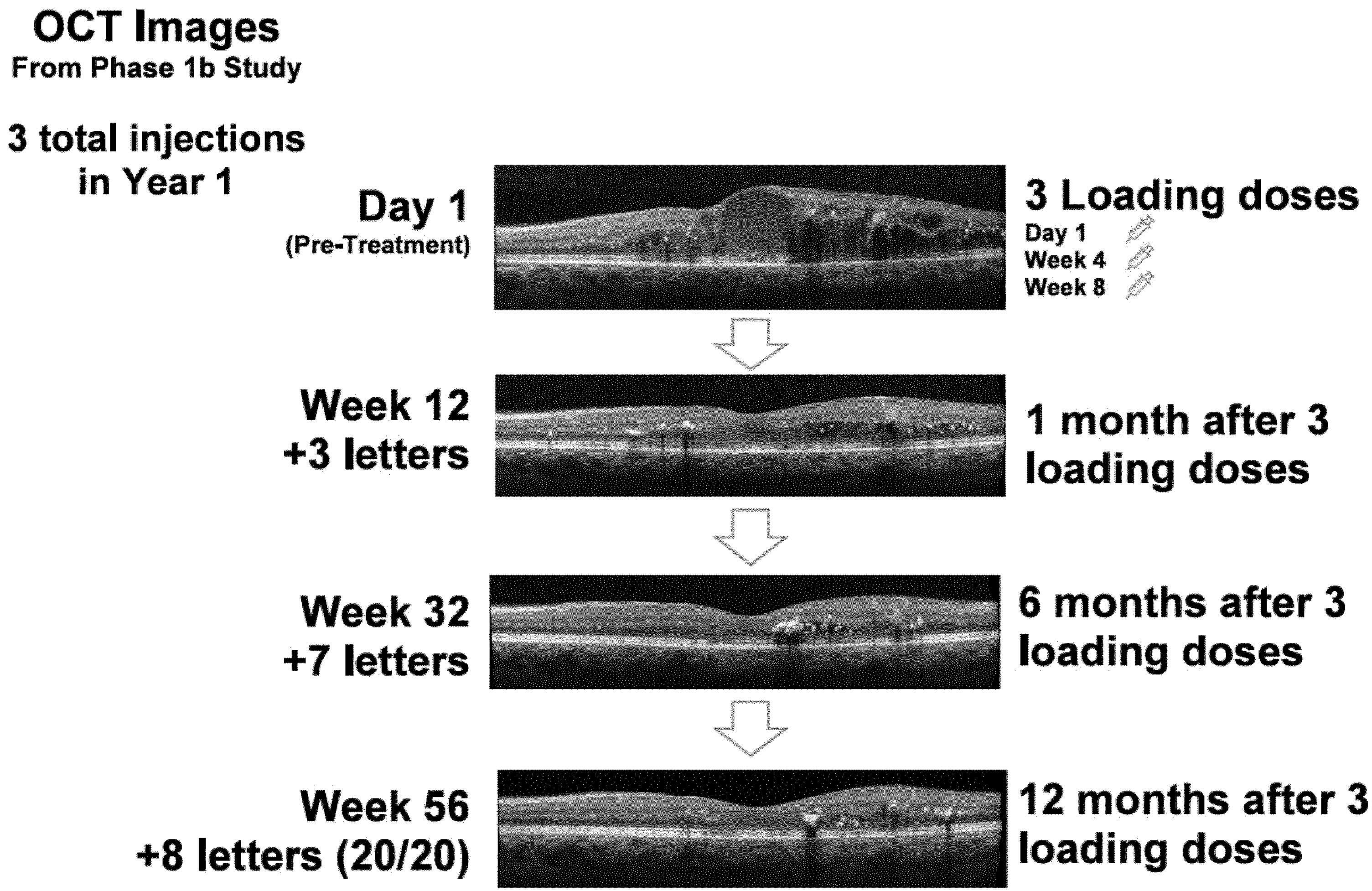
OCT Images
From Phase 1b Study
3 total injections in Year 1
Day 1
(Pre-Treatment)
3 Loading doses
Day 1
Week 4
Week 8
Week 12
+3 letters
1 month after 3 loading doses
Week 32
+7 letters
6 months after 3 loading doses
Week 56
+8 letters (20/20)
12 months after 3 loading doses

FIG. 52

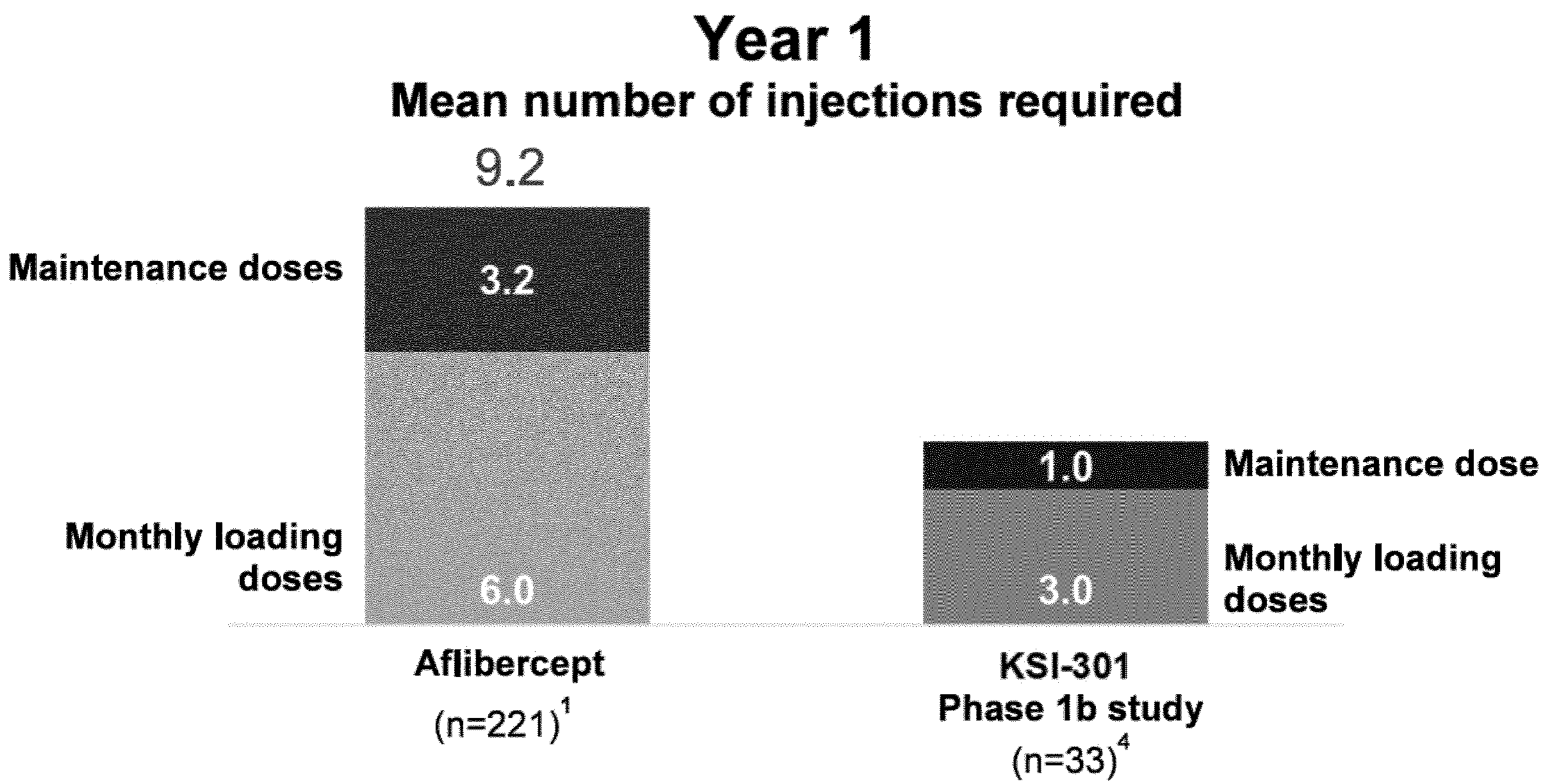


1. Wells JA. Aflibercept, bevacizumab, or ranibizumab for diabetic macular edema (DRCR Protocol T). N Engl J Med. 2015 Mar 26;372(13):1193-203 (supplemental data).
2. Interim data. Annualized injections based on the current monthly injection rate of all DME patients as of the data cutoff.

FIG. 53

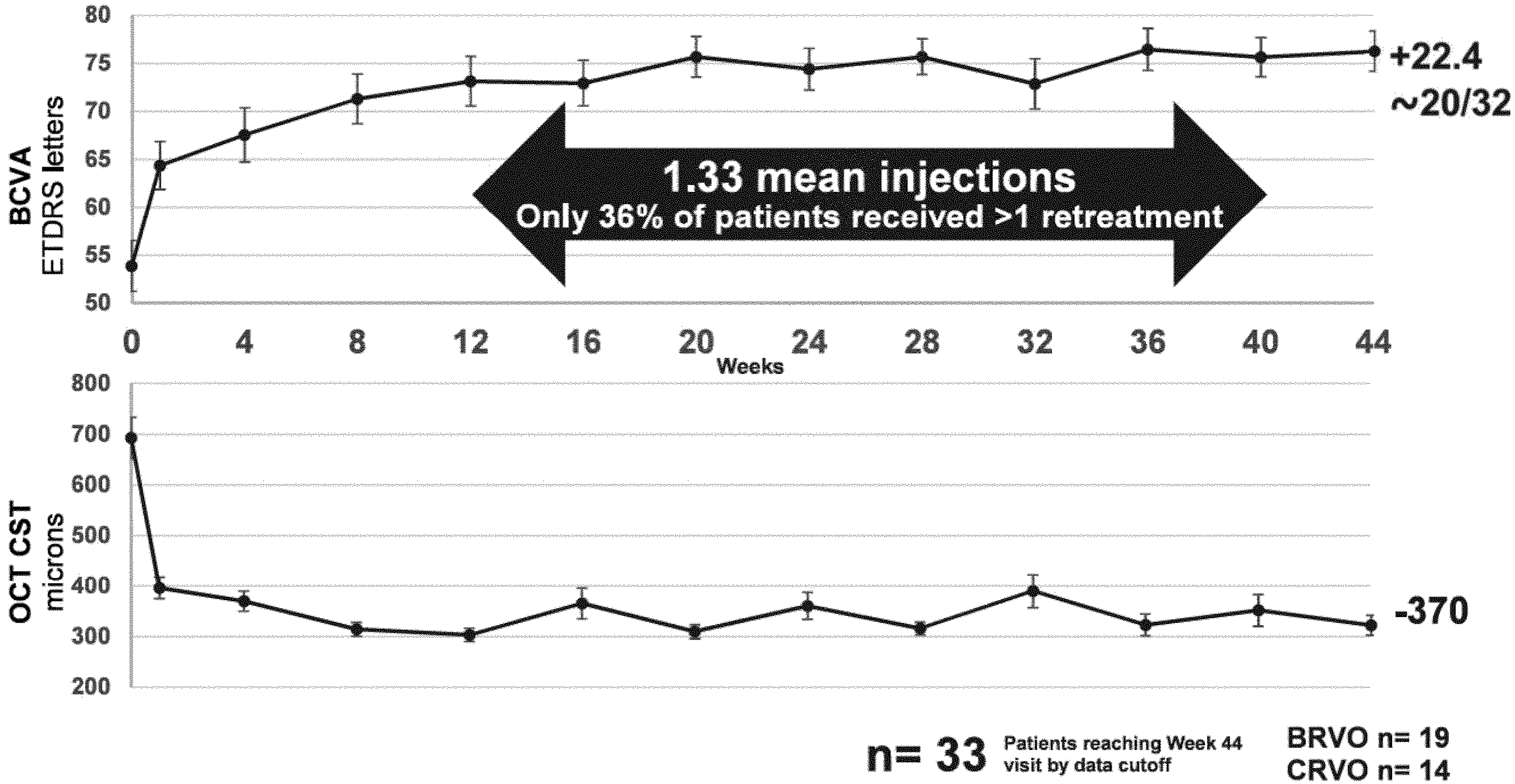


Interim data. Includes only randomized patients that reached Week 44 visit by the data cutoff date; 2.5 & 5 mg doses pooled. Observed data. Error bars represent standard error of the mean. OCT CST values are site reported. BCVA= best corrected visual acuity; OCT= optical coherence tomography; CST= central subfield thickness. Mean injections reflect the average number of injections received per patient between Week 12 and 40 (aflibercept per label mean number of injections 8.0).

FIG. 54A

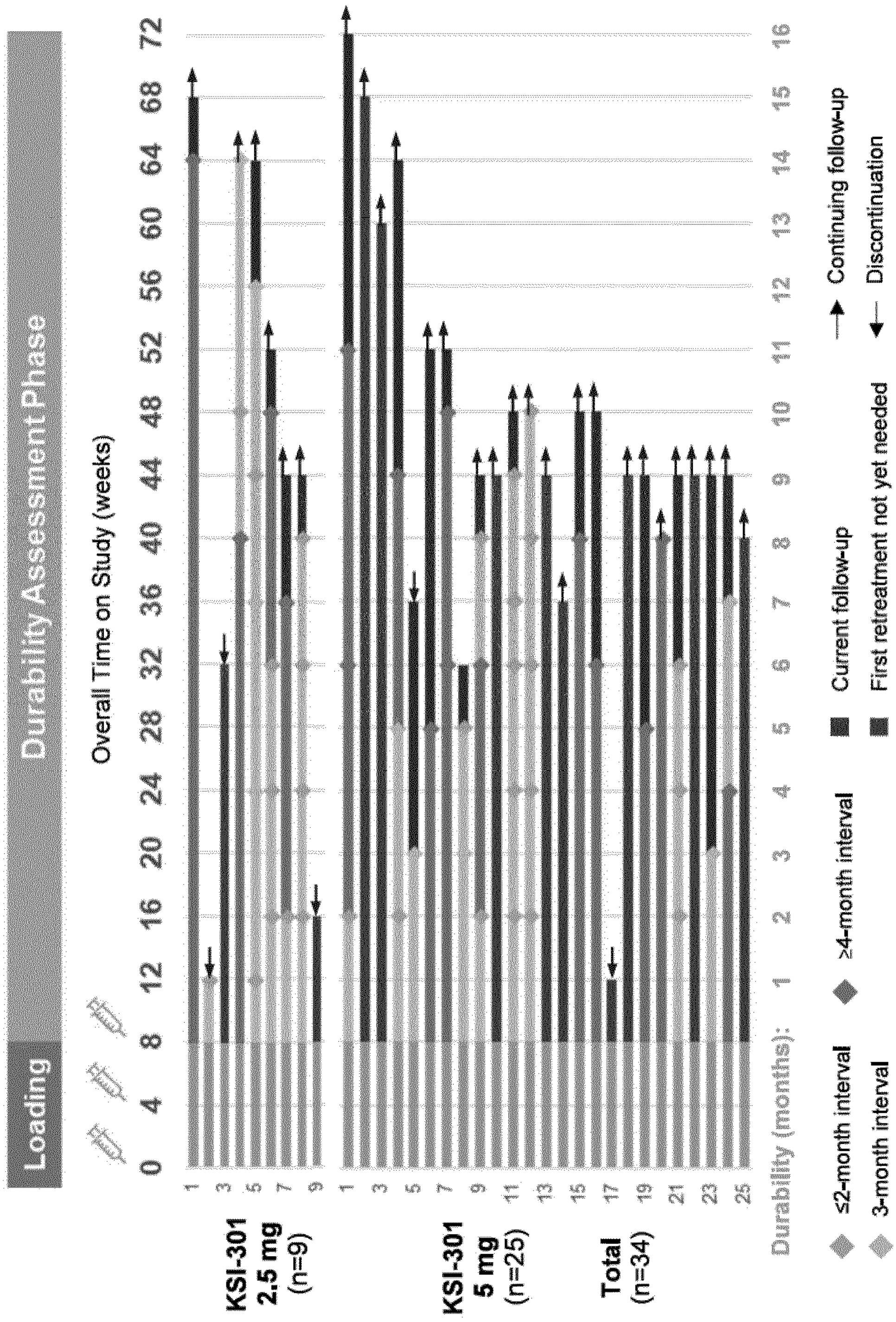


Interim data. Includes only randomized patients that reached the first retreatment opportunity (Week 12 visit) by the data cutoff date. Each bar represents an individual patient.

FIG. 54B

| First Retreatment | Percentage |
|---|---|
| At 1 month | 6% (2/34) |
| 2 months or longer | 94% (31/33) |
| 3 months or longer | 66% (21/32) |
| 4 months or longer | 56% (18/32) |

**81% have achieved a 4-month or longer treatment interval at least once during follow-up**

FIG. 55

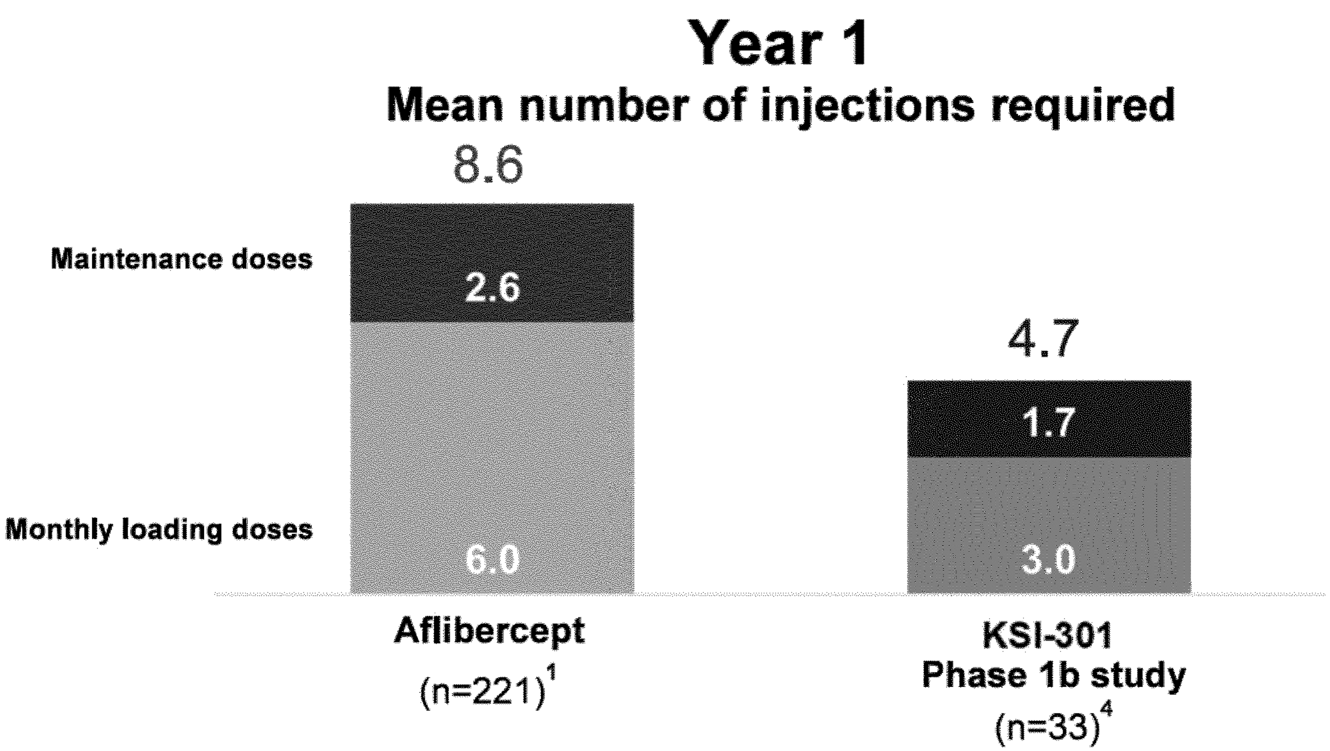


1. Injections averaged between the two pivotal aflibercept trials; n represents the total randomized in the aflibercept groups in both studies. Brown DM. Intravitreal Aflibercept Injection for Macular Edema Secondary to Central Retinal Vein Occlusion: 1-Year Results From the Phase 3 COPERNICUS Study. Am J Ophthalmol 2013;155:429–437.Korobelnik JF, et al. Intravitreal Aflibercept Injection for Macular Edema Resulting from Central Retinal Vein Occlusion. Ophthalmology 2014;121:202-208
2. Interim data. Annualized injections based on the current monthly injection rate of all RVO patients.

FIG. 56

**Case Example of KSI-301 in the Phase 1b Study**

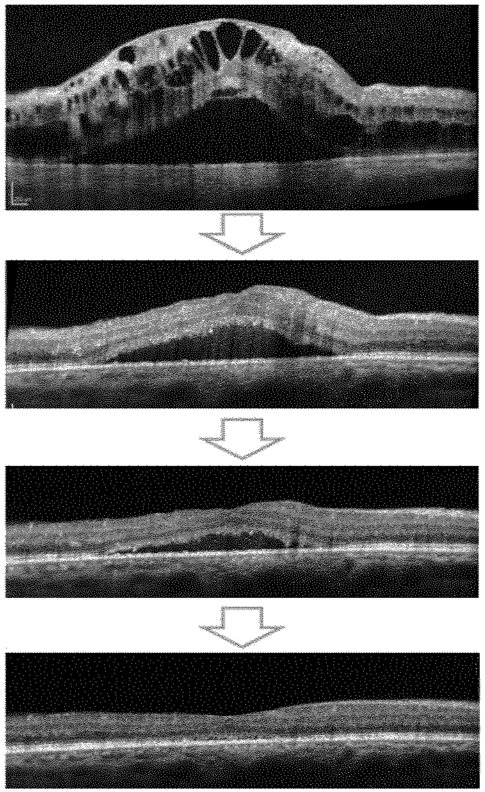

Day 1
1202 microns

Week 1
597 microns
1 week after 1 dose
+14 letters

Week 4
416 microns
1 month after 1 dose
+23 letters

Week 8
260 microns
1 month after 2 doses
+23 letters (20/25)

FIG. 57

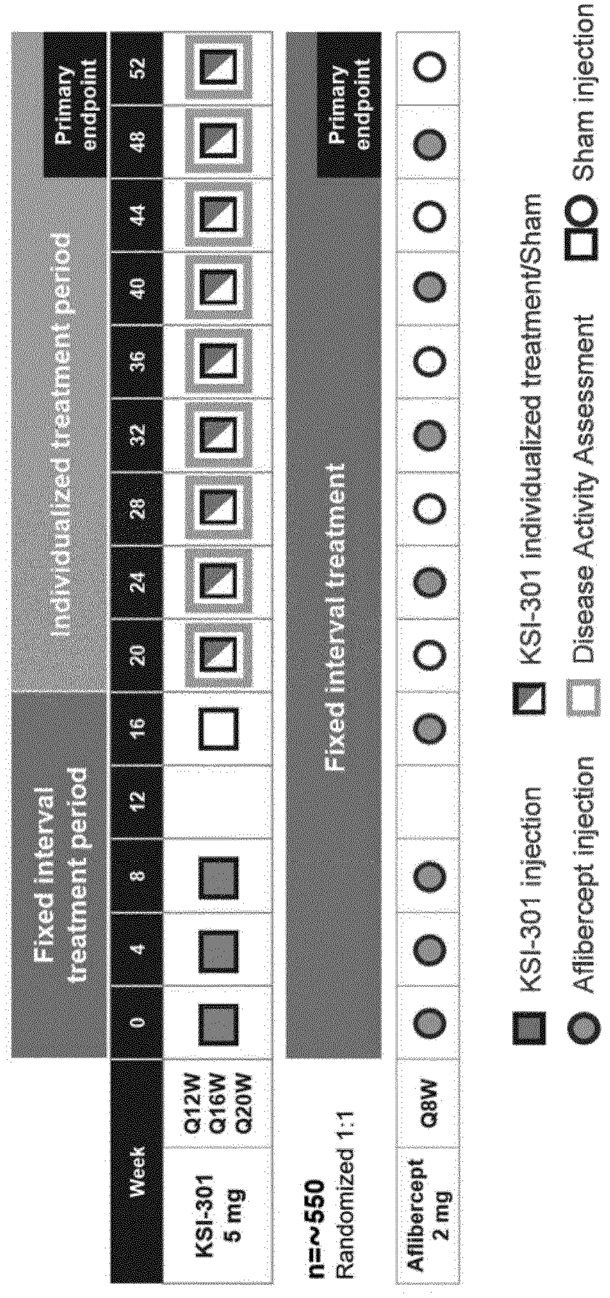
Fixed interval treatment period
Individualized treatment period
Primary endpoint
Week
0
4
8
12
16
20
24
28
32
36
40
44
48
52
KSI-301 5 mg
Q12W Q16W Q20W
n=~550
Randomized 1:1
Fixed interval treatment
Primary endpoint
Aflibercept 2 mg
Q8W
KSI-301 injection
Aflibercept injection
KSI-301 individualized treatment/Sham
Disease Activity Assessment
Sham injection

FIG. 58

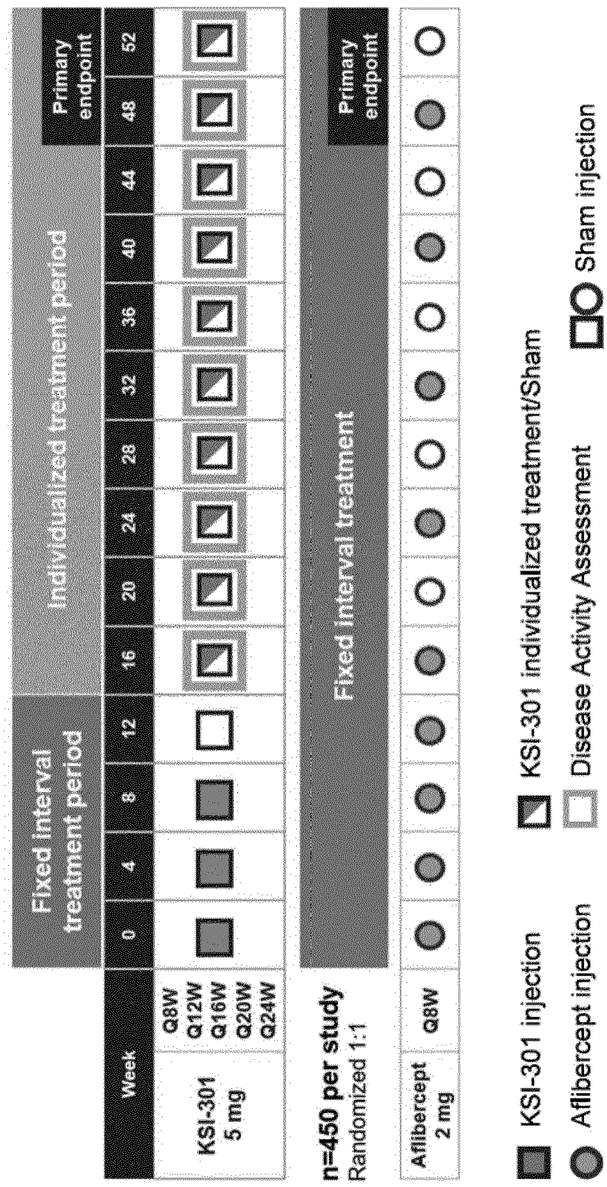
Fixed interval treatment period
Individualized treatment period
Primary endpoint
Week
0
4
8
12
16
20
24
28
32
36
40
44
48
52
KSI-301 5 mg
Q8W
Q12W
Q16W
Q20W
Q24W
n=450 per study
Randomized 1:1
Fixed interval treatment
Primary endpoint
Aflibercept 2 mg
Q8W
KSI-301 injection
Aflibercept injection
KSI-301 individualized treatment/Sham
Disease Activity Assessment
Sham injection

FIG. 59

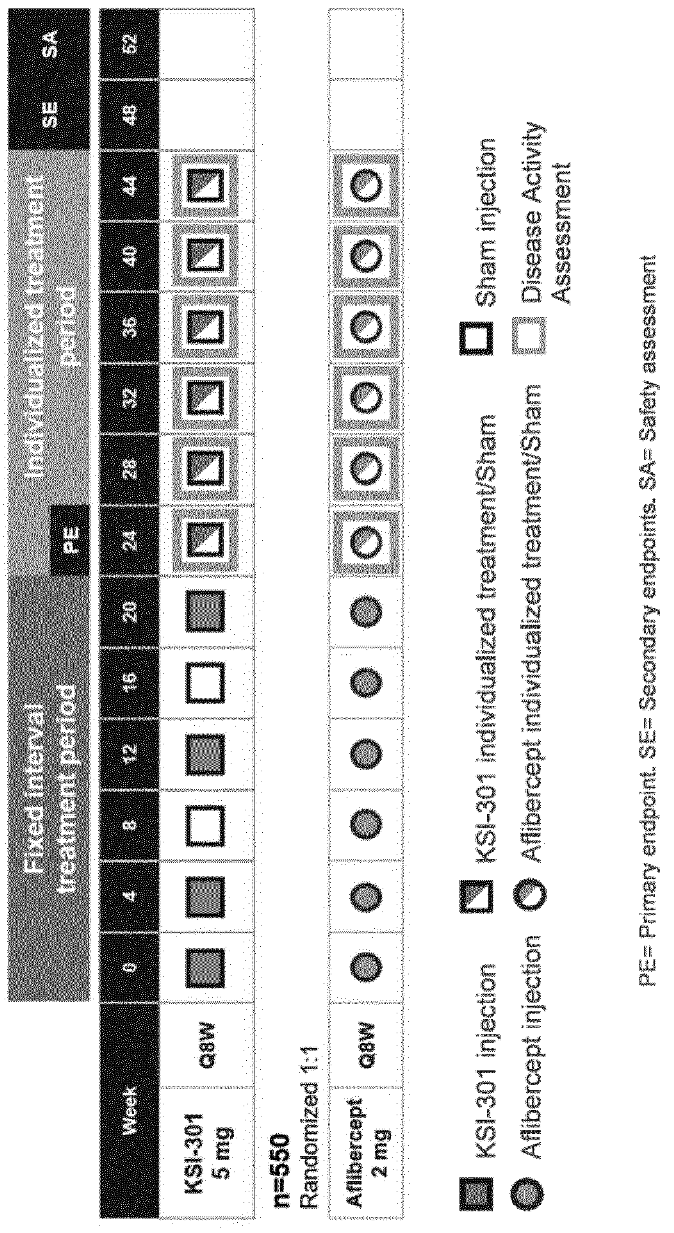
Fixed interval treatment period
Individualized treatment period
PE
SE
SA
Week
0
4
8
12
16
20
24
28
32
36
40
44
48
52
KSI-301 5 mg
Q8W
n=550
Randomized 1:1
Aflibercept 2 mg
Q8W
KSI-301 injection
Aflibercept injection
KSI-301 individualized treatment/Sham
Aflibercept individualized treatment/Sham
Sham injection
Disease Activity Assessment
PE= Primary endpoint. SE= Secondary endpoints. SA= Safety assessment

**Figure 60**

**Study KSI-CL-101 (Phase 1b): Mean Change in BCVA and CST from Baseline to Week 52 in Patients with DME (*Interim Results*)**

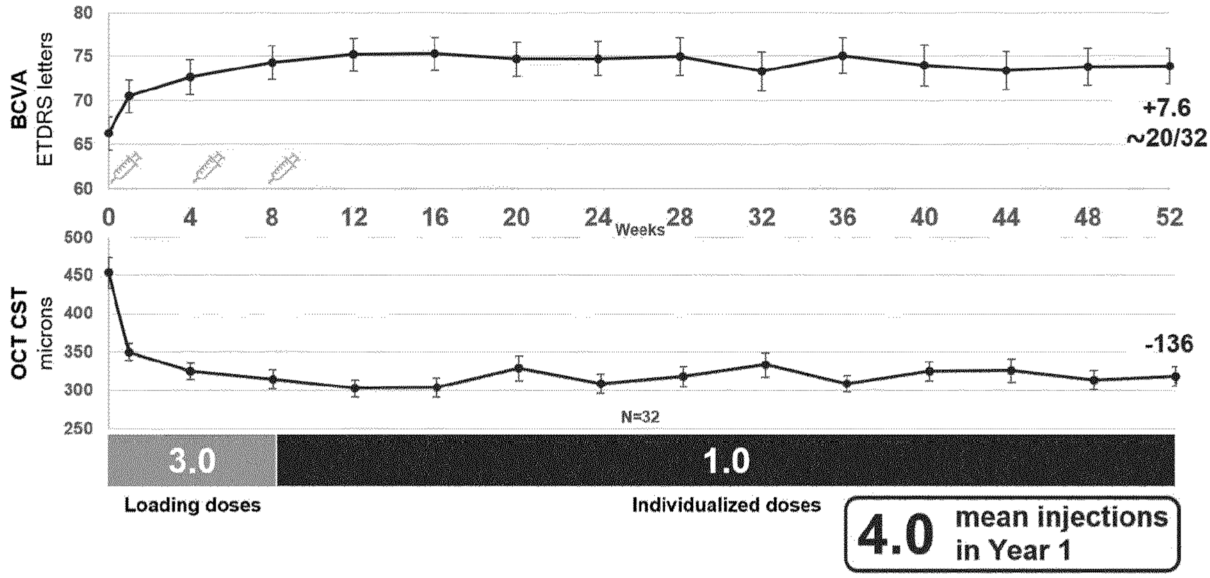


Abbreviations: BCVA = best-corrected visual acuity; CST = central subfield thickness; DME = diabetic macular edema; OCT = optical coherence tomography.

Interim data: 2.5 & 5 mg doses pooled.

Observed data, includes only patients that received all (3) loading doses and reached Week 12 or later. Error bars represent standard error of the mean. Individualized doses reflect the number of injections received per patient between Week 12 and 48 inclusive.

OCT CST values are site reported.

## Figure 61

**Study KSI-CL-101 (Phase 1b): KSI-301 Durability/Need for Retreatment from Baseline to Week 52 in Patients with DME (*Interim Results*)**

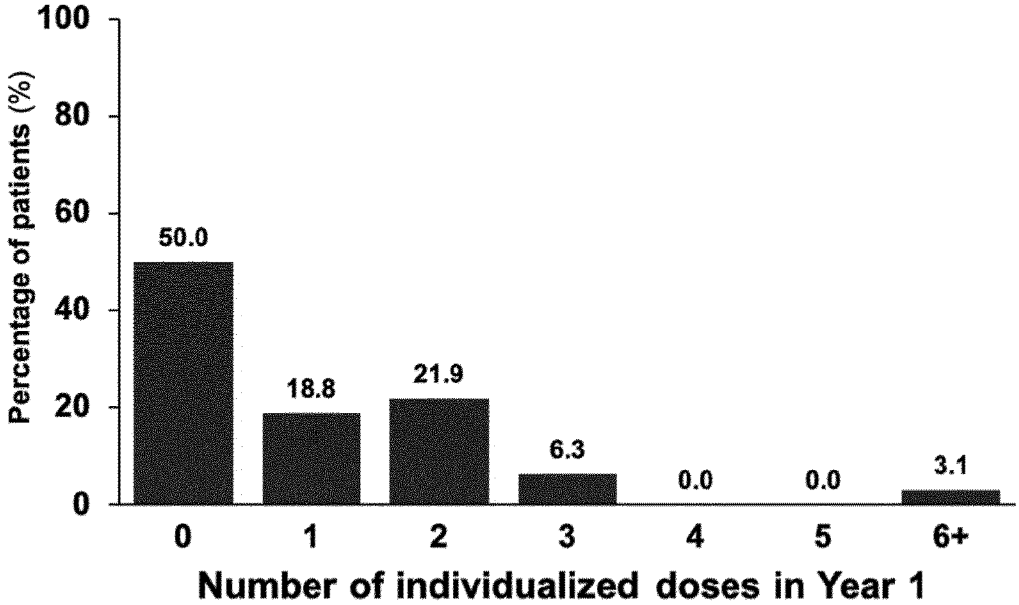


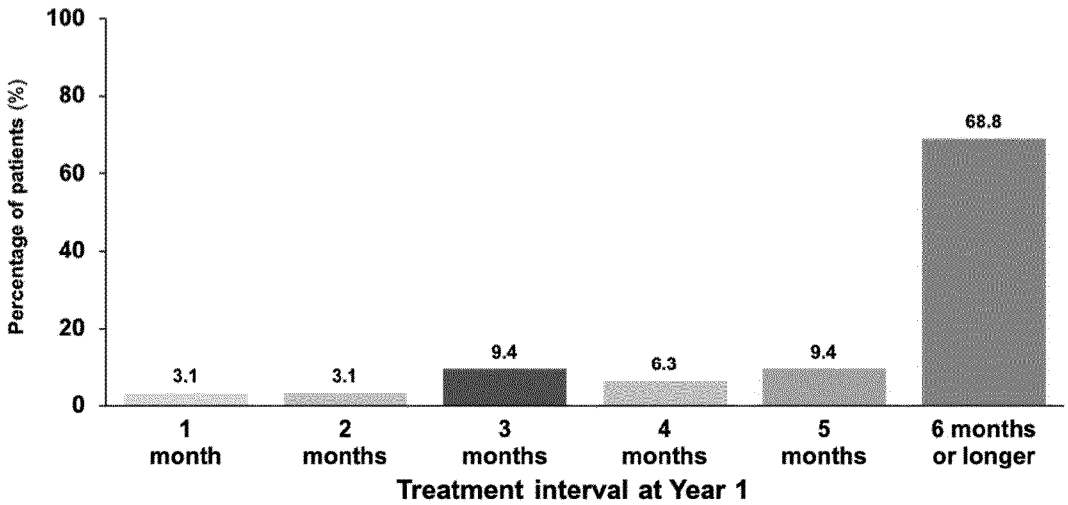


Interim data; 2.5 & 5 mg doses pooled (N = 32). Includes only patients that received all (3) loading doses and either a) received a dose before Week 52 or b) did not receive a dose and were followed for at least six months after the last loading dose (Week 32 visit). Individualized doses reflect the average number of injections received per patient between Week 12 and 48 inclusive. Treatment interval at Year 1 reflects the treatment interval ongoing at the Week 52 visit (where available) or the last interval before Week 52.

## Figure 62

### Study KSI-CL-101 (Phase 1b): Mean Change in BCVA and CST from Baseline to Week 52 in Patients with RVO (*Interim Results*)

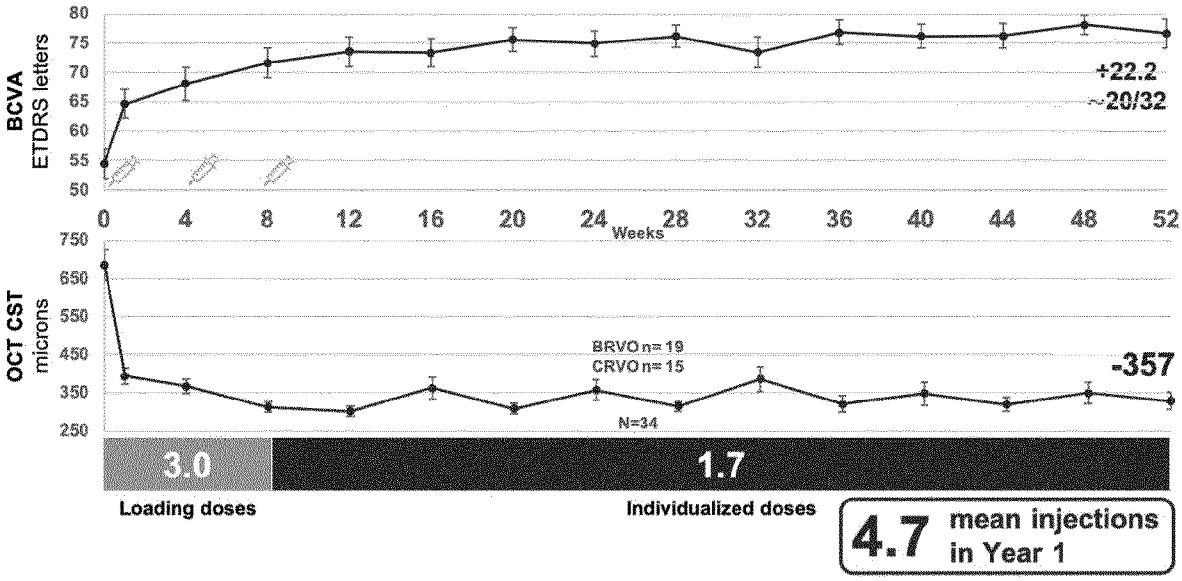

Figure 63

**Study KSI-CL-101 (Phase 1b): KSI-301 Durability/Need for Retreatment from Baseline to Week 52 in Patients with RVO (*Interim Results*)**

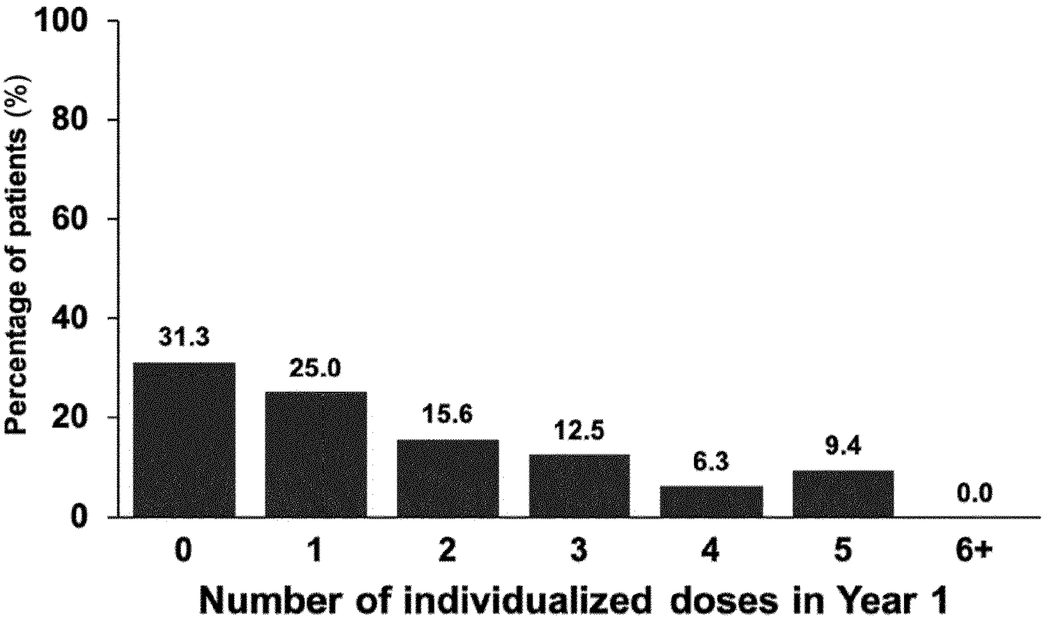


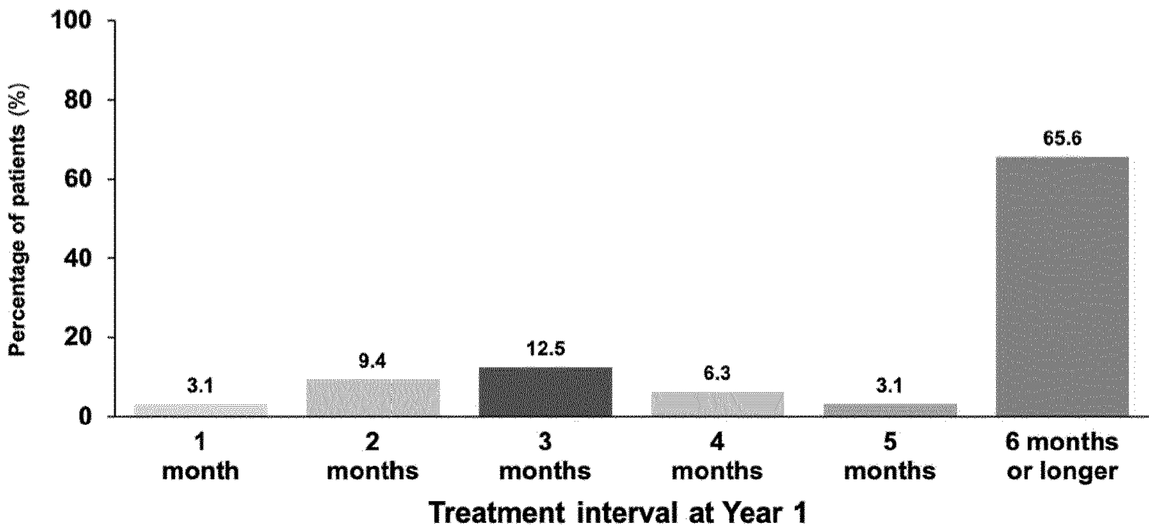

# Figure 64

**Heavy chains of OG1950**

EVQLVESGGGLVQPGGSLRLSCAASGYDFTHYGMNWVRQAPGKGLEWVGWINTYTGEPTYAADFKRRFTFSLDTSKS
TAYLQMNSLRAEDTAVYYCAKYPYYYGTSHWYFDVWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICNVNHKPSNTKVDKKVEPKSCDKTHT
CPPCPAPEAAGAPSVFLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEALHNHYTQKSLSC**SPGK** (SEQ ID NO:1)

Or

EVQLVESGGGLVQPGGSLRLSCAASGYDFTHYGMNWVRQAPGKGLEWVGWINTYTGEPTYAADFKRRFTFSLDTSKS
TAYLQMNSLRAEDTAVYYCAKYPYYYGTSHWYFDVWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICNVNHKPSNTKVDKKVEPKSCDKTHT
CPPCPAPEAAGAPSVFLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEALHNHYTQKSLSC**SPG** (SEQ ID NO:15)

Or

EVQLVESGGGLVQPGGSLRLSCAASGYDFTHYGMNWVRQAPGKGLEWVGWINTYTGEPTYAADFKRRFTFSLDTSKS
TAYLQMNSLRAEDTAVYYCAKYPYYYGTSHWYFDVWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICNVNHKPSNTKVDKKVEPKSCDKTHT
CPPCPAPEAAGAPSVFLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEALHNHYTQKSLSC**SP** (SEQ ID NO:16)

Or

EVQLVESGGGLVQPGGSLRLSCAASGYDFTHYGMNWVRQAPGKGLEWVGWINTYTGEPTYAADFKRRFTFSLDTSKS
TAYLQMNSLRAEDTAVYYCAKYPYYYGTSHWYFDVWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICNVNHKPSNTKVDKKVEPKSCDKTHT
CPPCPAPEAAGAPSVFLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEALHNHYTQKSLSC**S** (SEQ ID NO:17)

Or

EVQLVESGGGLVQPGGSLRLSCAASGYDFTHYGMNWVRQAPGKGLEWVGWINTYTGEPTYAADFKRRFTFSLDTSKS
TAYLQMNSLRAEDTAVYYCAKYPYYYGTSHWYFDVWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICNVNHKPSNTKVDKKVEPKSCDKTHT
CPPCPAPEAAGAPSVFLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEALHNHYTQKSLSC (SEQ ID NO:18)

**Light chain of OG1950**

DIQLTQSPSSLSASVGDRVTITCSASQDISNYLNWYQQKPGKAPKVLIYFTSSLHSGVPSRFSGSGSGTDFTLTISS
LQPEDFATYYCQQYSTVPWTFGQGTKVEIKRTVAAPSVFIFPPSDEQLKSGTASVVCLLNNFYPREAKVQWKVDNAL
QSGNSQESVTEQDSKDSTYSLSSTLTLSKADYEKHKVYACEVTHQGLSSPVTKSFNRGEC (SEQ ID NO:2)

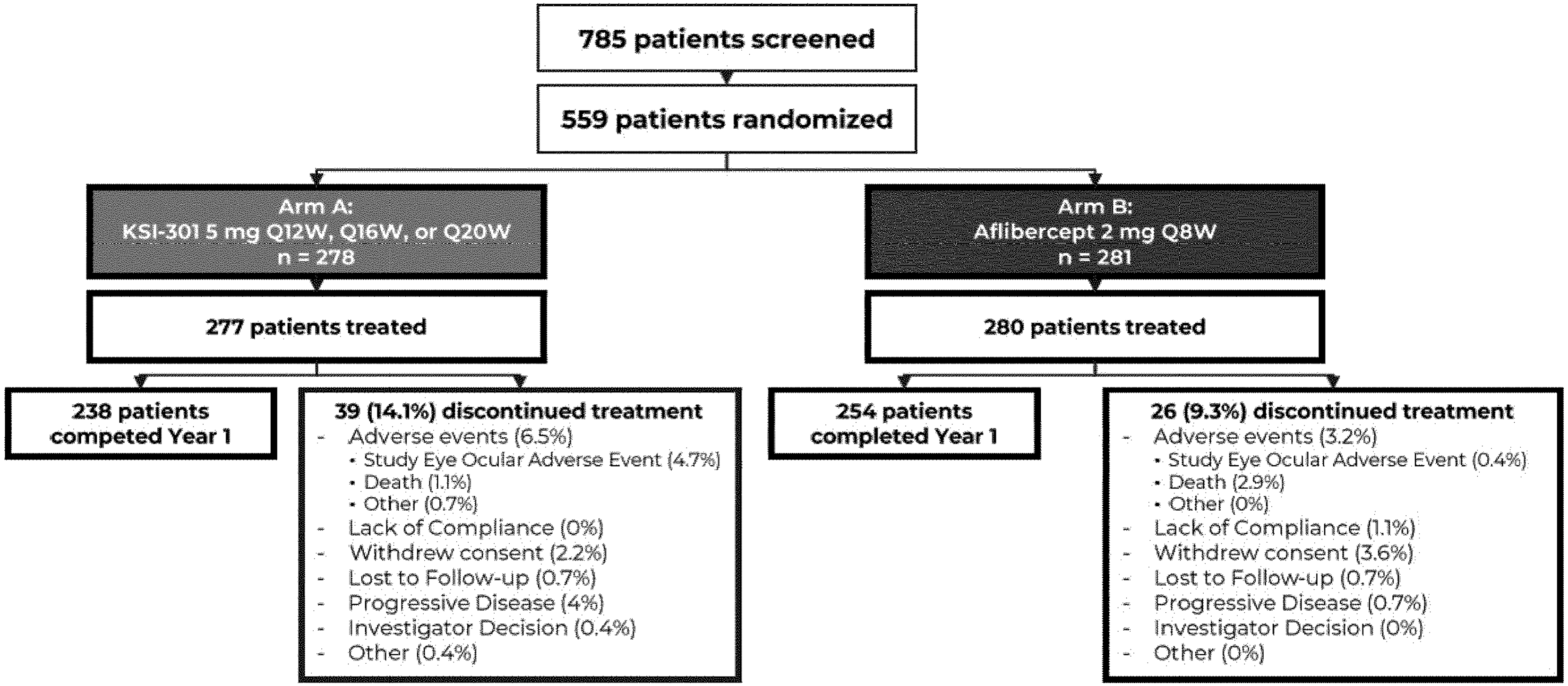


Q8W: every 8 weeks; Q12W: every 12 weeks; Q16W; every 16 weeks; Q20W: every 20 weeks.

FIG. 65

| Parameter | KSI-301 5 mg Q12W-Q20W (N=277) | Aflibercept 2 mg Q8W (N=280) |
|---|---|---|
| **Gender** | | |
| Female | 178 (64.3%) | 168 (60.0%) |
| **Age at Randomization, years** | | |
| Mean (SD) | 76.6 ( 7.35) | 76.2 ( 8.27) |
| **Ethnicity** | | |
| Hispanic or Latino | 17 ( 6.1%) | 9 ( 3.2%) |
| Not Hispanic or Latino | 260 (93.9%) | 271 (96.8%) |
| **Race** | | |
| American Indian or Alaska Native | 1 ( 0.4%) | 1 ( 0.4%) |
| Asian | 4 ( 1.4%) | 5 ( 1.8%) |
| Black or African American | 1 ( 0.4%) | 1 ( 0.4%) |
| Other | 0 | 1 ( 0.4%) |
| White | 271 (97.8%) | 272 (97.1%) |
| **Geographical Region** | | |
| Europe | 48 (17.3%) | 45 (16.1%) |
| USA | 229 (82.7%) | 235 (83.9%) |

N = Number of participants treated; The denominator for percentages is the number of participants treated within each treatment arm
Q8W: every 8 weeks; Q12W: every 12 weeks; Q20W: every 20 weeks.

FIG. 66

| Parameter | KSI-301 5 mg Q12W-Q20W (N=277) | Aflibercept 2 mg Q8W (N=280) |
|---|---|---|
| **BCVA, ETDRS Letters** | | |
| Mean (SD) | 63.6 ( 12.23) | 63.6 ( 12.34) |
| **BCVA Category** | | |
| ≤ 49 ETDRS Letters | 33 (11.9%) | 33 (11.8%) |
| 50 – 69 ETDRS Letters | 133 (48.0%) | 136 (48.6%) |
| 70 – 80 ETDRS Letters | 111 (40.1%) | 111 (39.6%) |
| **BCVA - Low Luminance VA Difference** | | |
| < 33 | 186 (67.1%) | 187 (66.8%) |
| ≥ 33 | 91 (32.9%) | 93 (33.2%) |
| **OCT Central Subfield Thickness from ILM to RPE, µm** | | |
| Mean (SD) | 350.4 (110.90) | 359.5 (112.81) |
| **OCT Intraretinal fluid visible in Central 1 mm** | | |
| Present | 121 (43.7%) | 109 (38.9%) |
| **OCT Subretinal fluid visible in Central 1 mm** | | |
| Present | 224 (80.9%) | 231 (82.5%) |
| **Intraocular Pressure, mmHg** | | |
| Mean (SD) | 15.1 ( 3.14) | 14.6 ( 3.08) |

N = Number of participants treated; The denominator for percentages is the number of participants treated within each treatment arm. AMD: age-related macular degeneration; BCVA: best corrected visual acuity; ETDRS: early treatment diabetic retinopathy study; OCT: optical coherence tomography; ILM: internal limiting membrane; RPE: retinal pigment epithelium; Q8W: every 8 weeks; Q12W: every 12 weeks; Q20W: every 20 weeks.

FIG. 67

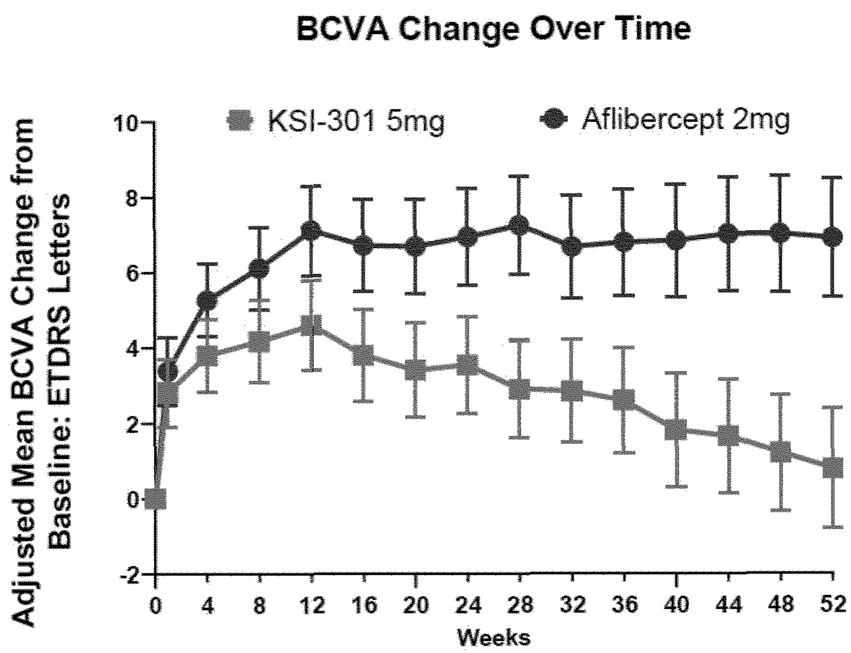


| | Average of weeks 48 & 52 |
|---|---|
| KSI-301 5mg | 1.0 (-0.5, 2.5)* |
| Aflibercept 2mg | 7.0 (5.5, 8.5)* |

Least square means BCVA change from baseline and 95% CI are based on MMRM model with treatment, visit, baseline BCVA categories, BCVA-low luminance VA baseline categories, geographical location categories, and treatment by visit interaction.Least square means CST change from baseline and 95% CI are based on MMRM model with treatment, visit, baseline OCT, baseline BCVA categories, BCVA-low luminance VA baseline categories, geographical location categories, and treatment by visit interaction. *Adjusted mean BCVA/CST change from baseline at year 1,averaged over weeks 48 and 52.
BCVA: best corrected visual acuity; ETDRS: early treatment diabetic retinopathy study; OCT: optical coherence tomography; CST: central subfield thickness.

FIG. 68A

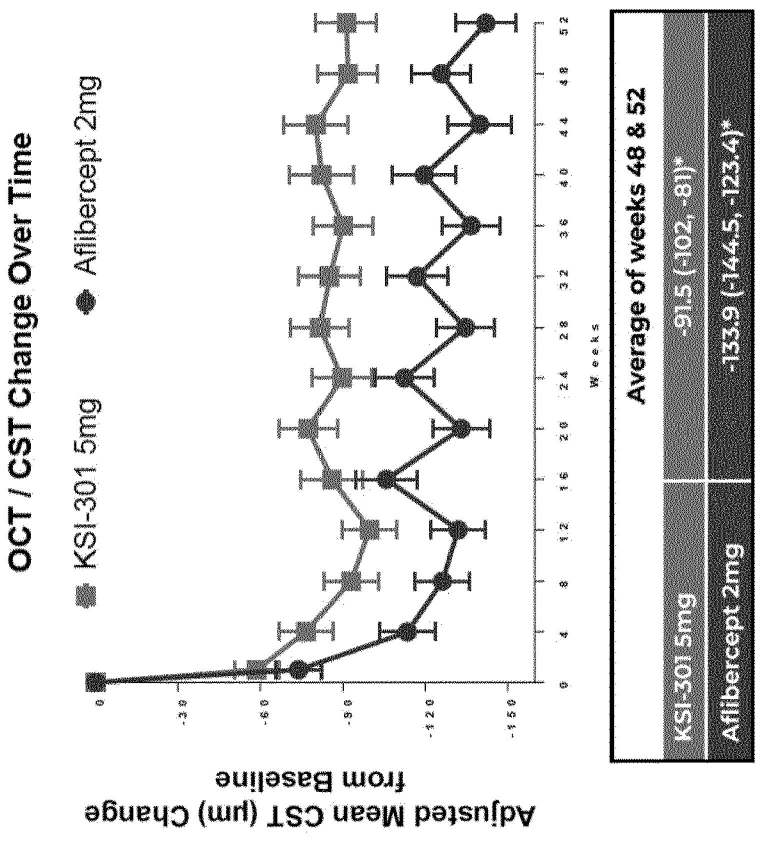
OCT / CST Change Over Time
KSI-301 5mg
Aflibercept 2mg
Adjusted Mean CST (µm) Change from Baseline
0
-30
-60
-90
-120
-150
0 4 8 12 16 20 24 28 32 36 40 44 48 52
Weeks
Average of weeks 48 & 52
KSI-301 5mg
-91.5 (-102, -81)*
Aflibercept 2mg
-133.9 (-144.5, -123.4)*

FIG. 68B

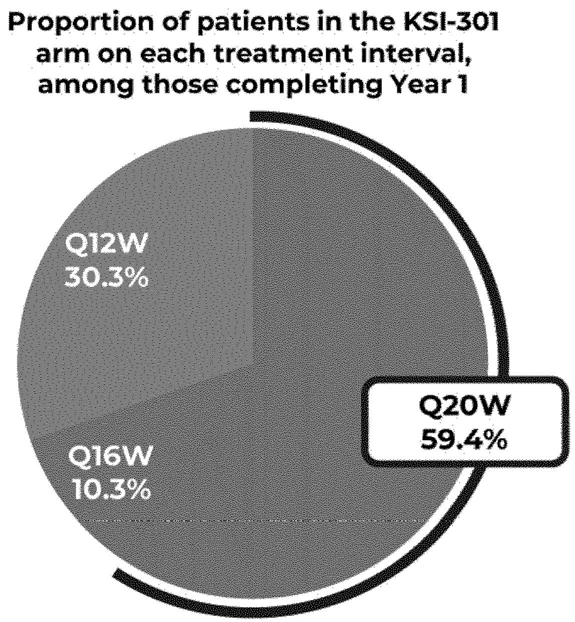


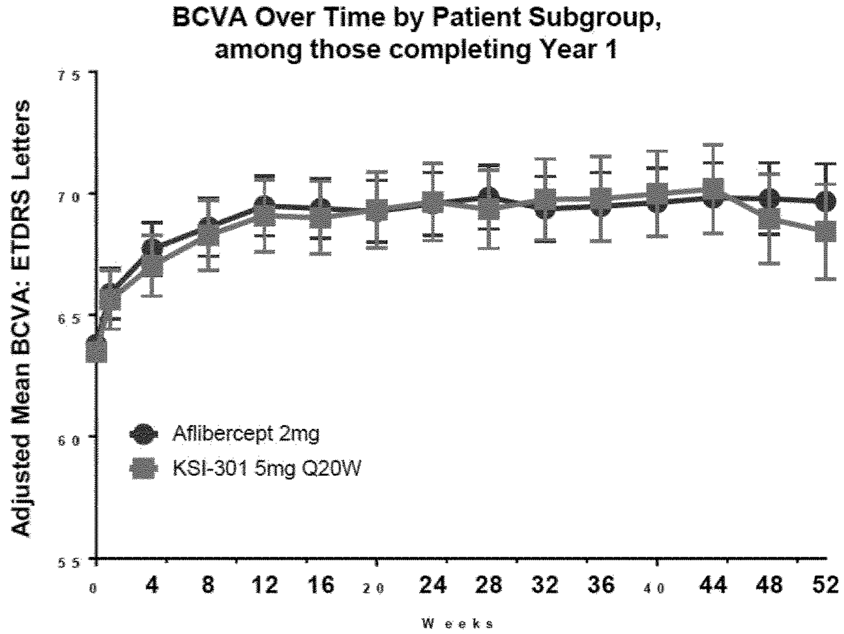


Least square means and 95% CI are based on MMRM model with treatment, visit, baseline BCVA, baseline BCVA categories, BCVA-low luminance VA baseline categories, geographical location categories, and treatment (KSI-301 Q20W, Q16W, Q12W, Aflibercept Q8W) by visit interaction.

Q12W: every 12 weeks; Q16W: every 16 weeks; Q20W: every 20 weeks. BCVA: best corrected visual acuity; ETDRS: early treatment diabetic retinopathy study.

FIG. 69

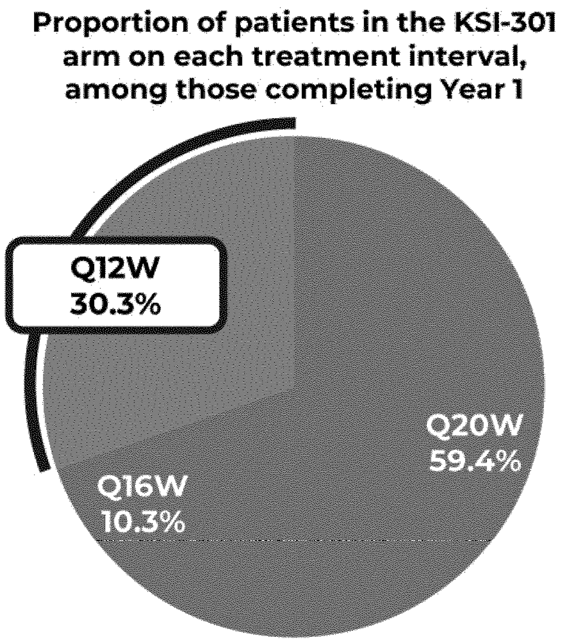


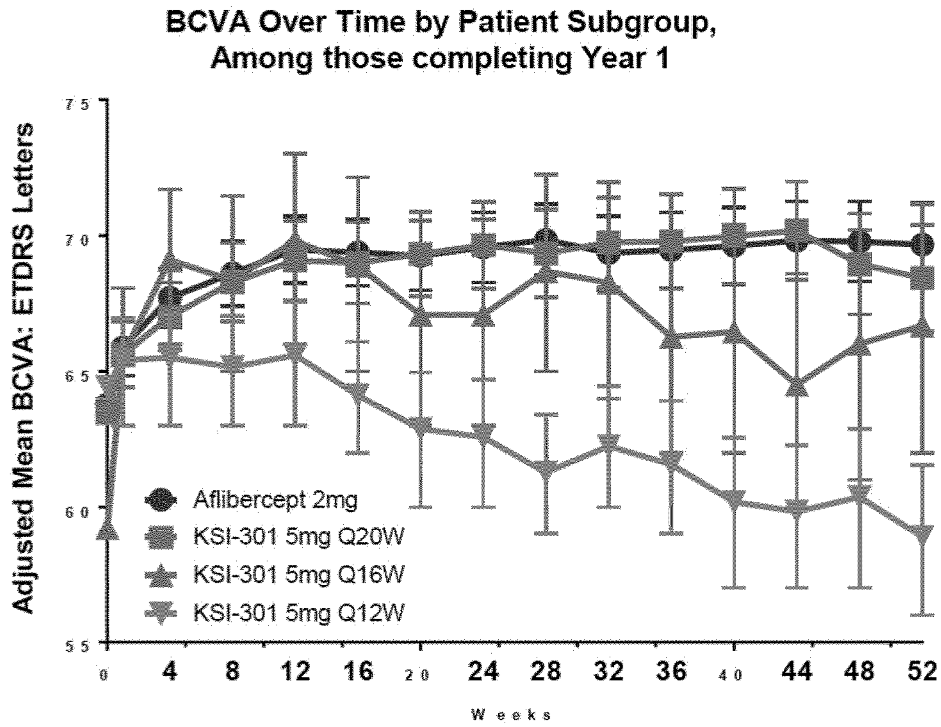


Least square means and 95% CI are based on MMRM model with treatment, visit, baseline BCVA, baseline BCVA categories, BCVA-low luminance VA baseline categories, geographical location categories, and treatment (KSI-301 Q20W, Q16W, Q12W, Aflibercept Q8W) by visit interaction.
Q12W: every 12 weeks; Q16W: every 16 weeks; Q20W: every 20 weeks; BCVA: best corrected visual acuity; ETDRS: early treatment diabetic retinopathy study.

FIG. 70

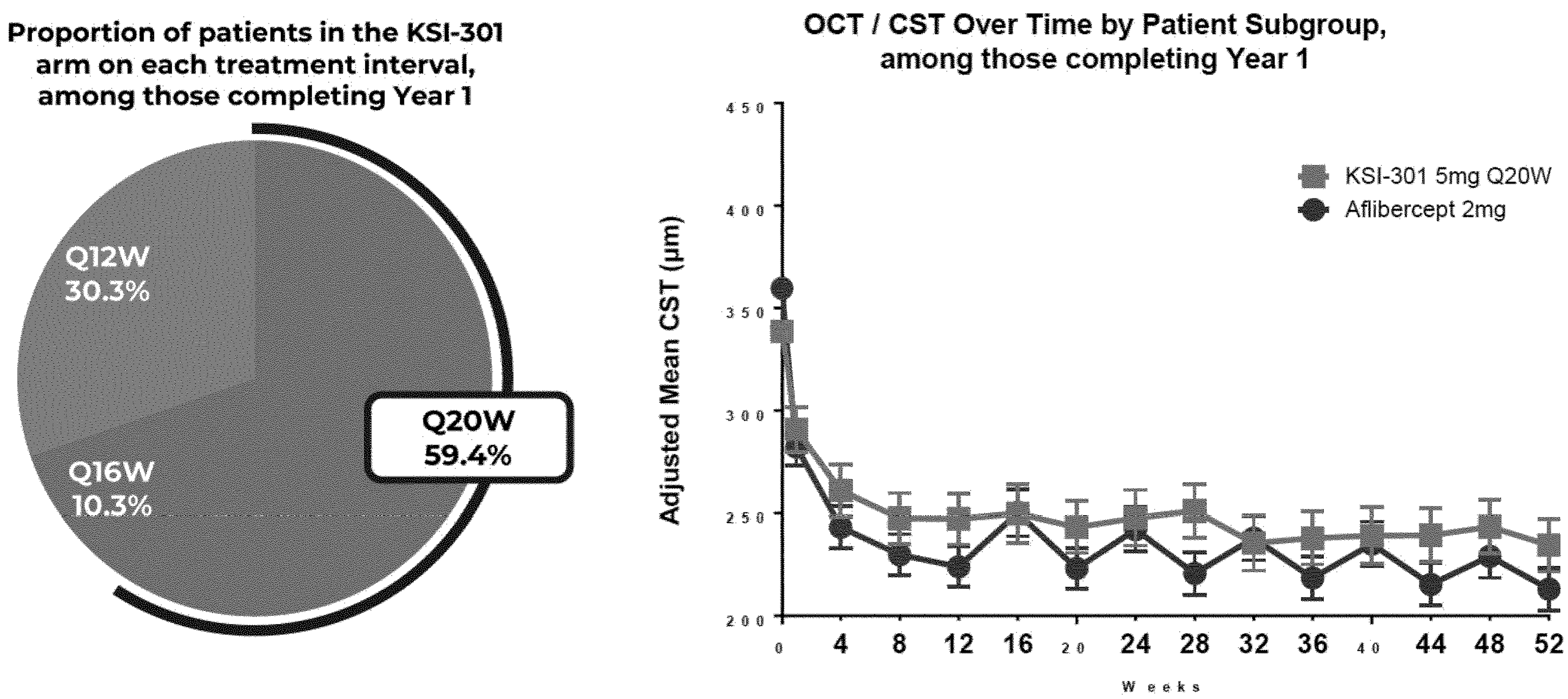
Proportion of patients in the KSI-301 arm on each treatment interval, among those completing Year 1
Q12W 30.3%
Q16W 10.3%
Q20W 59.4%
OCT / CST Over Time by Patient Subgroup, among those completing Year 1
KSI-301 5mg Q20W
Aflibercept 2mg
Adjusted Mean CST (µm)
450
400
350
300
250
200
0
4
8
12
16
20
24
28
32
36
40
44
48
52
Weeks

FIG. 71

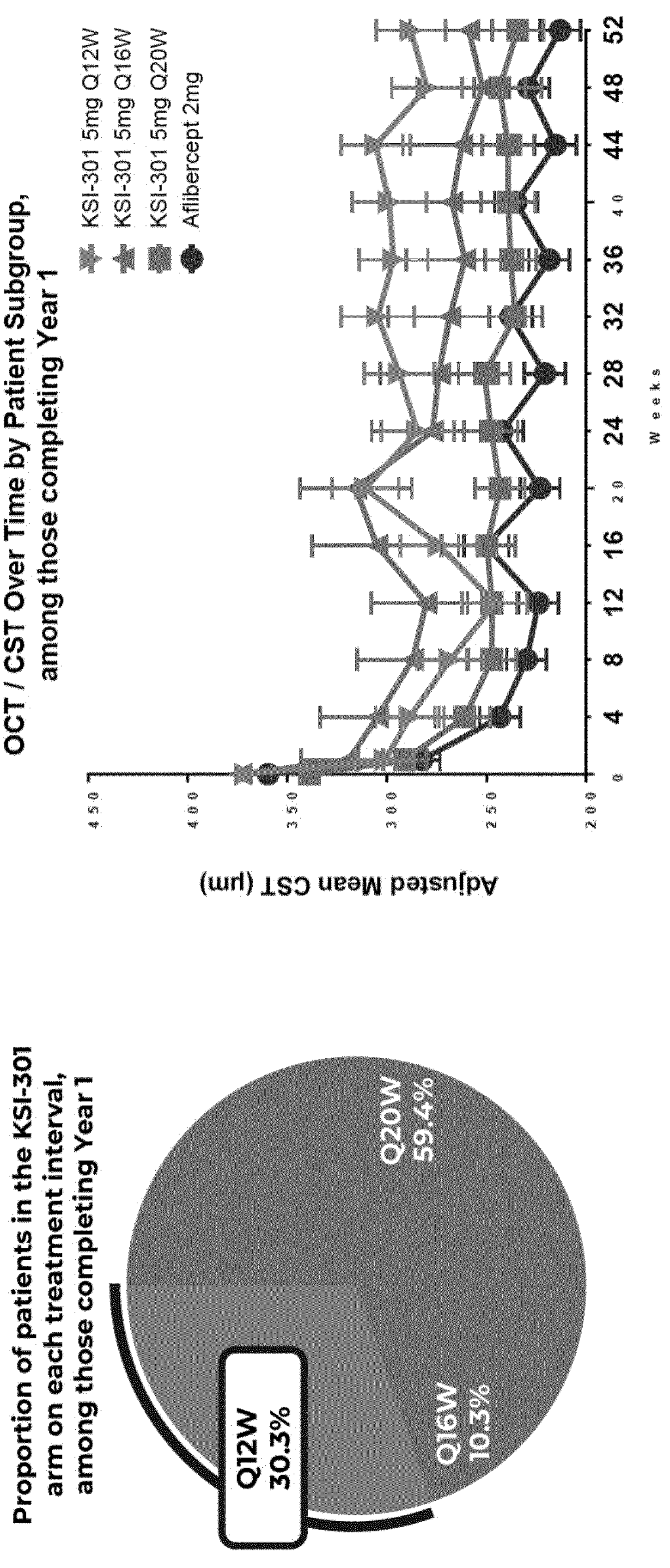
Proportion of patients in the KSI-301 arm on each treatment interval, among those completing Year 1
Q12W 30.3%
Q16W 10.3%
Q20W 59.4%
OCT / CST Over Time by Patient Subgroup, among those completing Year 1
KSI-301 5mg Q12W
KSI-301 5mg Q16W
KSI-301 5mg Q20W
Aflibercept 2mg
Adjusted Mean CST (µm)
Weeks
450
400
350
300
250
200
0
4
8
12
16
20
24
28
32
36
40
44
48
52

FIG. 72

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5530101 A
- US 5585089 A, Winter
- US 5225539 A, Carter
- US 6407213 B, Adair
- US 5859205 A
- US 6881557 B, Foote
- US 4634664 A, Östberg
- US 4634666 A, Engleman
- WO 9312227 A, Lonberg
- US 5877397 A
- US 5874299 A
- US 5814318 A
- US 5789650 A
- US 5770429 A
- US 5661016 A
- US 5633425 A
- US 5625126 A
- US 5569825 A
- US 5545806 A
- WO 9110741 A, Kucherlapati,
- WO 9117271 A, Dower
- WO 9201047 A, McCafferty
- US 5877218 A
- US 5871907 A
- US 5858657 A
- US 5837242 A
- US 5733743 A
- US 5565332 A
- WO 2013059137 A
- US 20170190766 A
- WO 20040204052004 A
- WO 2008155134 A1
- WO 2013093809 A
- US 7521541 B
- WO 2008020827 A
- US 8008453 B
- US 8455622 B
- US 20120213705 A
- US 20150158952 A
- WO 2009052249 A
- US 7893173 B
- WO 2017117464 A


### Non-patent literature cited in the description


- **HOUSTON JS.** *Methods in Enzymol.*, 1991, vol. 203, 46-96
- Fundamental Immunology. Raven Press, 1989
- Bispecific antibody: a tool for diagnosis and treatment of disease. **SONGSIVILAI S** ; **LACHMANN PC.** Clin Exp Immunol.. 1990, vol. 79, 315-321
- **KOSTELNY SA** ; **COLE MS** ; **TSO JY.** Formation of bispecific antibody by the use of leucine zippers. *J Immunol.*, 1992, vol. 148, 1547-1553
- **KABAT EA et al.** Sequences of Proteins of Immunological Interest. National Institutes of Health, 1987
- **CHOTHIA C** ; **LESK AM**. Canonical Structures for the Hypervariable Regions of Immunoglobulins. *J Mol Biol*, 1987, vol. 196, 901-917
- **CHOTHIA C et al.** Conformations of Immunoglobulin Hypervariable Regions. *Nature*, 1989, vol. 342, 877-883
- Epitope Mapping Protocols. Methods in Molecular Biology. 1996, vol. 66
- **JUNGHANS et al.** *Cancer Res.*, 1990, vol. 50, 1495
- **DE PASCALIS R** ; **IWAHASHI M** ; **TAMURA M et al.** Grafting "Abbreviated" Complementary-Determining Regions Containing Specificity-Determining Residues Essential for Ligand Contact to Engineer a Less Immunogenic Humanized Monoclonal Antibody. *J Immunol.*, 2002, vol. 169, 3076-3084
- **VAJDOS FF** ; **ADAMS CW** ; **BREECE TN** ; **PRESTA LG** ; **VOS AM** ; **SIDHU, SS**. omprehensive functional maps of the antigen-binding site of an anti-ErbB2 antibody obtained with shotgun scanning mutagenesis. *J Mol Biol.*, 2002, vol. 320, 415-428
- **IWAHASHI M** ; **MILENIC DE** ; **PADLAN EA et al.** CDR substitutions of a humanized monoclonal antibody (CC49): Contributions of individual CDRs to antigen binding and immunogenicity. *Mol Immunol.*, 1999, vol. 36, 1079-1091
- **TAMURA M** ; **MILENIC DE** ; **IWAHASHI M et al.** Structural correlates of an anticarcinoma antibody: Identification of specificity-determining regions (SDRs) and development of a minimally immunogenic antibody variant by retention of SDRs only. *J Immunol.*, 2000, vol. 164, 1432-1441

- **PADLAN EA.** A possible procedure for reducing the immunogenicity of antibody variable domains while preserving their ligand-binding properties. *Mol Immunol.*, 1991, vol. 28, 489-98
- **ÖSTBERG L** ; **PURSCH E**. Human x (mouse x human) hybridomas stably producing human antibodies. *Hybridoma*, 1983, vol. 2, 361-367
- *Nature*, 1994, vol. 148, 1547-1553
- *Nature Biotechnology*, 1996, vol. 14, 826
- **GREENE et al.** Protective Groups In Organic Synthesis. John Wiley and Sons, Inc., 1999
- **FERRARA et al.** *Nat. Med.*, 2003, vol. 9, 669-676
- **FERRARA N** ; **HILLAN KJ** ; **GERBER HP** ; **NOVOTNY W**. Discovery and development of bevacizumab, an anti-VEGF antibody for treating cancer. *Nat Rev Drug Discov.*, 2004, vol. 3 (5), 391-400
- **CAPON DJ** ; **CHAMOW SM** ; **MORDENTI J et al.** Designing CD4 immunoadhesions for AIDS therapy. *Nature*, 1989, vol. 337, 525-31
- **YEH P** ; **LANDAIS D** ; **LEMAITRE M et al.** Design of yeast-secreted albumin derivatives for human therapy: biological and antiviral properties of a serum albumin-CD4 genetic conjugate. *Proc Natl Acad Sci USA.*, 1992, vol. 89, 1904-08
- **FARES FA** ; **SUGANUMA N. NISHIMORI K et al.** Design of a long-acting follitropin agonist by fusing the C-terminal sequence of the chorionic gonadotropin beta subunit to the follitropin beta subunit. *Proc Natl Acad Sci USA.*, 1992, vol. 89, 4304-08
- **SCHELLENBERGER V** ; **WANG CW** ; **GEETHING NC et al.** A recombinant polypeptide extends the in vivo half-life of peptides and proteins in a tunable manner. *Nat Biotechnol.*, 2009, vol. 27, 1186-90
- **MCPHERSON DT** ; **MORROW C** ; **MINEHAN DS et al.** Production and purification of a recombinant elastomeric polypeptide, G(VPGVG19-VPGV, from Escheriachia coli.. *Biotechnol Prog*, 1992, vol. 8, 347-52
- **PRIOR CP** ; **LAI C-H** ; **SADEHGHI H et al.** *Modified transferrin fusion proteins*
- **SKERRA A** ; **THEOBALD I** ; **SCHLAPSKY M.** *Biological active proteins having increased in vivo and/or vitro stability*
- **THEOBALD I** ; **MACK H et al.** Fusion of a recombinant antibody fragment with a homo-amino acid polymer: effects on biophysical properties and prolonged plasma half-life. *Protein Eng Des Sel.*, 2007, vol. 20, 273-84
- **HUANG Y-S** ; **WEN X-F** ; **ZARO JL et al.** Engineering a pharmacologically superior form of granulocyte-colony-stimulating-factor by fusion with gelatin-like protein polymer. *Eur J. Pharm Biopharm.*, 2010, vol. 72, 435-41
- Intraocular Drug Delivery. Taylor & Francis, March 2006
- Peptide and Protein Drug Delivery. 1991, 247-301
- **JONES**. *Adv. Drug Delivery Rev.*, 1993, vol. 10, 29-90
- **RAETZ CR** ; **ULEVITCH RJ** ; **WRIGHT SD** ; **SIBLEY CH** ; **DING A** ; **NATHAN CF**. Gram-negative endotoxin: an extraordinary lipid with profound effects on eukaryotic signal transduction. *FASEB J.*, 1991, vol. 5 (12), 2652-2660
- **PETSCH D** ; **ANSPACH F.** Endotoxin removal from protein solutions.. *J Biotechnol.*, 2000, vol. 76, 97-119
- **OGIKUBO Y** ; **OGIKUBO Y** ; **NORIMATSU M** ; **NODA K** ; **TAKAHASHI J** ; **INOTSUME M** ; **TSUCHIYA M** ; **TAMURA Y.** Evaluation of the bacterial endotoxin test for quantifications of endotoxin contamination of porcine vaccines. *Biologics*, 2004, vol. 32, 88-93
- **FISKE JM** ; **ROSS A** ; **VANDERMEID RK** ; **MCMICHAEL JC** ; **ARUMUGHAM**. Method for reducing endotoxin in Moraxella catarrhalis UspA2 protein preparations. *J Chrom B.*, 2001, vol. 753, 269-278
- **DANESHIAM M** ; **GUENTHER A** ; **WENDEL A** ; **HARTUNG T** ; **VON AULOCK S.** In vitro pyrogen test for toxic or immunomodulatory drugs. *J Immunol Method*, 2006, vol. 313, 169-175
- **HIRAYAMA C** ; **SAKATA M.** Chromatographic removal of endotoxin from protein solutions by polymer particles. *J Chrom B*, 2002, vol. 781, 419-432
- **BERTHOLD W** ; **WALTER J.** Protein Purification: Aspects of Processes for Pharmaceutical Products. *Biologicals*, 1994, vol. 22, 135-150
- **PETSCH D** ; **ANSPACH FB.** Endotoxin removal from protein solutions. *J Biotech*, 2000, vol. 76, 97-119
- Penicillin and the control of deep intra-ocular infection. British J Ophthalmol. Rycroft. Rycroft BW., 1945, vol. 29, 57-87
- **CHRISTY NE** ; **SOMMER A.** Antibiotic prophylaxis of postoperative endophthalmitis. *Ann Ophthalmol*, 1979, vol. 11 (8), 1261-1265
- **MARTICORENA J** ; **ROMANO V** ; **GOMEZ-ULLA F.** Sterile Endophthalmitis after Intravitreal Injections. *Med Inflam.*, 2012, 928123
- **MARTICORENA et al.** *Bevacizumab (Avastin)*
- **HOFFMAN S et al.** International validation of novel pyrogen tests based on human monocytoid cells. *J. Immunol. Methods*, 2005, vol. 298, 161-173
- **DING JL** ; **HO BA**. New era in pyrogen testing. *Biotech.*, 2001, vol. 19, 277-281
- **ONG KG** ; **LELAN JM** ; **ZENG KF** ; **BARRETT G** ; **AOUROB M** ; **GRIMES CA**. A rapid highly-sensitive endotoxin detection system. *Biosensors and Bioelectronics*, 2006, vol. 21, 2270-2274

- **HAISHIMA Y ; HASEGAWA C ; YAGAMI T ; TSUCHIYA T ; MATSUDA R ; HAYASHI Y.** Estimation of uncertainty in kinetic-colorimetric assay of bacterial endotoxins. *J Pharm Biomed Analysis.*, 2003, vol. 32, 495-503